# EUROPÄISCHE PATENTSCHRIFT

(11) **EP 1 992 349 B1**
(45) Veröffentlichungstag und Bekanntmachung des Hinweises auf die Patenterteilung: **31.07.2013**
(21) Anmeldenummer: 08160848.1
(22) Anmeldetag: 21.03.2006
(51) Int. Cl.: A61K 31/551, A61P 25/06, C07D 401/04, C07D 401/14

(54) **CGRP-antagonisten, Verfahren zu deren Herstellung sowie deren Verwendung als Arzneimittel**
CGRP antagonists, their preparation and use as a medicament
Antagonistes CGRP, leur préparation et leur utilisation comme médicament

(30) Priorität: 23.03.2005 WO PCT/EP2005/003094
(43) Veröffentlichungstag der Anmeldung: 19.11.2008
(62) Teilanmeldung aus: 06723571.3
(73) Patentinhaber: Boehringer Ingelheim International GmbH, 55216 Ingelheim (DE); Boehringer Ingelheim Pharma GmbH & Co. KG, 55216 Ingelheim (DE)
(72) Erfinder: Mueller, Stephan, Georg, 88447 Warthausen (DE); Rudlof Klaus, Warthausen (DE); Lustenberger, Philipp, 4056 basel (CH); Stenkamp, Dirk, 88400 Mittelbiberach (DE); Santagostino, Marco, 88441 Mittelbiberach (DE); Paleari, Fabio, 20052 Monza (IT); Dreyer, Alexander, 88484 Gutenzell-Huerbel (DE); Arndt, Kirsten, 88400 Biberach (DE); Doods, Henri, 88447 Warthausen (DE); Schaenzle, gerhard, 88400 Biberach (DE)
(74) Vertreter: Simon, Elke Anna Maria

(56) Entgegenhaltungen:
- WO-A-2004/037810
- WO-A-2004/037811

## Beschreibung

Gegenstand der vorliegenden Erfindung sind neue CGRP-Antagonisten der allgemeinen Formel **I** 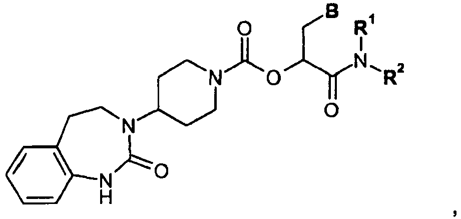 in der **B**, **R¹** und **R²** wie in Anspruch 1 definiert sind, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen, diese Verbindungen enthaltende Arzneimittel, deren Verwendung und Verfahren zu ihrer Herstellung.

### HINTERGRUND DER ERFINDUNG

In den internationalen Patentanmeldungen PCT/EP03/11762 und PCT/EP2005/003094 sowie in der US 2005/0215576 werden bereits CGRP-Antagonisten zur Behandlung von Migräne beschrieben.

Weiterhin offenbaren die Dokumente WO 2004/037810 und WO 2004/037811 CGRP-Antagonisten der allgemeinen Formel I, in denen der Substituent "B" jedoch eine andere Gruppe darstellt als in den Verbindungen der vorliegenden Anmeldung.

### DETAILLIERTE BESCHREIBUNG DER ERFINDUNG

In der obigen allgemeinen Formel I bedeuten in einer ersten Ausführungsform
- **B**: eine Gruppe ausgewählt aus 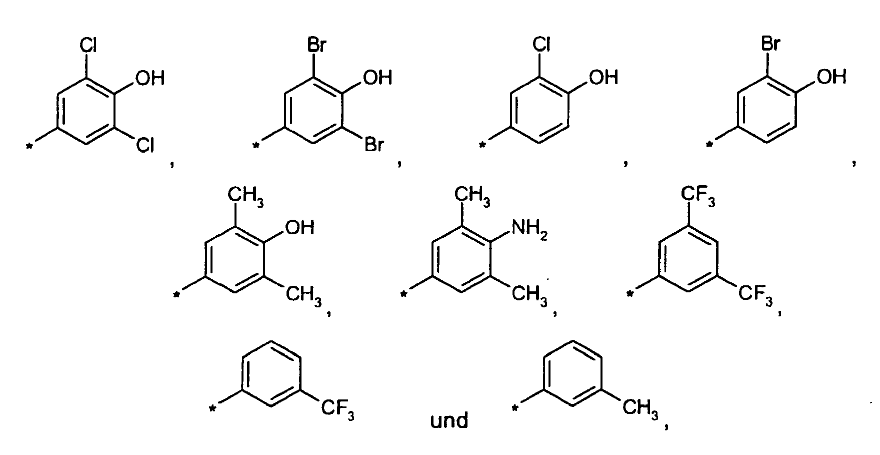
- **R¹** und **R²**: zusammen mit dem eingeschlossenen Stickstoffatom einen Rest der allgemeinen Formel **II** 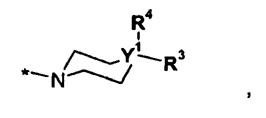
in der
- **Y¹**: das Kohlenstoffatom oder, wenn **R⁴** ein freies Elektronenpaar darstellt, auch das Stickstoffatom,
- **R³**: eine Cyclopentyl-, Cyclohexyl- oder Cycloheptylgruppe oder
- **R³**: einen Heterocyclus ausgewählt aus einer Morpholin-4-yl-, 1,1-Dioxo-thiomorholin-4-yl-, Piperidin-1-yl-, Piperidin-4-yl-, Piperazin-1-yl- oder Pyrrolidin-1-ylgruppe darstellt,
wobei die vorstehend genannten monocyclischen Heterocyclen im Ring ein- oder zweifach durch Hydroxy-, Methyl-, Ethyl-, Trifluormethyl-, Hydroxymethyl- oder Hydroxyethylgruppen, oder, gegebenenfalls zusätzlich, einfach durch eine Hydroxycyclopropyl-, Trifluormethylcarbonylmethyl-, Amino-, Carboxy-carbonyl-, Methoxycarbonyl-, Ethoxycarbonyl-carbonyl-, Carboxymethyl-, Carboxyethyl-, Ethoxycarbonylmethyl-, Ethoxycarbonylethyl-, Carboxy-ethylcarbonyl-, Ethoxycarbonyl-ethylcarbonyl-, Aminosulfonyl-, Methylaminosulfonyl-, Dimethylaminosulfonyl-, sulfonyl-, Methylsulfonyl-, Ethylsulfonyl-, Isopropylsulfonyl-, Cyclopropylsulfonyl-, (Hydroxyamino)-carbonylmethyl-, Hydroxy-(methyl)-aminocarbonyl-methyl- oder Methoxyaminocarbonyl-methylgruppe substituiert sein können, wobei die Substituenten gleich oder verschieden und an ein Ring-Kohlenstoff- oder RingStickstoffatom gebunden sein können, oder
die vorstehend genannten monocyclischen Heterocyclen im Ring einfach durch eine Carboxygruppe substituiert sein können, wenn diese Carboxygruppe nicht über ein Stickstoffatom gebunden ist,
- **R⁴**: das Wasserstoffatom, wenn **Y¹** das Kohlenstoffatom darstellt, oder
- **R⁴**: ein freies Elektronenpaar, wenn **Y¹** das Stickstoffatom darstellt,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen,
mit der Maßgabe, dass die folgenden Verbindungen, deren Tautomere, deren Diastereomere, deren Enantiomere, deren Hydrate, deren Gemische und deren Salze sowie die Hydrate der Salze vom Anspruchsumfang ausgeschlossen sind:

| **Nr.** | **Struktur** |
|---|---|
| (1) | 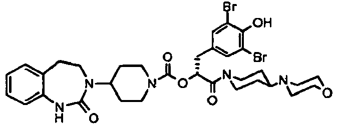 |
| (2) | 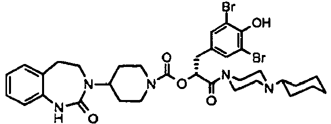 |
| (3) | 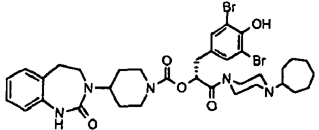 |
| (4) | 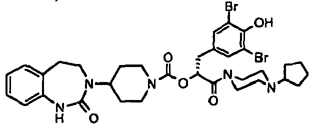 |
| (5) | 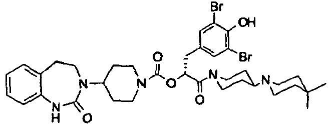 |
| (6) | 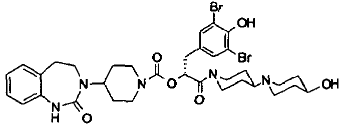 |
| (7) | 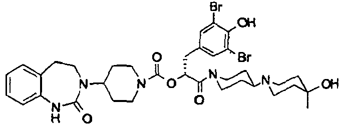 |
| (8) | 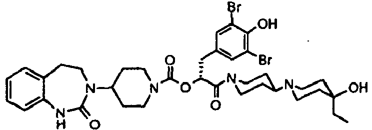 |
| (9) | 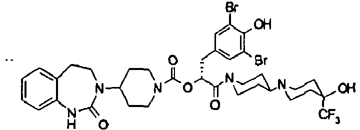 |
| (10) | 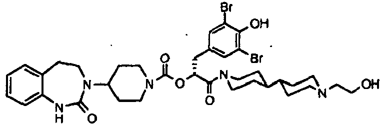 |
| (11) | 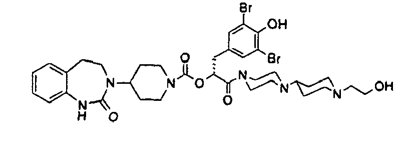 |
| (12) | 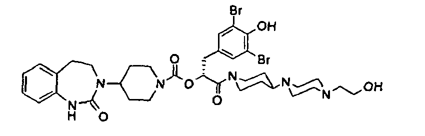 |
| (13) | 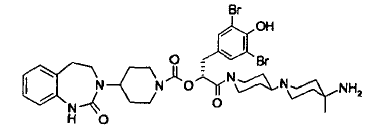 |
| (14) | 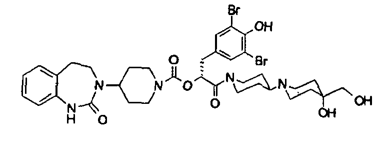 |
| (15) | 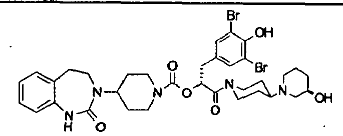 |
| (16) | 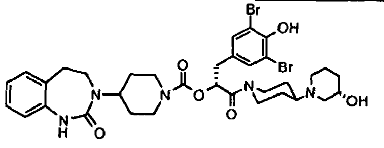 |
| (17) | 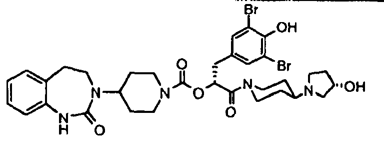 |
| (18) | 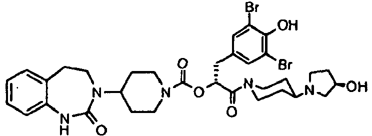 |
| (19) | 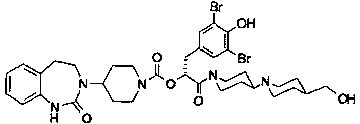 |
| (20) | 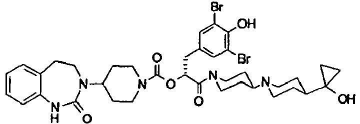 |
| (21) | 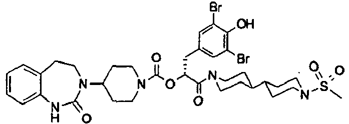 |
| (22) | 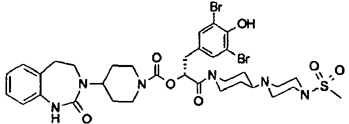 |
| (23) | 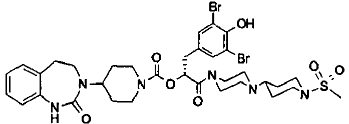 |
| (24) | 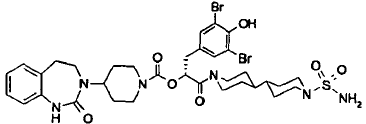 |
| (25) | 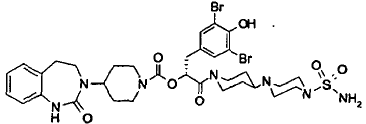 |
| (26) | 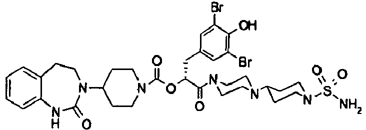 |
| (27) | 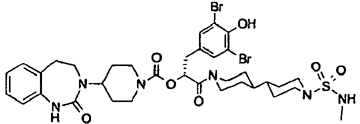 |
| (28) | 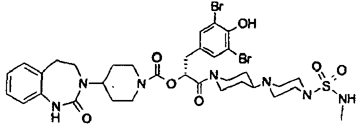 |
| (29) | 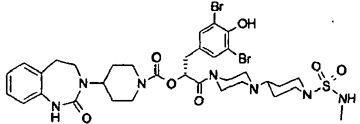 |
| (30) | 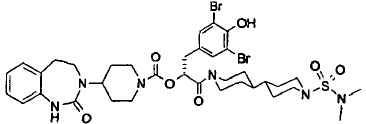 |
| (31) | 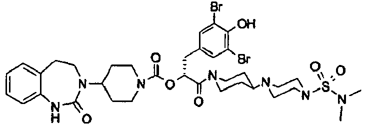 |
| (32) | 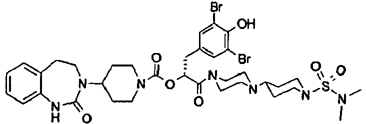 |
| (33) | 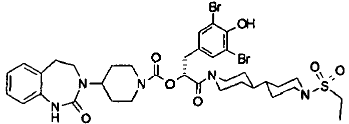 |
| (34) | 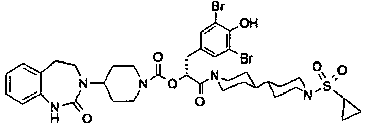 |
| (35) | 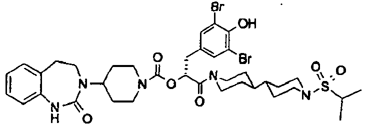 |
| (36) | 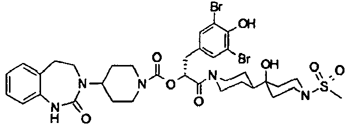 |
| (37) | 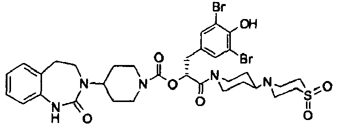 |
| (38) | 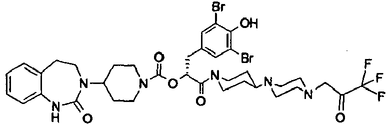 |
| (39) | 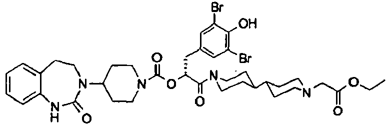 |
| (40) | 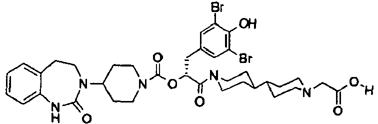 |
| (41) | 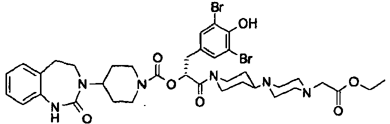 |
| (42) | 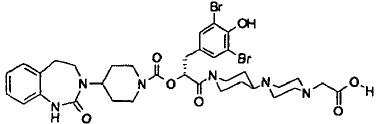 |
| (43) | 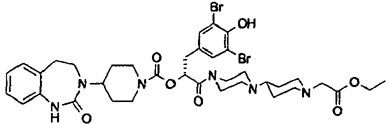 |
| (44) | 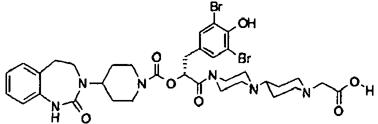 |
| (45) | 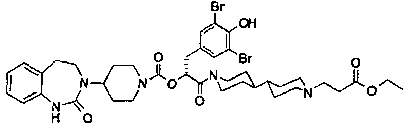 |
| (46) | 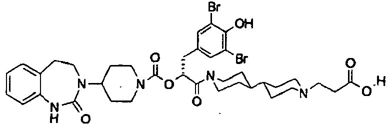 |
| (47) | 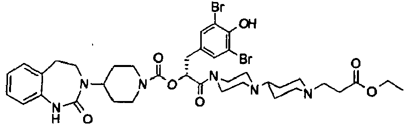 |
| (48) | 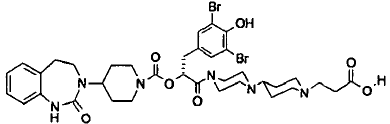 |
| (49) | 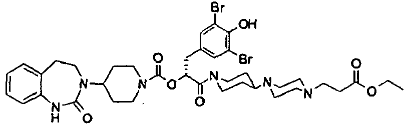 |
| (50) | 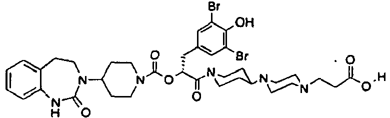 |
| (51) | 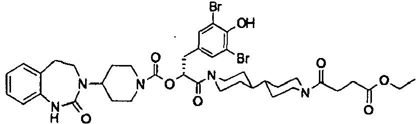 |
| (52) | 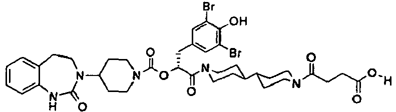 |
| (53) | 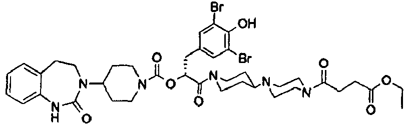 |
| (54) | 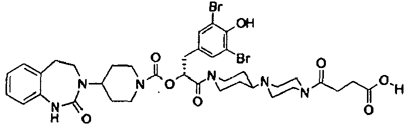 |
| (55) | 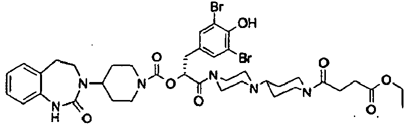 |
| (56) | 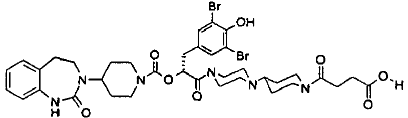 |
| (57) | 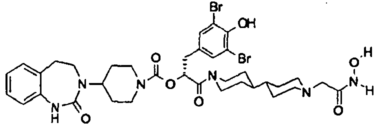 |
| (58) | 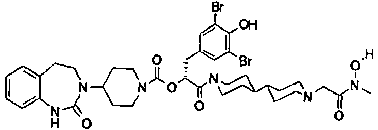 |
| (59) | 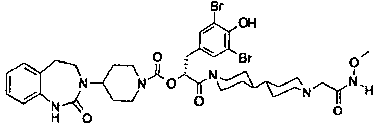 |
| (60) | 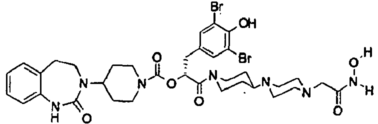 |
| (61) | 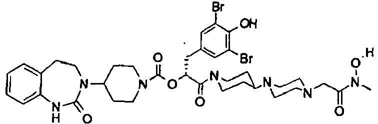 |
| (62) | 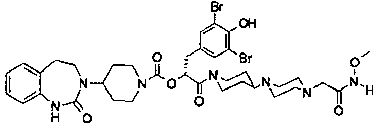 |
| (63) | 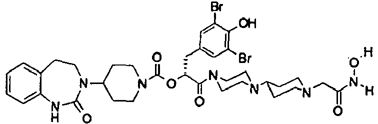 |
| (64) | 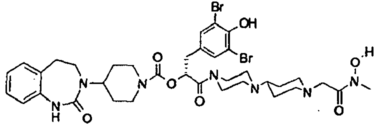 |
| (65) | 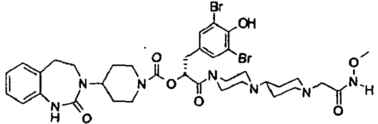 |
| (66) | 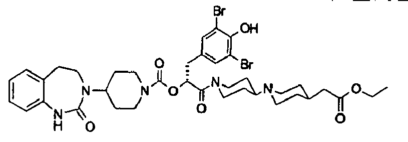 |
| (67) | 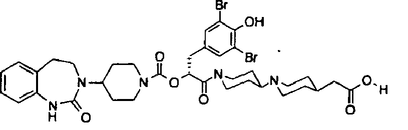 |
| (68) | 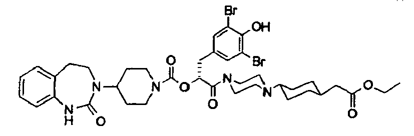 |
| (69) | 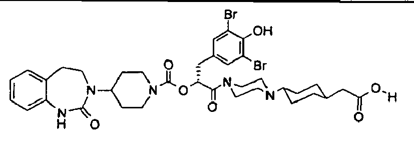 |
| (70) | 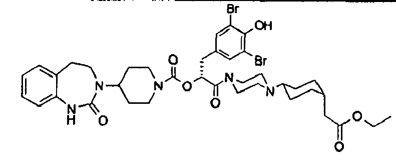 |
| (71) | 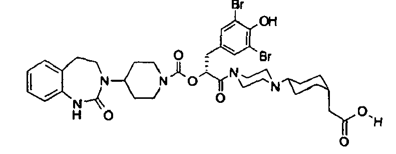 |
| (72) | 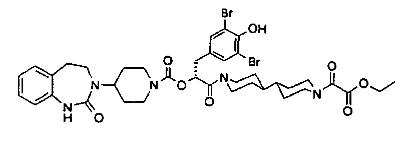 |
| (73) | 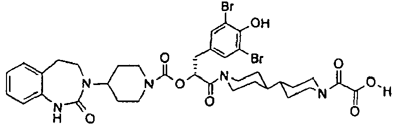 |
| (74) | 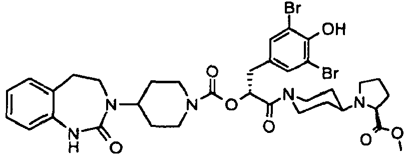 |
| (75) | 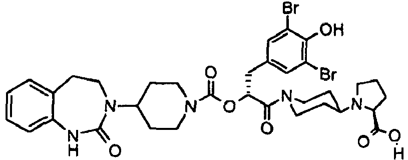 |
| (76) | 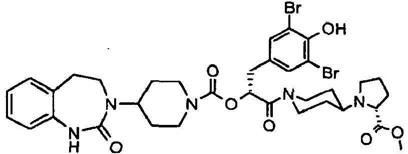 |
| (77) | 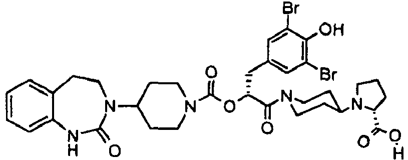 |
| (78) | 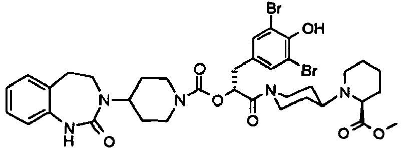 |
| (79) | 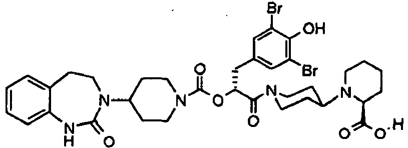 |
| (80) | 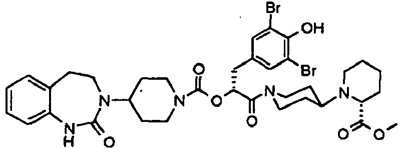 |
| (81) | 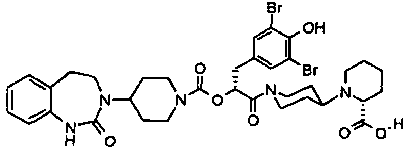 |
| (82) | 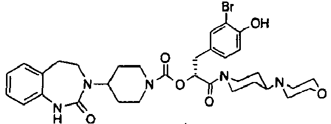 |
| (83) | 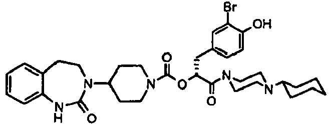 |
| (84) | 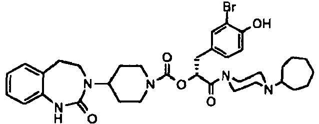 |
| (85) | 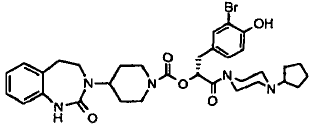 |
| (86) | 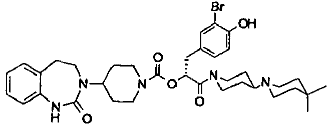 |
| (87) | 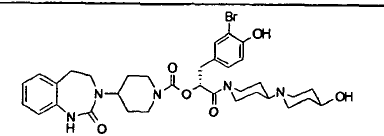 |
| (88) | 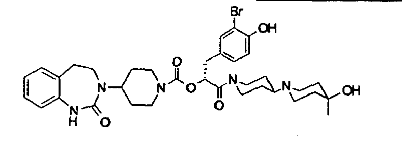 |
| (89) | 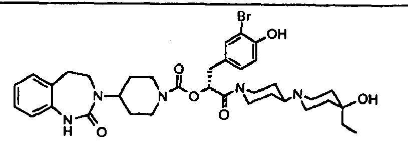 |
| (90) | 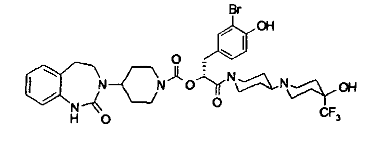 |
| (91) | 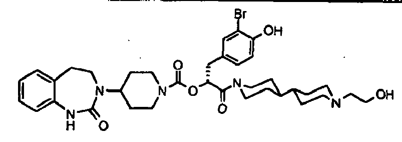 |
| (92) | 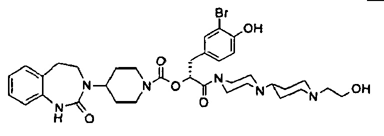 |
| (93) | 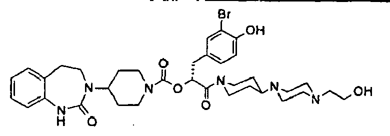 |
| (94) | 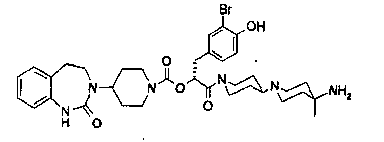 |
| (95) | 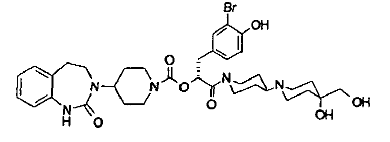 |
| (96) | 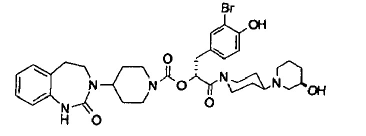 |
| (97) | 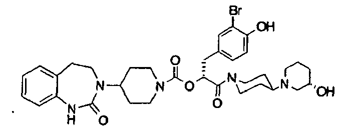 |
| (98) | 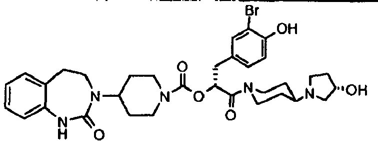 |
| (99) | 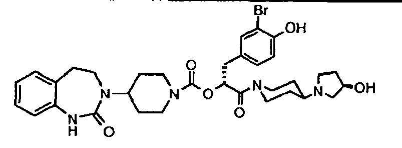 |
| (100) | 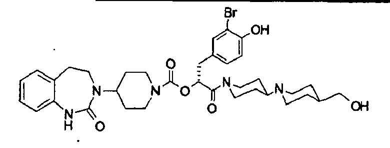 |
| (101) | 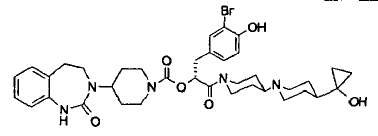 |
| (102) | 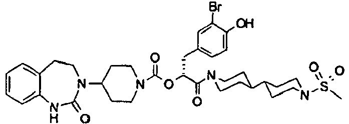 |
| (103) | 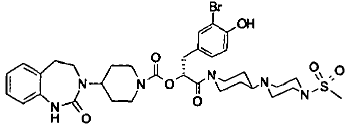 |
| (104) | 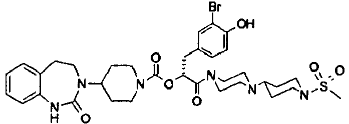 |
| (105) | 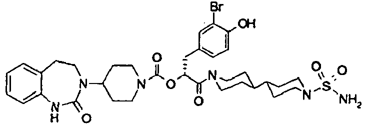 |
| (106) | 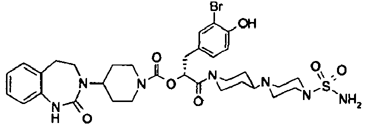 |
| (107) | 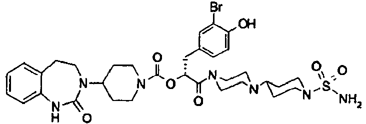 |
| (108) | 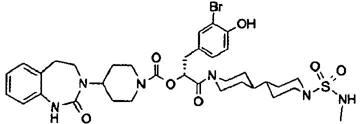 |
| (109) | 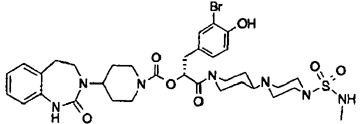 |
| (110) | 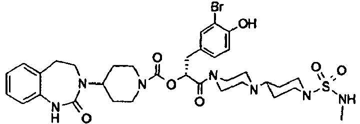 |
| (111) | 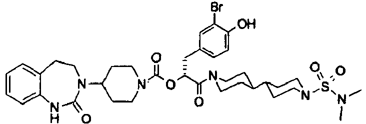 |
| (112) | 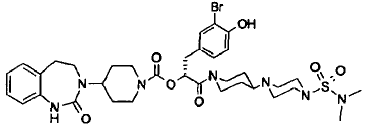 |
| (113) | 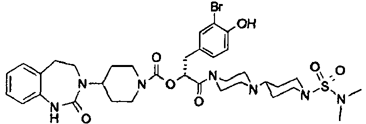 |
| (114) | 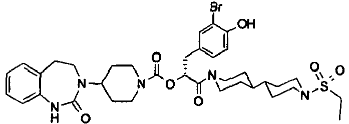 |
| (115) | 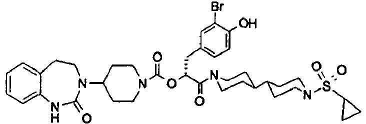 |
| (116) | 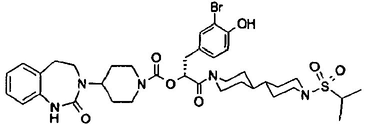 |
| (117) | 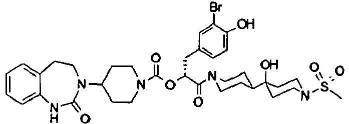 |
| (118) | 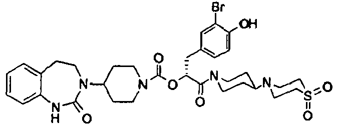 |
| (119) | 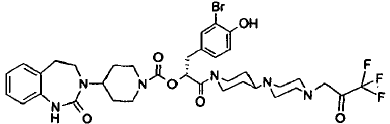 |
| (120) | 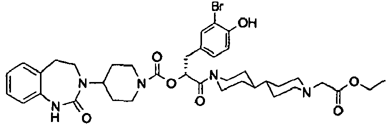 |
| (121) | 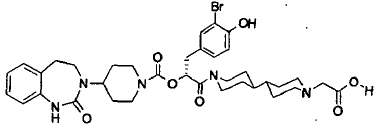 |
| (122) | 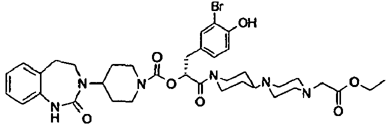 |
| (123) | 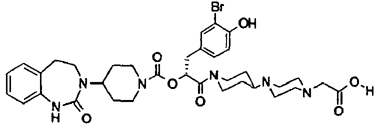 |
| (124) | 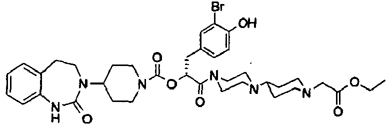 |
| (125) | 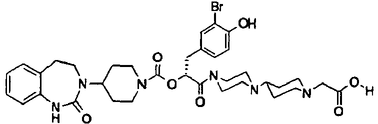 |
| (126) | 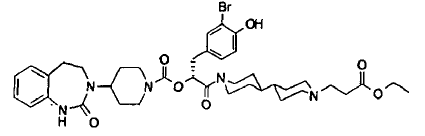 |
| (127) | 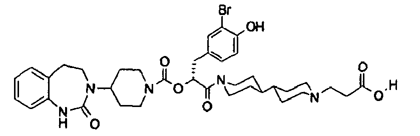 |
| (128) | 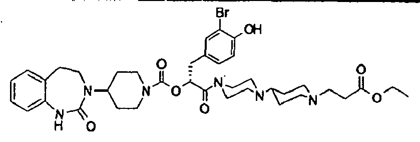 |
| (129) | 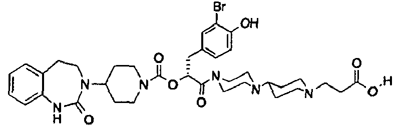 |
| (130) | 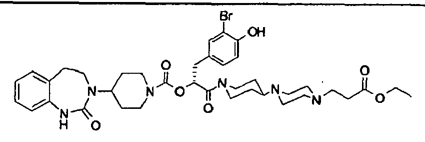 |
| (131) | 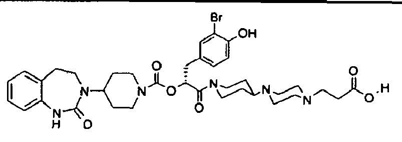 |
| (132) | 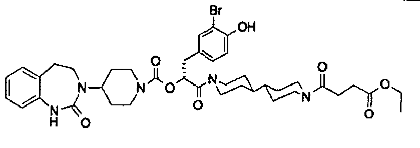 |
| (133) | 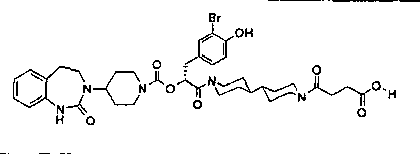 |
| (134) | 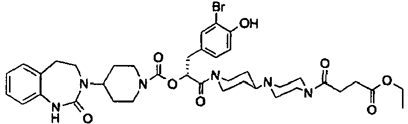 |
| (135) | 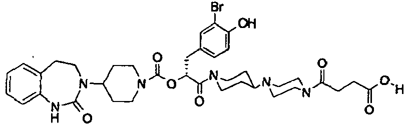 |
| (136) | 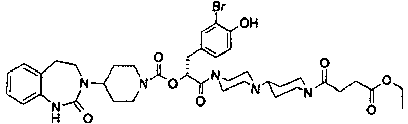 |
| (137) | 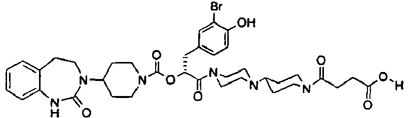 |
| (138) | 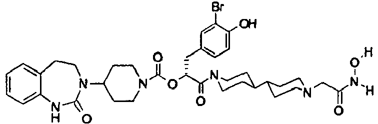 |
| (139) | 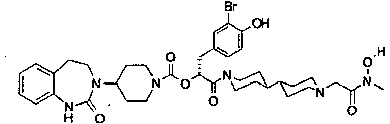 |
| (140) | 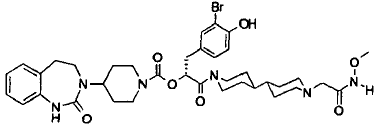 |
| (141) | 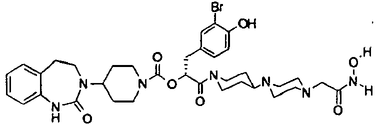 |
| (142) | 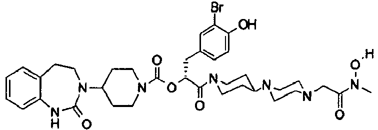 |
| (143) | 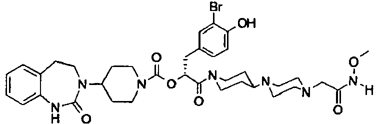 |
| (144) | 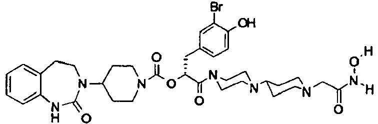 |
| (145) | 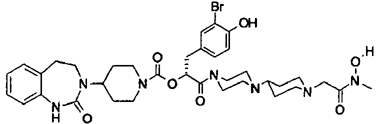 |
| (146) | 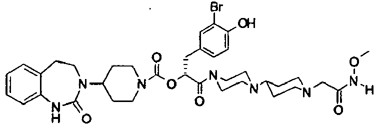 |
| (147) | 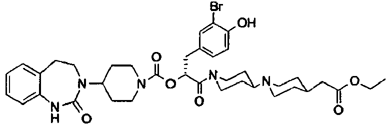 |
| (148) | 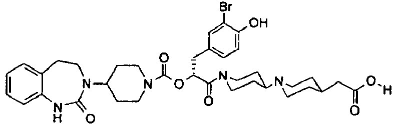 |
| (149) | 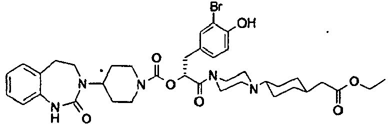 |
| (150) | 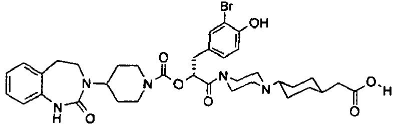 |
| (151) | 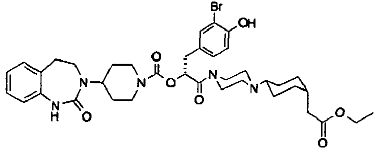 |
| (152) | 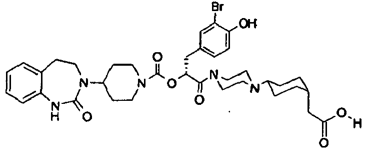 |
| (153) | 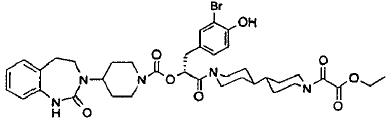 |
| (154) | 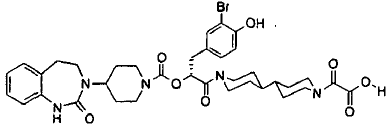 |
| (155) | 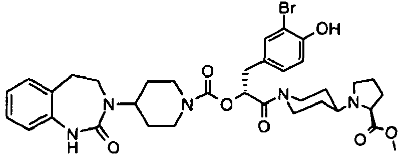 |
| (156) | 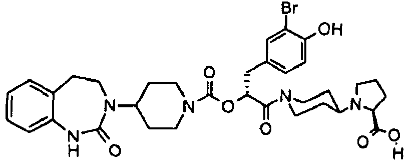 |
| (157) | 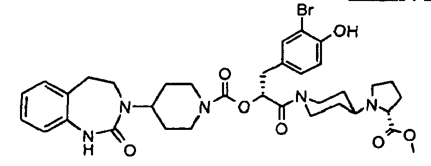 |
| (158) | 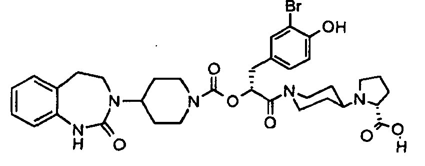 |
| (159) | 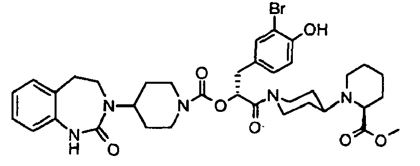 |
| (160) | 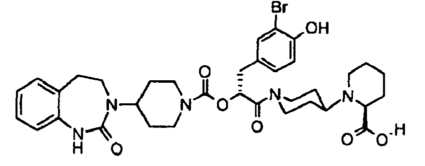 |
| (161) | 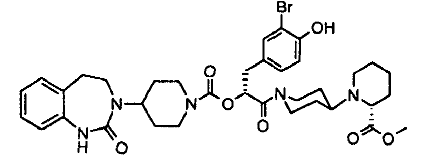 |
| (162) | 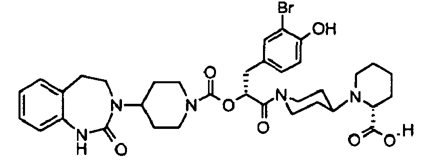 |
| (163) | 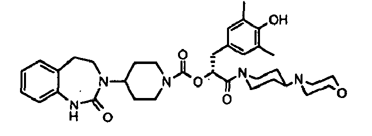 |
| (164) | 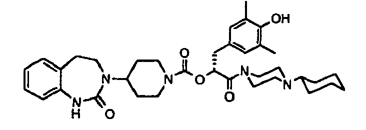 |
| (165) | 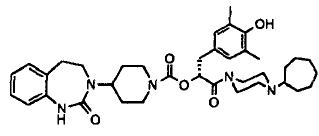 |
| (166) | 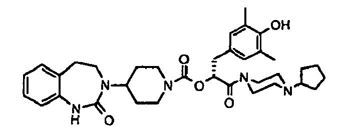 |
| (167) | 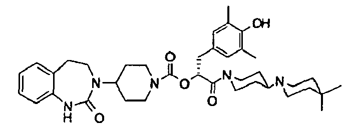 |
| (168) | 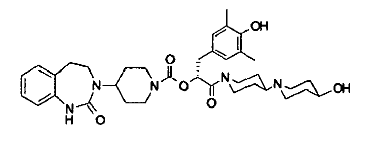 |
| (169) | 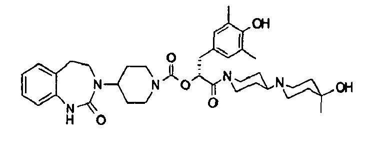 |
| (170) | 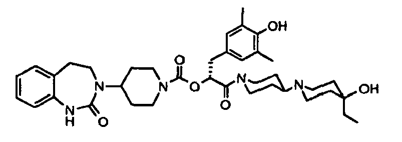 |
| (171) | 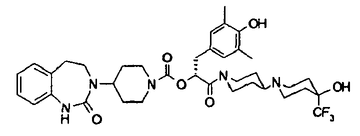 |
| (172) | 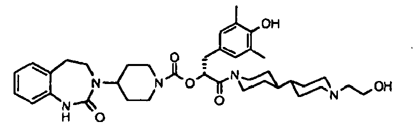 |
| (173) | 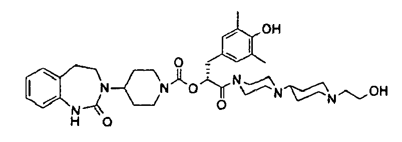 |
| (174) | 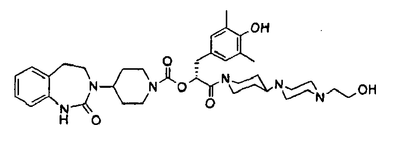 |
| (175) | 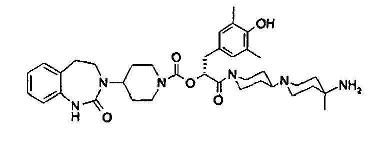 |
| (176) | 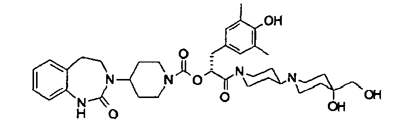 |
| (177) | 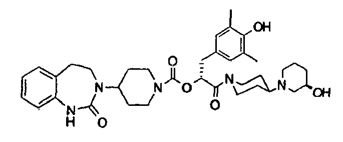 |
| (178) | 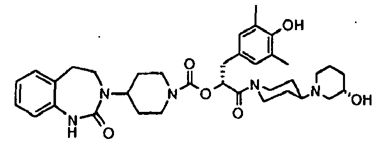 |
| (179) | 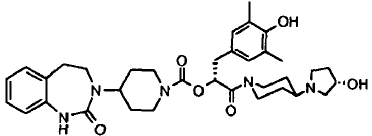 |
| (180) | 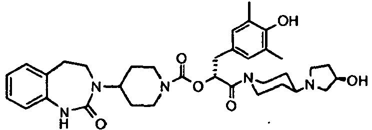 |
| (181) | 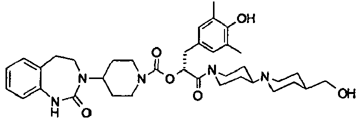 |
| (182) | 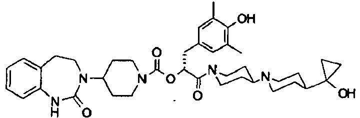 |
| (183) | 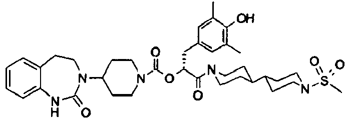 |
| (184) | 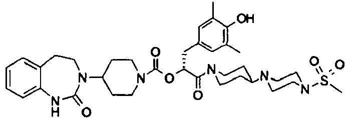 |
| (185) | 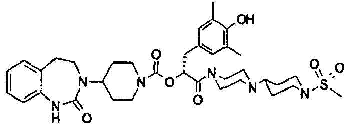 |
| (186) | 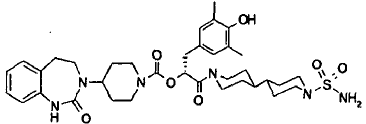 |
| (187) | 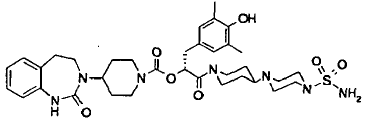 |
| (188) | 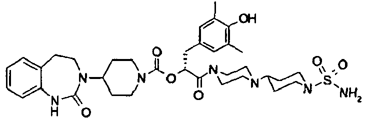 |
| (189) | 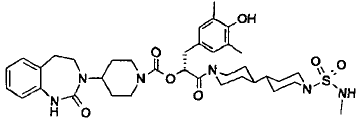 |
| (190) | 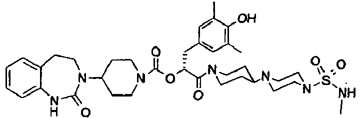 |
| (191) | 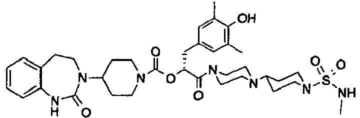 |
| (192) | 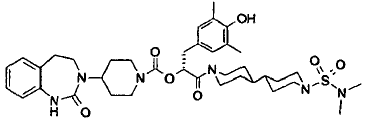 |
| (193) | 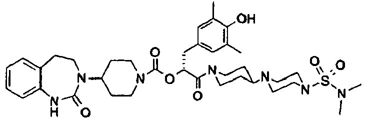 |
| (194) | 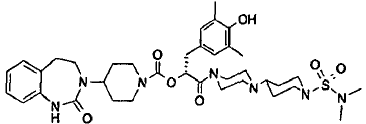 |
| (195) | 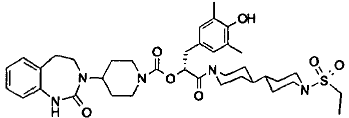 |
| (196) | 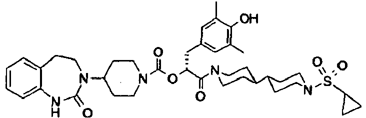 |
| (197) | 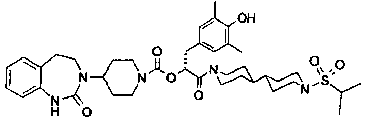 |
| (198) | 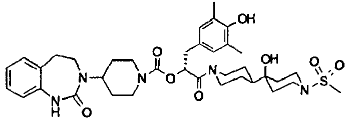 |
| (199) | 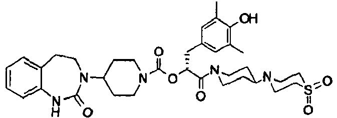 |
| (200) | 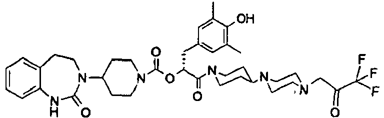 |
| (201) | 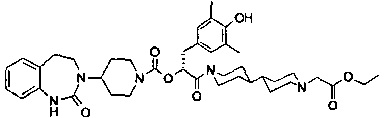 |
| (202) | 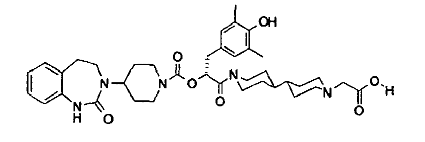 |
| (203) | 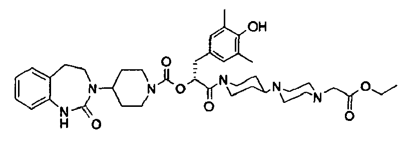 |
| (204) | 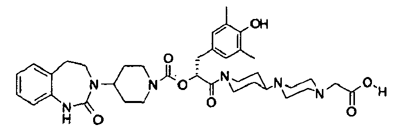 |
| (205) | 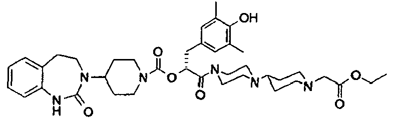 |
| (206) | 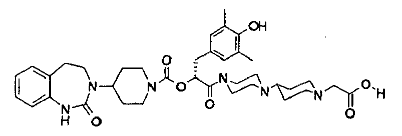 |
| (207) | 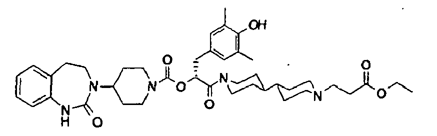 |
| (208) | 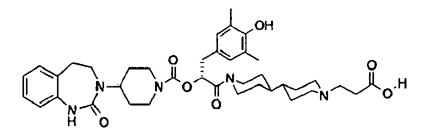 |
| (209) | 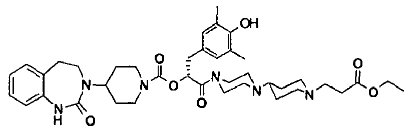 |
| (210) | 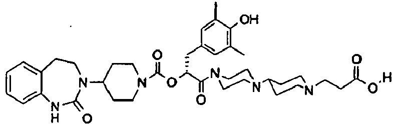 |
| (211) | 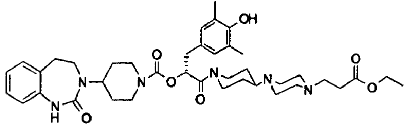 |
| (212) | 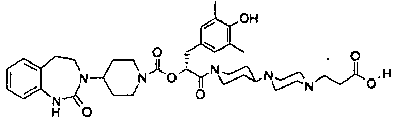 |
| (213) | 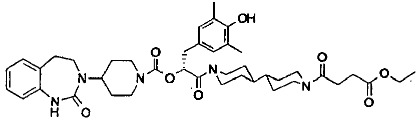 |
| (214) | 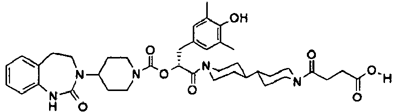 |
| (215) | 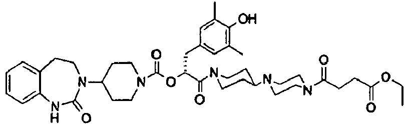 |
| (216) | 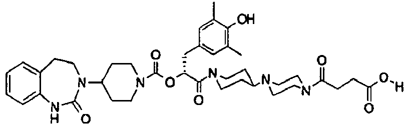 |
| (217) | 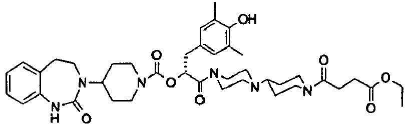 |
| (218) | 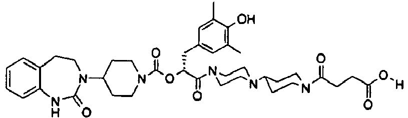 |
| (219) | 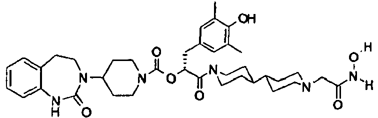 |
| (220) | 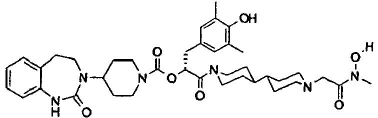 |
| (221) | 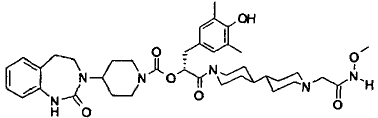 |
| (222) | 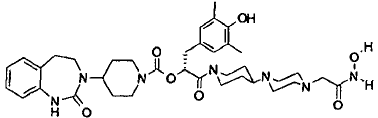 |
| (223) | 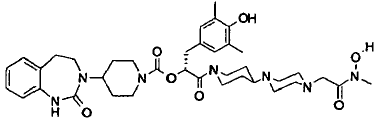 |
| (224) | 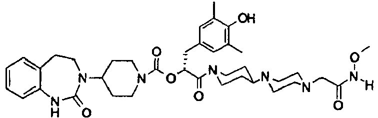 |
| (225) | 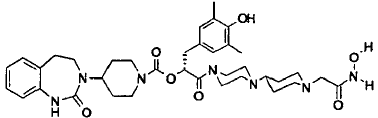 |
| (226) | 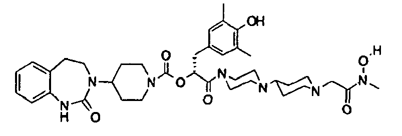 |
| (227) | 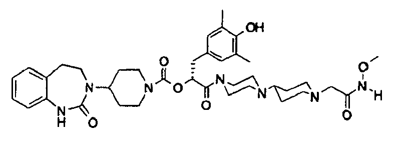 |
| (228) | 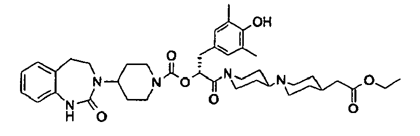 |
| (229) | 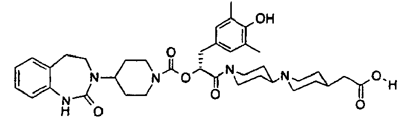 |
| (230) | 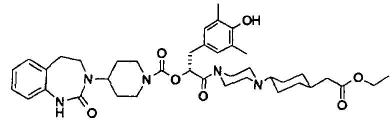 |
| (231) | 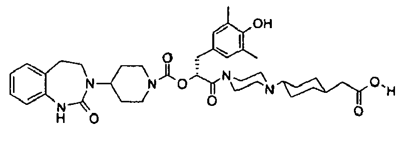 |
| (232) | 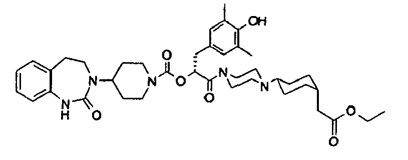 |
| (233) | 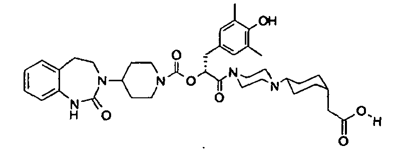 |
| (234) | 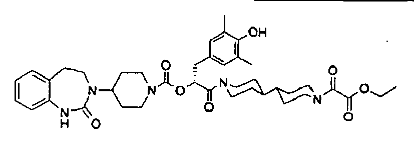 |
| (235) | 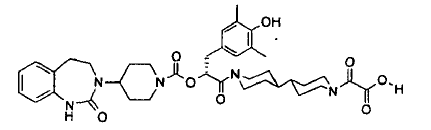 |
| (236) | 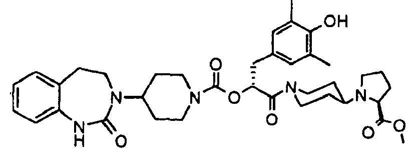 |
| (237) | 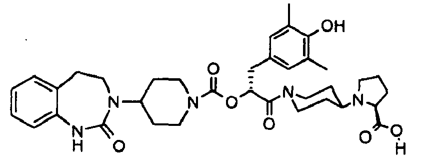 |
| (238) | 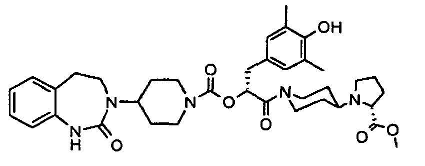 |
| (239) | 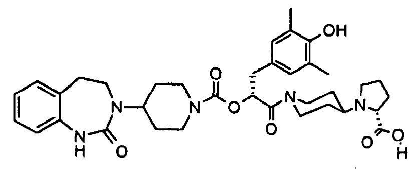 |
| (240) | 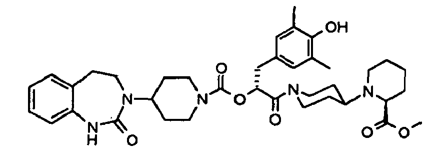 |
| (241) | 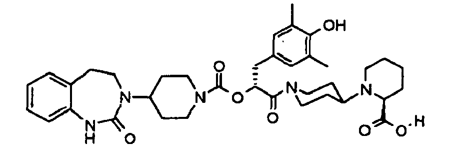 |
| (242) | 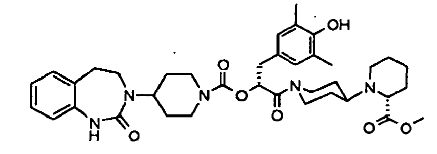 |
| (243) | 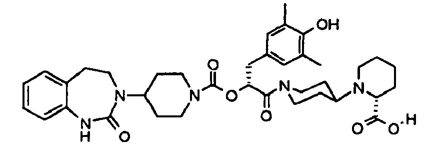 |
| (244) | 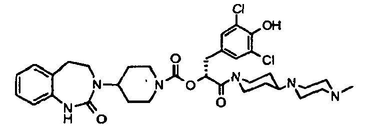 |
| (245) | 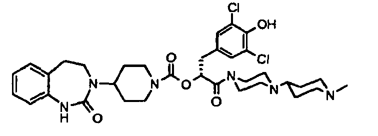 |
| (246) | 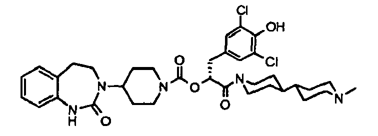 |
| (247) | 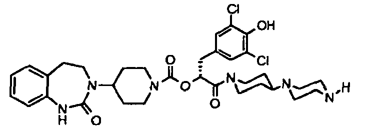 |
| (248) | 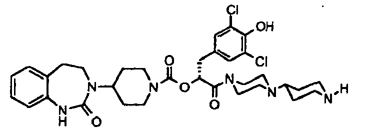 |
| (249) | 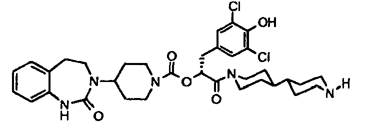 |
| (250) | 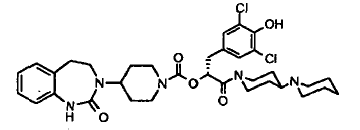 |
| (251) | 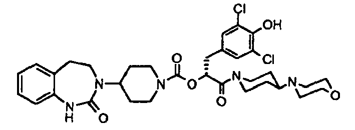 |
| (252) | 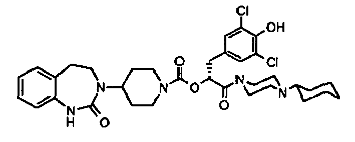 |
| (253) | 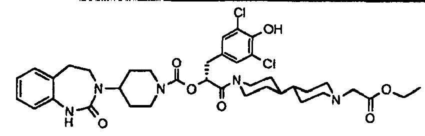 |
| (254) | 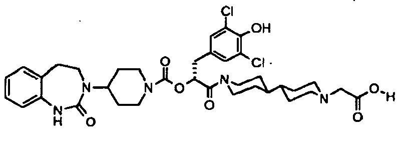 |
| (255) | 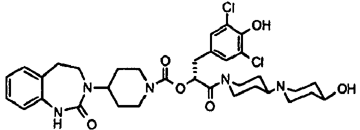 |
| (256) | 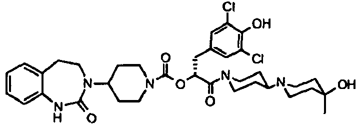 |
| (257) | 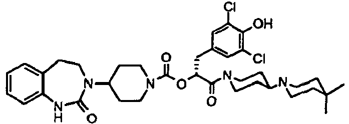 |
| (258) | 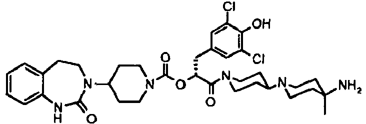 |
| (259) | 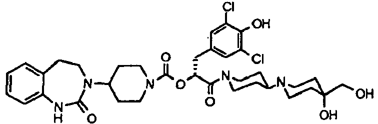 |
| (260) | 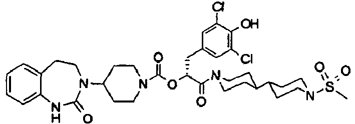 |
| (261) | 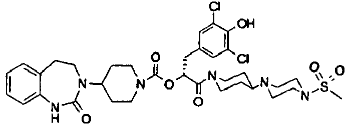 |
| (262) | 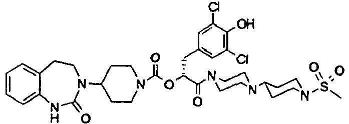 |
| (263) | 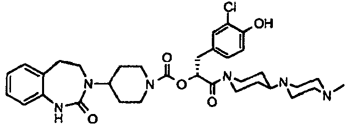 |
| (264) | 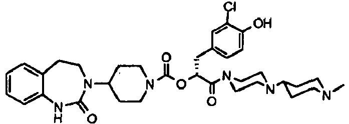 |
| (265) | 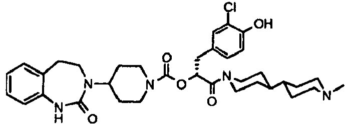 |
| (266) | 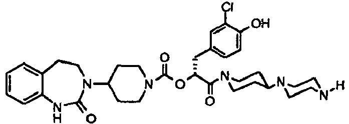 |
| (267) | 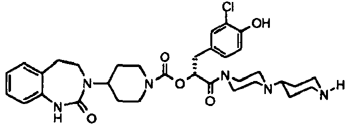 |
| (268) | 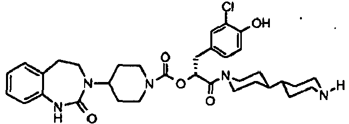 |
| (269) | 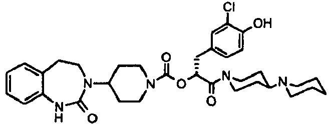 |
| (270) | 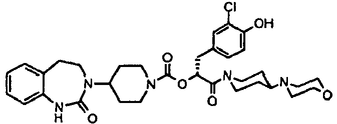 |
| (271) | 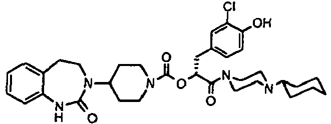 |
| (272) | 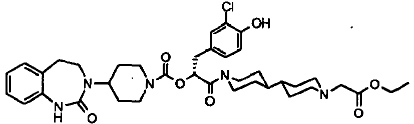 |
| (273) | 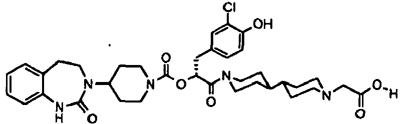 |
| (274) | 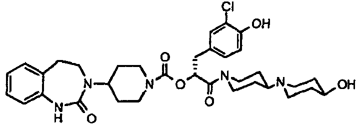 |
| (275) | 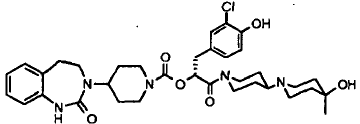 |
| (276) | 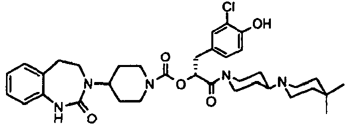 |
| (277) | 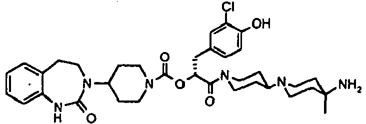 |
| (278) | 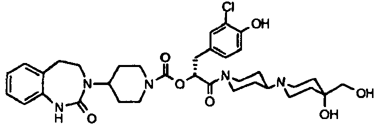 |
| (279) | 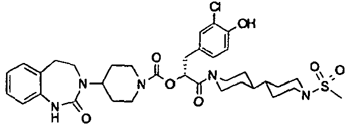 |
| (280) | 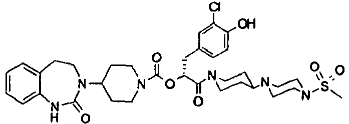 |
| (281) | 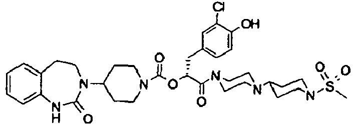 |
| (282) | 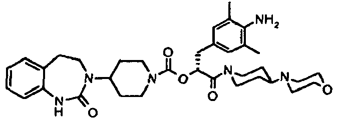 |
| (283) | 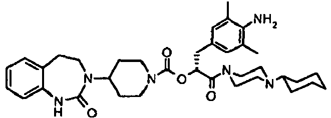 |
| (284) | 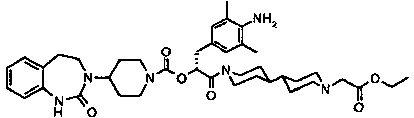 |
| (285) | 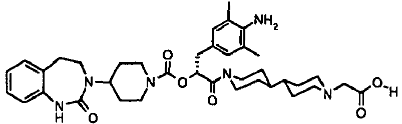 |
| (286) | 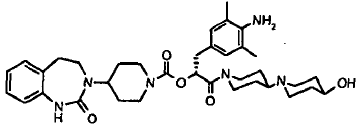 |
| (287) | 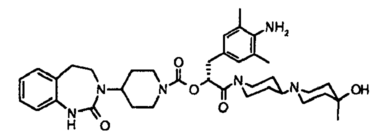 |
| (288) | 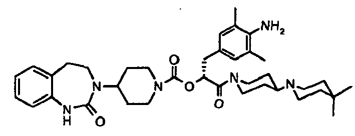 |
| (289) | 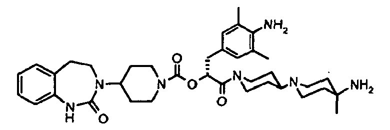 |
| (290) | 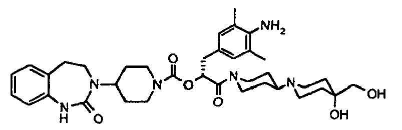 |
| (291) | 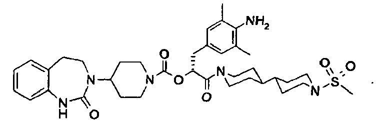 |
| (292) | 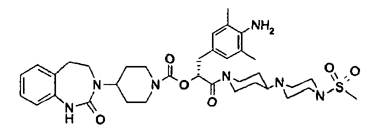 |
| (293) | 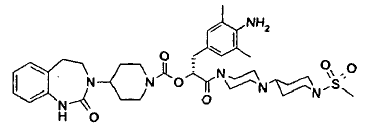 |
| (294) | 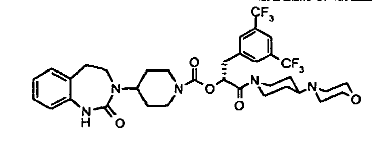 |
| (295) | 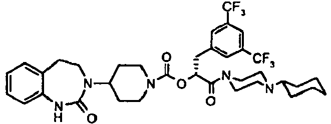 |
| (296) | 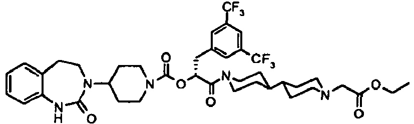 |
| (297) | 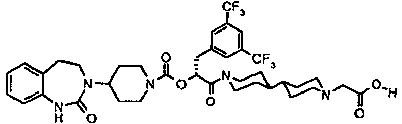 |
| (298) | 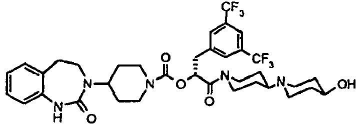 |
| (299) | 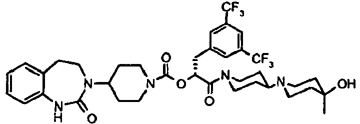 |
| (300) | 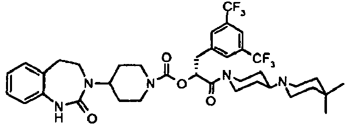 |
| (301) | 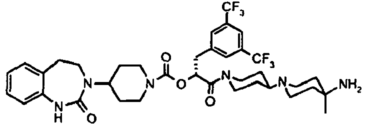 |
| (302) | 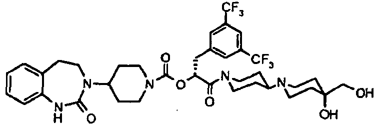 |
| (303) | 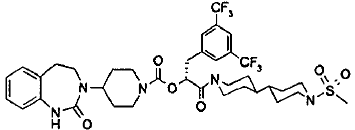 |
| (304) | 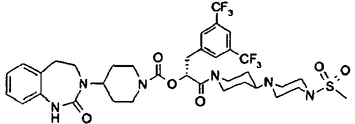 |
| (305) | 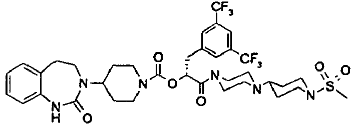 |
| (306) | 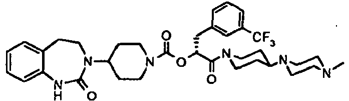 |
| (307) | 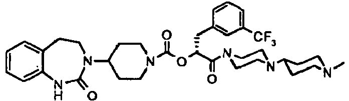 |
| (308) | 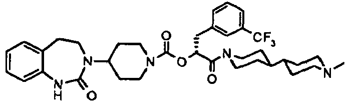 |
| (309) | 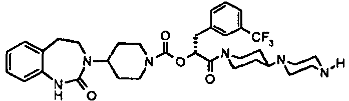 |
| (310) | 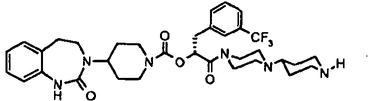 |
| (311) | 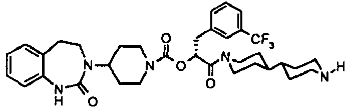 |
| (312) | 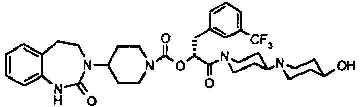 |
| (313) | 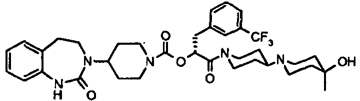 |
| (314) | 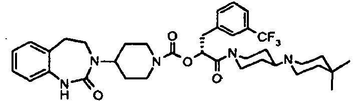 |
| (315) | 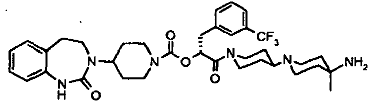 |
| (316) | 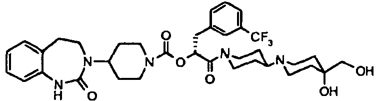 |
| (317) | 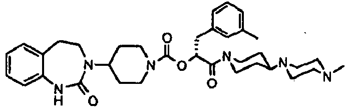 |
| (318) | 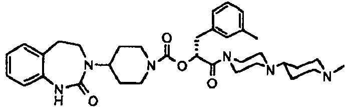 |
| (319) | 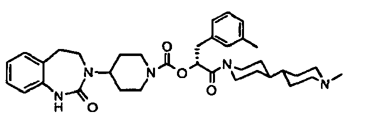 |
| (320) | 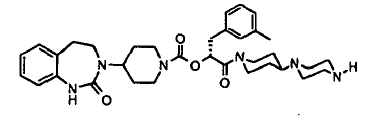 |
| (321) | 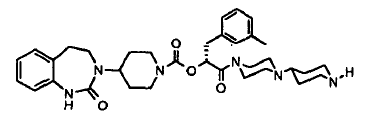 |
| (322) | 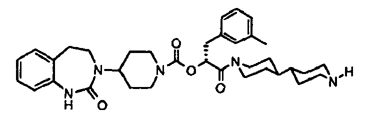 |
| (323) | 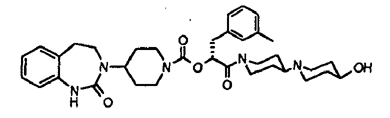 |
| (324) | 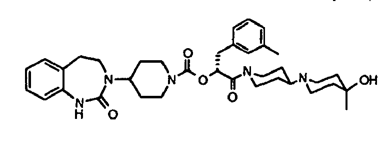 |
| (325) | 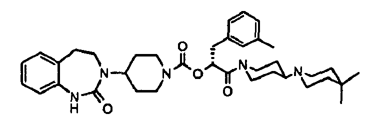 |
| (326) | 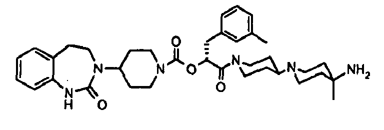 |
| (327) | 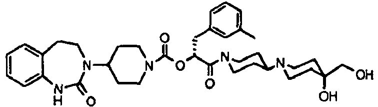 |

### VERWENDETE BEGRIFFE UND DEFINITIONEN

Im Rahmen dieser Anmeldung können bei der Definition von möglichen Substituenten, diese auch in Form einer Strukturformel dargestellt werden. Dabei wird, falls vorhanden, ein Stern (*) in der Strukturformel des Substituenten als der Verknüpfungspunkt zum Rest des Moleküls verstanden.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Verbindungen der allgemeinen Formel 1 können Säuregruppen besitzen, hauptsächlich Carboxylgruppen, und/oder basische Gruppen wie z.B. Aminofunktionen. Verbindungen der allgemeinen Formel **I** können deshalb als innere Salze, als Salze mit pharmazeutisch verwendbaren anorganischen Säuren wie beispielsweise Bromwasserstoffsäure, Phosphorsäure, Salpetersäure, Salzsäure, Schwefelsäure, Methansulfonsäure, Ethansulfonsäure, Benzolsulfonsäure, *p-*Toluolsulfonsäure oder organischen Säuren wie beispielsweise Äpfelsäure, Bernsteinsäure, Essigsäure, Fumarsäure, Maleinsäure, Mandelsäure, Milchsäure, Weinsäure, Zitronensäure oder als Salze mit pharmazeutisch verwendbaren Basen wie Alkali- oder Erdalkalimetallhydroxiden, beispielsweise Natriumhydroxid oder Kaliumhydroxid, oder Carbonaten, Ammoniak, Zink- oder Ammoniumhydroxiden oder organischen Aminen wie z.B. Diethylamin, Triethylamin, Ethanolamin, Diethanolamin, Triethanolamin, Cyclohexylamin, Dicyclohexylamin u.a. vorliegen.

Wie vorstehend genannt, können die Verbindungen der Formel **I** in ihre Salze, insbesondere für die pharmazeutische Anwendung, in ihre physiologisch und pharmakologisch verträglichen Salze überführt werden. Diese Salze können einerseits als physiologisch und pharmakologisch verträgliche Säureadditionssalze der Verbindungen der Formel **I** mit anorganischen oder organischen Säuren vorliegen. Andererseits kann die Verbindung der Formel **I,** wenn **B** eine phenolische OH-Gruppe enthält, durch Umsetzung mit anorganischen Basen auch in physiologisch und pharmakologisch verträgliche Salze mit Alkali- oder Erdalkalimetallkationen als Gegenion überführt werden. Zur Darstellung der Säureadditionssalze kommen beispielsweise Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure in Betracht. Ferner können Mischungen der vorgenannten Säuren eingesetzt werden. Zur Darstellung der Alkali- und Erdalkalimetallsalze der Verbindung der Formel **I,** kommen vorzugsweise die Alkali- und Erdalkalihydroxide und -hydride in Betracht, wobei die Hydroxide und Hydride der Alkalimetalle, besonders des Natriums und Kaliums bevorzugt, Natrium- und Kaliumhydroxid besonders bevorzugt sind.

Die erfindungsgemäßen Verbindungen können als Racemate vorliegen, sofern sie nur ein Chiralitätselement besitzen, sie können aber auch als reine Enantiomere, d.h. in (R)- oder (S)-Form gewonnen werden. Bevorzugt sind Verbindungen, die als Racemate bzw. als (R)-Form vorliegen.

Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel I vorhanden ist, sowie die einzelnen optisch aktiven Enatiomeren, aus denen sich die erwähnten Racemate zusammensetzen.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglycol- oder Methansulfonsäure.

### HERSTELLVERFAHREN

Die Verbindungen der allgemeinen Formel **I** werden nach prinzipiell bekannten Methoden hergestellt. Die folgenden Verfahren haben sich zur Herstellung der erfindungsgemäßen Verbindungen der allgemeinen Formel **I** besonders bewährt:
(a) Zur Herstellung von Verbindungen der allgemeinen Formel **I**, in der alle Reste wie eingangs erwähnt definiert sind:
   Umsetzung eines Piperidins der Formel **III** 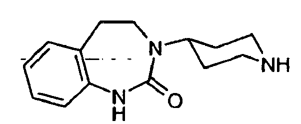 mit einem Kohlensäurederivat der allgemeinen Formel **IV** 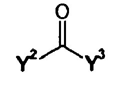 in der **Y²** und **Y³** nucleofuge Gruppen bedeuten, die gleich oder verschieden sein können, vorzugsweise das Chloratom, die p-Nitrophenoxy- oder Trichlormethoxygruppe,
      und mit einer Verbindung der allgemeinen Formel V 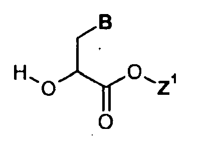 in der **B** wie eingangs erwähnt definiert ist und **Z¹** eine Schutzgruppe für eine Carboxygruppe darstellt, beispielsweise eine C₁₋₆-Alkyl- oder Benzylgruppe, wobei die Alkylgruppen geradkettig oder verzweigt sein können und die Benzylgruppe durch ein oder zwei Methoxygruppen substituiert sein kann. Bevorzugt ist für **Z¹** die Methyl-, Ethyl-, *tert-*Butyl oder Benzylgruppe.
   In einer ersten Stufe werden die Verbindungen der allgemeinen Formel **III** in einem Lösungsmittel, beispielsweise in Dichlormethan, THF, Pyridin oder deren Mischungen, bei einer Temperatur zwischen -20 bis 50°C in Gegenwart einer Base, beispielsweise Triethylamin, Pyridin oder Ethyldiisopropylamin, mit den Kohlensäurederivaten der allgemeinen Formel **IV** zur Reaktion gebracht. Die dabei entstehende Zwischenstufe kann aufgereinigt oder ohne Reinigung weiter umgesetzt werden.
      Die Umsetzung dieser Zwischenstufen mit Verbindungen der allgemeinen Formel **V** erfolgt ebenfalls in einem der oben genannten Lösungsmittel, und bei den oben genannten Temperaturen, in Gegenwart einer Base, wie Triethylamin oder Pyridin, mit oder ohne Zusatz eines Aktivierungsreagenz, wie z.B. 4-Dimethylaminopyridin. Zur Aktivierung können die Verbindungen der allgemeinen Formel **V** auch mittels eines Metallhydrides, wie z.B. NaH oder KH, deprotoniert werden, wobei in diesem Fall auf die Gegenwart der Base oder des Aktivierungsreagenzes verzichtet werden kann.
   Die Ausgangsverbindungen der Formel **III** und **IV** sind entweder käuflich, literaturbekannt oder können nach literaturbekannten Methoden hergestellt werden.
   Verfahren zur Herstellung von Verbindungen der allgemeinen Formel **III** sind beispielsweise in der EP 1 619 187 A1 beschrieben.
(b) Zur Herstellung von Verbindungen der allgemeinen Formel **I,** in der alle Reste wie eingangs erwähnt definiert sind:
   Kupplung einer Carbonsäure der allgemeinen Formel **VI** 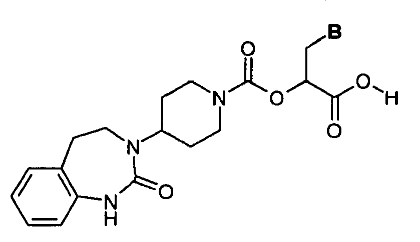 in der **B** wie eingangs erwähnt definiert ist, mit einem Amin der allgemeinen Formel **VII**

      H-N**R¹R²**,

      in der **R¹** und **R²** wie eingangs definiert sind. Vor Durchführung der Reaktion können in den Resten **R¹** und **R²** des Amins der allgemeinen Formel **VII** gegebenenfalls vorhandene Carbonsäurefunktionen, primäre oder sekundäre Aminofunktionen oder Hydroxyfunktionen durch übliche Schutzreste geschützt und gegebenenfalls verwendete Schutzreste nach Durchführung der Reaktion nach dem Fachmann geläufigen Methoden wieder abgespalten werden.
      Die Kupplung wird bevorzugt unter Verwendung von aus der Peptidchemie bekannten Verfahren (siehe z. B. Houben-Weyl, Methoden der Organischen Chemie, Bd. 15/2) durchgeführt, wobei zum Beispiel Carbodiimide, wie z. B. Dicyclohexylcarbodiimid (DCC), Diisopropylcarbodiimid (DIC) oder Ethyl-(3-dimethylamino-propyl)-carbodiimid, O-(1*H*-Benzotriazol-1-yl)-*N,N-N',N'*-tetramethyluronium-hexa-fluorphosphat (HBTU) oder -tetrafluorborat (TBTU) oder 1*H*-Benzotriazol-1-yl-oxy-tris-(dimethylamino)-phosphonium-hexafluorphosphat (BOP) eingesetzt werden. Durch Zugabe von 1-Hydroxybenzotriazol (HOBt) oder von 3-Hydroxy-4-oxo-3,4-dihydro-1,2,3-benzotriazin (HOObt) kann die Reaktionsgeschwindigkeit gesteigert werden. Die Kupplungen werden normalerweise mit äquimolaren Anteilen der Kupplungskomponenten sowie des Kupplungsreagenz in Lösungsmitteln wie Dichlormethan, Tetrahydrofuran, Acetonitril, Dimethylformamid (DMF), Dimethylacetamid (DMA), N-Methylpyrrolidon (NMP) oder Gemischen aus diesen und bei Temperaturen zwischen -30 und +30°C, bevorzugt -20 und +25°C, durchgeführt. Sofern erforderlich wird als zusätzliche Hilfsbase *N*-Ethyldiisopropylamin (Hünig-Base) bevorzugt.
      Als weiteres Kupplungsverfahren zur Synthese von Verbindungen der allgemeinen Formel **I** wird das sogenannte "Anhydridverfahren" (siehe auch: M. Bodanszky, "Peptide Chemistry", Springer-Verlag 1988, S. 58-59; M. Bodanszky, "Principles of Peptide Synthesis", Springer-Verlag 1984, S. 21-27) eingesetzt. Bevorzugt wird das "gemischte Anhydridverfahren" in der Variante nach Vaughan (J.R. Vaughan Jr., J. Amer. Chem.Soc. 73, 3547 (1951)), bei der unter Verwendung von Chlorkohlensäureisobutylester in Gegenwart von Basen, wie 4-Methylmorpholin oder 4-Ethylmorpholin, das gemischte Anhydrid aus der zu kuppelnden Carbonsäure der allgemeinen Formel **VI** und dem Kohlensäuremonoisobutylester erhalten wird. Die Herstellung dieses gemischten Anhydrids und die Kupplung mit den Aminen der allgemeinen Formel **VII** erfolgt im Eintopfverfahren, unter Verwendung der vorstehend genannten Lösungsmittel und bei Temperaturen zwischen -20°C und +25°C, bevorzugt zwischen 0°C und +25°C.
(c) Zur Herstellung von Verbindungen der allgemeinen Formel **I,** in der alle Reste wie eingangs erwähnt definiert sind:
   Kupplung einer Verbindung der allgemeinen Formel **VIII** 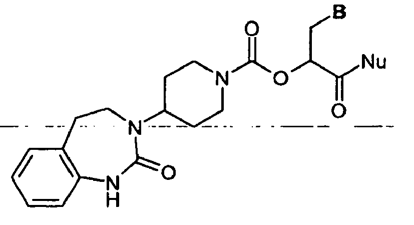 in der **B** wie eingangs erwähnt definiert ist und **Nu** eine Austrittsgruppe, beispielsweise ein Halogenatom, wie das Chlor-, Brom- oder Iodatom, eine Alkylsulfonyloxygruppe mit 1 bis 10 Kohlenstoffatomen im Alkylteil, eine gegebenenfalls durch Chlor- oder Bromatome, durch Methyl- oder Nitrogruppen mono-, di- oder trisubstituierte Phenylsulfonyloxy- oder Naphthylsulfonyloxygruppe, wobei die Substituenten gleich oder verschieden sein können, eine 1*H*-Imidazol-1-yl-, eine gegebenenfalls durch eine oder zwei Methylgruppen im Kohlenstoffgerüst substituierte 1*H*-Pyrazol-1-yl-, eine 1*H*-1,2,4-Triazol-1-yl-, 1*H*-1,2,3-Triazol-1-yl-, 1*H*-1,2,3,4-Tetrazol-1-yl-, eine Vinyl-, Propargyl-, *p*-Nitrophenyl-, 2,4-Dinitrophenyl-, Trichlorphenyl-, Pentachlorphenyl-, Pentafluorphenyl-, Pyranyl- oder Pyridinyl-, eine Dimethylaminyloxy-, 2(1H)-Oxopyridin-1-yl-oxy-, 2,5-Dioxopyrrolidin-1-yloxy-, Phthalimidyloxy-, 1 H-Benzo-triazol-1-yloxy- oder Azidgruppe bedeutet, mit einem Amin der allgemeinen Formel **VII**

      H-N**R¹R²,**

      in der **R¹** und **R²** wie eingangs erwähnt definiert sind.
   Vor Durchführung der Reaktion können in den Resten **R¹** und **R²** des Amins der allgemeinen Formel **VII** gegebenenfalls vorhandene Carbonsäurefunktionen, primäre oder sekundäre Aminofunktionen oder Hydroxyfunktionen durch übliche Schutzreste geschützt und gegebenenfalls verwendete Schutzreste nach Durchführung der Reaktion nach dem Fachmann geläufigen Methoden wieder abgespalten werden.

Die Umsetzung wird unter Schotten-Baumann- oder Einhorn-Bedingungen durchgeführt, das heißt, die Komponenten werden in Gegenwart von wenigstens einem Äquivalent einer Hilfsbase bei Temperaturen zwischen -50°C und +120°C, bevorzugt -10°C und +30°C, und gegebenenfalls in Gegenwart von Lösungsmitteln zur Reaktion gebracht. Als Hilfsbasen kommen bevorzugt Alkali- und Erdalkalihydroxide, beispielsweise Natriumhydroxid, Kaliumhydroxid oder Bariumhydroxid, Alkalicarbonate, z. B. Natriumcarbonat, Kaliumcarbonat oder Cäsiumcarbonat, Alkaliacetate, z.B. Natrium- oder Kaliumacetat, sowie tertiäre Amine, beispielsweise Pyridin, 2,4,6-Trimethylpyridin, Chinolin, Triethylamin, N-Ethyldiisopropylamin, *N*-Ethyldicyclohexylamin, 1,4-Di-azabicyclo[2,2,2]octan oder 1,8-Diaza-bicyclo[5,4,0]undec-7-en, als Lösungsmittel beispielsweise Dichlormethan, Tetrahydrofuran, 1,4-Dioxan, Acetonitril, Dimethylformamid, Dimethylacetamid, N-Methylpyrrolidon oder Gemische davon in Betracht; werden als Hilfsbasen Alkali- oder Erdalkalihydroxide, Alkalicarbonate oder -acetate verwendet, kann dem Reaktionsgemisch auch Wasser als Cosolvens zugesetzt werden.

Die erfindungsgemäßen neuen Verbindungen der allgemeinen Formel **I** enthalten ein oder mehrere Chiralitätszentren. Sind beispielsweise zwei Chiralitätszentren vorhanden, dann können die Verbindungen in Form zweier diastereomerer Antipodenpaare auftreten. Die Erfindung umfasst die einzelnen Isomeren ebenso wie ihre Gemische.

Die Trennung der jeweiligen Diastereomeren gelingt auf Grund ihrer unterschiedlichen physikochemischen Eigenschaften, z.B. durch fraktionierte Kristallisation aus geeigneten Lösemitteln, durch Hochdruckflüssigkeits- oder Säulenchromatographie unter Verwendung chiraler oder bevorzugt achiraler stationärer Phasen.

Die Trennung von unter die allgemeine Formel I fallenden Racematen gelingt beispielsweise durch HPLC an geeigneten chiralen stationären Phasen (z. B. Chiral AGP, Chiralpak AD). Racemate, die eine basische oder saure Funktion enthalten, lassen sich auch über die diastereomeren, optisch aktiven Salze trennen, die bei Umsetzung mit einer optisch aktiven Säure, beispielsweise (+)- oder ~(-)-Weinsäure, (+)- oder ~(-)-Diacetylweinsäure, (+)- oder~(-)-Monomethyltartrat~oder (+)- oder (-)-Camphersulfonsäure, bzw. einer optisch aktiven Base, beispielsweise mit (*R*)-(+)-1-Phenylethylamin, (*S*-)~(-)-1-Phenylethyl-amin oder (S)-Brucin, entstehen.

Nach einem üblichen Verfahren zur Isomerentrennung wird das Racemat einer Verbindung der allgemeinen Formel **I** mit einer der vorstehend angegebenen optisch aktiven Säuren bzw. Basen in äquimolarer Menge in einem Lösemittel umgesetzt und die erhaltenen kristallinen, diastereomeren, optisch aktiven Salze unter Ausnutzung ihrer verschiedenen Löslichkeit getrennt. Diese Umsetzung kann in jeder Art von Lösemitteln durchgeführt werden, solange sie einen ausreichenden Unterschied hinsichtlich der Löslichkeit der Salze aufweisen. Vorzugsweise werden Methanol, Ethanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, oder mit einer geeigneten Säure, beispielsweise mit verdünnter Salzsäure oder wässeriger Methansulfonsäure, vorsichtig neutralisiert und dadurch die entsprechende freie Verbindung in der (+)- oder (-)-Form erhalten.

Jeweils nur das (R)- oder (S)-Enantiomer bzw. ein Gemisch zweier optisch aktiver, unter die allgemeine Formel **I** fallender, diastereomerer Verbindungen wird auch dadurch erhalten, dass man die oben beschriebenen Synthesen mit jeweils einer geeigneten (R)-bzw. (*S*)-konfigurierten Reaktionskomponente durchführt.

Die für die Synthese benötigten Hydroxycarbonsäuren der allgemeinen Formel **V** 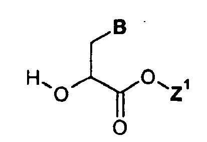 in der **B** wie eingangs erwähnt definiert ist und **Z¹** eine Schutzgruppe für eine Carboxygruppe darstellt, beispielsweise eine C₁₋₆-Alkyl- oder Benzylgruppe, wobei die Alkylgruppen geradkettig oder verzweigt sein können und die Benzylgruppe durch ein oder zwei Methoxygruppen substituiert sein kann, wobei **Z¹** vorzugsweise die Methyl-, Ethyl-, *tert-*Butyl oder Benzylgruppe bedeutet, können aus Verbindungen der allgemeinen Formel **IX** 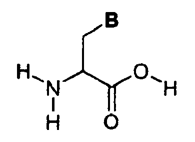 gewonnen werden, in der **B** wie eingangs erwähnt definiert ist.

Durch Diazotierung von Verbindungen der allgemeinen Formel **IX** mit einem geeigneten Diazotierungsreagenz, bevorzugt Natriumnitrit in saurem Milieu, können die Verbindungen der allgemeinen Formel **V** erhalten werden. Bei Einsatz enantiomerenreiner Verbindungen werden die entsprechenden enantiomerenreinen Hydroxycarbonsäureverbindungen erhalten, wobei die Reaktion unter Retention der Konfiguration abläuft.

Ein alternativer Zugang zu Verbindungen der allgemeinen Formel **V** besteht in der Umsetzung von Aldehyden der allgemeinen Formel **X** 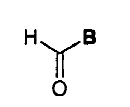 in der **B** wie eingangs erwähnt definiert ist, mit N-Acetylglycin in Acetanhydrid als Lösungsmittel in Gegenwart von Alkaliacetat, bevorzugt Natrium- oder Kaliumacetat bei geeigneter Temperatur, bevorzugt bei 80 bis 130°C.

Die primär entstehenden Azlactone werden ohne Isolierung zu den Verbindungen der allgemeinen Formel **XI** 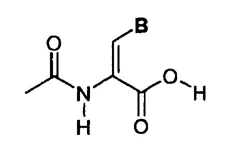 in der **B** wie eingangs erwähnt definiert ist, hydrolysiert.

Durch weitere Umsetzung in Gegenwart von wässrigen Mineralsäuren, wie beispielsweise Schwefel-, Phosphor- oder Chlorwasserstoffsäure, bevorzugt jedoch von Chlorwasserstoffsäure, werden Verbindungen der allgemeinen Formel **XII** 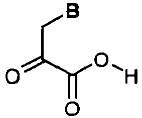 in der **B** wie eingangs erwähnt definiert ist, erhalten. Diese werden dann mit geeigneten Reduktionsmitteln in die Verbindungen der allgemeinen Formel **V** überführt.

Als Reduktionsmittel können Alkaliborhydride, wie Natrium- oder Kaliumborhydrid verwendet werden. Weitere Reduktionsmittel stellen Chlordialkylborane, wie Chlordicyclohexylboran, dar. Werden chirale Chlordialkylborane, wie z.B. B-Chlordüsopinocampheylboran benutzt, können die Verbindungen der allgemeinen Formel **V** in enantiomerenreiner Form isoliert werden.

Die weitere Umsetzung von Verbindungen der allgemeinen Formel **V,** in denen **Z¹** das Wasserstoffatom bedeutet, zu Verbindungen der allgemeinen Formel **V,** in denen **Z¹** wie voranstehend erwähnt definiert ist, erfolgt im alkoholischen Milieu, bevorzugt in Methanol oder Ethanol, in Gegenwart einer geeigneten Säure, wie Chlorwasserstoffsäure. Die Reaktion kann alternativ durch Umsetzung in alkoholischen Lösungsmitteln, bevorzugt Methanol, mit Thionychlorid erfolgen.

Ein weiterer Zugang zu Verbindungen der allgemeinen Formel **V** besteht in der Alkylierung einer Verbindung der Formel **XIII** 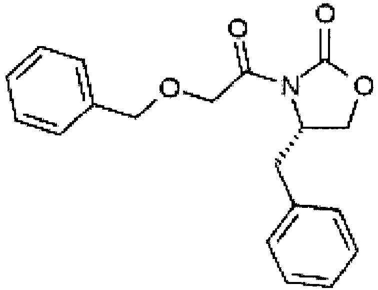 mit einem Aryl- oder Heteroaryl-methylhalogenid der allgemeinen Formel **XIV** 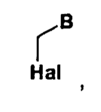 in der **Hal** ein Chlor-, Brom- oder Jodatom bedeutet, und **B** wie eingangs erwähnt definiert ist, in Analogie zu literaturbekannten Methoden (Michael T. Crimmins, Kyle A. Emmitte und Jason D. Katz, Org. Lett. 2, 2165-2167 [2000]).
Die entstehenden diastereomeren Produkte können dann mit Hilfe physikochemischer Methoden, bevorzugt mit Hilfe chromatographischer Methoden oder Umkristallisation, getrennt werden. Die hydrolytische Abspaltung des chiralen Auxiliars und Abspaltung der Benzylschutzgruppe eröffnet ebenfalls einen Zugang zu enantiomerenreinen Hydroxycarbonsäureverbindungen der allgemeinen Formel **V**.
Alle Verbindungen der allgemeinen Formel **I,** die primäre oder sekundäre Amino-, Hydroxy- oder Hydroxycarbonylfunktionen enthalten, werden bevorzugt aus mit Schutzgruppen versehenen Vorstufen gewonnen. Als Schutzgruppen für Aminofunktionen kommen beispielsweise eine Benzyloxycarbonyl-, 2-Nitrobenzyloxycarbonyl-, 4-Nitrobenzyloxycarbonyl-, 4-Methoxy-benzyloxycarbonyl-, 2-Chlorbenzyloxycarbonyl-, 3-Chlorbenzyloxycarbonyl-, 4-Chlorbenzyloxycarbonyl-, 4-Biphenylyl-α,α-dimethyl-benzyloxycarbonyl- oder 3,5-Dimethoxy-α,α-dimethyl-benzyloxycarbonylgruppe, eine Alkoxycarbonylgruppe mit insgesamt 1 bis 5 Kohlenstoffatomen im Alkylteil, beispielsweise die Methoxycarbonyl-, Ethoxycarbonyl-, *n*-Propoxycarbonyl-, Isopropoxycarbonyl-, n-Butoxycarbonyl-, 1-Methylpropoxycarbonyl-, 2-Methylpropoxy-carbonyl- oder *tert*-Butyloxycarbonylgruppe, die Allyloxycarbonyl-, 2,2,2-Trichlor-(1,1-dimethylethoxy)carbonyl- oder 9-Fluorenylmethoxycarbonylgruppe oder eine Formyl-, Acetyl- oder Trifluoracetylgruppe in Frage.
Als Schutzgruppe für Hydroxyfunktionen kommt beispielsweise eine Trimethylsilyl-, Triethylsilyl-, Triisopropyl-, *tert*-Butyldimethylsilyl- oder *tert*-Butyldiphenylsilylgruppe, eine tert-Butyl-, Benzyl-, 4-Methoxybenzyl- oder 3,4-Dimethoxybenzylgruppe in Frage.
Als Schutzgruppe für Hydroxycarbonylfunktionen kommt beispielsweise eine Alkylgruppe mit insgesamt 1 bis 5 Kohlenstoffatomen, beispielsweise die Methyl-, Ethyl-, *n*-Propyl-, Isopropyl-, *n*-Butyl-, *tert*-Butyl, Allyl-, 2,2,2-Trichlorethyl-, Benzyl- oder 4-Methoxybenzylgruppe in Frage.

Die neuen Verbindungen der allgemeinen Formel **I** und deren physiologisch verträgliche Salze weisen wertvolle pharmakologische Eigenschaften auf, die auf ihre selektiven CGRP-antagonistischen Eigenschaften zurückgehen. Ein weiterer Gegenstand der Erfindung sind diese Verbindungen enthaltende Arzneimittel, deren Verwendung und deren Herstellung.

Die voranstehend genannten neuen Verbindungen und deren physiologisch verträgliche Salze besitzen CGRP-antagonistische Eigenschaften und zeigen gute Affinitäten in CGRP-Rezeptorbindungsstudien. Die Verbindungen weisen in den nachstehend beschriebenen pharmakologischen Testsystemen CGRP-antagonistische Eigenschaften auf.

Zum Nachweis der Affinität der voranstehend genannten Verbindungen zu humanen CGRP-Rezeptoren und ihrer antagonistischen Eigenschaften wurden die folgenden Versuche durchgeführt:

### A. Bindungsstudien mit (den humanen CGRP-Rezeptor exprimierenden) SK-N-MC-Zellen

SK-N-MC-Zellen werden in "Dulbecco's modified Eagle Medium" kultiviert. Das Medium konfluenter Kulturen wird entfernt. Die Zellen werden zweimal mit PBS-Puffer (Gibco 041-04190 M) gewaschen, durch Zugabe von PBS-Puffer, versetzt mit 0.02% EDTA, abgelöst und durch Zentrifugation isoliert. Nach Resuspension in 20 ml "Balanced Salts Solution" [BSS (in mM): NaCl 120, KCl 5.4, NaHCO₃ 16.2, MgSO₄ 0.8, NaHPO₄ 1.0, CaCl₂ 1.8, D-Glucose 5.5, HEPES 30, pH 7.40] werden die Zellen zweimal bei 100 x g zentrifugiert und in BSS resuspendiert. Nach Bestimmung der Zellzahl werden die Zellen mit Hilfe eines Ultra-Turrax homogenisiert und für 10 Minuten bei 3000 x g zentrifugiert. Der Überstand wird verworfen und das Pellet in Tris-Puffer (10 mM Tris, 50 mM NaCl, 5 mM MgCl₂, 1 mM EDTA, pH 7.40), angereichert mit 1% Rinderserum-Albumin und 0.1% Bacitracin, rezentrifugiert und resuspendiert (1 ml /1000000 Zellen). Das Homogenat wird bei -80°C eingefroren. Die Membranpräparationen sind bei diesen Bedingungen für mehr als 6 Wochen stabil.

Nach Auftauen wird das Homogenat 1:10 mit Assay-Puffer (50 mM Tris, 150 mM NaCl, 5 mM MgCl₂, 1 mM EDTA, pH 7.40) verdünnt und 30 Sekunden lang mit einem Ultra-Turrax homogenisiert. 230 µl des Homogenats werden für 180 Minuten bei Raumtemperatur mit 50 pM ¹²⁵I-Iodotyrosyl-Calcitonin-Gene-Related Peptide (Amersham) und ansteigenden Konzentrationen der Testsubstanzen in einem Gesamtvolumen von 250 µl inkubiert. Die Inkubation wird durch rasche Filtration durch mit Polyethylenimin (0.1%) behandelte GF/B-Glasfaserfilter mittels eines Zellharvesters beendet. Die an Protein gebundene Radioaktivität wird mit Hilfe eines Gammacounters bestimmt. Als nichtspezifische Bindung wird die gebundene Radioaktivität nach Gegenwart von 1 µM humanem CGRP-alpha während der Inkubation definiert.

Die Analyse der Konzentrations-Bindungskurven erfolgt mit Hilfe einer computergestützten nichtlinearen Kurvenanpassung.

Die eingangs erwähnten Verbindungen zeigen in dem beschriebenen Test IC₅₀-Werte ≤ 10000 nM.

### B. CGRP-Antagonismus in SK-N-MC-Zellen

SK-N-MC-Zellen (1 Mio. Zellen) werden zweimal mit 250 µl Inkubationspuffer (Hanks HEPES, 1 mM 3-Isobutyl-1-methylxanthin, 1% BSA, pH 7.4) gewaschen und bei 37°C für 15 Minuten vorinkubiert. Nach Zugabe von CGRP (10 µl) als Agonist in steigenden Konzentrationen (10̃¹¹ bis 10̃⁶ M) bzw. zusätzlich von Substanz in 3 bis 4 verschiedenen Konzentrationen wird nochmals 15 Minuten inkubiert.

Intrazelluläres cAMP wird anschließend durch Zugabe von 20 µl 1M HCl und Zentrifugation (2000 x g, 4°C für 15 Minuten) extrahiert. Die Überstände werden in flüssigem Stickstoff eingefroren und bei 20°C gelagert.

Die cAMP-Gehalte der Proben werden mittels Radioimmunassay (Fa. Amersham) bestimmt und die pA₂-Werte antagonistisch wirkender Substanzen graphisch ermittelt.

Die erfindungsgemäßen Verbindungen zeigen in dem beschriebenen in *vitro*-Testmodell CGRP-antagonistische Eigenschaften in einem Dosisbereich zwischen 10̃¹² bis 10̃⁵ M.

### INDIKATIONSGEBIETE

Aufgrund ihrer pharmakologischen Eigenschaften eignen sich die erfindungsgemäßen Verbindungen und deren Salze mit physiologisch verträglichen Säuren somit zur akuten und prophylaktischen Behandlung von Kopfschmerzen, insbesondere Migräne-, Cluster-Kopfschmerz sowie Spannungskopfschmerzen. Weiterhin beeinflussen die erfindungsgemäßen Verbindungen auch die folgenden Erkrankungen positiv:
Nicht-insulinabhängigen Diabetes mellitus ("NIDDM"), cardiovaskuläre Erkrankungen, Morphintoleranz, Clostridiumtoxin-bedingte Durchfallerkrankungen, Erkrankungen der Haut, insbesondere thermische und strahlenbedingte Hautschäden inklusive Sonnenbrand, Lichen, Prurigo, pruriginöse Toxidermien sowie schwerer Juckreiz, entzündliche Erkrankungen, z.B. entzündliche Gelenkerkrankungen (Osteoarthritis, rheumatoide Arthritis, neurogene Arthritis), generalisierter Weichteilrheumatismus (Fibromyalgie), neurogene Entzündungen der oralen Mucosa, entzündliche Lungenerkrankungen, allergische Rhinitis, Asthma, COPD, Erkrankungen, die mit einer überschießenden Gefäßerweiterung und dadurch bedingter verringerter Gewebedurchblutung einhergehen, z.B. Schock und Sepsis, chronische Schmerzerkrankungen, wie z.B. diabetische Neuropathien, durch Chemotherapien induzierte Neuropathien, HIV-induzierte Neuropathien, postherpetische Neuropathien durch Gewebetrauma induzierte Neuropathien, trigeminale Neuralgien, temporomandibuläre Dysfunktionen, CRPS (complex regional pain syndrome), Rückenschmerzen, und viszerale Erkrankungen, wie z.B. irritable bowel syndrome (IBS), inflammatory bowel syndrome. Darüber hinaus zeigen die erfindungsgemäßen Verbindungen eine lindernde Wirkung auf Schmerzzustände im allgemeinen. Die Symptomatik menopausaler, durch Gefäßerweiterung und erhöhten Blutfluss verursachter Hitzewallungen östrogendefizienter Frauen sowie hormonbehandelter Prostatakarzinompatienten und Kastraten wird durch die CGRP-Antagonisten der vorliegenden Anwendung präventiv und akut-therapeutisch günstig beeinflusst, wobei sich dieser Therapieansatz vor der Hormonsubstitution durch Nebenwirkungsarmut auszeichnet.

Vorzugsweise eignen sich die erfindungsgemäßen Verbindungen zur akuten und prophylaktischen Behandlung von Migräne- und Cluster-Kopfschmerz, zur Behandlung des irritable bowel syndroms (IBS) und zur präventiven und akut-therapeutischen Behandlung von Hitzewallungen östrogendefizienter Frauen.

Die zur Erzielung einer entsprechenden Wirkung erforderliche Dosierung beträgt zweckmäßigerweise bei intravenöser oder subkutaner Gabe 0.0001 bis 3 mg/kg Körpergewicht, vorzugsweise 0.01 bis 1 mg/kg Körpergewicht, und bei oraler, nasaler oder inhalativer Gabe 0.01 bis 10 mg/kg Körpergewicht, vorzugsweise 0.1 bis 10 mg/kg Körpergewicht, jeweils ein- bis dreimal täglich.

Sofern die Behandlung mit CGRP-Antagonisten oder/und CGRP-Release-Hemmern in Ergänzung zu einer üblichen Hormonsubstitution erfolgt, empfiehlt sich eine Verringerung der vorstehend angegebenen Dosierungen, wobei die Dosierung dann 1/5 der vorstehend angegebenen Untergrenzen bis zu 1/1 der vorstehend angegebenen Obergrenzen betragen kann.

Ein weiterer Gegenstand der Erfindung ist die Verwendung der erfindungsgemäßen Verbindungen als wertvolle Hilfsmittel zur Erzeugung und Reinigung (Affinitätschromatographie) von Antikörpern sowie, nach geeigneter radioaktiver Markierung, beispielsweise durch Tritiierung geeigneter Vorstufen, beispielsweise durch katalytische Hydrierung mit Trithium oder Ersatz von Halogenatomen durch Tritium, in RIA- und ELISA-Assays und als diagnostische bzw. analytische Hilfsmittel in der Neurotransmitter-Forschung.

### KOMBINATIONEN

Als Kombinationspartner denkbare Wirkstoffklassen sind z.B. Antiemetica, Prokinetica, Neuroleptica, Antidepressiva, Neurokinin-Antagonisten, Anticonvulsiva, Histamin-H1-Rezeptorantagonisten, β-Blocker, α-Agonisten und α-Antagonisten, Ergotalkaloiden, schwachen Analgetica, nichtsteroidalen Antiphlogistica, Corticosteroiden, Calcium-Antagonisten, 5-HT_{1B/1D}-Agonisten oder andere Antimigränemitteln, die zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/-Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Lösungen, Dosieraerosole oder Zäpfchen eingearbeitet werden können.

Für die oben erwähnten Kombinationen kommen somit als weitere Wirksubstanzen beispielsweise die nicht-steroidalen Antiphlogistika Aceclofenac, Acemetacin, Acetylsalicylsäure, Acetaminophen (Paracetamol), Azathioprin, Diclofenac, Diflunisal, Fenbufen, Fenoprofen, Flurbiprofen, Ibuprofen, Indometacin, Ketoprofen, Leflunomid, Lornoxicam, Mefenaminsäure, Naproxen, Phenylbutazon, Piroxicam, Sulfasalazin, Zomepirac oder deren pharmazeutisch verträgliche Salze sowie Meloxicam und andere selektive COX2-Inhibitoren, wie beispielsweise Rofecoxib, Valdecoxib, Parecoxib, Etoricoxib und Celecoxib, in Betracht sowie Substanzen, die frühere oder spätere Schritte in der Prostaglandin-Synthese inhibieren oder Prostaglandinrezeptor Antagonisten wie z.B. EP2-Rezeptor Antagonisten und IP-Rezeptor Antagonisten.

Weiterhin können Ergotamin, Dihydroergotamin, Metoclopramid, Domperidon, Diphenhydramin, Cyclizin, Promethazin, Chlorpromazin, Vigabatrin, Timolol, Isomethepten, Pizotifen, Botox, Gabapentin, Pregabalin, Duloxetin, Topiramat, Riboflavin, Montelukast, Lisinopril, Micardis, Prochlorperazin, Dexamethason, Flunarizin, Dextropropoxyphen, Meperidin, Metoprolol, Propranolol, Nadolol, Atenolol, Clonidin, Indoramin, Carbamazepin, Phenytoin, Valproat, Amitryptilin, Imipramine, Venlafaxine, Lidocain oder Diltiazem und andere 5-HT_{1B/1D}-Agonisten wie z.B. Almotriptan, Avitriptan, Eletriptan, Frovatriptan, Naratriptan, Rizatriptan, Sumatriptan und Zolmitriptan verwendet werden.

Außerdem können CGRP-Antagonisten mit Vanilloid-Rezeptor Antagonisten, wie z.B. VR-1 Antagonisten, Glutamatrezeptor Antagonisten, wie z.B. mGlu5-Rezeptor Antagonisten, mGlu1-Rezeptor Antagonisten, iGlu5-Rezeptor Antagonisten, AMPA-Rezeptor Antagonisten, Purinrezeptor Blockern, wie z.B. P2X3 Antagonisten, NO-Synthase Inhibitoren, wie z.B. iNOS Inhibitoren, Calciumkanal-Blockern, wie z.B. PQ-typ Blockern, N-typ Blockern, Kaliumkanalöffnern, wie z.B. KCNQ Kanalöffnern, Natriumkanal Blockern, wie z.B. PN3 Kanal Blockern, NMDA-Rezeptor Antagonisten, Acid-sensing Ionenkanal Antagonisten, wie z.B. ASIC3 Antagonisten, Bradykinin Rezeptor Antagonisten wie z.B. B1-Rezeptor Antagonisten, Cannabinoid-Rezeptor Agonisten, wie z.B. CB2 Agonisten, CB1 Agonisten, Somatostatin-Rezeptor Agonisten, wie z.B. sst2 Rezeptor Agonisten gegeben werden.

Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 20 bis 100 mg Sumatriptan.

### DARREICHUNGSFORMEN

Die erfindungsgemäß hergestellten Verbindungen können entweder alleine oder gegebenenfalls in Kombination mit anderen Wirksubstanzen zur Behandlung von Migräne intravenös, subkutan, intramuskulär, intraartikulär, intrarektal, intranasal, durch Inhalation, topisch, transdermal oder oral erfolgen, wobei zur Inhalation insbesondere Aerosolformulierungen geeignet sind. Die Kombinationen können entweder simultan oder sequentiell verabreicht werden.

Geeignete Anwendungsformen sind beispielsweise Tabletten, Kapseln, Lösungen, Säfte, Emulsionen oder Inhalationspulver oder -aerosole. Hierbei soll der Anteil der pharmazeutisch wirksamen Verbindung(en) jeweils im Bereich von 0.1 bis 90 Gew.-%, bevorzugt 0.5 bis 50 Gew.-% der Gesamtzusammensetzung liegen, d.h. in Mengen die ausreichend sind, um den voranstehend angegebenen Dosierungsbereich zu erreichen.

Die orale Gabe kann in Form einer Tablette, als Pulver, als Pulver in einer Kapsel (z.B. Hartgelatinekapsel), als Lösung oder Suspension erfolgen. Im Fall einer inhalativen Gabe kann die Wirkstoffkombination als Pulver, als wässrige oder wässrig-ethanolische Lösung oder mittels einer Treibgasformulierung erfolgen.

Bevorzugt sind deshalb pharmazeutische Formulierungen gekennzeichnet durch den Gehalt an einer oder mehrerer Verbindungen der Formel **I** gemäß der obigen bevorzugten Ausführungsformen.

Besonders bevorzugt ist es, wenn die Verbindungen der Formel **I** oral verabreicht werden, ganz besonders bevorzugt ist es, wenn die Verabreichung ein oder zweimal täglich erfolgt. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein Geschmack verbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie *p*-Hydroxybenzoate, enthalten.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmacksaufbesserern oder Farbstoffen versetzt werden.

Ebenfalls bevorzugt ist es, wenn die Verbindungen der Formel **I** inhalativ verabreicht werden, besonders bevorzugt ist es, wenn die Verabreichung ein oder zweimal täglich erfolgt. Hierzu müssen die Verbindungen der Formel **I** in inhalierbaren Darreichungsformen bereitgestellt werden. Als inhalierbare Darreichungsformen kommen Inhalationspulver, treibgashaltige Dosieraerosole oder treibgasfreie Inhalationslösungen in Betracht, die gegebenenfalls im Gemisch mit gebräuchlichen physiologisch verträglichen Hilfsstoffen vorliegen.

Im Rahmen der vorliegenden Erfindung sind von dem Begriff treibgasfreie Inhalationslösungen auch Konzentrate oder sterile, gebrauchsfertige Inhalationslösungen umfasst. Die im Rahmen der vorliegenden Erfindung einsetzbaren Darreichungsformen werden im nachfolgenden Teil der Beschreibung detailliert beschrieben.

### EXPERIMENTELLER TEIL

Für die hergestellten Verbindungen liegen in der Regel ¹H-NMR und/oder Massenspektren vor. Wenn nicht anders angegeben, werden R_{f}-Werte unter Verwendung von DC-Fertigplatten Kieselgel 60 F254 (E. Merck, Darmstadt, Artikel-Nr. 1.05714) ohne Kammersättigung bestimmt.
Die bei den Fliessmitteln angegebenen Verhältnisse beziehen sich auf Volumeneinheiten der jeweiligen Lösungsmittel. Die angegebenen Volumeneinheiten bei NH₃ beziehen sich auf eine konzentrierte Lösung von NH₃ in Wasser.
Soweit nicht anders vermerkt sind die bei den Aufarbeitungen der Reaktionslösungen verwendeten Säure-, Basen- und Salzlösungen wässrige Systeme der angegebenen Konzentrationen. Zu chromatographischen Reinigungen wird Kieselgel der Firma Millipore (MATREX*^{™}*, 35-70 µm) verwendet.
Die angegebenen HPLC-Daten werden unter nachstehend angeführten Parametern gemessen:

### Methode A:

| Zeit (min) | Volumenprozent Wasser (mit 0.1% Ameisensäure) | Volumenprozent Acetonitril (mit 0.1% Ameisensäure) |
|---|---|---|
| 0 | 95 | 5 |
| 4.5 | 10 | 90 |
| 5 | 10 | 90 |
| 5.5 | 90 | 10 |

Analytische Säule: Zorbax-Säule (Agilent Technologies), SB (Stable Bond) C18; 3.5 µm; 4.6 x 75 mm; Säulentemperatur: 30°C; Fluss: 1.6 mL / min; Injektionsvolumen: 5 µL; Detektion bei 254 nm

### Methode B:

| Zeit (min) | Volumenprozent Wasser (mit 0. 1 % Ameisensäure) | Volumenprozent Acetonitril (mit 0. 1 % Ameisensäure) |
|---|---|---|
| 0 | 95 | 5 |
| 9 | 10 | 90 |
| 10 | 10 | 90 |
| 11 | 95 | 5 |

Analytische Säule: Zorbax-Säule (Agilent Technologies), SB (Stable Bond) C18; 3.5 µm; 4.6 x 75 mm; Säulentemperatur: 30°C; Fluss: 0.8 mL / min; Injektionsvolumen: 5 µL; Detektion bei 254 nm

### Methode C:

| Zeit (min) | Volumenprozent Wasser (mit 0. 1 % Ameisensäure) | Volumenprozent Acetonitril (mit 0. 1 % Ameisensäure) |
|---|---|---|
| 0 | 95 | 5 |
| 4 | 50 | 50 |
| 4.5 | 10 | 90 |
| 5 | 10 | 90 |
| 5.5 | 90 | 10 |

Analytische Säule: Zorbax-Säule (Agilent Technologies), SB (Stable Bond) C18; 3.5 µm; 4.6 x 75 mm; Säulentemperatur: 30°C; Fluss: 1.6 mL / min; Injektionsvolumen: 5 µL; Detektion bei 254 nm

### Methode D:

| Zeit (min) | Volumenprozent Wasser (mit 0.04% TFA) | Volumenprozent Acetonitril (mit 0.04% TFA) |
|---|---|---|
| 0 | 80 | 20 |
| 30 | 20 | 80 |

Analytische Säule: Waters Symmetry C8, 5 µM, 4.6 x 150 mm; Säulentemperatur: 25°C, Fluss: 1.3 mL/min, Injektionsvolumen: 5 µL, Detektion bei 254 nm.

### Methode E:

| Zeit (min) | Volumenprozent Wasser (mit 0.04% TFA) | Volumenprozent Acetonitril (mit 0.04% TFA) |
|---|---|---|
| 0 | 80 | 20 |
| 15 | 20 | 80 |
| 17 | 20 | 80 |

Analytische Säule: Symmetry C8 Waters - 4.6 X 150 mm; 5 micron, Fluss: 1.3 mL/min, Säulentemperatur: 25°C, Detektion bei 254 nm.
Bei präparativen HPLC-Reinigungen werden in der Regel die gleichen Gradienten verwendet, die bei der Erhebung der analytischen HPLC-Daten benutzt wurden.
Die Sammlung der Produkte erfolgt massengesteuert, die Produkt enthaltenden Fraktionen werden vereinigt und gefriergetrocknet.
Falls nähere Angaben zur Konfiguration fehlen, bleibt offen, ob es sich um reine Enantiomere handelt oder ob partielle oder gar völlige Racemisierung eingetreten ist.

In den Versuchsbeschreibungen werden die folgenden Abkürzungen verwendet:
- Cyc: Cyclohexan
- DCM: Dichlormethan
- DIPE: Diisopropylether
- DMF: N,N-Dimethylformamid
- EtOAc: Essigsäureethylester
- EtOH: Ethanol
- HATU: *O*-(7-Azabenztriazol-1-yl)-*N,N,N',N'*-tetramethyluronium-hexafluorophosphat
- AcOH: Essigsäure
- i.vac.: in vacuo (im Vakuum)
- MeOH: Methanol
- MTBE: *tert*-Butylmethylether
- NaOAc: Natriumacetat
- NMP: N-Methylpyrroldinon
- PE: Petrolether
- RT: Raumtemperatur
- TBTU: O-(Benzotriazol-1-yl)-*N*,*N*,*N*',*N*'-tetramethyluronium-tetrafluoroborat
- TFA: Trifluoressigsäure
- THF: Tetrahydrofuran

### Herstellung der Ausgangsverbindungen:

### Amin A1

### [4,4']Bipiperidinyl-1-yl-oxo-essiasäureethylester

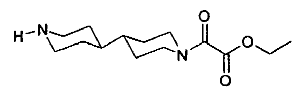

### A1a) 1'-Ethoxyoxalyl-[4,4']bipiperidinyl-1-carbonsäure-tert-butylester

Zu einer Lösung von 4.0 g (14.9 mmol) [4,4']Bipiperidinyl-1-carbonsäure-*tert*-butylester und 2.15 mL (15.4 mmol) Triethylamin in 80 mL DCM wurden unter Eiskühlung langsam 1.68 mL (15.0 mmol) Chlor-oxo-essigsäureethylester zugetropft und das Reaktionsgemisch 1 h bei RT nachgerührt. Zur Reaktionslösung wurde wenig Wasser gegeben, die organische Phase abgetrennt und über Na₂SO₄ getrocknet. Nach Entfernen des Trocken- und Lösungsmittels wurde der Rückstand in EtOAc aufgenommen und über Kieselgel filtriert, wobei das Produkt mit EtOAc eluiert wurde. Nach Einengen i.vac. erhielt man das Produkt, welches ohne Reinigung weiter umgesetzt wurde.

| | |
|---|---|
| Ausbeute: | 3.1 g (57% der Theorie) |
| ESI-MS: | (M+NH₄)⁺ = 386 |

### A1b) [4,4']Bipiperidinyl-1-yl-oxo-essigsäureethylester

Zu einer Lösung von 3.08 g (8.36 mmol) 1'-Ethoxyoxalyl-[4,4']bipiperidinyl-1-carbonsäure-*tert*-butylester in 40 mL DCM wurden 5 mL TFA zugetropft und das Reaktionsgemisch 4 h bei RT gerührt. Man engte i.vac. ein, nahm den Rückstand in 50 mL DCM auf und rührte diese Lösung langsam in eine eisgekühlte Lösung von 4 g Na₂CO₃ in 20 mL Wasser ein. Nach beendeter Zugabe wurde weitere 15 min gerührt, die organische Phase abgetrennt, die wässrige Phase noch zweimal mit DCM extrahiert und die vereinigten organischen Phasen über Na₂SO₄ getrocknet. Nach Entfernen des Trocken- und Lösungsmittels erhielt man das Produkt als Hydrogencarbonat-Salz.

| | |
|---|---|
| Ausbeute: | 2.33 g (84% der Theorie) |
| ESI-MS: | (M+H)⁺ = 269 |

### Amin A2

### 4-[4,4']Bipiperidinyl-1-yl-4-oxo-butansäureethylester

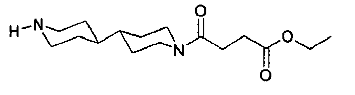

### A2a) 1'-(3-Carboxy-propionyl)-[4,4']bipiperidinyl-1-carbonsäure-tert-butylester

Zu einer Lösung von 10.0 g (37.0 mmol) [4,4']Bipiperidinyl-1-carbonsäure-*tert*-butylester in 100 mL THF wurde eine Lösung von 4.07 g (41.0 mmol) Bernsteinsäureanhydrid in 50 mL THF langsam zugetropft und das Reaktionsgemisch über Nacht bei RT gerührt. Zur Vervollständigung der Reaktion wurden weitere 2.0 g (20.0 mmol) Bernsteinsäureanhydrid zugegeben, das Reaktionsgemisch 4 h bei 50°C und über Nacht bei RT gerührt. Man versetzte vorsichtig mit 200 mL 7.5% K₂CO₃-Lösung und 200 mL EtOAc, trennte die organische Phase ab, extrahierte diese nochmals mit 100 mL 7.5% K₂CO₃-Lösung und säuerte die vereinigten wässrigen Phasen mit 10% Zitronensäure-Lösung an. Man extrahierte die wässrige Phase mit 300 mL EtOAc, trennte die organische Phase ab und entfernte i.vac. das Lösungsmittel.

| | |
|---|---|
| Ausbeute: | 11.7 g (85% der Theorie) |
| ESI-MS: | (M-H)⁻ = 367 |

### A2b) 4-[4,4']Bipiperidinyl-1-yl-4-oxo-butansäureethylester

Eine Lösung von 11.7 g (31.7 mmol) 1'-(3-Carboxy-propionyl)-[4,4']bipiperidinyl-1-carbonsäure-*tert*-butylester in 250 mL ethanolischer HCl (1.25 M) wurde über Nacht bei RT gerührt. Man engte i.vac. zur Trockene ein, nahm den Rückstand in 100 mL EtOAc und 100 mL 15% K₂CO₃-Lösung auf, trennte die organische Phase ab und trocknete diese über Na₂SO₄. Nach Entfernen des Trocken- und Lösungsmittels wurde das Produkt ohne Reinigung weiter umgesetzt.

| | |
|---|---|
| Ausbeute: | 4.3 g (46% der Theorie) |
| ESI-MS: | (M+H)⁺ = 297 |

### Herstellung der Endverbindungen:

### Beispiel 1

### 4-(2-Oxo-1,2,4,5-tetrahydro-1,3-benzdiazepin-3-yl)-piperidin-1-carbonsäure-(R)-2-[4-(4-methyl-pi perazin-1-yl)-piperid in-1-yl]-2-oxo-1-(3-trifluormethyl-benzyl)-ethylester

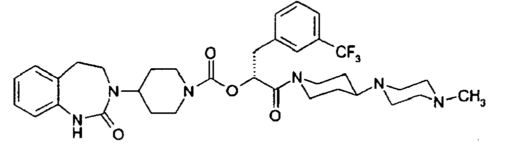

### 1a) (Z,E)-2-Acetylamino-3-(3-trifluormethyl-phenyl)-acrylsäure

Eine Lösung von 15.8 g (115 mmol) 3-Trifluormethyl-benzaldehyd und 21.3 g (182 mmol) N-Acetylglycin in 60 mL Essigsäureanhydrid wurde 4 h auf 120°C (Badtemperatur) erhitzt. Nach dem Abkühlen wurden langsam 40 mL Wasser zugegeben und 1 h bei 80°C gerührt. Nach dem Abkühlen wurde mit 400 mL Wasser und 150 mL Toluol versetzt, 1 h gerührt, der entstandene Niederschlag abfiltriert, dieser mit Toluol gewaschen und bei 50°C getrocknet.

| | |
|---|---|
| Ausbeute: | 16.7 g (53% der Theorie) |
| ESI-MS: | (M+H)⁺ = 274 |
| R_{f} = | 0.4 (Kieselgel, DCM/MeOH/AcOH 90:10:1) |

### 1b) 2-Oxo-3-(3-trifluormethyl-phenyl)-propionsäure

Zu einer auf 80°C (Badtemperatur) erhitzten Suspension von 16.6 g (60.8 mmol) (*Z,E*)-2-Acetylamino-3-(3-trifluormethyl-phenyl)-acrylsäure in 200 mL 1,4-Dioxan wurden 200 mL 4 M HCL zugetropft, das Reaktionsgemisch 1.5 h unter Rückfluss erhitzt und anschließend über Nacht bei RT gerührt. Zur Vervollständigung der Reaktion wurde weitere 4 h unter Rückfluss erhitzt. Das organische Lösungsmittel wurde i.vac. entfernt, der wässrige Rückstand mit NaOH bis zur alkalischen Reaktion versetzt, mit MTBE gewaschen und mit HCl sauer gestellt. Man extrahierte erschöpfend mit EtOAc und trocknete die vereinigten organischen Phasen über MgSO₄. Nach Entfernen des Trocken- und Lösungsmittels erhielt man das gewünschte Produkt, welches ohne Reinigung weiter umgesetzt wurde.

| | |
|---|---|
| Ausbeute: | 5.7 g (40% der Theorie) |
| ESI-MS: | (M-H)⁻ = 231 |
| R_{f} = | 0.19 (Kieselgel, DCM/MeOH/Cyc/NH₃ 70:15:15:2) |

### 1c) (R)-2-Hydroxy-3-(3-trifluormethyl-phenyl)-propionsäure

Unter Stickstoffatmosphäre wurden zu einer auf -35°C gekühlten Lösung von 5.70 g (24.6 mmol) 2-Oxo-3-(3-trifluormethyl-phenyl)-propionsäure und 4.4 mL (31.4 mmol) Triethylamin in 60 mL THF innerhalb von 15 min eine Lösung von 11.8 g (36.8 mmol) (1*R*)-B-Chlordiisopinocampheylboran in 40 mL THF zugetropft und die Reaktionslösung über Nacht bei RT gerührt. Anschließend wurde die Reaktionslösung unter Eiskühlung vorsichtig mit 20 mL 1 M NaOH alkalisch gestellt und 30 min nachgerührt. Man versetzte mit 40 mL EtOAc, trennte die wässrige Phase ab, wusch die organische Phase dreimal mit je 15 mL 1 M NaOH-Lösung und einmal mit 15 mL Wasser. Die vereinigten wässrigen Phasen wurden mit halbkonzentrierter HCl sauer gestellt, zweimal mit je 40 mL EtOAc extrahiert und die vereinigten organischen Phasen über MgSO₄ getrocknet. Nach Entfernen des Trocken- und Lösungsmittels erhielt man das Produkt, welches ohne Reinigung weiter umgesetzt wurde.

| | |
|---|---|
| Ausbeute: | 4.25 g (74% der Theorie) |
| ESI-MS: | (M-H)⁻ = 233 |
| R_{f} = | 0.35 (Kieselgel, DCM/MeOH/Cyc/NH₃ 70:15:15:2) |

### 1d) (R)-2-Hydroxy-3-(3-trifluormethyl-phenyl)-propionsäuremethylester

Eine Lösung von 4.20 g (17.9 mmol) (R)-2-Hydroxy-3-(3-trifluormethyl-phenyl)-propionsäure in 80 mL methanolischer HCl wurde 70 h bei RT gerührt. Die Reaktionslösung wurde i.vac. eingeengt, der Rückstand in Wasser aufgenommen und erschöpfend mit EtOAc extrahiert. Die vereinigten organischen Phasen wurden dreimal mit 15% K₂CO₃-Lösung und einmal mit Wasser gewaschen und über MgSO₄ getrocknet. Nach Entfernen des Trocken- und Lösungsmittels wurde der Rückstand ohne Reinigung weiter umgesetzt.

| | |
|---|---|
| Ausbeute: | 2.47 g (55% der Theorie) |
| ESI-MS: | (M+H)⁺ = 249 |
| R_{f} = | 0.73 (Kieselgel, DCM/MeOH/Cyc/NH₃ 70:15:15:2) |

### 1e) 4-(2-Oxo-1,2,4,5-tetrahydro-1,3-benzdiazepin-3-yl)-piperidin-1-carbonsäure-(R)-1-methoxycarbonyl-2-(3-trifluormethyl-phenyl)-ethylester

Unter Stickstoffatmosphäre wurden zu einer Lösung von 1.64 g (13.4 mmol) 4-Dimethylaminopyridin in 40 mL Pyridin 2.10 g (10.4 mmol) Chlorameisensäure-4-nitrophenylester gegeben und 3 h bei RT gerührt. Dann wurden 2.47 g (9.95 mmol) (*R*)-2-Hydroxy-3-(3-trifluormethyl-phenyl)-propionsäuremethylester in 20 mL Pyridin zugegeben und das Reaktionsgemisch 2.5 h bei RT gerührt. Die Reaktionslösung wurde mit 4.1 g (10.9 mmol) 3-Piperidin-4-yl-1,3,4,5-tetrahydro-1,3-benzdiazepin-2-on (Reinheit 65%) versetzt und 20 h bei RT gerührt. Man engte i.vac. ein, nahm den Rückstand in 60 mL EtOAc auf, wusch die organische Phase zweimal mit 15% K₂CO₃-Lösung, viermal mit gesättigter NaHCO₃-Lösung und trocknete diese über Na₂SO₄. Nach Entfernen des Trocken- und Lösungsmittels wurde der Rückstand chromatographisch (Kieselgel, Cyc/EtOAc 1:2) gereinigt.

| | |
|---|---|
| Ausbeute: | 3.16 g (61 % der Theorie) |
| ESI-MS: | (M+H)⁺ = 520 |
| R_{f} = | 0.93 (Kieselgel, EtOAc/MeOH/NH₃ 80:20:2) |

### 1f) 4-(2-Oxo-1,2,4,5-tetrahydro-1,3-benzdiazepin-3-yl)-piperidin-1-carbonsäure-(R)-1-carboxy-2-(3-trifluormethyl-phenyl)-ethylester

Zu einer Lösung von 3.10 g (5.97 mmol) 4-(2-Oxo-1,2,4,5-tetrahydro-1,3-benzdiazepin-3-yl)-piperidin-1-carbonsäure-(R)-1-methoxycarbonyl-2-(3-trifluor-methyl-phenyl)-ethylester in 80 mL THF wurde eine Lösung von 0.22 g (9.00 mmol) LiOH in 40 mL Wasser gegeben und das Reaktionsgemisch 1 h bei RT gerührt. Das organische Lösungsmittel wurde i.vac. entfernt, der wässrige Rückstand mit 80 mL Wasser versetzt und mit 1 M HCl sauer gestellt. Der ausgefallene Niederschlag wurde abgesaugt und über Nacht bei 35°C getrocknet.

| | |
|---|---|
| Ausbeute: | 2.80 g (93% der Theorie) |
| ESI-MS: | (M+H)⁺ = 506 |
| R_{f} = | 0.58 (Kieselgel, EtOAc/MeOH/NH₃ 70:30:3) |

### 1g) 4-(2-Oxo-1,2,4,5-tetrahydro-1,3-benzdiazepin-3-yl)-piperidin-1-carbonsäure-(R)-2-[4-(4-methyl-piperazin-1-yl)-piperidin-1-yl]-2-oxo-1-(3-trifluormethyl-benzyl)-ethylester

Zu einer Lösung von 80.0 mg (0.16 mmol) 4-(2-Oxo-1,2,4,5-tetrahydro-1,3-benzdiazepin-3-yl)-piperidin-1-carbonsäure-(R)-1-carboxy-2-(3-trifluormethyl-phenyl)-ethylester, 61 mg (0.19 mmol) TBTU und 27 µL (0.20 mmol) Triethylamin in 1 mL DMF wurden bei RT 30 mg (0.16 mmol) 1-Methyl-4-piperidin-4-yl-piperazin gegeben und die Reaktionslösung 70 h bei RT gerührt. Diese wurde ohne weitere Aufarbeitung via HPLC gereinigt; die das Produkt enthaltenden Fraktionen wurden vereinigt und lyophilisiert.

| | |
|---|---|
| Ausbeute: | 67 mg (63% der Theorie) |
| ESI-MS: | (M+H)⁺ = 671 |
| R_{f} = | 0.4 (Kieselgel, DCM/MeOH/Cyc/NH₃ 70:15:15:2) |

Analog wurden folgende Verbindungen aus jeweils 80 mg (Beispiele 1.1 bis 1.4) bzw. 140 mg (Beispiele 1.5 und 1.6) 4-(2-Oxo-1,2,4,5-tetrahydro-1,3-benzdiazepin-3-yl)-piperidin-1-carbonsäure-(*R*)-1-carboxy-2-(3-trifluor-methyl-phenylrethylester und der entsprechenden Menge an Amin hergestellt:

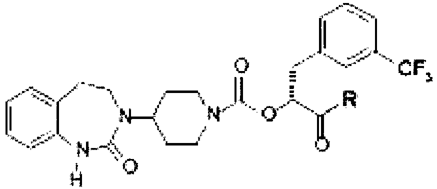

| **Beispiel** | **R** | **Ausbeute (%)** | **Massenspektrum** | **R_{f} ^{##}) (Methode)** |
|---|---|---|---|---|
| **1.1** | 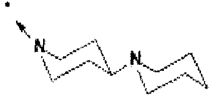 | 93 | 656 | 0.68 |
| | | | [M+H]⁺ | |
| **1.2** | 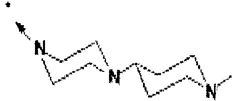 | 64 | 671 | 0.35 |
| | | | [M+H]⁺ | |
| **1.3** | 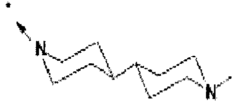 | 74 | 670 | 0.53 |
| | | | [M+H]⁺ | |
| **1.4** | 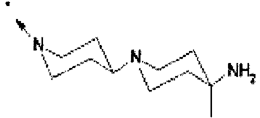 | 48 | 685 | 5.1 min |
| | | | [M+H]⁺ | (B) |
| **1.5** | 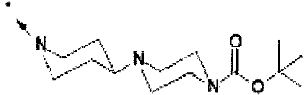 | 62 | 757 | 0.51 |
| | | | [M+H]⁺ | |
| **1.6** | 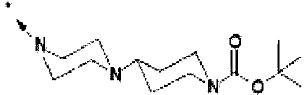 | 71 | 757 | 0.50 |
| | | | [M+H]⁺ | |

| | | | | |
|---|---|---|---|---|
| ^{##}) (kieselgel, DCM/MeOH/ Cyc/NH₃ 70:15:15:2) oder Retentionszeit HPLC | | | | |

### Beispiel 1.7

### 4-(2-Oxo-1,2,4,5-tetrahydro-1,3-benzdiazepin-3-ylrpiperidin-1-carbonsäure-(R)-1-(3-methyl-benzyl)-2-oxo-2-(4-piperazin-1-yl-piperidin-1-yl)-ethylester

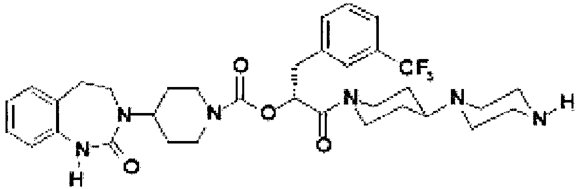

Eine Lösung von 131 mg (0.17 mmol) 4-{1-[(*R*)-2-[4-(2-Oxo-1,2,4,5-tetrahydro-1,3-benzdiazepin-3-yl)-piperidin-1-carbonyloxy]-3-(3-trifluormethyl-phenyl)-propionyl]-piperidin-4-yl}-piperazin-1-carbonsäure-*tert*-butylester (Beispiel 1.5) in 1.5 mL 4 M HCI wurde über Nacht bei RT gerührt. Die Reaktionslösung wurde ohne weitere Aufarbeitung via HPLC gereinigt. Die das Produkt enthaltenden Fraktionen wurden vereinigt und lyophilisiert.
Ausbeute: 75 mg (67% der Theorie)
ESI-MS: (M+H)⁺ = 657
R_{f} = 0.38 (Kieselgel, DCM/MeOH/Cyc/NH₃ 70:15:15:2)

### Beispiel 1.8

### 4-(2-Oxo-1,2,4,5-tetrahydro-1,3-benzdiazepin-3-yl)-piperidin-1-carbonsäure-(R)-2-oxo-2-(4-piperidin-4-yl-piperazin-1-yl)-1-(3-trifluormethyl-benzyl)-ethylester

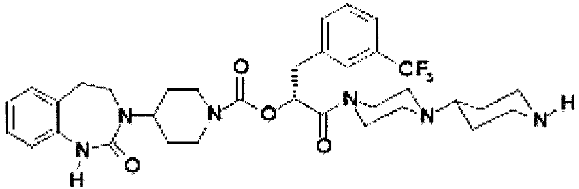

Analog Beispiel 1.7 wurde aus 149 mg (0.20 mmol) 4-(2-Oxo-1,2,4,5-tetrahydro-1,3-benzdiazepin-3-yl)-piperidin-1-carbonsäure-(*R*)-1-(3-trifluormethyl-benzyl)-2-[4-(1-*tert*-butoxycarbonyl-piperidin-4-yl)-piperazin-1-yl]-2-oxo-ethylester (Beispiel 1.6) das Produkt erhalten.
Ausbeute: 66 mg (51 % der Theorie)
ESI-MS: (M+H)⁺ = 657
R_{f} = 0.18 (Kieselgel, DCM/MeOH/Cyc/NH₃ 70:15:15:2)

### Beispiel 2

### 4-(2-Oxo-1,2,4,5-tetrahydro-1,3-benzdiazepin-3-yl)-piperidin-1-carbonsäure-(R)-2-[4-(4-methyl-piperazin-1-yl)-piperidin-1-yl]-2-oxo-1-(3-methyl-benzyl)-ethylester

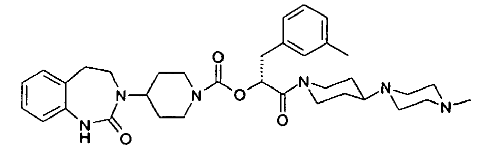

### 2a) (Z,E)-2-Acetylamino-3-m-tolyl-acrylsäure

Analog Beispiel 1a konnte aus 25.0 g (212 mmol) 3-Methyl-benzaldehyd und 24.9 g (212 mmol) N-Acetylglycin das Produkt erhalten werden.

| | |
|---|---|
| Ausbeute: | 26.0 g (56% der Theorie) |
| ESI-MS: | (M+H)⁺ = 220 |
| Retentionszeit (HPLC): | 5.4 min (Methode B) |

### 2b) 2-Oxo-3-m-tolyl-propionsäure

Zu einer Lösung von 13.0 g (59.3 mmol) (*Z,E*)-2-Acetylamino-3-*m*-tolyl-acrylsäure in 200 mL 1,4-Dioxan wurden 400 mL 4 M HCl gegeben und das Reaktionsgemisch 2.5 h unter Rückfluss erhitzt. Das organische Lösungsmittel wurde i.vac. eingeengt, der ausgefallene Niederschlag abgesaugt und getrocknet. Dieser wurde in 300 mL EtOAc aufgenommen, die organische Phase zweimal mit je 200 mL Wasser gewaschen und über Na₂SO₄ getrocknet. Nach Entfernen des Trocken- und Lösungsmittels wurde der Rückstand ohne Reinigung weiter umgesetzt.

| | |
|---|---|
| Ausbeute: | 9.2 g (88% der Theorie) |
| ESI-MS: | (M-H)⁻ = 177 |
| Retentionszeit (HPLC): | 7.3 min (Methode B) |

### 2c) (R)-2-Hydroxy-3-m-tolyl-propionsäure

Analog Beispiel 1c konnte aus 9.24 g (51.9 mmol) 2-Oxo-3-*m*-tolyl-propionsäure und 24.0 g (74.8 mmol) (1*R*)-B-Chlordiisopinocampheylboran das Produkt erhalten werden.

| | |
|---|---|
| Ausbeute: | 8.4 g (90% der Theorie) |
| ESI-MS: | (M-H)⁻ = 179 |
| Retentionszeit (HPLC): | 7.2 min (Methode B) |

### 2d) (R)-2-Hydroxy-3-m-tolyl-propionsäuremethylester

Zu einer auf 0°C gekühlten Lösung von 8.40 g (46.6 mmol) (R)-2-Hydroxy-3-m-tolyl-propionsäure in 200 mL MeOH wurden 3.74 mL (51.28 mmol) SOCl₂ langsam zugetropft und das Reaktionsgemisch nach beendeter Zugabe 1 h bei RT gerührt. Man engte i.vac. ein und reinigte den Rückstand chromatographisch (Kieselgel, Cyc/EtOAc 3:1).

| | |
|---|---|
| Ausbeute: | 6.28 g (69% der Theorie) |
| ESI-MS: | (M+H)⁺ = 195 |
| Retentionszeit (HPLC): | 6.9 min (Methode B) |

### 2e) 4-(2-Oxo-1,2,4,5-tetrahydro-1,3-benzdiazepin-3-yl)-piperidin-1-carbonsäure-(R)-1-methoxycarbonyl-2-m-tolyl-ethylester

Analog Beispiel 1e konnte aus 1.12 g (5.76 mmol) (R)-2-Hydroxy-3-m-tolyl-propionsäure-methylester und 1.41 g (5.76 mmol) 3-Piperidin-4-yl-1,3,4,5-tetrahydro-1,3-benzdiazepin-2-on das Produkt erhalten werden.

| | |
|---|---|
| Ausbeute: | 2.07 g (77% der Theorie) |
| ESI-MS: | (M+H)⁺ = 466 |
| Retentionszeit (HPLC): | 9.0 min (Methode B) |

### 2f) 4-(2-Oxo-1,2,4,5-tetrahydro-1,3-benzdiazepin-3-yl)-piperidin-1-carbonsäure-(R)-1-carboxy-2-m-tolyl-ethylester

Analog Beispiel 1f konnte aus 2.07 g (4.45 mmol) 4-(2-Oxo-1,2,4,5-tetrahydro-1,3-benz-diazepin-3-yl)-piperidin-1-carbonsäure-(R)-1-methoxycarbonyl-2-m-tolyl-ethylester und 0.16 g (6.72 mmol) LiOH das Produkt erhalten werden.

| | |
|---|---|
| Ausbeute: | 1.86 g (93% der Theorie) |
| ESI-MS: | (M+H)⁺ = 452 |
| Retentionszeit (HPLC): | 8.0 min (Methode B) |

### 2g) 4-(2-Oxo-1,2,4,5-tetrahydro-1,3-benzdiazepin-3-yl)-piperidin-1-carbonsäure-(R)-2-[4-(4-methyl-piperazin-1-yl)-piperidin-1-yl]-2-oxo-1-(3-methyl-benzyl)-ethylester

Eine Lösung von 80.0 mg (0.18 mmol) 4-(2-Oxo-1,2,4,5-tetrahydro-1,3-benzdiazepin-3-yl)-piperidin-1-carbonsäure-(R)-1-carboxy-2-m-tolyl-ethylester, 57 mg (0.18 mmol) TBTU und 31 µL (0.22 mmol) Triethylamin in 1 mL DMF wurden bei RT 1 h gerührt. Dann erfolgte die Zugabe von 33 mg (0.18 mmol) 1-Methyl-4-piperidin-4-yl-piperazin zur Reaktionslösung, welche anschließend über Nacht bei RT gerührt wurde. Diese wurde ohne weitere Aufarbeitung via HPLC gereinigt; die das Produkt enthaltenden Fraktionen wurden vereinigt und lyophilisiert.

| | |
|---|---|
| Ausbeute: | 46.7 mg (43% der Theorie) |
| ESI-MS: | (M+H)⁺ = 617 |
| Retentionszeit (HPLC): | 5.5 min (Methode B) |

Analog wurden folgende Verbindungen aus jeweils 80 mg 4-(2-Oxo-1,2,4,5-tetrahydro-1,3-benzdiazepin-3-yl)-piperidin-1-carbonsäure-(*R*)-1-carboxy-2-*m*-tolyl-ethylester und der entsprechenden Menge an Amin hergestellt:

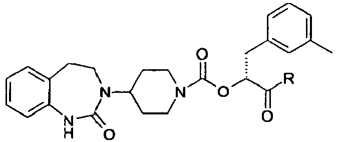

| **Bespiel** | **R** | **Ausbeute (%)** | **Massenspektrum** | **Retentionszeit HPLC (Methode)** |
|---|---|---|---|---|
| **2.1** | 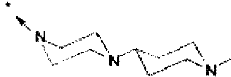 | 80 | 617 | 5.0 min |
| | | | [M+H]⁺ | (B) |
| **2.2** | 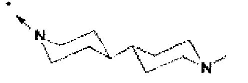 | 75 | 616 | 6.2 min |
| | | | [M+H]⁺ | (B) |

### Beispiel 3

### 4-(2-Oxo-1,2,4,5-tetrahydro-1,3-benzdiazepin-3-yl)-piperidin-1-carbonsäure-(R)-1-(3,5-bis-trifluormethyl-benzyl)-2-(1'-ethoxycarbonylmethyl-4,4'-bipiperidinyl-1-yl)-2-oxo-ethylester

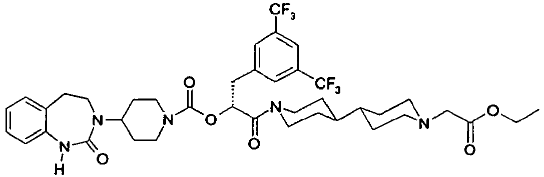

### 3a) 2-Acetylamino-3-(3,5-bis-trifluormethyl-phenyl)acrylsäuremethylester

Unter Stickstoffatmosphäre wurden 50.0 g (171 mmol) 3,5-Bis-(trifluormethyl)-brombenzol und 25.0 g (171 mmol) 2-Acetylamino-acrylsäuremethylester in 475 mL Triethylamin und 250 mL Acetonitril mit 3.9 g (12.4 mmol) Tri-o-tolyl-phosphan und 2.8 g (12.5 mmol) Pd(OAc)₂ versetzt und 18 h bei 80°C gerührt. Nach beendeter Umsetzung wurde das Reaktionsgemisch i.vac. auf ca. 200 mL eingeengt, mit 400 mL EtOAc und 400 mL Wasser versetzt, der Niederschlag abgesaugt und die Phasen getrennt. Die organische Phase wurde über Na₂SO₄ getrocknet, mit Aktivkohle versetzt, filtriert und zur Trockene eingeengt. Der Rückstand wurde mit DIPE verrührt, abgesaugt und i.vac getrocknet.

| | |
|---|---|
| Ausbeute: | 19.5 g (32% der Theorie) |
| ESI-MS: | (M+H)⁺ = 356 |
| R_{f}= | 0.76 (Kieselgel, PE/EtOAc 1:1) |

### 3b) 3-(3,5-Bis-trifluormethyl-phenyl)-2-oxo-propionsäure

19.5 g (54.9 mmol) 2-Acetylamino-3-(3,5-bis-trifluormethyl-phenyl)-acrylsäuremethylester in 100 mL 1,4-Dioxan wurden auf 100°C Badtemperatur erwärmt, mit 100 mL 4 M HCl versetzt und 8 h bei 100°C Badtemperatur gerührt. Das Reaktionsgemisch wurde i.vac. eingeengt, die Kristalle abgesaugt, mit Wasser gewaschen und diese im Trockenschrank bei 50°C getrocknet.

| | |
|---|---|
| Ausbeute: | 16.1 g (98% der Theorie) |
| ESI-MS: | (M-H)⁻ = 299 |
| R_{f} = | 0.18 (Kieselgel, EtOAc) |

### 3c) (R)-3-(3,5-Bis-trifluormethyl-phenyl)-2-hydroxy-propionsäure

16.1 g (53.6 mmol) 3-(3,5-Bis-trifluormethyl-phenyl)-2-oxo-propionsäure in 9.5 (70.0 mmol) Triethylamin und 100 mL THF wurden bei -35°C mit einer Lösung aus 26.0 (81.1 mmol) (1*R*)-B-Chlordiisopinocampheylboran in 40 mL THF innerhalb von 30 min versetzt, 1 h bei dieser Temperatur und über Nacht bei RT nachgerührt. Nach beendeter Umsetzung wurde das Reaktionsgemisch bei 0°C mit 160 mL 1 M NaOH alkalisch gestellt, 15 min nachgerührt, mit 100 mL MTBE versetzt und die Phasen getrennt. Die organische Phase wurde mit 50 mL Wasser und 50 mL 1 M NaOH gewaschen. Die vereinigten wässrigen Phasen wurden mit 4 M HCl sauer gestellt, erschöpfend mit MTBE extrahiert, die vereinigten organischen Phasen über Na₂SO₄ getrocknet, über Aktivkohle abgesaugt und i.vac. eingeengt. Das Produkt wurde ohne Reinigung weiter umgesetzt.

| | |
|---|---|
| Ausbeute: | 12.5 g (77% der Theorie) |
| ESI-MS: | (M-H)⁻ = 301 |
| R_{f} = | 0.45 (Kieselgel, EtOAc) |

### 3d) (R)-3-(3,5-Bis-trifluormethyl-phenyl)-2-hydroxy-propionsäuremethylester

12.5 g (41.4 mmol) (R)-3-(3,5-Bis-trifluormethyl-phenyl)-2-hydroxy-propionsäure in 150 mL methanolischer HCI (1.25 M) wurden 4 h bei RT gerührt und anschließend i.vac. eingeengt. Der Rückstand wurde in EtOAc aufgenommen und mit gesättigter NaHCO₃-Lösung gewaschen, die organische Phase über Na₂SO₄ getrocknet, über Aktivkohle abgesaugt und i.vac. eingeengt. Der Rückstand wurde mit PE verrührt, abgesaugt und i.vac. eingeengt. Das Produkt wurde ohne Reinigung weiter umgesetzt.

| | |
|---|---|
| Ausbeute: | 11.4 g (87% der Theorie) |
| ESI-MS: | (M+H)⁺ = 316 |
| R_{f} = | 0.80 (Kieselgel, PE/EtOAc 1:1) |

### 3e) 4-(2-Oxo-1,2,4,5-tetrahydro-1,3-benzdiazepin-3-yl)-piperidin-1-carbonsäure-(R)-2-(3,5-bis-trifluormethyl-phenyl)-1-methoxycarbonyl-ethylester

Analog Beispiel 1e konnte aus 6.0 g (8.2 mmol) (R)-3-(3,5-Bis-trifluormethyl-phenyl)-2-hydroxy-propionsäuremethylester und 5.13 g (20.9 mmol) 3-Piperidin-4-yl-1,3,4,5-tetra-hydro-1,3-benzdiazepin-2-on das Produkt erhalten werden. Die Reinigung erfolgte chromatographisch (Kieselgel, Gradient PE/EtOAc 1:1 zu 1:9 ).

| | |
|---|---|
| Ausbeute: | 5.1 g (46% der Theorie) |
| ESI-MS: | (M+H)⁺ = 588 |
| R_{f} = | 0.63 (Kieselgel, DCM/MeOH/Cyc/NH₃ 70:15:15:2) |

### 3f) 4-(2-Oxo-1,2,4,5-tetrahydro-1,3-benzdiazepin-3-yl)-piperidin-1-carbonsäure-(R)-2-(3,5-bis-trifluormethyl-phenyl)-1-carboxy-ethylester

Zu einer Lösung von 5.0 g (8.5 mmol) 4-(2-Oxo-1,2,4,5-tetrahydro-1,3-benzdiazepin-3-yl)-piperidin-1-carbonsäure-(*R*)-2-(3,5-bis-trifluormethyl-phenyl)-1-methoxy-carbonyl-ethylester in 50 mL THF wurde eine Lösung von 307 mg (12.8 mmol) LiOH in 5 mL Wasser gegeben und das Reaktionsgemisch über Nacht bei RT gerührt. Man engte i.vac. ein, nahm den Rückstand in Wasser auf, säuerte mit 1 M HCl an, filtrierte den Niederschlag ab und trocknete diesen im Vakuumtrockenschrank bei 40°C.

| | |
|---|---|
| Ausbeute: | 4.5 g (92% der Theorie) |
| ESI-MS: | (M+H)⁺ = 574 |
| R_{f} = | 0.32 (Kieselgel,DCM/MeOH/Cyc/NH₃ 70:15:15:2) |

### 3g) 4-(2-Oxo-1,2,4,5-tetrahydro-1,3-benzdiazepin-3-yl)-piperidin-1-carbonsäure-(R)-1-(3,5-bis-trifluormethyl-benzyl)-2-(1'-ethoxycarbonylmethyl-4,4'-bipiperidinyl-1-yl)-2-oxo-ethylester

Zu einer Lösung von 100 mg (0.17 mmol) 4-(2-Oxo-1,2,4,5-tetrahydro-benzdiazepin-3-yl)-piperidin-1-carbonsäure-(*R*)-2-(3,5-bis-trifluormethyl-phenyl)-1-carboxy-ethylester, 61 mg (0.20 mmol) TBTU und 33 µL (0.16 mmol) Ethyldiisopropylamin in 1 mL DMF wurden bei RT 50 mg (0.20 mmol) [4,4']Bipiperidinyl-1-yl-essigsäureethylester gegeben und das Reaktionsgemisch über Nacht bei RT gerührt. Die Reaktionslösung wurde über einen Spritzenfilter filtriert und ohne weitere Aufarbeitung direkt via HPLC gereinigt. Die das Produkt enthaltenden Fraktionen wurden vereinigt und lyophilisiert.

| | |
|---|---|
| Ausbeute: | 55 mg (39% der Theorie) |
| ESI-MS: | (M+H)⁺ = 810 |
| Retentionszeit (HPLC-MS): | 7.3 min (Methode B) |

### Beispiel 3.1

### 4-(2-Oxo-1,2,4,5-tetrahydro-1,3-benzdiazepin-3-yl)-piperidin-1-carbonsäure-(R)-1-(3,5-bis-trifluormethyl-benzyl)-2-(1'-carboxymethyl-4,4'-bipiperidinyl-1-yl)-2-oxo-ethylester

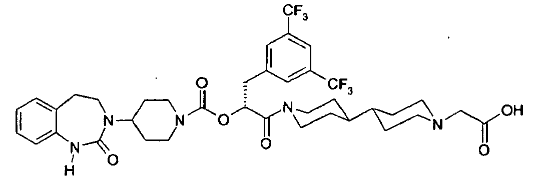

Zu einer Lösung von 35 mg (0.04 mmol) 4-(2-Oxo-1,2,4,5-tetrahydro-1,3-benzdiazepin-3-yl)-piperidin-1-carbonsäure-(*R*)-1-(3,5-bis-trifluormethyl-benzyl)-2-(1'-ethoxycarbonylmethyl-4,4'-bipiperidinyl-1-yl)-2-oxo-ethylester (Beispiel 3g) in 5 mL THF wurde eine Lösung von 1.5 mg (0.06 mmol) LiOH in 1 mL Wasser gegeben und die Reaktionslösung über Nacht bei RT gerührt. Man engte i.vac. ein, nahm den Rückstand in Wasser auf, säuerte mit 1 N HCl an, filtrierte den Niederschlag ab und trocknete diesen im Vakuumtrockenschrank.

| | |
|---|---|
| Ausbeute: | 15 mg (44% der Theorie) |
| ESI-MS: | (M+H)⁺ = 782 |
| R_{f}= | 0.41 (Kieselgel, DCM/MeOH/Cyc/NH₃70:15:15:2) |

### Beispiel 3.2

### 4-(2-Oxo-1,2,4,5-tetrahydro-1,3-benzdiazepin-3-yl)-piperidin-1-carbonsäure-(R)-2-(4-amino-4-methyl-1,4'-bipiperidinyl-1'-yl)-1-(3,5-bis-trifluormethyl-benzyl)-2-oxo-ethylester

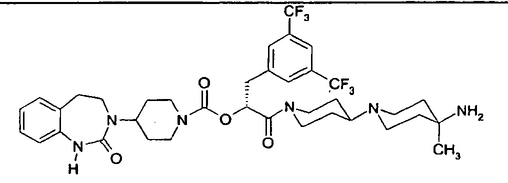

Zu einer Lösung von 80.0 mg (0.14 mmol) 4-(2-Oxo-1,2,4,5-tetrahydro-1,3-benzdiazepin-3-yl)-piperidin-1-carbonsäure-(*R*)-2-(3,5-bis-trifluormethyl-phenyl)-1-carboxy-ethylester, 58 mg (0.18 mmol) TBTU und 140 µL (1.00 mmol) Triethylamin in 1.8 mL DMF wurden bei RT 52 mg (0.14 mmol) (4-Methyl-[1,4']bipiperidinyl-4-yl)-carbaminsäure-*tert*-butylester (eingesetzt als Bis-Hydrochlorid-Salz) gegeben und die Reaktionslösung über Nacht bei RT geschüttelt. Diese wurde ohne weitere Aufarbeitung via HPLC gereinigt; die das Produkt enthaltenden Fraktionen wurden vereinigt und lyophilisiert. Das Kupplungsprodukt wurde in 4 mL DCM gelöst, die Lösung mit 0.5 mL TFA versetzt, 5 h bei RT gerührt und anschließend über Nacht bei RT belassen, wobei das Lösungsmittel verdunstete. Der Rückstand wurde in 2 mL 15% K₂CO₃-Lösung aufgenommen, zweimal mit je 2 mL DCM extrahiert und die vereinigten organischen Phasen über Nacht geschüttelt wobei das Lösungsmittel verdunstete. Der Rückstand wurde via HPLC gereinigt; die das Produkt enthaltenden Fraktionen wurden vereinigt und lyophilisiert, wobei dieses als Formiat-Salz anfiel.

| | |
|---|---|
| Ausbeute: | 47 mg (42% der Theorie) |
| ESI-MS: | (M+H)⁺ = 753 |
| Retentionszeit (HPLC): | 5.4 min (Methode B) |

### Beispiel 4

### 4-(2-Oxo-1,2,4,5-tetrahydro-1,3-benzdiazepin-3-yl)-piperidin-1-carbonsäure-(R)-1-(4-amino-3,5-dimethyl-benzyl)-2-(4-morpholin-4-yl-piperidin-1-yl)-2-oxo-ethylester

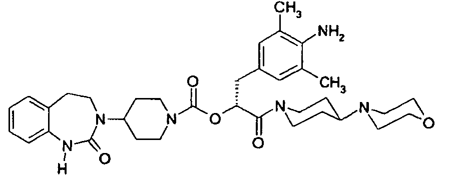

### 4a) (Z,E)-2-Acetylamino-3-(4-amino-3,5-dimethyl-phenyl)-acrylsäuremethylester

Unter einer Stickstoffatmosphäre wurde zu einer Mischung aus 7.2 g (32.1 mmol) Pd(OAc)₂ und 10.1 g (32.1 mmol) Tri-o-tolyl-phosphan in 1.2 L Triethylamin und 600 mL Acetonitril zuerst eine Lösung von 90.0 g (441 mmol) 4-Brom-2,6-dimethyl-phenylamin in 200 mL Acetonitril gegeben und anschließend eine Lösung von 65.0 g (445 mmol) 2-Acetylamino-acrylsäuremethylester in 200 mL Acetonitril zugetropft. Nach beendeter Zugabe wurde 18 h bei 80°C gerührt. Zur Vervollständigung der Reaktion wurde das Reaktionsgemisch erneut mit 4.0 g (17.8 mmol) Pd(OAc)₂ und 5.0 g (16.4 mmol) Tri-o-tolyl-phosphan versetzt und weitere 5 h bei 80°C gehalten. Man engte i.vac auf ca. 200 mL ein, versetzte den Rückstand mit 400 mL EtOAc, filtrierte den Rückstand (A) ab und trocknete die organische Phase über Na₂SO₄. Nach Entfernen des Trockenmittels durch Filtration über Aktivkohle wurde das Filtrat auf ca. 100 mL eingedampft, die ausgefallene Substanz abgesaugt, mit 30 mL EtOAc nach gewaschen und getrocknet.
Obiger Rückstand A wurde mit 1 L DCM, mit Na₂SO₄ und Aktivkohle versetzt und über Celite filtriert. Das Filtrat wurde eingeengt, der Rückstand mit 350 mL Diethylether versetzt, der entstandene Niederschlag abgesaugt, dieser mit 100 mL Diethylether gewaschen und getrocknet. Beide Produktfraktionen wurden vereinigt.

| | |
|---|---|
| Ausbeute: | 74.8 g (65% der Theorie) |
| ESI-MS: | (M+H)⁺ = 263 |
| R_{f} = | 0.51 (Kieselgel, EtOAc) |

### 4b) 3-(4-Amino-3,5-dimethyl-phenyl)-2-oxo-propionsäure

Eine Suspension von 74.0 g (282 mmol) (*Z*,*E*)-2-Acetylamino-3-(4-amino-3,5-dimethylphenyl)-acrylsäuremethylester in 500 mL 1,4-Dioxan wurde auf 100°C erhitzt und mit 460 mL 4 M HCl versetzt, wobei sich eine Lösung bildete. Man erhitzte weitere 8 h bei 100°C und engte die abgekühlte Lösung i.vac. auf ca. 200 mL ein, wobei das Produkt kristallisierte. Man filtrierte, wusch den Rückstand mit 50 mL Wasser und trocknete das Produkt bei 50°C.

| | |
|---|---|
| Ausbeute: | 43.6 g (63% der Theorie) |
| ESI-MS: | (M+H)⁺ = 208 |
| R_{f} = | 0.68 (Kieselgel, PE/EtOAc 1:1) |

### 4c) (R)-3-(4-Amino-3,5-dimethyl-phenyl)-2-hydroxy-propionsäuremethylester

Unter Stickstoffatmosphäre wurde eine Mischung aus 20.0 g (82.1 mmol) 3-(4-Amino-3,5-dimethyl-phenyl)-2-oxo-propionsäure und 25.7 mL (189 mmol) Triethylamin in 400 mL THF auf -35°C gekühlt. Dann wurde eine Lösung von 40.0 g (125 mmol) (1*R*)-B-Chlor-diisopinocampheylboran in 100 mL THF so zugetropft, dass die Reaktionstemperatur zwischen -35°C und -25°C blieb. Man hielt das Reaktionsgemisch 1 h bei dieser Temperatur, entfernte das Kältebad und ließ das Reaktionsgemisch über Nacht bei RT rühren. THF wurde i.vac. eingeengt, der Rückstand mit methanolischer HCl (1.25 M) versetzt und 2 h bei RT gerührt. Man engte i.vac. ein, nahm den Rückstand in 2 M HCl auf und extrahierte erschöpfend mit EtOAc. Die wässrige Phase wurde mit halbkonzentrierter NaOH alkalisch gestellt und erschöpfend mit EtOAc extrahiert. Die vereinigten organischen Phasen wurden über Na₂SO₄ getrocknet, über Aktivkohle abgesaugt und eingeengt. Das Produkt konnte als braunes Öl erhalten werden.

| | |
|---|---|
| Ausbeute: | 8.3 g (45% der Theorie) |
| ESI-MS: | (M+H)⁺ = 224 |
| R_{f} = | 0.46 (Kieselgel, PE/EtOAc 1:1) |

### 4d) 4-(2-Oxo-1,2,4,5-tetrahydro-1,3-benzdiazepin-3-yl)-piperidin-1-carbonsäure-(R)-2-(4-amino-3,5-dimethyl-phenyl)-1-methoxycarbonyl-ethylester

Analog Beispiel 1e konnte aus 4.0 g (17.9 mmol) (*R*)-3-(4-Amino-3,5-dimethyl-phenyl)-2-hydroxy-propionsäuremethylester und 4.8 g (19.6 mmol) 3-Piperidin-4-yl-1,3,4,5-tetra-hydro-1,3-benzdiazepin-2-on das gewünschte Produkt erhalten werden.

| | |
|---|---|
| Ausbeute: | 3.2 g (36% der Theorie) |
| ESI-MS: | (M+H)⁺ = 495 |
| R_{f} = | 0.35 (Kieselgel, DCM/MeOH/NH₃ 90:10:1) |

### 4e) 4-(2-Oxo-1,2,4,5-tetrahydro-1,3-benzdiazepin-3-yl)-piperidin-1-carbonsäure-(R)-2-(4-amino-3,5-dimethyl-phenyl)-1-carboxy-ethylester

Zu einer Lösung von 6.7 g (13.6 mmol) 4-(2-Oxo-1,2,4,5-tetrahydro-1,3-benzdiazepin-3-yl)-piperidin-1-carbonsäure-(*R*)-2-(4-amino-3,5-dimethyl-phenyl)-1-methoxycarbonyl-ethylester in 50 mL THF wurde eine Lösung von 500 mg (20.9 mmol) LiOH in 10 mL Wasser gegeben und das Reaktionsgemisch über Nacht bei RT gerührt. Zur Vervollständigung der Reaktion wurden weitere 300 mg (12.5 mmol) LiOH zugegeben und die Reaktionslösung 3 h bei 40°C gerührt. Man engte i.vac. ein, nahm den Rückstand in 15% K₂CO₃-Lösung auf und extrahierte erschöpfend mit DCM. Die wässrige Phase wurde mit 4 M HCI sauer gestellt, erschöpfend mit DCM extrahiert und die vereinigten organischen Phasen über Na₂SO₄getrocknet. Nach Entfernen des Trocken- und Lösungsmittels wurde der Rückstand ohne Reinigung weiter umgesetzt.

| | |
|---|---|
| Ausbeute: | 4.2 g (65% der Theorie) |
| ESI-MS: | (M+H)⁺ = 481 |
| R_{f} = | 0.21 (Kieselgel, DCM/MeOH/Cyc/NH₃ 70:15:15:2) |

### 4f) 4-(2-Oxo-1,2,4,5-tetrahydro-1,3-benzdiazepin-3-yl)-Piperidin-1-carbonsäure-(R)-1-(4-amino-3,5-dimethyl-benzyl)-2-(4-morpholin-4-yl-piperidin-1-yl)-2-oxo-ethylester

Analog Beispiel 3g wurde aus 80 mg (0.17 mmol) 4-(2-Oxo-1,2,4,5-tetrahydro-1,3-benz-diazepin-3-yl)-piperidin-1-carbonsäure-(R)-2-(4-amino-3,5-dimethyl-phenyl)-1-carboxy-ethylester und 32 mg (0.19 mmol) 4-Piperidin-4-yl-morpholin das Produkt erhalten.

| | |
|---|---|
| Ausbeute: | 52 mg (49% der Theorie) |
| ESI-MS: | (M+H)⁺ = 633 |
| Retentionszeit (HPLC): | 4.9 min (Methode B) |

### Beispiel 4.1

### 4-(2-Oxo-1,2,4,5-tetrahydro-1,3-benzdiazepin-3-yl)-piperidin-1-carbonsäure-(R)-1-(4-amino-3,5-dimethyl-benzyl)-2-(1'-ethoxycarbonylmethyl-4,4'-bipiperidinyl-1-yl)-2-oxo-ethylester

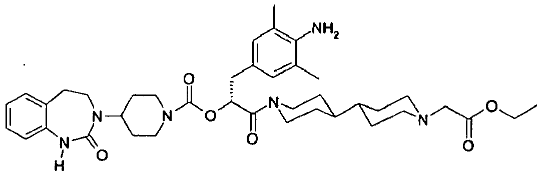

Analog Beispiel 3g wurde aus 100 mg (0.21 mmol) 4-(2-Oxo-1,2,4,5-tetrahydro-1,3-benz-diazepin-3-yl)-piperidin-1-carbonsäure-(*R*)-2-(4-amino-3,5-dimethyl-phenyl)-1-carboxy-ethylester und 59 mg (0.23 mmol) [4,4']Bipiperidinyl-1-yl-essigsäure-ethylester das Produkt erhalten.

| | |
|---|---|
| Ausbeute: | 58 mg (39% der Theorie) |
| ESI-MS: | (M+H)⁺ = 717 |
| Retentionszeit (HPLC): | 4.7 min (Methode B) |

### Beispiel 4.2

### 4-(2-Oxo-1,2,4,5-tetrahydro-1,3-benzdiazepin-3-yl)-piperidin-1-carbonsäure-(R)-1-(4-amino-3,5-dimethyl-benzyl)-2-(1'-carboxymethyl-4,4'-bipiperidinyl-1-yl)-2-oxo-ethylester

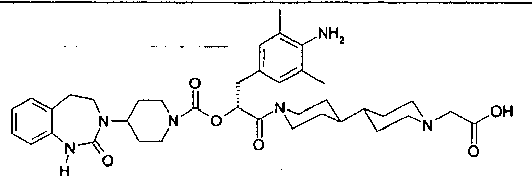

Zu einer Lösung von 55 mg (0.08 mmol) 4-(2-Oxo-1,2,4,5-tetrahydro-1,3-benzdiazepin-3-yl)-piperidin-1-carbonsäure-(*R*)-1-(4-amino-3,5-dimethyl-benzyl)-2-(1'-ethoxycarbonylmethyl-4,4'-bipiperidinyl-1-yl)-2-oxo-ethylester (Beispiel 4.1) in 5 mL THF wurde eine Lösung von 3.1 mg (0.13 mmol) LiOH in 1 mL Wasser gegeben und das Reaktionsgemisch über Nacht bei RT gerührt. Man engte i.vac. ein, nahm den Rückstand in 1 mL DMF auf und reinigte das Rohprodukt via HPLC. Die das Produkt enthaltenden Fraktionen wurden vereinigt und lyophilisiert.

| | |
|---|---|
| Ausbeute: | 22 mg (42% der Theorie) |
| ESI-MS: | (M+H)⁺ = 689 |
| Retentionszeit (HPLC): | 4.8 min (Methode B) |

### Beispiel 5

### 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-(4-morpholin-4-yl-piperidin-1-yl)-2-oxo-ethylester

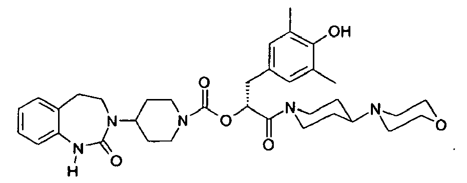

### 5a) 2-Benzyloxy-5-brom-1,3-dimethylbenzol

Zu einer Lösung von 50.0 g (249 mmol) 2,6-Dimethyl-4-bromphenol in 500 mL DMF wurden 39.9 g (286 mmol) K₂CO₃ gegeben und 20 min nachgerührt. Dann wurden 34.0 mL (286 mmol) Benzylchlorid langsam zugetropft und das Reaktionsgemisch 3 h bei 100°C Badtemperatur gerührt. Nach beendeter Umsetzung wurde auf 500 mL Wasser gegossen und mit EtOAc erschöpfend extrahiert. Die organischen Phasen wurden vereint, über Na₂SO₄ getrocknet und i. vac. eingeengt.

| | |
|---|---|
| Ausbeute: | quantitativ |
| GC-MS: | (M⁺) = 290/292 (Br) |
| R_{f} = | 0.87 (Kieselgel, Cyc/EtOAc 3:1) |

### 5b) 2-Acetylamino-3-(4-benzyloxy-3,5-dimethyl-phenyl)-acrylsäuremethylester

Unter Stickstoffatmosphäre wurde eine Mischung aus 40.0 g (137 mmol) 2-Benzyloxy-5-brom-1,3-dimethylbenzol und 24.1 g (165 mmol) 2-Acetylamino-acrylsäuremethylester in 420 mL Triethylamin und 200 mL Acetonitril mit 3.5 g (11.2 mmol) Tri-o-tolyl-phosphan und 2.5 g (11.1 mmol) Pd(OAc)₂ versetzt und 18 h bei 80°C gerührt. Der Niederschlag wurde abgesaugt, das Filtrat i.vac. eingeengt und mit 800 mL DCM und 800 mL Wasser versetzt. Die organische Phase wurde abgetrennt, über Na₂SO₄ abgesaugt, das Lösungsmittel i.vac. entfernt, der Rückstand mit EtOAc verrührt, abgesaugt und i.vac getrocknet.

| | |
|---|---|
| Ausbeute: | 31.1 g (64% der Theorie) |
| ESI-MS: | (M+H)⁺ = 354 |
| Retentionszeit (HPLC-MS): | 8.6 min (Methode B) |

### 5c) 3-(4-Benzyloxy-3,5-dimethyl-phenyl)-2-oxo-propionsäure

31.1 g (88.1 mmol) 2-Acetylamino-3-(4-benzyloxy-3,5-dimethyl-phenyl)-acrylsäuremethylester in 150 mL 1,4-Dioxan wurden mit 125 mL 4 M HCl versetzt, 7 h unter Rückfluss und über Nacht bei RT gerührt. Der Niederschlag wurde abgesaugt, mit Wasser gewaschen und bei 45°C im Vakuumtrockenschrank getrocknet.

| | |
|---|---|
| Ausbeute: | 14.3 g (54% der Theorie) |
| EI-MS: | (M)⁺=298 |
| Retentionszeit (HPLC-MS): | 9.0 min (Methode B) |

### 5d) (R)-3-(4-Benzoyl-3,5-dimethyl-phenyl)-2-hydroxy-propionsäure

Unter einer Stickstoffatmosphäre wurde eine Lösung von 14.3 g (47.8 mmol) 3-(4-Benzyloxy-3,5-dimethyl-phenyl)-2-oxo-propionsäure und 8.3 mL (59.8 mmol) Triethylamin in 170 mL THF bei -35°C mit einer Lösung von 22.1 (69.0 mmol) (1*R*)-*B*-Chlordiisopino-campheylboran in 70 mL THF innerhalb von 30 min versetzt. Nach beendeter Zugabe wurde das Kältebad entfernt und die Reaktionslösung über Nacht bei RT gerührt. Das Reaktionsgemisch wurde bei 0°C mit 70 mL 1 M NaOH alkalisch gestellt, mit 100 mL MTBE versetzt, 15 min nachgerührt und die Phasen getrennt. Die organische Phase wurde mit 50 mL Wasser und dreimal mit je 50 mL 1 M NaOH gewaschen. Die vereinigten wässrigen Phasen wurden mit halbkonz. HCl sauer gestellt, mit EtOAc erschöpfend extrahiert und die vereinigten organischen Phasen über Na₂SO₄ getrocknet. Nach Entfernen des Trocken- und Lösungsmittels wurde der Rückstand ohne Reinigung weiter umgesetzt.

| | |
|---|---|
| Ausbeute: | 14.0 g (98% der Theorie) |
| ESI-MS: | (M-H)⁻ = 299 |
| Retentionszeit (HPLC-MS): | 7.9 min (Methode B) |

### 5e) (R)-3-(4-Benzyloxy-3,5-dimethyl-phenyl)-2-hydroxy-propionsäuremethylester

Zu einer auf 0°C gekühlten Lösung von 14.0 g (23.3 mmol) (R)-3-(4-Benzoyl-3,5-dimethylphenyl)-2-hydroxy-propionsäure in 150 mL MeOH wurden tropfenweise 2.0 mL (27.4 mmol) SOCl₂ zugegeben und das Reaktionsgemisch 1 h bei RT nachgerührt. Die Reaktionslösung wurde i.vac. eingeengt und der Rückstand chromatographisch (Kieselgel, Cyc/EtOAc 3:1) gereinigt.

| | |
|---|---|
| Ausbeute: | 5.7 g (78% der Theorie) |
| ESI-MS: | (M+NH4)⁺ = 332 |
| Retentionszeit (HPLC-MS): | 9.1 min (Methode B) |

### 5f) 4-(2-Oxo-1,2,4,5-tetrahydro-1,3-benzdiazepin-3-yl)-piperidin-1-carbonsäure-(R)-2-(4-benzyloxy-3,5-dimethyl-phenyl)-1-methoxycarbonyl-ethylester

Unter Stickstoffatmosphäre wurde zu einer Lösung von 1.17 g (9.58 mmol) 4-Dimethylaminopyridin in 50 mL Pyridin 1.93 g (9.58 mmol) Chlorameisensäure-4-nitrophenylester gegeben, 1.5 h bei RT gerührt, mit 3.0 g (9.58 mmol) (R)-3-(4-Benzyloxy-3,5-dimethylphenyl)-2-hydroxy-propionsäuremethylester versetzt und 20 min bei RT gerührt. Anschließend wurden 2.35 g (9.58 mmol) 3-Piperidin-4-yl-1,3,4,5-tetrahydro-1,3-benzdiazepin-2-on zugegeben und 20 h bei RT gerührt. Das Reaktionsgemisch wurde i. vac. eingeengt, der Rückstand in EtOAc aufgenommen, die organische Phase mit 10% KHSO₄ und gesättigter NaHCO₃-Lösung gewaschen und über Na₂SO₄ getrocknet. Nach Entfernen des Trocken- und Lösungsmittels wurde der Rückstand chromatographisch (Kieselgel, Gradient Cyc/EtOAc 1:1 zu 1:2) gereinigt.

| | |
|---|---|
| Ausbeute: | 3.21 g (57% der Theorie) |
| ESI-MS: | (M+H)⁺ = 586 |
| Retentionszeit (HPLC-MS): | 10.4 min (Methode B) |

### 5g) 4-(2-Oxo-1,2,4,5-tetrahydro-1,3-benzdiazepin-3-yl)-piperidin-1-carbonsäure-(R)-2-(4-benzyloxy-3,5-dimethyl-phenyl)-1-carboxy-ethylester

Eine Lösung von 3.21 g (5.48 mmol) 4-(2-Oxo-1,2,4,5-tetrahydro-1,3-benzdiazepin-3-yl)-piperidin-1-carbonsäure-(*R*)-2-(4-benzyloxy-3,5-dimethyl-phenyl)-1-methoxy-carbonyl-ethylester in 80 mL THF wurde mit einer Lösung von 200 mg (8.35 mmol) LiOH in 40 mL Wasser versetzt und 1 h bei RT gerührt. Das Reaktionsgemisch wurde i.vac. eingeengt, der Rückstand in 100 mL Wasser aufgenommen, mit 2 M HCl sauer gestellt, der Niederschlag abgesaugt und im Vakuumtrockenschrank bei 40°C getrocknet.

| | |
|---|---|
| Ausbeute: | quantitativ |
| ESI-MS: | (M+H)⁺ = 572 |
| Retentionszeit (HPLC-MS): | 9.2 min (Methode B) |

### 5h) 4-(2-Oxo-1,2,4,5-tetrahydro-1,3-benzdiazepin-3-yl)-piperidin-1-carbonsäure-(R)-2-(4-hydroxy-3,5-dimethyl-phenyl)-1-carboxy-ethylester

3.72 g (6.51 mmol) 4-(2-Oxo-1,2,4,5-tetrahydro-1,3-benzdiazepin-3-yl)-piperidin-1-carbonsäure-(R)-2-(4-benzyloxy-3,5-dimethyl-phenyl)-1-carboxy-ethylester in 50 mL DCM wurden mit 300 mg 10% Pd/C versetzt und bei RT und 3000 hPa Wasserstoffdruck bis zum Reaktionsstillstand geschüttelt. Der Katalysator wurde abgesaugt und das Lösungsmittel i. vac. eingeengt. Der Rückstand wurde mit DIPE verrieben und abgesaugt.

| | |
|---|---|
| Ausbeute: | 2.41 g (77% der Theorie) |
| ESI-MS: | (M+H)⁺ = 482 |
| Retentionszeit (HPLC-MS): | 7.0 min (Methode B) |

### 5i) 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-(4-morpholin-4-yl-piperidin-1-yl)-2-oxo-ethylester

Eine Lösung von 70 mg (0.15 mmol) 4-(2-Oxo-1,2,4,5-tetrahydro-1,3-benzdiazepin-3-yl)-piperidin-1-carbonsäure-(*R*)-2-(4-hydroxy-3,5-dimethyl-phenyl)-1-carboxy-ethylester, 51 mg (0.16 mmol) TBTU und 25 µL (0.18 mmol) Triethylamin in 1 mL DMF wurde bei RT 1 h gerührt. Dann erfolgte die Zugabe von 25 mg (0.15 mmol) 4-Piperidin-4-yl-morpholin zur Reaktionslösung, welche anschließend 16 h bei RT gerührt wurde. Die Reaktionslösung wurde ohne weitere Aufarbeitung via HPLC gereinigt; die das Produkt enthaltenden Fraktionen wurden vereinigt und lyophilisiert.

| | |
|---|---|
| Ausbeute: | 35 mg (38% der Theorie) |
| ESI-MS: | (M+H)⁺ = 634 |
| Retentionszeit (HPLC-MS): | 5.6 min (Methode B) |

### Beispiel 5.1

### 4-(2-Oxo-1,2,4,5-tetrahydro-1,3-benzdiazepin-3-yl)-piperidin-1-carbonsäure-(R)-2-(4-hydroxy-1,4'-bipiperidinyl-1'-yl)-1-(4-hydroxy-3,5-dimethyl-benzyl)-2-oxo-ethylester

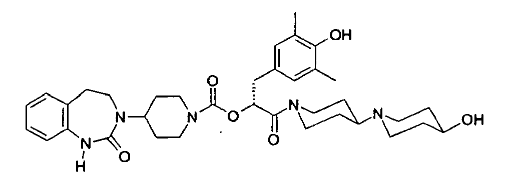

Analog Beispiel 5i wurde aus 70 mg (0.15 mmol) 4-(2-Oxo-1,2,4,5-tetrahydro-1,3-benz-diazepin-3-yl)-piperidin-1-carbonsäure-(*R*)-2-(4-hydroxy-3,5-dimethyl-phenyl)-1-carboxy-ethylester und 27 mg (0.15 mmol) [1,4']Bipiperidinyl-4-ol das Produkt erhalten.

| | |
|---|---|
| Ausbeute: | 35 mg (37% der Theorie) |
| ESI-MS: | (M+H)⁺ = 648 |
| Retentionszeit (HPLC): | 5.5 min (Methode B) |

### Beispiel 5.2

### 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-(4-hydroxy-4-methyl-[1,4']bipiperidinyl-1'-yl)-2-oxo-ethylester

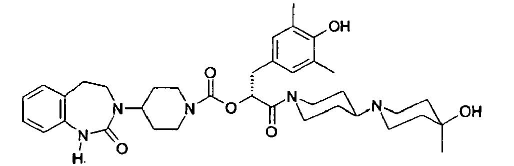

### 5.2a) 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-oxo-2-(4-oxo-piperidin-1-yl)-ethylester

Zu einer Lösung von 400 mg (0.83 mmol) 4-(2-Oxo-1,2,4,5-tetrahydro-1,3-benzdiazepin-3-yl)-piperidin-1-carbonsäure-(*R*)-2-(4-hydroxy-3,5-dimethyl-phenyl)-1-carboxy-ethylester, 320 mg (1.00 mmol) TBTU und 260 µL (1.87 mmol) Triethylamin in 10 mL DMF wurden bei RT 130 mg (0.83 mmol) Piperidin-4-on (eingesetzt als Hydrat des Hydrochloridsalzes) gegeben und die Reaktionsmischung 16 h bei RT gerührt. Man engte i.vac. ein, nahm den Rückstand in EtOAc auf, wusch die organische Phase mit halbgesättigter NaHCO₃-Lösung und trocknete über Na₂SO₄. Nach Entfernen des Trocken- und Lösungsmittels wurde der Rückstand chromatographisch (Kieselgel, EtOAc) gereinigt. Die das Produkt enthaltenden Fraktionen wurden eingeengt, der Rückstand mit DIPE verrührt, abgesaugt und getrocknet.

| | |
|---|---|
| Ausbeute: | 333 mg (71% der Theorie) |
| ESI-MS: | (M+H)⁺ = 563 |
| Retentionszeit (HPLC-MS): | 6.9 min (Methode B) |

### 5.2b) 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-(4-hydroxy-4-methyl-[1,4']bipiperi-dinyl-1'-yl)-2-oxo-ethylester

Eine Lösung von 50 mg (0.09 mmol) 4-(2-Oxo-1,2,4,5-tetrahydro-1,3-benzdiazepin-3-yl)-piperidin-1-carbonsäure-(*R*)-1-(4-hydroxy-3,5-dimethyl-benzyl)-2-oxo-2-(4-oxo-piperidin-1-yl)-ethylester in 1.5 mL DCM wurde mit 21 mg (0.18 mmol) 4-Methyl-piperidin-4-ol und 10.3 µL (0.19 mmol) AcOH versetzt, auf 0°C abgekühlt und 2 h gerührt. Dann wurden 28 mg (0.19 mmol) Natrium-triacetoxyborhydrid zugegeben und über Nacht bei 0°C gerührt. Nach Entfernen des Lösungsmittels wurde der Rückstand mit 2 mL DMF versetzt und via HPLC gereinigt. Die das Produkt enthaltenden Fraktionen wurden vereinigt und lyophilisiert.

| | |
|---|---|
| Ausbeute: | 25 mg (42% der Theorie) |
| ESI-MS: | (M+H)⁺ = 662 |
| Retentionszeit (HPLC-MS): | 2.90 min (Methode A) |

### Beispiel 5.3

### 4-(2-Oxo-1,2,4,5-tetrahydro-1,3-benzdiazepin-3-yl)-piperidin-1-carbonsäure-(R)-2-(4,4-dimethyl-[1,4']bipiperidinyl-1'-yl)-1-(4-hydroxy-3,5-dimethyl-benzyl)-2-oxo-ethylester

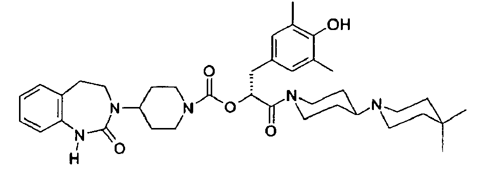

Analog Beispiel 5.2b konnte aus 50.0 mg (0.09 mmol) 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-oxo-2-(4-oxo-piperidin-1-yl)-ethylester (Beispiel 5.2a) und 31.0 mg (0.18 mmol) 4,4-Dimethylpiperidin das Produkt erhalten werden.

| | |
|---|---|
| Ausbeute: | 18.3 mg (31 % der Theorie) |
| ESI-MS: | (M+H)⁺ = 660 |
| Retentionszeit (HPLC-MS): | 3.2 min (Methode A) |

### Beispiel 5.4

### 4-(2-Oxo-1,2,4,5-tetrahydro-1,3-benzdiazepin-3-yl)-piperidin-1-carbonsäure-(R)-2-(4-amino-4-methyl-[1,4']bipiperidinyl-1'-yl)-1-(4-hydroxy-3,5-dimethyl-benzyl)-2-oxo-ethylester

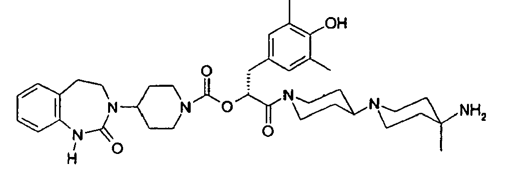

Eine Lösung von 150 mg (0.27 mmol) 4-(2-Oxo-1,2,4,5-tetrahydro-1,3-benzdiazepin-3-yl)-piperidin-1-carbonsäure-(*R*)-1-(4-hydroxy-3,5-dimethyl-benzyl)-2-oxo-2-(4-oxo-piperidin-1-yl)-ethylester (Beispiel 5.2a) in 4 mL DCM wurde mit 120 mg (0.53 mmol) (4-Methylpiperidin-4-yl)-carbaminsäure-*tert*-butylester und 31 µL (0.56 mmol) AcOH versetzt, auf 0°C abgekühlt und 2 h gerührt. Dann wurden 85 mg (0.56 mmol) Natriumtriacetoxyborhydrid zugegeben und über Nacht bei 0°C gerührt. Dann wurde die Reaktionslösung mit 0.5 mL TFA versetzt und erneut über Nacht bei RT gerührt. Nach Entfernen der Lösemittel wurde der Rückstand in 2 mL DMF gelöst und via HPLC gereinigt. Die das Produkt enthaltenden Fraktionen wurden vereinigt und lyophilisiert, wobei das Produkt als TFA-Salz anfiel.

| | |
|---|---|
| Ausbeute: | 94 mg (46% der Theorie) |
| ESI-MS: | (M+H)⁺ = 661 |
| Retentionszeit (HPLC-MS): | 2.5 min (Methode A) |

### Beispiel 5.5

### 4-(2-Oxo-1,2,4,5-tetrahydro-1,3-benzdiazepin-3-yl)-piperidin-1-carbonsäure-(R)-2-(1'-ethoxycarbonylmethyl-4,4'-bipiperidinyl-1-yl)-1-(4-hydroxy-3,5-dimethyl-benzyl)-2-oxo-ethylester

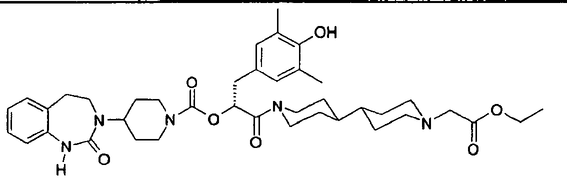

Zu einer Lösung von 200 mg (0.42 mmol) 4-(2-Oxo-1,2,4,5-tetrahydro-1,3-benzdiazepin-3-yl)-piperidin-1-carbonsäure-(*R*)-2-(4-hydroxy-3,5-dimethyl-phenyl)-1-carboxy-ethylester (Beispiel 5h), 148 mg (0.46 mmol) TBTU und 64 µL (0.46 mmol) Triethylamin in 10 mL THF und 1 mL DMF wurden bei RT 117 mg (0.46 mmol) [4,4']Bipiperidinyl-1-yl-essigsäureethylester gegeben und das Reaktionsgemisch über Nacht bei RT geschüttelt. Die Reaktionslösung wurde i.vac. eingeengt, der Rückstand im MeOH aufgenommen und via HPLC gereinigt. Die das Produkt enthaltenden Fraktionen wurden vereinigt, i.vac. eingeengt, der Rückstand mit DIPE verrieben, abgesaugt und getrocknet.

| | |
|---|---|
| Ausbeute: | 222 mg (74% der Theorie) |
| ESI-MS: | (M+H)⁺ = 718 |
| Retentionszeit (HPLC): | 3.1 min (Methode A) |

### Beispiel 5.6

### 4-(2-Oxo-1,2,4,5-tetrahydro-1,3-benzdiazepin-3-yl)-piperidin-1-carbonsäure-(R)-2-(1'-carboxymethyl-4,4'-bipiperidinyl-1-yl)-1-(4-hydroxy-3,5-dimethyl-benzyl)-2-oxo-ethylester

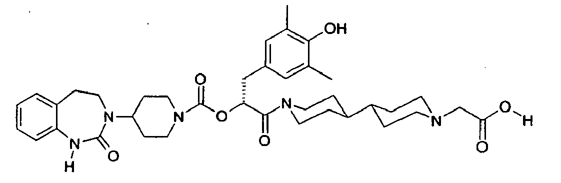

Zu einer Lösung von 100 mg (0.14 mmol) 4-(2-Oxo-1,2,4,5-tetrahydro-1,3-benzdiazepin-3-yl)-piperidin-1-carbonsäure-(*R*)-2-(1'-ethoxycarbonylmethyl-4,4'-bipiperidinyl-1-yl)-1-(4-hydroxy-3,5-dimethyl-benzyl)-2-oxo-ethylester in 3 mL THF wurde eine Lösung von 3.8 mg (0.16 mmol) LiOH in 1 mL Wasser gegeben und die Reaktionslösung über Nacht bei RT gerührt. Das organische Lösungsmittel wurde im Stickstoffstrom entfernt, der Rückstand mit je 1 mL Wasser und Acetonitril versetzt und mit Ameisensäure sauer gestellt. Die Reinigung des Produktes erfolgte via HPLC; die das Produkt enthaltenden Fraktionen wurden vereinigt und lyophilisiert.

| | |
|---|---|
| Ausbeute: | 56 mg (58% der Theorie) |
| ESI-MS: | (M-H)⁻ = 688 |
| Retentionszeit (HPLC): | 3.0 min (Methode A) |

### Beispiel 5.7

### 4-(2-Oxo-1,2,4,5-tetrahydro-1,3-benzdiazepin-3-yl)-piperidin-1-carbonsäure-(R)-2-(4-cyclohexyl-piperazin-1-yl)-1-(4-hydroxy-3,5-dimethyl-benzyl)-2-oxo-ethylester

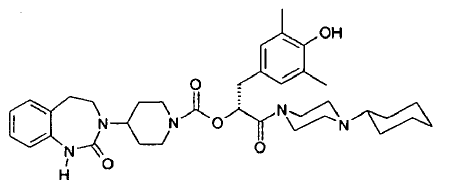

Analog Beispiel 3g konnte aus 69 mg (0.14 mmol) 4-(2-Oxo-1,2,4,5-tetrahydro-1,3-benz-diazepin-3-yl)-piperidin-1-carbonsäure-(*R*)-2-(4-hydroxy-3,5-dimethyl-phenyl)-1-carboxy-ethylester (Beispiel 5h) und 24 mg (0.14 mmol) 1-Cyclohexyl-piperazin das Produkt erhalten werden.

| | |
|---|---|
| Ausbeute: | 51 mg (91 % der Theorie) |
| ESI-MS: | (M+H)⁺ = 632 |
| Retentionszeit (HPLC): | 3.1 min (Methode A) |

### Beispiel 5.8

### 4-(2-Oxo-1,2,4,5-tetrahydro-1,3-benzdiazepin-3-yl)-piperidin-1-carbonsäure-(R)-2-[4-(4-ethoxycarbonylmethyl-piperazin-1-yl)-piperidin-1-yl]-1-(4-hydroxy-3,5-dimethyl-benzyl)-2-oxo-ethylester

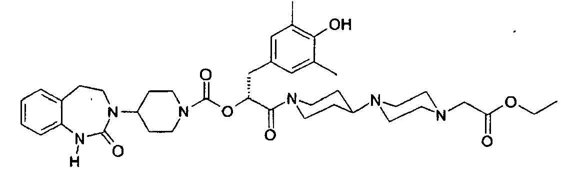

Analog Beispiel 5i konnte aus 150 mg (0.31 mmol) 4-(2-Oxo-1,2,4,5-tetrahydro-1,3-benzdiazepin-3-yl)-piperidin-1-carbonsäure-(*R*)-2-(4-hydroxy-3,5-dimethyl-phenyl)-1-carboxy-ethylester (Beispiel 5h) und 87 mg (0.34 mmol) (4-Piperidin-4-yl-piperazin-1-yl)-essigsäureethylester das Produkt erhalten werden.

| | |
|---|---|
| Ausbeute: | 64 mg (28% der Theorie) |
| ESI-MS: | (M+H)⁺ = 719 |
| Retentionszeit (HPLC): | 3.6 min (Methode A) |

### Beispiel 5.9

### 4-(2-Oxo-1,2,4,5-tetrahydro-1,3-benzdiazepin-3-yl)-piperidin-1-carbonsäure-(R)-2-[4-(4-carboxymethyl-piperazin-1-yl)-piperidin-1-yl]-1-(4-hydroxy-3,5-dimethyl-benzyl)-2-oxo-ethylester

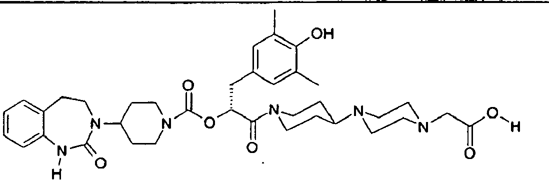

Zu einer Lösung von 40.0 mg (0.06 mmol) 4-(2-Oxo-1,2,4,5-tetrahydro-1,3-benzdiazepin-3-yl)-piperidin-1-carbonsäure-(*R*)-2-[4-(4-ethoxycarbonylmethyl-piperazin-1-yl)-piperidin-1-yl]-1-(4-hydroxy-3,5-dimethyl-benzyl)-2-oxo-ethylester in 5 mL THF wurde eine Lösung von 2.3 mg (0.09 mmol) LiOH in 5 mL Wasser gegeben und das Reaktionsgemisch 2 h bei RT gerührt. Man engte i.vac. ein, nahm den Rückstand in 1 mL DMF auf und reinigte diesen via HPLC; die das Produkt enthaltenden Fraktionen wurden vereinigt und lyophilisiert.

| | |
|---|---|
| Ausbeute: | 20 mg (38% der Theorie) |
| ESI-MS: | (M+H)⁺ = 691 |
| Retentionszeit (HPLC): | 2.6 min (Methode A) |

### Beispiel 5.10

### 4-(2-Oxo-1,2,4,5-tetrahydro-1,3-benzdiazepin-3-yl)-piperidin-1-carbonsäure-(R)-2-[4-(1-ethoxycarbonylmethyl-piperidin-4-yl)-piperazin-1-yl]-1-(4-hydroxy-3,5-dimethyl-benzyl)-2-oxo-ethylester

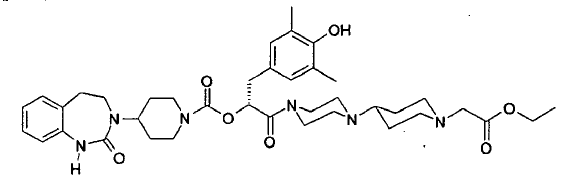

### 5.10a) 4-(2-Oxo-1,2,4,5-tetrahydro-1,3-benzdiazepin-3-yl)-piperidin-1-carbonsäure-(R)-1-(4-benzyloxy-3,5-dimethyl-benzyl)-2-[4-(1-ethoxycarbonylmethyl-piperidin-4-yl)-piperazin-1-yl]-2-oxo-ethylester

Eine Lösung von 8.00 g (14.0 mmol) 4-(2-Oxo-1,2,4,5-tetrahydro-1,3-benzodiazepin-3-yl)-piperidin-1-carbonsäure-(*R*)-2-(4-benzyloxy-3,5-dimethyl-phenyl)-1-carboxy-ethylester (Beispiel 5g), 5.17 g (16.1 mmol) TBTU und 8.84 mL (63.0 mmol) Triethylamin in 100 mL DMF wurde bei RT 1 h gerührt. Dann erfolgte die Zugabe von 5.28 g (16.1 mmol) (4-Piperazin-1-yl-piperidin-1-yl)-essigsäureethylester (eingesetzt als Bis-Hydrochlorid-Salz) zum Reaktionsgemisch, welches anschließend 1 h bei RT gerührt wurde. Man versetzte mit 150 mL 15% K₂CO₃-Lösung, extrahierte mit 200 mL EtOAc, trennte die organische Phase ab und extrahierte diese mit 150 mL 10% Zitronensäure-Lösung. Die wässrige Phase wurde mit K₂CO₃ alkalisch gestellt, mit 200 mL EtOAc extrahiert und die organische Phase über Na₂SO₄ getrocknet. Nach Entfernen des Trocken- und Lösungsmittels wurde der Rückstand chromatographisch (Kieselgel, EtOH) gereinigt. Die das Produkt enthaltenden Fraktionen wurden vereinigt, i.vac. eingeengt, der Rückstand mit DIPE verrührt, abgesaugt und getrocknet.

| | |
|---|---|
| Ausbeute: | 8.58 g (76% der Theorie) |
| ESI-MS: | (M+H)⁺ = 809 |
| Retentionszeit (HPLC): | 3.7 min (Methode A) |

### 5.10b) 4-(2-Oxo-1,2,4,5-tetrahydro-1,3-benzdiazepin-3-yl)-piperidin-1-carbonsäure-(R)-2-[4-(1-ethoxycarbonylmethyl-piperidin-4-yl)-piperazin-1-yl]-1-(4-hydroxy-3,5-dimethyl-benzyl)-2-oxo-ethylester

Eine Suspension von 4.00 g (4.94 mmol) 4-(2-Oxo-1,2,4,5-tetrahydro-1,3-benzdiazepin-3-yl)-piperidin-1-carbonsäure-(*R*)-1-(4-benzyloxy-3,5-dimethyl-benzyl)-2-[4-(1-ethoxycarbonylmethyl-piperidin-4-yl)-piperazin-1-yl]-2-oxo-ethylester und 400 mg 10% Pd/C in 50 mL EtOH wurde bei RT und 3000 hPa Wasserstoffdruck bis zur theoretischen Aufnahme an Wasserstoff hydriert. Der Katalysator wurde abfiltriert, das Filtrat i.vac. eingeengt, der Rückstand mit DIPE verrührt, abgesaugt und getrocknet.

| | |
|---|---|
| Ausbeute: | 3.40 g (96% der Theorie) |
| ESI-MS: | (M+H)⁺ = 719 |
| Retentionszeit (HPLC): | 2.5 min (Methode A) |

### Beispiel 5.11

### 4-(2-Oxo-1,2,4,5-tetrahydro-1,3-benzdiazepin-3-yl)-piperidin-1-carbonsäure-(R)-2-[4-(1-carboxymethyl-piperidin-4-yl)-piperazin-1-yl]-1-(4-hydroxy-3,5-dimethyl-benzyl)-2-oxo-ethylester

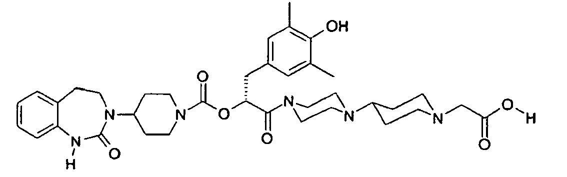

Zu einer Lösung von 2.50 g (3.48 mmol) 4-(2-Oxo-1,2,4,5-tetrahydro-1,3-benzdiazepin-3-yl)-piperidin-1-carbonsäure-(*R*)-2-[4-(1-ethoxycarbonylmethyl-piperidin-4-yl)-piperazin-1-yl]-1-(4-hydroxy-3,5-dimethyl-benzyl)-2-oxo-ethylester in 30 mL THF wurde bei RT eine Lösung von 126 mg (5.25 mmol) LiOH in 10 mL Wasser gegeben und das Reaktionsgemisch über Nacht bei RT gerührt. Man engte i.vac. zur Trockene ein und reinigte den Rückstand chromatographisch (Kieselgel, Gradient DCM zu DCM/MeOH/NH₃ 70:30:3). Die das Produkt enthaltenden Fraktionen wurden vereinigt, i.vac. eingeengt, der Rückstand mit DIPE verrieben, abgesaugt und getrocknet.

| | |
|---|---|
| Ausbeute: | 1.80 g (75% der Theorie) |
| ESI-MS: | (M+H)⁺ = 691 |
| R_{f} = | 0.10 (Kieselgel, DCM/MeOH/Cyc/NH₃ 70:15:15:2) |

### Beispiel 5.12

### 4-(2-Oxo-1,2,4,5-tetrahydro-1,3-benzdiazepin-3-yl)-piperidin-1-carbonsäure-(R)-2-{4-[1-(2-ethoxycarbonyl-ethyl)-piperidin-4-yl]-piperazin-1-yl]-1-(4-hydroxy-3,5-dimethyl-benzyl)-2-oxo-ethylester

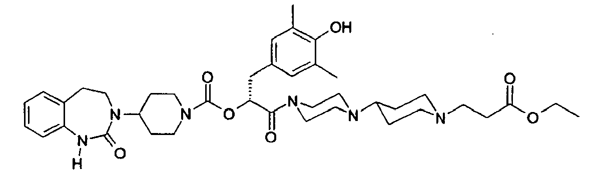

Zu einer Lösung von 400 mg (0.83 mmol) 4-(2-Oxo-1,2,4,5-tetrahydro-1,3-benzdiazepin-3-yl)-piperidin-1-carbonsäure-(*R*)-2-(4-hydroxy-3,5-dimethyl-phenyl)-1-carboxy-ethylester, 299 mg (0.93 mmol) TBTU und 477 µL (3.40 mmol) Triethylamin in 5 mL DMF wurden 352 mg (0.93 mmol) 3-(4-Piperazin-1-yl-piperidin-1-yl)-propionsäureethylester (eingesetzt als Bis-Hydrochlorid-Salz) gegeben und das Reaktionsgemisch 2 h bei RT gerührt. Die Reaktionslösung wurde mit 15% K₂CO₃-Lösung versetzt, erschöpfend mit DCM extrahiert und die vereinigten organischen Phasen über Na₂SO₄ getrocknet. Nach Entfernen des Trocken- und Lösungsmittels wurde der Rückstand via HPLC gereinigt; die das Produkt enthaltenden Fraktionen wurden vereinigt und lyophilisiert.

| | |
|---|---|
| Ausbeute: | 305 mg (50% der Theorie) |
| ESI-MS: | (M+H)⁺ = 733 |
| Retentionszeit (HPLC): | 2.6 min (Methode A) |

### Beispiel 5.13

### 4-(2-Oxo-1,2,4,5-tetrahydro-1,3-benzdiazepin-3-yl)-piperidin-1-carbonsäure-(R)-2-{4-[1-(2-carboxy-ethyl)-piperidin-4-yl]-piperazin-1-yl}-1-(4-hydroxy-3,5-dimethyl-benzyl)-2-oxo-ethylester

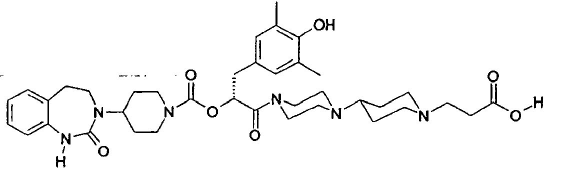

Analog Beispiel 5.11 konnte aus 2.60 g (3.55 mmol) 4-(2-Oxo-1,2,4,5-tetrahydro-1,3-benzdiazepin-3-yl)-piperidin-1-carbonsäure-(*R*)-2-{4-[1-(2-ethoxycarbonyl-ethyl)-piperidin-4-yl]-piperazin-1-yl}-1-(4-hydroxy-3,5-dimethyl-benzyl)-2-oxo-ethylester und 128 mg (5.33 mmol) LiOH das Produkt erhalten werden.

| | |
|---|---|
| Ausbeute: | 1.60 g (64% der Theorie) |
| ESI-MS: | (M+H)⁺ = 705 |
| R_{f} = | 0.07 (Kieselgel, DCM/MeOH/Cyc/NH₃ 70:15:15:2) |

### Beispiel 5.14

### 4-(2-Oxo-1,2,4,5-tetrahydro-1,3-benzdiazepin-3-yl)-piperidin-1-carbonsäure-(R)-2-[1'-(2-ethoxycarbonyl-ethyl)-4,4'-bipiperidinyl-1-yl]-1-(4-hydroxy-3,5-dimethyl-benzyl)-2-oxo-ethylester

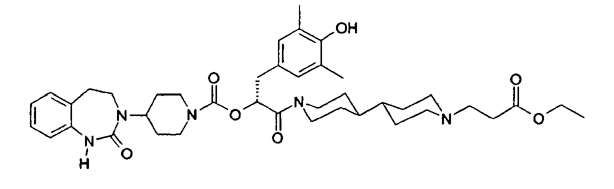

Eine Lösung von 80 mg (0.17 mmol) 4-(2-Oxo-1,2,4,5-tetrahydro-1,3-benzdiazepin-3-yl)-piperidin-1-carbonsäure-(*R*)-2-(4-hydroxy-3,5-dimethyl-phenyl)-1-carboxy-ethylester (Beispiel 5h), 59 mg (0.18 mmol) TBTU und 76 µL (0.54 mmol) Triethylamin in 1 mL DMF wurde bei RT 1 h gerührt. Dann erfolgte die Zugabe von 49 mg (0.18 mmol) 3-[4,4']Bipiperidinyl-1-yl-propionsäureethylester zum Reaktionsgemisch, welches anschließend 2 h bei RT gerührt wurde. Dieses wurde ohne weitere Aufarbeitung via HPLC gereinigt; die das Produkt enthaltenden Fraktionen wurden vereinigt und lyophilisiert.

| | |
|---|---|
| Ausbeute: | 11 mg (9% der Theorie) |
| ESI-MS: | (M+H)⁺ = 732 |
| Retentionszeit (HPLC): | 3.4 min (Methode C) |

### Beispiel 5.15

### 4-(2-Oxo-1,2,4,5-tetrahydro-1,3-benzdiazepin-3-yl)-piperidin-1-carbonsäure-(R)-2-[1'-(2-carboxy-ethyl)-4,4'-bipiperidinyl-1-yl]-1-(4-hydroxy-3,5-dimethyl-benzyl)-2-oxo-ethylester

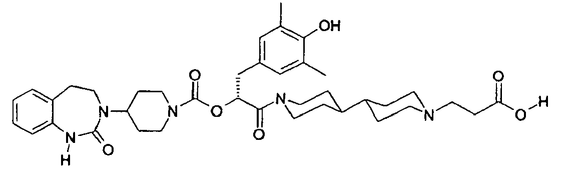

Analog Beispiel 5.11 konnte aus 2.50 g (3.42 mmol) 4-(2-Oxo-1,2,4,5-tetrahydro-1,3-benzdiazepin-3-yl)-piperidin-1-carbonsäure-(*R*)-2-[1'-(2-ethoxycarbonyl-ethyl)-4,4'-bipiperidinyl-1-yl]-1-(4-hydroxy-3,5-dimethyl-benzyl)-2-oxo-ethyl ester und 128 mg (5.33 mmol) LiOH das Produkt erhalten werden.

| | |
|---|---|
| Ausbeute: | 1.70 g (71 % der Theorie) |
| ESI-MS: | (M+H)⁺ = 704 |
| R_{f} = | 0.20 (Kieselgel, DCM/MeOH/Cyc/NH₃ 70:15:15:2) |

### Beispiel 5.16

### 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-{1'-[(hydroxy-methyl-carbamoyl)-methyl]-4,4'-bipiperidinyl-1-yl}-2-oxo-ethylester

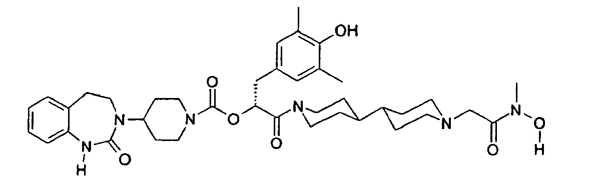

Eine Lösung von 80.0 mg (0.12 mmol) 4-(2-Oxo-1,2,4,5-tetrahydro-1,3-benzdiazepin-3-yl)-piperidin-1-carbonsäure-(*R*)-2-(1'-carboxymethyl-4,4'-bipiperidinyl-1-yl)-1-(4-hydroxy-3,5-dimethyl-benzyl)-2-oxo-ethylester (Bespiel 5.6), 44.6 mg (0.14 mmol) TBTU und 64.6 µL (0.46 mmol) Triethylamin in 1.2 mL DMF wurde 1 h bei RT gerührt. Dann erfolgte die Zugabe von 29.1 mg (0.35 mmol) N-Methylhydroxylamin (eingesetzt als Hydrochlorid-Salz) zum Reaktionsgemisch, welches anschließend 20 h bei RT gerührt wurde. Zum Reaktionsgemisch wurden 5 Tropfen AcOH gegeben, dieses über einen Spritzenfilter filtriert und via HPLC gereinigt. Die das Produkt enthaltenden Fraktionen wurden vereinigt, mit 30 mL EtOAc und 5% NaHCO₃-Lösung versetzt, die organische Phase abgetrennt und über Na₂SO₄ getrocknet. Nach Entfernen des Trocken- und Lösungsmittels wurde der Rückstand chromatographisch (Kieselgel, DCM/MeOH/NH₃ 90:10:1) gereinigt. Die das Produkt enthaltenden Fraktionen wurden vereinigt, i.vac. eingeengt, der Rückstand mit DIPE verrieben, abgesaugt und an der Luft bis zur Gewichtskonstanz getrocknet.

| | |
|---|---|
| Ausbeute: | 12.4 mg (15% der Theorie) |
| ESI-MS: | (M+H)⁺ = 719 |
| R_{f} = | 0.16 (Kieselgel, DCM/MeOH/NH₃ 90:10:1) |
| Retentionszeit (HPLC): | 3.0 min (Methode A) |

### Beispiel 5.17

### 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-[1'-(methoxycarbamoyl-methyl)-4,4'-bipiperidinyl-1-yl]-2-oxo-ethylester

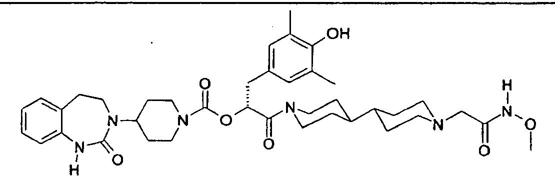

Analog Beispiel 5.16 konnte aus 80.0 mg (0.12 mmol) 4-(2-Oxo-1,2,4,5-tetrahydro-1,3-benzdiazepin-3-yl)-piperidin-1-carbonsäure-(*R*)-2-(1'-carboxymethyl-4,4'-bipiperidinyl-1-yl)-1-(4-hydroxy-3,5-dimethyl-benzyl)-2-oxo-ethylester (Bespiel 5.6) und 29.1 mg (0.35 mmol) O-Methylhydroxyamin (eingesetzt als Hydrochlorid-Salz) das Produkt erhalten werden.

| | |
|---|---|
| Ausbeute: | 13.0 mg (16% der Theorie) |
| ESI-MS: | (M+H)⁺ = 719 |
| R_{f} = | 0.27 (Kieselgel, DCM/MeOH/NH₃ 90:10:1) |
| Retentionszeit (HPLC): | 3.0 min (Methode A) |

### Beispiel 5.18

### 4-(2-Oxo-1,2,4,5-tetrahydro-1,3-benzdiazepin-3-yl)-piperidin-1-carbonsäure-(R)-2-(4-cyclopentyl-piperazin-1-yl)-1-(4-hydroxy-3,5-d imethyl-benzyl)-2-oxo-ethylester

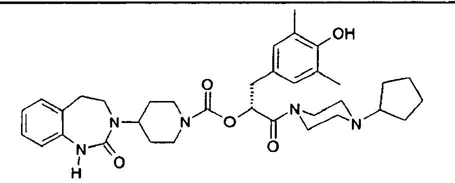

### 5.18a) 4-(2-Oxo-1,2,4,5-tetrahydro-1,3-benzdiazepin-3-yl)-piperidin-1-carbonsäure-(R)-1-(4-benzyloxy-3,5-dimethyl-benzyl)-2-(4-benzyl-piperazin-1-yl)-2-oxo-ethylester

Eine Lösung von 1.54 g (2.69 mmol) 4-(2-Oxo-1,2,4,5-tetrahydro-1,3-benzdiazepin-3-yl)-piperidin-1-carbonsäure-(R)-2-(4-benzyloxy-3,5-dimethyl-phenyl)-1-carboxy-ethylester (Beispiel 5g), 0.95 g (2.96 mmol) TBTU und 0.47 mL (3.37 mmol) Triethylamin in 20 mL THF und 2 ml DMF wurde 1 h bei RT gerührt. Dann erfolgte die Zugabe von 0.52 mL (2.96 mmol) N-Benzylpiperazin zum Reaktionsgemisch, welches anschließend 14 h bei RT gerührt wurde. Zum Reaktionsgemisch wurden 30 mL EtOAc gegeben, mit halbgesättigter NaHCO₃-Lösung gewaschen und die organische Phase über Na₂SO₄ getrocknet. Nach Entfernen des Trocken- und Lösungsmittels wurde der Rückstand chromatographisch (Kieselgel, EtOAc/Cyc 95:5) gereinigt. Die das Produkt enthaltenden Fraktionen wurden eingeengt, der Rückstand mit DIPE verrieben, abgesaugt und getrocknet.

| | |
|---|---|
| Ausbeute: | 1.62 g (82% der Theorie) |
| ESI-MS: | (M+H)⁺ = 730 |
| Retentionszeit (HPLC): | 4.3 min (Methode A) |

### 5.18b) 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-oxo-2-piperazin-1-yl-ethylester

Eine Suspension von 1.62 g (2.22 mmol) 4-(2-Oxo-1,2,4,5-tetrahydro-1,3-benzdiazepin-3-yl)-piperidin-1-carbonsäure-(R)-1-(4-benzyloxy-3,5-dimethyl-benzyl)-2-(4-benzyl-piperazin-1-yl)-2-oxo-ethylester und 150 mg 10% Pd/C in 40 mL MeOH wurde bei RT und 3000 hPa Wasserstoffdruck bis zur theoretischen Aufnahme an Wasserstoff hydriert. Der Katalysator wurde abgesaugt, das Lösungsmittel i.vac. eingeengt und der Rückstand chromatographisch (Kieselgel, EtOAc + 15% MeOH/NH₃ 9:1) gereinigt.

| | |
|---|---|
| Ausbeute: | 1.03 g (85% der Theorie) |
| ESI-MS: | (M+H)⁺ = 550 |
| Retentionszeit (HPLC): | 2.7 min (Methode A) |

### 5.18c) 4-(2-Oxo-1,2,4,5-tetrahydro-1,3-benzdiazepin-3-yl)-piperidin-1-carbonsäure-(R)-2-(4-cyclopentyl-piperazin-1-yl)-1-(4-hydroxy-3,5-dimethyl-benzyl)-2-oxo-ethylester

Eine Lösung von 100 mg (0.18 mmol) 4-(2-Oxo-1,2,4,5-tetrahydro-1,3-benzdiazepin-3-yl)-piperidin-1-carbonsäure-(*R*)-1-(4-hydroxy-3,5-dimethyl-benzyl)-2-oxo-2-piperazin-1-yl-ethylester, 48 µL (0.55 mmol) Cyclopentanon und 20 µL (0.37 mmol) AcOH in 2 mL THF/MeOH (2:1) wurde über Nacht bei RT gerührt. Dann erfolgte die Zugabe von 24 mg (0.36 mmol) Natriumcyanoborhydrid zur auf 0°C gekühlten Reaktionslösung, welche anschließend 4 h bei 0°C und über Nacht bei RT gerührt wurde. Die Lösungsmittel wurden bei 40°C entfernt, der Rückstand in 1 mL DMF gelöst und via HPLC gereinigt; die das Produkt enthaltenden Fraktionen wurden vereinigt und lyophilisiert.

| | |
|---|---|
| Ausbeute: | 48 mg (43% der Theorie) |
| ESI-MS: | (M+H)⁺ = 618 |
| Retentionszeit (HPLC): | 3.2 min (Methode A) |

### Beispiel 5.19

### 4-(2-Oxo-1,2,4,5-tetrahydro-1,3-benzdiazepin-3-yl)-piperidin-1-carbonsäure-(R)-2-(4-cycloheptyl-piperazin-1-yl)-1-(4-hydroxy-3,5-d imethyl-benzyl)-2-oxo-ethylester

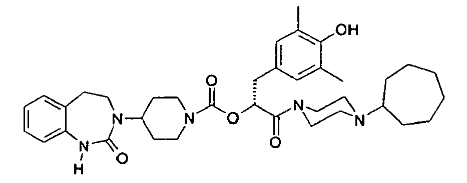

Analog Beispiel 5.18c konnte aus 100 mg (0.18 mmol) -(2-Oxo-1,2,4,5-tetrahydro-1,3-benzdiazepin-3-yl)-piperidin-1-carbonsäure-(R)-1-(4-hydroxy-3,5-dimethyl-benzyl)-2-oxo-2-piperazin-1-yl-ethylester, 40.8 mg (0.36 mmol) Cycloheptanon und 12 mg (0.18 mmol) Natriumcyanoborhydrid das Produkt erhalten werden.

| | |
|---|---|
| Ausbeute: | 21 mg (18% der Theorie) |
| ESI-MS: | (M+H)⁺ = 646 |
| Retentionszeit (HPLC): | 3.5 min (Methode A) |

### Beispiel 5.20

### 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-(1'-methansulfonyl-4,4'-bipiperidinyl-1-yl)-2-oxo-ethylester

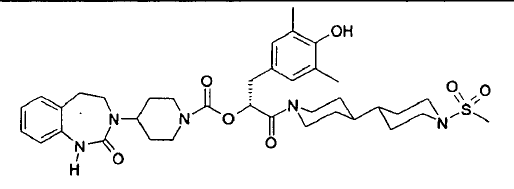

Eine Lösung von 100 mg (0.21 mmol) 4-(2-Oxo-1,2,4,5-tetrahydro-1,3-benzdiazepin-3-yl)-piperidin-1-carbonsäure-(*R*)-1-carboxy-2-(4-hydroxy-3,5-dimethyl-phenyl)-ethylester, 73 mg (0.23 mmol) TBTU und 36 µL (0.26 mmol) Triethylamin in 1 mL DMF wurde 1 h bei RT gerührt. Dann erfolgte die Zugabe von 56 mg (0.23 mmol) 1-Methansulfonyl-[4,4']bipiperidinyl zum Reaktionsgemisch, welches anschließend 5 h bei RT gerührt wurde. Das Reaktionsgemisch wurde ohne weitere Aufarbeitung via HPLC gereinigt; die das Produkt enthaltenden Fraktionen wurden vereinigt und lyophilisiert.

| | |
|---|---|
| Ausbeute: | 63 mg (43% der Theorie) |
| ESI-MS: | (M-H)⁻ = 708 |
| Retentionszeit (HPLC): | 4.0 min (Methode A) |

### Beispiel 5.21

### 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-[4-(4-methansulfonyl-piperazin-1-yl)-piperidin-1-yl]-2-oxo-ethylester

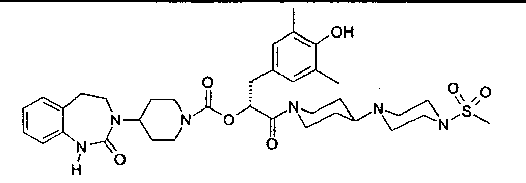

Analog Beispiel 5.20 konnte aus 100 mg (0.21 mmol) 4-(2-Oxo-1,2,4,5-tetrahydro-1,3- benzdiazepin-3-yl)-piperidin-1-carbonsäure-(R)-1-carboxy-2-(4-hydroxy-3,5-dimethylphenyl)-ethylester und 57 mg (0.23 mmol) 1-Methansulfonyl-4-piperidin-4-yl-piperazin das Produkt erhalten werden.

| | |
|---|---|
| Ausbeute: | 43 mg (29% der Theorie) |
| ESI-MS: | (M+H)⁺ = 711 |
| Retentionszeit (HPLC): | 3.1 min (Methode A) |

### Beispiel 5.22

### 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-{4-[4-(2-hydroxy-ethyl)-piperazin-1-yl]-piperidin-1-yl}-2-oxo-ethylester

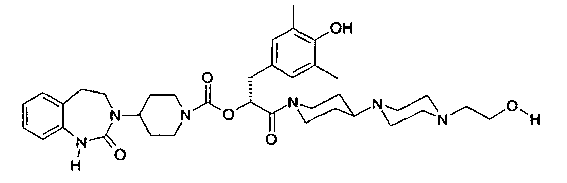

Analog Beispiel 5.20 konnte aus 80 mg (0.17 mmol) 4-(2-Oxo-1,2,4,5-tetrahydro-1,3- benzdiazepin-3-yl)-piperidin-1-carbonsäure-(*R*)-1-carboxy-2-(4-hydroxy-3,5-dimethylphenyl)-ethylester und 52 mg (0.18 mmol) 2-(4-Piperidin-4-yl-piperazin-1-yl)-ethanol das Produkt erhalten werden.

| | |
|---|---|
| Ausbeute: | 63 mg (56% der Theorie) |
| ESI-MS: | (M+H)⁺ = 677 |
| Retentionszeit (HPLC): | 2.4 min (Methode A) |

### Beispiel 5.23

### 4-(2-Oxo-1,2,4,5-tetrahydro-1,3-benzdiazepin-3-yl)-piperidin-1-carbonsäure-(R)-2-[1'-(3- ethoxycarbonyl-propionyl)-4,4'-bipiperidinyl-1-yl]-1-(4-hydroxy-3,5-dimethyl-benzyl)-2-oxo-ethylester

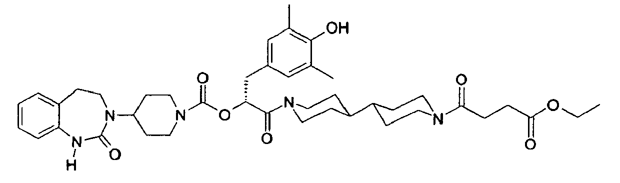

Zu einer Lösung von 300 mg (0.62 mmol) 4-(2-Oxo-1,2,4,5-tetrahydro-1,3-benzdiazepin-3-yl)-piperidin-1-carbonsäure-(R)-1-carboxy-2-(4-hydroxy-3,5-dimethyl-phenyl)-ethylester, 225 mg (0.70 mmol) TBTU und 97 µL (0.70 mmol) Triethylamin in 5 mL DMF wurden bei RT 207 mg (0.70 mmol) 4-[4,4']Bipiperidinyl-1-yl-4-oxo-buttersäureethylester (Amin A2) gegeben und das Reaktionsgemisch über Nacht bei RT geschüttelt. Dieses wurde ohne weitere Aufarbeitung direkt via HPLC gereinigt; die das Produkt enthaltenden Fraktionen wurden vereinigt und lyophilisiert.

| | |
|---|---|
| Ausbeute: | 170 mg (36% der Theorie) |
| ESI-MS: | (M+H)⁺ = 760 |
| Retentionszeit (HPLC): | 4.1 min (Methode A) |

### Beispiel 5.24

### 4-(2-Oxo-1,2,4,5-tetrahydro-1,3-benzdiazepin-3-yl)-piperidin-1-carbonsäure-(R)-2-[1'-(3-carboxy-propionyl)-4,4'-bipiperidinyl-1-yl]-1-(4-hydroxy-3,5-dimethyl-benzyl)-2-oxo-ethylester

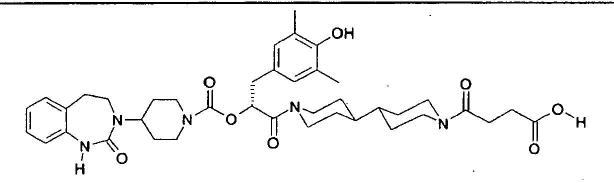

Zu einer Lösung von 20.0 mg (0.03 mmol) 4-(2-Oxo-1,2,4,5-tetrahydro-1,3-benzdiazepin-3-yl)-piperidin-1-carbonsäure-(*R*)-2-[1'-(3-ethoxycarbonyl-propionyl)-4,4'-bipiperidinyl-1-yl]-1-(4-hydroxy-3,5-dimethyl-benzyl)-2-oxo-ethylester in 1 mL THF wurde eine Lösung 0.96 mg (0.04 mmol) LiOH in 1 mL Wasser gegeben und das Reaktionsgemisch bei RT 2 h gerührt. Die Lösungsmittel wurden im Stickstoffstrom entfernt, der Rückstand in Wasser/Acetonitril aufgenommen und lyophilisiert. Das Produkt fiel als Li-Salz an.

| | |
|---|---|
| Ausbeute: | 17 mg (88% der Theorie) |
| ESI-MS: | (M+H)⁺ = 732 |
| R_{f} = | 0.13 (Kieselgel, DCM/MeOH/NH₃ 90:10:1) |

### Beispiel 5.25

### 4-(2-Oxo-1,2,4,5-tetrahydro-1,3-benzdiazepin-3-yl)-piperidin-1-carbonsäure-(R)-2-(1'- ethoxyoxalyl-4,4'-bipiperidinyl-1-yl)-1-(4-hydroxy-3,5-dimethyl-benzyl)-2-oxo-ethylester

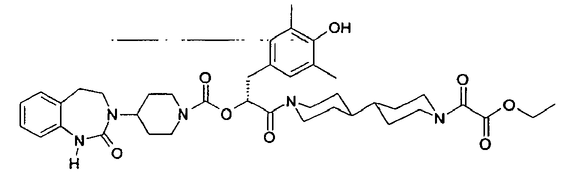

Zu einer Lösung von 300 mg (0.62 mmol) 4-(2-Oxo-1,2,4,5-tetrahydro-1,3-benzdiazepin-3-yl)-piperidin-1-carbonsäure-(R)-1-carboxy-2-(4-hydroxy-3,5-dimethyl-phenyl)-ethylester, 225 mg (0.70 mmol) TBTU und 197 µL (1.40 mmol) Triethylamin in 5 mL DMF wurden bei RT 231 mg (0.70 mmol) [4,4']Bipiperidinyl-1-yl-oxo-essigsäureethylester (Amin A1, eingesetzt als Hydrogencarbonat-Salz) gegeben und das Reaktionsgemisch 3 h bei RT gerührt. Man engte i.vac. ein, nahm den Rückstand in DCM auf, wusch die organische Phase mit 15% K₂CO₃-Lösung und trocknete diese über Na₂SO₄. Nach Entfernen des Trocken- und Lösungsmittels wurde der Rückstand via HPLC gereinigt; die das Produkt enthaltenden Fraktionen wurden vereinigt, i.vac. eingeengt, der Rückstand mit DIPE verrieben, abgesaugt und getrocknet.

| | |
|---|---|
| Ausbeute: | 360 mg (79% der Theorie) |
| ESI-MS: | (M+H)⁺ = 732 |
| Retentionszeit (HPLC): | 4.0 min (Methode A) |

### Beispiel 5.26

### 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-(1'-oxalyl-4,4'-bipiperidinyl-1-yl)-2-oxo-ethylester

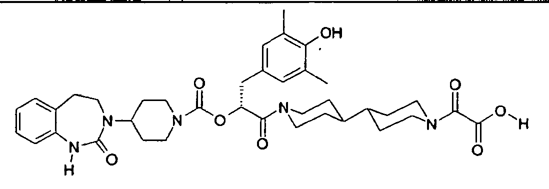

Zu einer Lösung von 20.0 mg (0.03 mmol) 4-(2-Oxo-1,2,4,5-tetrahydro-1,3-benzdiazepin-3-yl)-piperidin-1-carbonsäure-(*R*)-2-(1'-ethoxyoxalyl-4,4'-bipiperidinyl-1-yl)-1-(4-hydroxy-3,5-dimethyl-benzyl)-2-oxo-ethylester in 1 mL THF wurde eine Lösung 0.96 mg (0.04 mmol) LiOH in 1 mL Wasser gegeben und das Reaktionsgemisch bei RT 2 h gerührt. Die Lösungsmittel wurden im Stickstoffstrom entfernt, der Rückstand in Wasser/Acetonitril aufgenommen und lyophilisiert. Das Produkt fiel als Li-Salz an.

| | |
|---|---|
| Ausbeute: | 19 mg (99% der Theorie) |
| ESI-MS: | (M+H)⁺ = 704 |
| R_{f} = | 0.10 (Kieselgel, DCM/MeOH/NH₃ 90:10:1) |

### Beispiel 6

### 4-(2-Oxo-1,2,4,5-tetrahydro-1,3-benzdiazepin-3-yl)-piperidin-1-carbonsäure-(R)-2-(4-cyclohexyl-piperazin-1-yl)-1-(3,5-dibrom-4-hyd roxy-benzyl)-2-oxo-ethylester

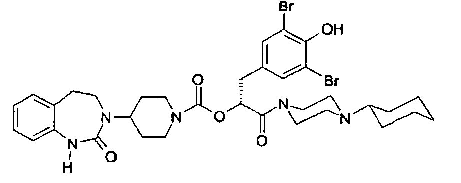

### 6a) (Z,E)-3-(4-Acetoxy-3,5-dibrom-phenyl)-2-acetylamino-acrylsäure

Unter Stickstoffatmosphäre wurde eine Mischung aus 30.0 g (107 mmol) 3,5-Dibrom-4-hydroxy-benzaldehyd, 18.8 g (268 mmol) N-Acetylglycin und 13.2 g (161 mmol) NaOAc in 120 mL Acetanhydrid 1.5 h auf 130°C erhitzt. Man kühlte auf 90°C ab und tropfte langsam 15 mL Wasser so zu, dass die Temperatur 100°C nicht überschritt. Nach beendeter Zugabe wurde weitere 2 h bei 90°C gehalten, auf 70°C gekühlt, mit 300 mL Wasser versetzt und 30 min gerührt. Der Niederschlag wurde abfiltriert, mit Wasser gewaschen und bei 50°C getrocknet. Das Rohprodukt wurde ohne Reinigung weiter umgesetzt.

| | |
|---|---|
| Ausbeute: | 35.7 g (79% der Theorie) |
| ESI-MS: | (M+H)⁺ = 420/422/424 (2 Br) |
| R_{f} = | 0.20 (Kieselgel, DCM/MeOH/AcOH 90:10:1) |

### 6b) 3-(3,5-Dibrom-4-hydroxy-phenyl)-2-oxo-propionsäure

Unter Eiskühlung wurde zu einer Lösung von 35.7 g (84.8 mmol) (*Z,E*)-3-(4-Acetoxy-3,5-dibrom-phenyl)-2-acetylamino-acrylsäure in 290 mL NMP 325 mL 4 M HCl gegeben und das Reaktionsgemisch 1.5 h auf 120°C erhitzt (Badtemperatur). Man kühlte auf 0°C ab, versetzte mit 1.4 L Wasser und rührte weitere 30 min. Der entstandene Niederschlag wurde abfiltriert und getrocknet.

| | |
|---|---|
| Ausbeute: | 20.5 g (72% der Theorie) |
| ESI-MS: | (M-H)⁻ = 335/337/339 (2 Br) |
| R_{f} = | 0.35 (Kieselgel, DCM/MeOH/AcOH 80:20:2) |

### 6c) (R)-3-(3,5-Dibrom-4-hydroxy-phenyl)-2-hydroxy-propionsäure

Unter Argonatmosphäre wurde zu einer Lösung von 14.5 g (42.9 mmol) 3-(3,5-Dibrom-4-hydroxy-phenyl)-2-oxo-propionsäure in 250 mL THF 6 mL (43.1 mmol) Triethylamin gegeben und diese auf -32°C abgekühlt. Dann wurde eine Lösung von 22.6 g (70.5 mmol) (1*R*)-*B*-Chlordiisopinocampheylboran in 90 mL THF so zugetropft, dass die Temperatur -20°C nicht überschritt. Man rührte 30 min bei -30°C und ließ das Reaktionsgemisch innerhalb 2.5 h auf 0°C erwärmen. Zur Vervollständigung der Reaktion wurde erneut auf - 32°C gekühlt, eine Lösung von 5.8 g (18.1 mmol) (1*R*)-B-Chlordiisopinocampheylboran in 40 mL THF so zugetropft, dass die Temperatur-20°C nicht überschritt und anschließend das Reaktionsgemisch über Nacht im Eisbad gerührt. Nach erneutem Abkühlen auf-32°C wurden weitere 2.5 g (7.8 mmol) (1*R*)-*B*-Chlordüsopinocampheylboran in 20 mL THF zugetropft, das Reaktionsgemisch 30 min bei dieser Temperatur gerührt, innerhalb von 2.5 h auf 0°C erwärmt und anschließend 66 h bei RT gerührt. Zur Reaktionslösung wurden 100 mL 10% NaOH so zugegeben, dass die Temperatur 25°C nicht überschritt, weitere 30 min gerührt, mit MTBE versetzt, die organische Phase abgetrennt und diese erneut mit 20 mL 10% NaOH-Lösung extrahiert. Die vereinigten wässrigen Phasen wurden mehrmals mit MTBE gewaschen, mit 20% HCl angesäuert und mit Diethylether/EtOAc (1:1) erschöpfend extrahiert. Die vereinigten organischen Phasen wurden mit Aktivkohle versetzt und filtriert. Das Produkt wurde ohne Reinigung weiter umgesetzt.

| | |
|---|---|
| Ausbeute: | 12.7 g (87% der Theorie) |
| ESI-MS: | (M-H)⁻ = 337/339/341 (2 Br) |
| R_{f} = | 0.4 (Kieselgel, DCM/MeOH/AcOH 80:20:2) |
| Retentionszeit (HPLC-MS): | 6.4 min (Methode D) |

### 6d) (R)-3-(3,5-Dibromo-4-hydroxy-phenyl)-2-hydroxy-propionsäuremethylester

Eine Lösung aus 14.0 g (34.8 mmol) (*R*)-3-(3,5-Dibrom-4-hydroxy-phenyl)-2-hydroxy-propionsäure in 100 mL methanolischer HCl (6 M) wurde bei RT 3 h gerührt. Man engte i.vac. ein und reinigte den Rückstand chromatographisch (Kieselgel, n-Hexan/EtOAc 7:3).

| | |
|---|---|
| Ausbeute: | 7.0 g (57% der Theorie) |
| ESI-MS: | (M-H)⁻ = 351/353/355 (2 Br) |
| Retentionszeit (HPLC-MS): | 9.8 min (Methode D) |

### 6e) (R)-3-[3,5-Dibrom-4-(2-trimethylsilanyl-ethoxymethoxy)-Phenyl]-2-hydroxy- propionsäuremethylester

Unter Stickstoffatmosphäre wurden zu einer Lösung von 6.78 g (19.2 mmol) (R)-3-(3,5-Dibrom-4-hydroxy-phenyl)-2-hydroxy-propionsäuremethylester in 100 mL Acetonitril 11.1 g (76.6 mmol) 40% KF/Al₂O₃ gegeben und die resultierende Suspension für einige min bei RT gerührt. Anschließend wurde eine Lösung von 4.07 mL (23.0 mmol) (2-Chlormethoxy-ethyl)-trimethylsilan in 20 mL Acetonitril zugegeben und das Reaktionsgemisch 20 h bei RT gerührt. Man filtrierte über Celite, engte das Lösungsmittel i.vac. ein und reinigte Rückstand chromatographisch (Kieselgel, *n*-Hexan/EtOAc 7:3).

| | |
|---|---|
| Ausbeute: | 5.49 g (59% der Theorie) |
| R_{f} = | 0.45 (Kieselgel, *n*-Hexan/EtOAc 1:1) |

### 6f) 4-(2-Oxo-1,2,4,5-tetrahydro-1,3-benzdiazepin-3-yl)-piperidin-1-carbonsäure-(R)-2-[3,5-dibrom-4-(2-trimethylsilanyl-ethoxymethoxy)-phenyl]-1-methoxy-carbonyl-ethylester

Unter Stickstoffatmosphäre wurde zu einer auf 15°C gekühlten Lösung von 1.99 g (9.56 mmol) Chlorameisensäure-4-nitrophenylester in 80 mL Acetonitril 1.23 g (10.0 mmol) 4-Dimethylaminopyridin gegeben. Die entstandene Suspension wurde auf -7°C gekühlt und langsam mit einer Lösung von 4.63 g (9.56 mmol) (R)-3-[3,5-Dibrom-4-(2-trimethyl-silanyl-ethoxymethoxy)-phenyl]-2-hydroxy-propionsäuremethyl-ester in 20 mL Acetonitril versetzt. Man ließ weitere 15 min bei dieser Temperatur rühren, gab 2.35 g (9.56 mmol) 3-Piperidin-4-yl-1,3,4,5-tetrahydro-1,3-benzdiazepin-2-on zu und ließ das Reaktionsgemisch 2.5 h bei RT rühren. Man engte i.vac. ein, nahm den Rückstand in EtOAc auf, wusch die organische Phase mit 10% Zitronensäure- und 10% Na₂CO₃-Lösung und trocknete diese über Na₂SO₄. Nach Entfernen des Trocken- und Lösungsmittels wurde der Rückstand chromatographisch (Kieselgel, Gradient n-Hexan/EtOAc 1:1 zu 2:8) gereinigt.

| | |
|---|---|
| Ausbeute: | 4.35 g (69% der Theorie) |
| ESI-MS: | (M+H)⁺ = 754/756/758 (2 Br) |
| Retentionszeit (HPLC): | 29.2 min (Methode D) |

### 6g) 4-(2-Oxo-1,2,4,5-tetrahydro-1,3-benzdiazepin-3-yl)-piperidin-1-carbonsäure-(R)-2-(3,5-dibrom-4-hydroxy-phenyl)-1-methoxycarbonyl-ethylester

Unter Stickstoffatmosphäre wurden zu einer Lösung von 4.30 g (5.69 mmol) 4-(2-Oxo-1,2,4,5-tetrahydro-1,3-benzdiazepin-3-yl)-piperidin-1-carbonsäure-(*R*)-2-[3,5-dibrom-4-(2-trimethylsilanyl-ethoxymethoxy)-phenyl]-1-methoxy-carbonyl-ethylester in 40 mL THF und 40 mL MeOH 5.46 mL methanolische H₂SO₄ (0.5 M) gegeben und die Reaktionslösung 6 h bei RT gerührt. Das Reaktionsgemisch wurde i.vac eingeengt und der Rückstand ohne Reinigung weiter umgesetzt.

| | |
|---|---|
| Ausbeute: | quantitativ |
| ESI-MS: | (M+H)⁺ = 624/626/628 (2 Br) |
| Retentionszeit (HPLC): | 17.3 min (Methode D) |

### 6h) 4-(2-Oxo-1,2,4,5-tetrahydro-1,3-benzdiazepin-3-yl)-piperidin-1-carbonsäure-(R)-1-carboxy-2-(3,5-dibrom-4-hydroxy-phenyl)-ethylester

Zu einer Lösung von 4-(2-Oxo-1,2,4,5-tetrahydro-1,3-benzdiazepin-3-yl)-piperidin-1-carbonsäure-(*R*)-2-(3,5-dibrom-4-hydroxy-phenyl)-1-methoxycarbonyl-ethylester (Rohprodukt aus Beispiel 6g) in 80 mL THF wurde eine Lösung von 0.51 g (21.3 mmol) LiOH gegeben und das Reaktionsgemisch 3 h bei RT gerührt. Man zog i.vac. das THF ab, wusch die wässrige Phase mit EtOAc, säuerte mit 10% HCl an und extrahierte die wässrige Phase erschöpfend mit EtOAc. Die vereinigten organischen Phasen wurden i.vac. eingeengt, in Diethylether suspendiert, filtriert, der Rückstand getrocknet und anschließend chromatographisch (Kieselgel, DCM/MeOH/AcOH 90:10:1) gereinigt.

| | |
|---|---|
| Ausbeute: | 3.5 g (100% der Theorie) |
| ESI-MS: | (M+H)⁺ = 610/612/614 (2 Br) |
| Retentionszeit (HPLC): | 14.1 min (Methode D) |

### 6i) 4-(2-Oxo-1,2,4,5-tetrahydro-1,3-benzdiazepin-3-yl)-piperidin-1-carbonsäure-(R)-2-(4-cyclohexyl-piperazin-1-yl)-1-(3,5-dibrom-4-hydroxy-benzyl)-2-oxo-ethylester

Zu einer Lösung von 100 mg (0.16 mmol) 4-(2-Oxo-1,2,4,5-tetrahydro-1,3-benzdiazepin-3-yl)-piperidin-1-carbonsäure-(*R*)-1-carboxy-2-(3,5-dibrom-4-hydroxy-phenyl)-ethylester, 58 mg (0.18 mmol) TBTU und 25 µL (0.18 mmol) Triethylamin in 1 mL DMF wurden bei RT 30 mg (0.18 mmol) 1-Cyclohexyl-piperazin gegeben und das Reaktionsgemisch über Nacht bei RT gerührt. Die Reaktionslösung wurde über einen Spritzenfilter filtriert und ohne weitere Aufarbeitung direkt via HPLC gereinigt. Die das Produkt enthaltenden Fraktionen wurden vereinigt und lyophilisiert.

| | |
|---|---|
| Ausbeute: | 64 mg (51 % der Theorie) |
| ESI-MS: | (M+H)⁺ = 760/762/764 (2 Br) |
| Retentionszeit (HPLC-MS): | 3.3 min (Methode A) |

### Beispiel 6.1

### 4-(2-Oxo-1,2,4,5-tetrahydro-1,3-benzdiazepin-3-yl)-piperidin-1-carbonsäure-(R)-1-(3,5-dibrom-4-hydroxy-benzyl)-2-(4-morpholin-4-yl-piperidin-1-yl)-2-oxo-ethylester

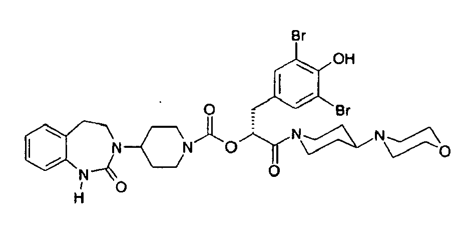

Analog Beispiel 6i konnte aus 100 mg (0.16 mmol) 4-(2-Oxo-1,2,4,5-tetrahydro-1,3-benz-diazepin-3-yl)-piperidin-1-carbonsäure-(R)-1-carboxy-2-(3,5-dibrom-4-hydroxy-phenyl)-ethylester und 31 mg (0.18 mmol) 4-Piperidin-4-yl-morpholin das Produkt erhalten werden.

| | |
|---|---|
| Ausbeute: | 72 mg (58% der Theorie) |
| ESI-MS: | (M+H)⁺ = 762/764/766 (2 Br) |
| Retentionszeit (HPLC-MS): | 3.1 min (Methode A) |

### Beispiel 6.2

### 4-(2-Oxo-1,2,4,5-tetrahydro-1,3-benzdiazepin-3-yl)-piperidin-1-carbonsäure-(R)-1-(3,5-dibrom-4-hydroxy-benzyl)-2-[4-(4-ethoxycarbonylmethyl-piperazin-1-yl)-piperid in-1-yl]-2-oxo-ethylester

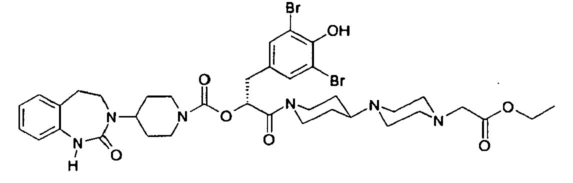

Analog Beispiel 6i konnte aus 100 mg (0.16 mmol) 4-(2-Oxo-1,2,4,5-tetrahydro-1,3-benz-diazepin-3-yl)-piperidin-1-carbonsäure-(*R*)-1-carboxy-2-(3,5-dibrom-4-hydroxy-phenyl)-ethylester und 46 mg (0.18 mmol) (4-Piperidin-4-yl-piperazin-1-yl)-essigsäureethylester das Produkt erhalten werden.

| | |
|---|---|
| Ausbeute: | 5 mg (4% der Theorie) |
| ESI-MS: | (M+H)⁺ = 847/849/851 (2 Br) |
| Retentionszeit (HPLC-MS): | 2.9 min (Methode A) |

### Beispiel 7

### 4-(2-Oxo-1,2,4,5-tetrahydro-1,3-benzdiazepin-3-yl)-piperidin-1-carbonsäure-(R)-1-(3-brom-4-hydroxy-benzyl)-2-(4-cyclohexyl-piperazin-1-yl)-2-oxo-ethylester

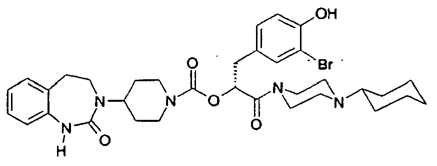

### 7a) (Z,E)-3-(4-Acetoxy-3-brom-phenyl)-2-acetylamino-acrylsäure

Hergestellt analog Beispiel 6a aus 75.0 g (366 mmol) 3-Brom-4-hydroxy-benzaldehyd und 64.2 g (548 mmol) N-Acetylglycin. Nach Abkühlen des Reaktionsgemisches fiel das Produkt aus, welches filtriert, mit Wasser gewaschen und getrocknet wurde.

| | |
|---|---|
| Ausbeute: | 69.8 g (56% der Theorie) |
| Retentionszeit (HPLC): | 7.6 min (Methode D) |

### 7b) 3-(3-Brom-4-hydroxy-phenyl)-2-oxo-propionsäure

Unter Eiskühlung wurde zu einer Lösung von 69.7 g (204 mmol) (*Z,E*)-3-(4-Acetoxy-3-brom-phenyl)-2-acetylamino-acrylsäure in 300 mL NMP 750 mL 4 M HCl gegeben und das Reaktionsgemisch 2.5 h auf 95°C erhitzt (Badtemperatur). Man kühlte über Nacht auf RT ab, versetzte mit 2 L Wasser, extrahierte dreimal mit je 300 mL EtOAc, wusch die vereinigten organischen Phasen zweimal mit je 1 L Wasser und trocknete über Na₂SO₄. Nach Entfernen des Trocken- und Lösungsmittels wurde der Rückstand ohne Reinigung weiter umgesetzt.

| | |
|---|---|
| Ausbeute: | 45.8 g (87% der Theorie) |
| Retentionszeit (HPLC): | 7.8 min (Methode D) |

### 7c) (R)-3-(3-Brom-4-hydroxy-phenyl)-2-hydroxy-propionsäure

Unter Argonatmosphäre wurde zu einer Lösung von 45.0 g (174 mmol) 3-(3-Brom-4-hydroxy-phenyl)-2-oxo-propionsäure in 350 mL THF 29 mL (356 mmol) Triethylamin gegeben und diese auf -27°C abgekühlt. Dann wurde eine Lösung und 114 g (356 mmol) (1*R*)-*B*-Chlordiisopinocampheylboran in 200 mL THF so zugetropft, dass die Temperatur - 20°C nicht überschritt. Man rührte 15 min bei -30°C und ließ das Reaktionsgemisch innerhalb 1 h auf RT erwärmen. Zur Reaktionslösung wurden 200 mL 10% NaOH so zugegeben, dass die Temperatur 25°C nicht überschritt, weitere 15 min gerührt, mit 400 mL Wasser verdünnt, mit 400 mL MTBE versetzt und die wässrige Phase abgetrennt. Diese wurde mit 400 mL MTBE gewaschen, mit 150 mL 4 M HCl angesäuert, zweimal mit je 400 mL EtOAc extrahiert, die vereinigten organischen Phasen mit gesättigter NaCl-Lösung gewaschen und über Na₂SO₄ getrocknet. Nach Entfernen des Trocken- und Lösungsmittels wurde der Rückstand ohne Reinigung weiter umgesetzt.

| | |
|---|---|
| Ausbeute: | 53.7 g (89% der Theorie) |
| Retentionszeit (HPLC): | 4.0 min (Methode D) |

### 7d) (R)-3-(3-Brom-4-hydroxy-phenyl)-2-hydroxy-propionsäuremethylester

Zu einer Lösung von 53.6 g (154 mmol) (R)-3-(3-Brom-4-hydroxy-phenyl)-2-hydroxy-propionsäure in 250 mL MeOH wurden 2.5 mL konzentrierte Schwefelsäure gegeben und das Reaktionsgemisch 4 h bei RT gerührt. Man engte i.vac. ein, nahm den Rückstand in 250 mL EtOAc auf, wusch die organische Phase zweimal mit je 100 mL gesättigter NaHCO₃- und gesättigter NaCl-Lösung und trocknete über Na₂SO₄. Nach Entfernen des Trocken- und Lösungsmittels wurde der Rückstand ohne Reinigung weiter umgesetzt.

| | |
|---|---|
| Ausbeute: | quantitativ |
| Retentionszeit (HPLC): | 6.8 min (Methode D) |

### 7e) (R)-3-[3-Brom-4-(2-trimethylsilanyl-ethoxymethoxy)-phenyl]-2-hydroxy-propionsäuremethylester

Zu einer Lösung von 10.2 g (34.6 mmol) (R)-3-(3-Brom-4-hydroxy-phenyl)-2-hydroxy-propionsäuremethylester in 100 mL DCM wurden 6.7 mL (39.1 mmol) Ethyldiisopropylamin gegeben und das Reaktionsgemisch im Eisbad gekühlt. Dann erfolgte die Zugabe einer Lösung von 7.9 mL (44.6 mmol) (2-Chlormethoxy-ethyl)-trimethylsilan in 20 mL DCM. Das Reaktionsgemisch wurde 3 h bei RT gerührt und dann zur Vervollständigung der Reaktion mit weiteren 0.67 mL Ethyldiisopropylamin und 0.8 mL (4.5 mmol) (2-Chlormethoxy-ethyl)-trimethylsilan versetzt und 1.5 h bei RT gerührt. Das Reaktionsgemisch wurde mit 5% Na₂CO₃- und gesättigter NaCl-Lösung gewaschen und über Na₂SO₄ getrocknet. Nach Entfernen des Trocken- und Lösungsmittels wurde der Rückstand chromatographisch (Kieselgel, Cyc/EtOAc 75:25) gereinigt.

| | |
|---|---|
| Ausbeute: | 9.6 g (68% der Theorie) |
| Retentionszeit (HPLC): | 15.1 min (Methode E) |

### 7f) 4-(2-Oxo-1,2,4,5-tetrahydro-1,3-benzdiazepin-3-yl)-piperidin-1-carbonsäure-(R)-2-[3-brom-4-(2-trimethylsilanyl-ethoxymethoxy)-phenyl]-1-methoxycarbonyl-ethylester

Hergestellt analog Beispiel 6f aus 4.55 g (11.2 mmol) (R)-3-[3-Brom-4-(2-trimethylsilanyl-ethoxymethoxy)-phenyl]-2-hydroxy-propionsäure-methylester und 2.75 g (11.2 mmol) 3-Piperidin-4-yl-1,3,4,5-tetrahydro-1,3-benzdiazepin-2-on.

| | |
|---|---|
| Ausbeute: | 5.46 g (72% der Theorie) |
| Retentionszeit (HPLC): | 16.5 min (Methode E) |

### 7g) 4-(2-Oxo-1,2,4,5-tetrahydro-1,3-benzdiazepin-3-yl)-piperidin-1-carbonsäure-(R)-2-(3-brom-4-hydroxy-phenyl)-1-methoxycarbonyl-ethylester

Hergestellt analog Beispiel 6g aus 5.40 g (7.98 mmol) 4-(2-Oxo-1,2,4,5-tetrahydro-1,3-benzdiazepin-3-yl)-piperidin-1-carbonsäure-(*R*)-2-[3-brom-4-(2-trimethylsilanyl-ethoxy-methoxy)-phenyl]-1-methoxycarbonyl-ethylester und 7.7 mL (4.2 mmol) methanolische Schwefelsäure (0.5 M). Das Rohprodukt (5.44 g) wurde ohne Reinigung weiter umgesetzt.

| | |
|---|---|
| Retentionszeit (HPLC): | 9.9 min (Methode E) |

### 7h) 4-(2-Oxo-1,2,4,5-tetrahydro-1,3-benzdiazepin-3-yl)-piperidin-1-carbonsäure-(R)-2-(3-brom-4-hydroxy-phenyl)-1-carboxy-ethylester

Zu einer Lösung von 5.44 g des Rohproduktes aus Beispiel 7g in 80 mL THF wurde eine Lösung von 0.84 g (34.1 mmol) LiOH in 20 mL Wasser gegeben und das Reaktionsgemisch 1 h bei RT gerührt. Das organische Lösungsmittel wurde i.vac. entfernt, die wässrige Phase mit EtOAc gewaschen, mit 10% HCl angesäuert und mit EtOAc erschöpfend extrahiert. Die vereinigten organischen Phasen wurden mit gesättigter NaCl-Lösung gewaschen und über Na₂SO₄ getrocknet. Nach Entfernen des Trocken- und Lösungsmittels wurde der Rückstand mit 90 mL Diethylether verrieben, filtriert, der Feststoff mit Diethylether gewaschen und bei 45°C getrocknet.

| | |
|---|---|
| Ausbeute: | 4.10 g (89% der Theorie über 2 Stufen) |
| Retentionszeit (HPLC): | 8.2 min (Methode E) |

### 7i) 4-(2-Oxo-1,2,4,5-tetrahydro-1,3-benzdiazepin-3-yl)-Piperidin-1-carbonsäure-(R)-1-(3-brom-4-hydroxy-benzyl)-2-oxo-2-[4-(tetrahydro-pyran-4-yl)-piperazin-1-yl]-ethylester

Hergestellt analog Beispiel 5.20 aus 100 mg (0.19 mmol) 4-(2-Oxo-1,2,4,5-tetrahydro-1,3-benzdiazepin-3-yl)-piperidin-1-carbonsäure-(*R*)-2-(3-brom-4-hydroxy-phenyl)-1-carboxyethyl ester und 35 mg (0.21 mmol) 1-Cyclohexyl-piperazin.

| | |
|---|---|
| Ausbeute: | 62 mg (49% der Theorie) |
| ESI-MS: | (M+H)⁺ = 682/684 (Br) |
| Retentionszeit (HPLC-MS): | 3.2 min (Methode A) |

### Beispiel 7.1

### 4-(2-Oxo-1,2,4,5-tetrahydro-1,3-benzdiazepin-3-yl)-piperidin-1-carbonsäure-(R)-1-(3-brom-4-hydroxy-benzyl)-2-(4-morpholin-4-yl-piperidin-1-yl)-2-oxo-ethylester

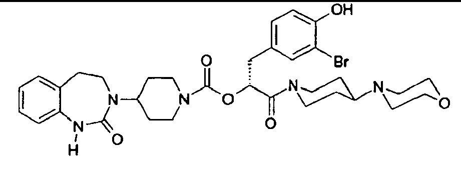

Hergestellt analog Beispiel 5.20 aus 100 mg (0.19 mmol) 4-(2-Oxo-1,2,4,5-tetrahydro-1,3-benzdiazepin-3-yl)-piperidin-1-carbonsäure-(R)-2-(3-brom-4-hydroxy-phenyl)-1-carboxy-ethylester und 36 mg (0.21 mmol) 4-Piperidin-4-yl-morpholin.

| | |
|---|---|
| Ausbeute: | 66 mg (51 % der Theorie) |
| ESI-MS: | (M+H)⁺ = 684/686 (Br) |
| Retentionszeit (HPLC-MS): | 2.9 min (Methode A) |

### Beispiel 7.2

### 4-(2-Oxo-1,2,4,5-tetrahydro-1,3-benzdiazepin-3-yl)-piperidin-1-carbonsäure-(R)-1-(3-brom-4-hydroxy-benzyl)-2-(1'-ethoxycarbonylmethyl-4,4'-bipiperidinyl-1-yl)-2-oxo-ethylester

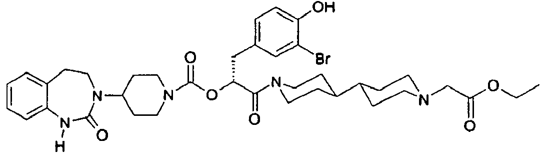

Hergestellt analog Beispiel 5.20 aus 150 mg (0.28 mmol) 4-(2-Oxo-1,2,4,5-tetrahydro-1,3-benzdiazepin-3-yl)-piperidin-1-carbonsäure-(R)-2-(3-brom-4-hydroxy-phenyl)-1-carboxy-ethylester und 79 mg (0.31 mmol) [4,4']Bipiperidinyl-1-yl-essigsäureethylester.

| | |
|---|---|
| Ausbeute: | 63 mg (29% der Theorie) |
| ESI-MS: | (M+H)⁺ = 768/770 (Br) |
| Retentionszeit (HPLC-MS): | 3.2 min (Methode A) |

### Beispiel 7.3

### 4-(2-Oxo-1,2,4,5-tetrahydro-1,3-benzdiazepin-3-yl)-piperidin-1-carbonsäure-(R)-1-(3-brom-4-hydroxy-benzyl)-2-[4-(4-ethoxycarbonylmethyl-piperazin-1-yl)-piperid in-1-yl]-2-oxo-ethylester

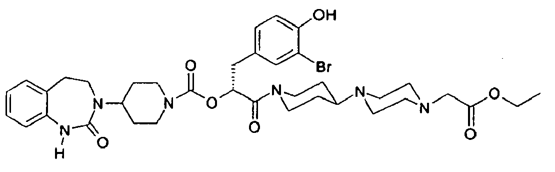

Hergestellt analog Beispiel 5.20 aus 150 mg (0.28 mmol) 4-(2-Oxo-1,2,4,5-tetrahydro-1,3-benzdiazepin-3-yl)-piperidin-1-carbonsäure-(*R*)-2-(3-brom-4-hydroxy-phenyl)-1-carboxy-ethylester und 79 mg (0.31 mmol) (4-Piperidin-4-yl-piperazin-1-yl)-essigsäureethylester.

| | |
|---|---|
| Ausbeute: | 82 mg (38% der Theorie) |
| ESI-MS: | (M+H)⁺ = 769/771 (Br) |
| Retentionszeit (HPLC-MS): | 3.1 min (Methode A) |

### Beispiel 7.4

### 4-(2-Oxo-1,2,4,5-tetrahydro-1,3-benzdiazepin-3-yl)-piperidin-1-carbonsäure-(R)-1-(3-brom-4-hydroxy-benzyl)-2-[4-(4-carboxymethyl-piperazin-1-yl)-piperidin-1-yl]-2-oxo-ethylester

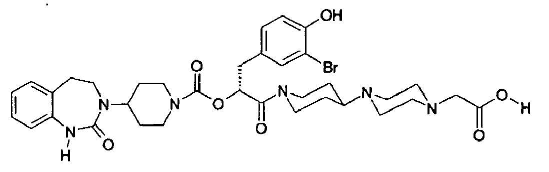

Zu einer Lösung von 50 mg (0.07 mol) 4-(2-Oxo-1,2,4,5-tetrahydro-1,3-benzdiazepin-3-yl)-piperidin-1-carbonsäure-(*R*)-1-(3-brom-4-hydroxy-benzyl)-2-[4-(4-ethoxy-carbonyl-methyl-piperazin-1-yl)-piperidin-1-yl]-2-oxo-ethylester in 5 mL THF wurde bei RT eine Lösung von 3.0 mg (0.12 mmol) LiOH in 5 mL Wasser gegeben und das Reaktionsgemisch 1 h gerührt. Das organische Lösungsmittel wurde i.vac. entfernt, der wässrige Rückstand mit 1 M HCl sauer gestellt und erneut i.vac. eingeengt. Der Rückstand wurde via HPLC gereinigt; die das Produkt enthaltenden Fraktionen wurden vereinigt und lyophilisiert.

| | |
|---|---|
| Ausbeute: | 28 mg (59% der Theorie) |
| ESI-MS: | (M+H)⁺ = 741/743 (Br) |
| Retentionszeit (HPLC-MS): | 2.5 min (Methode A) |

### Beispiel 8

### 4-(2-Oxo-1,2,4,5-tetrahydro-1,3-benzdiazepin-3-yl)-piperidin-1-carbonsäure-(R)-1-(3,5-dichlor-4-hydroxy-benzyl)-2-[4-(4-methyl-piperazin-1-yl)-piperidin-1-yl]-2-oxo-ethylester

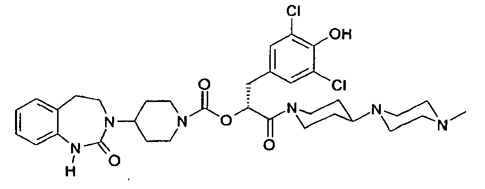

### 8a) 2,6-Dichlor-4-iod-phenol

Zu einer Lösung von 40.0 g (245 mmol) 2,6-Dichlor-phenol in 180 mL EtOH wurden Lösungen von 40.7 g (245 mmol) Natriumiodid in 160 mL Wasser und 9.6 mL (143 mmol) Ethan-1,2-diamin in 16 mL Wasser zugegeben und 15 min bei RT gerührt. Dann erfolgte die Zugabe von 62.3 g (245 mmol) Iod in kleinen Portionen zur Reaktionsmischung. Zur Vervollständigung der Reaktion wurden nach 3 h bei RT weitere 31.1 g (122 mmol) Iod und 4.8 mL (72 mmol) Ethan-1,2-diamin zugegeben und das Reaktionsgemisch über Nacht bei RT gerührt. Man versetzte mit gesättigter NaHSO₃-Lösung bis zur sauren Reaktion, extrahierte dreimal mit je 400 mL EtOAc, wusch die vereinigten organischen Phasen mit gesättigter NaCl-Lösung und trocknete über Na₂SO₄. Nach Entfernen des Trocken- und Lösungsmittels wurde der Rückstand ohne Reinigung weiter umgesetzt.

| | |
|---|---|
| Ausbeute: | 69.0 g (97% der Theorie) |
| R_{f} = | 0.5 (Kieselgel, n-Hexan/EtOAc 4:1) |

### 8b) [2-(2,6-Dichlor-4-iod-phenoxymethoxy)-ethyl]-trimethylsilan

Unter Stickstoffatmosphäre wurden zu einer Lösung von 28.0 g (96.9 mmol) 2,6-Dichlor-4-iod-phenol in 800 mL Acetonitril 33.5 g (242 mmol) K₂CO₃ und 20.7 mL (117 mmol) (2-Chlormethoxy-ethyl)-trimethylsilan gegeben und das Reaktionsgemisch 1 h unter Rückfluss erhitzt. Das Reaktionsgemisch wurde i.vac. eingeengt, der Rückstand in 300 mL Wasser aufgenommen, dreimal mit je 300 mL EtOAc extrahiert und über Na₂SO₄ getrocknet. Nach Entfernen des Trocken- und Lösungsmittels wurde der Rückstand ohne Reinigung weiter umgesetzt.

| | |
|---|---|
| Ausbeute: | 38.4 g (95% der Theorie) |
| R_{f} = | 0.83 (Kieselgel, n-Hexan/EtOAc 4:1) |

### 8c) 3,5-Dichlor-4-hydroxy-benzaldehyd

Unter Argonatmosphäre wurden zu einer auf -20°C gekühlten Lösung von 30.0 g (71.6 mmol) [2-(2,6-Dichlor-4-iod-phenoxymethoxy)-ethyl]-trimethylsilan in 200 mL THF 39.4 mL (78.8 mmol) Isopropylmagnesiumchlorid (2 M in THF), verdünnt mit 50 mL THF, langsam zugetropft. Nach beendeter Zugabe wurden bei -10°C 11.0 mL (143 mmol) DMF zugegeben und das Reaktionsgemisch langsam auf RT erwärmen lassen. Nach beendeter Reaktion (DC-Kontrolle) wurde mit 100 mL 2 M HCL versetzt und die Reaktionslösung über Nacht bei RT gerührt. Man extrahierte zweimal mit je 300 mL EtOAc, wusch die vereinigten organischen Phasen mit gesättigter NaCl-Lösung und trocknete über Na₂SO₄. Nach Entfernen des Trocken- und Lösungsmittels wurde der Rückstand durch zweimaliges Umkristallisieren aus Cyc/EtOAc (1:1) gereinigt.

| | |
|---|---|
| Ausbeute: | 12.0 g (88% der Theorie) |

### 8d) (Z,E)-3-(4-Acetoxy-3,5-dichlor-phenyl)-2-acetylamino-acrylsäure

Eine Mischung aus 12.2 g (64 mmol) 3,5-Dichlor-4-hydroxy-benzaldehyd, 11.2 g (96 mmol) N-Acetylglycin und 7.86 g (96 mmol) Natriumacetat in 50 mL Acetanhydrid wurde 3 h auf 130°C (Badtemperatur) erhitzt. Man kühlte das Reaktionsgemisch auf 90°C ab und versetzte so mit 5 mL Wasser, dass die Innentemperatur 100°C nicht überschritt. Man ließ 2 h bei 90°C rühren und versetzte mit 200 mL Wasser, wobei sich ein Niederschlag bildete. Dieser wurde abfiltriert, gut mit Wasser gewaschen und getrocknet.

| | |
|---|---|
| Ausbeute: | 12.0 g (57% der Theorie) |
| R_{f} = | 0.84 (Kieselgel, Cyc/EtOAc 1:1) |

### 8e) 3-(3,5-Dichlor-4-hydroxy-phenyl)-2-oxo-propionsäure

Zu einer Lösung von 12.0 g (36.1 mmol) (*Z,E*)-3-(4-Acetoxy-3,5-dichlor-phenyl)-2-acetylamino-acrylsäure in 70 mL NMP wurden bei RT 131 mL 4 M HCl gegeben und das Reaktionsgemisch 4 h auf 130°C erhitzt (Badtemperatur). Nach Abkühlen auf 0°C wurden 200 mL Wasser zugegeben und über Nacht gerührt, wobei sich ein Niederschlag bildete. Dieser wurde abfiltriert und getrocknet (2.9 g; Reinheit 95%). Das Filtrat wurde dreimal mit je 300 mL Cyc/EtOAc (1:3) extrahiert, die vereinigten organischen Phasen getrocknet und i.vac. eingeengt. Nach Entfernen des Trocken- und Lösungsmittels wurden 4 g des Produktes (Reinheit: 80%) erhalten

| | |
|---|---|
| Ausbeute: | 6.0 g (66% der Theorie) |
| ESI-MS: | (M+H)⁺ = 250/252/254 (2Cl) |
| R_{f} = | 0.30 (Kieselgel, DCM/MeOH/AcOH 90:10:1) |

### 8f) (R)-3-(3,5-Dichlor-4-hydroxy-phenyl)-2-hydroxy-propionsäure

Hergestellt analog Beispiel 7c aus 3.00 g (10.0 mmol) 3-(3,5-Dichlor-4-hydroxy-phenyl)-2-oxo-propionsäure (Reinheit: 80%) und 6.34 g (20.0 mmol) (1*R*)-B-Chlordiisopino-campheylboran. Das Rohprodukt (1.9 g) wurde ohne Reinigung weiter umgesetzt.

### 8g) (R)-3-(3,5-Dichlor-4-hydroxy-phenyl)-2-hydroxy-propionsäuremethylester

Zu einer auf 0°C gekühlten Lösung des Rohproduktes aus Beispiel 8f in 30 mL MeOH wurden 2 mL konzentrierte Schwefelsäure gegeben und das Reaktionsgemisch 2 h bei dieser Temperatur gerührt. Man neutralisierte mit festem KHCO₃, verdünnte mit Wasser und extrahierte erschöpfend mit EtOAc. Die vereinigten organischen Phasen wurden mit gesättigter NaCl-Lösung gewaschen und über Na₂SO₄ getrocknet. Nach Entfernen des Trocken- und Lösungsmittels wurde das Produkt ohne Reinigung weiter umgesetzt.

| | |
|---|---|
| Ausbeute: | 1.8 g (75% der Theorie über 2 Stufen) |

### 8h) (R)-3-[3,5-Dichlor-4-(2-trimethylsilanyl-ethoxymethoxy)-phenyl]-2-hydroxy-propionsäuremethylester

Unter Stickstoffatmosphäre wurden zu einer Lösung von 2.64 g (9.96 mmol) (R)-3-(3,5-Dichlor-4-hydroxy-phenyl)-2-hydroxy-propionsäuremethylester in 20 mL Acetonitril 5.79 g (76.6 mmol) 40% KF/Al₂O₃ gegeben und die resultierende Suspension für einige min bei RT gerührt. Anschließend wurde eine Lösung von 2.12 mL (12.0 mmol) (2-Chlormethoxy-ethyl)-trimethylsilan in 20 mL Acetonitril zugegeben und das Reaktionsgemisch 20 h bei RT gerührt. Man filtrierte über Celite und engte das Lösungsmittel i.vac. ein. Der Rückstand wurde ohne Reinigung weiter umgesetzt.

| | |
|---|---|
| Ausbeute: | 3.80 g (97% der Theorie) |

### 8i) 4-(2-Oxo-1,2,4,5-tetrahydro-1,3-benzdiazepin-3-yl)-piperidin-1-carbonsäure-(R)-2-[3,5-dichlor-4-(2-trimethylsilanyl-ethoxymethoxy)-phenyl]-1-methoxycarbonyl-ethylester

Hergestellt analog Beispiel 6f aus 3.80 g (8.65 mmol) (R)-3-[3,5-Dichlor-4-(2-trimethyl-silanyl-ethoxymethoxy)-phenyl]-2-hydroxy-propionsäuremethylester und 2.12 g (8.65 mmol) 3-Piperidin-4-yl-1,3,4,5-tetrahydro-1,3-benzdiazepin-2-on. Das Produkt wurde chromatographisch (Kieselgel, DCM/MeOH 98:2) gereinigt.

| | |
|---|---|
| Ausbeute: | 3.10 g (54% der Theorie) |

### 8k) 4-(2-Oxo-1,2,4,5-tetrahydro-1,3-benzdiazepin-3-yl)-piperidin-1-carbonsäure-(R)-2-(3,5-dichlor-4-hydroxy-phenyl)-1-methoxycarbonyl-ethylester

Hergestellt analog Beispiel 6g aus 3.10 g (4.65 mmol) 4-(2-Oxo-1,2,4,5-tetrahydro-1,3-benzdiazepin-3-yl)-piperidin-1-carbonsäure-(*R*)-2-[3,5-dichlor-4-(2-trimethyl-silanyl-ethoxy-methoxy)-phenyl]-1-methoxycarbonyl-ethylester und 4.48 mL methanolischer Schwefelsäure (0.5 M).

| | |
|---|---|
| Ausbeute: | 2.49 g (100% der Theorie) |

### 8l) 4-(2-Oxo-1,2,4,5-tetrahydro-1,3-benzdiazepin-3-yl)-piperidin-1-carbonsäure-(R)-1-carboxy-2-(3,5-dichlor-4-hydroxy-phenyl)-ethylester

Zu einer Lösung von 2.49 g (4.64 mmol) 4-(2-Oxo-1,2,4,5-tetrahydro-1,3-benzdiazepin-3-yl)-piperidin-1-carbonsäure-(R)-2-(3,5-dichlor-4-hydroxy-phenyl)-1-methoxycarbonyl-ethylester in 50 mL THF wurde eine Lösung von 0.42 g (17.3 mmol) LiOH in 20 mL Wasser gegeben und das Reaktionsgemisch bei RT 1 h gerührt. Das organische Lösungsmittel wurde i.vac. entfernt, die wässrige Phase mit EtOAc gewaschen, mit 10% HCI angesäuert, erschöpfend mit EtOAc extrahiert, die vereinigten organischen Phasen mit gesättigter NaCI-Lösung gewaschen und über Na₂SO₄ getrocknet. Nach Entfernen des Trocken- und Lösungsmittels wurde der Rückstand mit Diethylether verrieben, abgesaugt und getrocknet.

| | |
|---|---|
| Ausbeute: | 1.60 g (66% der Theorie) |
| R_{f} = | 0.05 (Kieselgel, DCM/MeOH 9:1) |

### 8m) 4-(2-Oxo-1,2,4,5-tetrahydro-1,3-benzdiazepin-3-yl)-piperidin-1-carbonsäure-(R)-1-(3,5-dichlor-4-hydroxy-benzyl)-2-[4-(4-methyl-piperazin-1-yl)-piperidin-1-yl]-2-oxo-ethylester

Unter Stickstoffatmosphäre wurde eine Mischung aus 150 mg (0.29 mmol) 4-(2-Oxo-1,2,4,5-tetrahydro-1,3-benzdiazepin-3-yl)-piperidin-1-carbonsäure-(*R*)-1-carboxy-2-(3,5-dichlor-4-hydroxy-phenyl)-ethylester, 63.8 mg (0.34 mmol) 1-Methyl-4-piperidin-4-yl-piperazin und 0.17 mL (0.98 mmol) Ethyldiisopropylamin in 10 mL DMF bei RT 5 min gerührt. Dann erfolgte die Zugabe von 124 mg (0.32 mmol) HATU zum Reaktionsgemisch, welches anschließend 4 h gerührt wurde. Dieses wurde i.vac. eingeengt und der Rückstand chromatographisch (Kieselgel, Gradient DCM zu DCM/MeOH 85:15) gereinigt. Die das Produkt enthaltenden Fraktionen wurden vereinigt, i.vac. eingeengt und über Aluminiumoxid filtriert, wobei das Produkt mit DCM/MeOH 9:1 eluiert wurde. Man engte i.vac. ein, verrieb den Rückstand mit Diethylether, filtrierte und trocknete das Produkt.

| | |
|---|---|
| Ausbeute: | 43 mg (22% der Theorie) |
| ESI-MS: | (M+H)⁺ = 687/689 (2CI) |
| Retentionszeit (HPLC-MS): | 2.8 min (Methode A) |

Analog wurden folgende Verbindungen aus jeweils 150 mg 4-(2-Oxo-1,2,4,5-tetrahydro-1,3-benzdiazepin-3-yl)-piperidin-1-carbonsäure-(*R*)-1-carboxy-2-(3,5-dichlor-4-hydroxyphenyl)-ethylester und der entsprechenden Menge an Amin hergestellt:

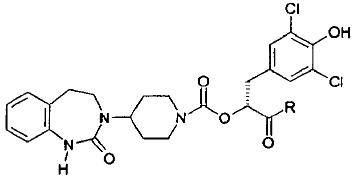

| **Beispiel** | **R** | **Ausbeute (%)** | **Massen-spektrum** | **Retentionszeit HPLC (Methode)** |
|---|---|---|---|---|
| **8.1** | 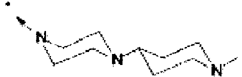 | 21 | 687/689 | 2.6 |
| | | | [M+H]⁺ | (A) |
| **8.2** | 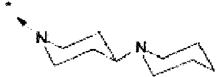 | 39 | 672/74 | 3.1 |
| | | | [M+H]⁺ | (A) |
| **8.3** | 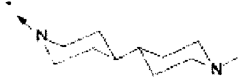 | 38 | 686/688 | 3.1 |
| | | | [M+H]⁺ | (A) |

### Beispiel 8.4

### 4-(2-Oxo-1,2,4,5-tetrahydro-1,3-benzdiazepin-3-yl)-piperidin-1-carbonsäure-(R)-1-(3,5-di-chlor-4-hydroxy-benzyl)-2-oxo-2-(4-piperidin-4-yl-piperazin-1-yl)-ethylester

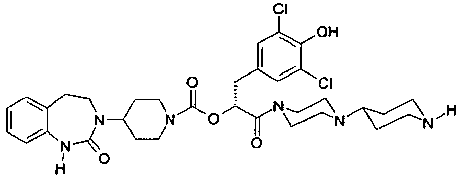

Unter Stickstoffatmosphäre wurde eine Mischung aus 150 mg (0.29 mmol) 4-(2-Oxo-1,2,4,5-tetrahydro-1,3-benzdiazepin-3-yl)-piperidin-1-carbonsäure-(*R*)-1-carboxy-2-(3,5-dichlor-4-hydroxy-phenyl)-ethylester, 105 mg (0.34 mmol) 4-Piperazin-1-yl-piperidin-1-carbonsäure-*tert*-butylester (eingesetzt als Hydrochlorid-Salz) und 0.17 mL (0.98 mmol) Ethyldiisopropylamin in 10 mL DMF bei RT 5 min gerührt. Dann erfolgte die Zugabe von 124 mg (0.32 mmol) HATU zum Reaktionsgemisch, welches anschließend 4 h gerührt wurde. Dieses wurde i.vac. eingeengt und der Rückstand chromatographisch (Kieselgel, Gradient DCM zu DCM/MeOH 85:15) gereinigt. Die das Produkt enthaltenden Fraktionen wurden vereinigt und i.vac. eingeengt. Der Rückstand wurde in 10 mL Ameisensäure aufgenommen und 3 h bei RT gerührt. Man engte i.vac. ein, nahm den Rückstand in gesättigter NH₃-Lösung auf, extrahierte erschöpfend mit EtOAc, wusch die vereinigten organischen Phasen mit gesättigter NaCI-Lösung und trocknete über Na₂SO₄. Nach Entfernen des Trocken- und Lösungsmittels wurde der Rückstand mit Diethylether verrieben, abgesaugt und getrocknet.

| | |
|---|---|
| Ausbeute: | 47 mg (24% der Theorie) |
| ESI-MS: | (M+H)⁺ = 673/675 (2Cl) |
| Retentionszeit (HPLC-MS): | 2.6 min (Methode A) |

### Beispiel 8.5

### 4-(2-Oxo-1,2,4,5-tetrahydro-1,3-benzdiazepin-3-yl)-piperidin-1-carbonsäure-(R)-2-4,4'-bipiperidinyl-1-yl-1-(3,5-dichlor-4-hydroxy-benzyl)-2-oxo-ethylester

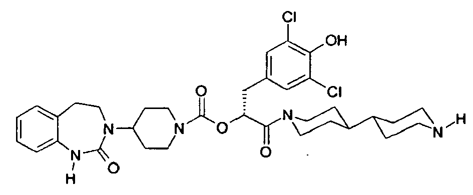

Hergestellt analog Beispiel 8.4 aus 150 mg (0.29 mmol) 4-(2-Oxo-1,2,4,5-tetrahydro-1,3-benzdiazepin-3-yl)-piperidin-1-carbonsäure-(*R*)-1-carboxy-2-(3,5-dichlor-4-hydroxy-phenyl)-ethylester und 92 mg (0.34 mmol) [4,4']Bipiperidinyl-1-carbonsäure-tert-butylester.

| | |
|---|---|
| Ausbeute: | 84 mg (44% der Theorie) |
| ESI-MS: | (M+H)⁺ = 672/674 (2Cl) |
| Retentionszeit (HPLC-MS): | 3.1 min (Methode A) |

## Patentansprüche

1. CGRP-Antagonisten der allgemeinen Formel **I** 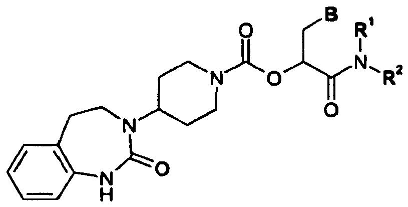 in der
**B** eine Gruppe ausgewählt aus 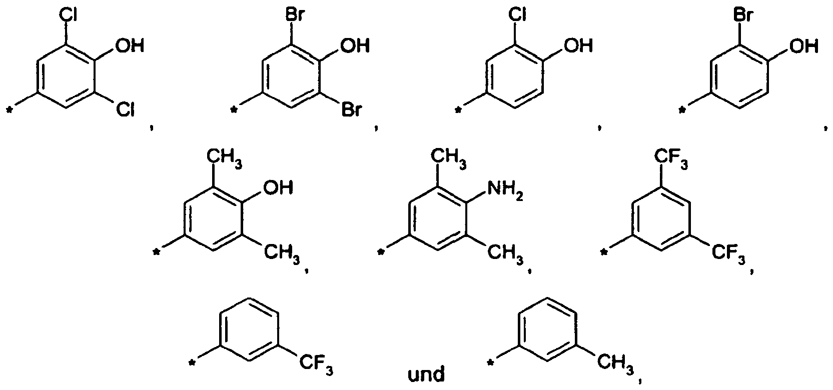
**R¹** und **R²** zusammen mit dem eingeschlossenen Stickstoffatom einen Rest der allgemeinen Formel **II** 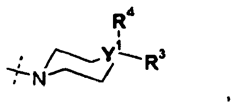
in der
**Y¹** das Kohlenstoffatom oder, wenn **R⁴** ein freies Elektronenpaar darstellt, auch das Stickstoffatom,
**R³** eine Cyclopentyl-, Cyclohexyl- oder Cycloheptylgruppe oder
**R³** einen Heterocyclus ausgewählt aus einer Morpholin-4-yl-, 1,1-Dioxo-thiomorholin-4-yl-, Piperidin-1-yl-, Piperidin-4-yl-, Piperazin-1-yl- oder Pyrrolidin-1-ylgruppe darstellt,
wobei die vorstehend genannten monocyclischen Heterocyclen im Ring ein- oder zweifach durch Hydroxy-, Methyl-, Ethyl-, Trifluormethyl-, Hydroxymethyl- oder Hydroxyethylgruppen, oder, gegebenenfalls zusätzlich, einfach durch eine Hydroxycyclopropyl-, Trifluormethylcarbonylmethyl-, Amino-, Carboxy-carbonyl-, Methoxycarbonyl-, Ethoxycarbonyl-carbonyl-, Carboxymethyl-, Carboxyethyl-, Ethoxycarbonylmethyl-, Ethoxycarbonylethyl-, Carboxy-ethylcarbonyl-, Ethoxycarbonyl-ethylcarbonyl-, Aminosulfonyl-, Methylaminosulfonyl-, Dimethylaminosulfonyl-, Methylsulfonyl-, Ethylsulfonyl-, Isopropylsulfonyl-, Cyclopropylsulfonyl-, (Hydroxyamino)-carbonylmethyl-, Hydroxy-(methyl)-aminocarbonyl-methyl- oder Methoxyaminocarbonyl-methylgruppe substituiert sein können, wobei die Substituenten gleich oder verschieden und an ein Ring-Kohlenstoff- oder RingStickstoffatom gebunden sein können, oder
die vorstehend genannten monocyclischen Heterocyclen im Ring einfach durch eine Carboxygruppe substituiert sein können, wenn diese Carboxygruppe nicht über ein Stickstoffatom gebunden ist, und
**R⁴** das Wasserstoffatom, wenn **Y¹** das Kohlenstoffatom darstellt, oder
**R⁴** ein freies Elektronenpaar, wenn **Y¹** das Stickstoffatom darstellt, bedeuten,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen, mit der Maßgabe, dass die folgenden Verbindungen, deren Tautomere, deren Diastereomere, deren Enantiomere, deren Hydrate, deren Gemische und deren Salze sowie die Hydrate der Salze vom Anspruchsumfang ausgeschlossen sind:
| **Nr.** | **Struktur** |
|---|---|
| (1) | 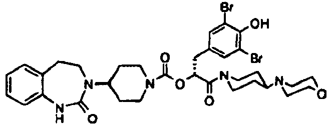 |
| (2) | 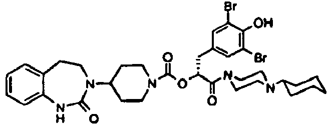 |
| (3) | 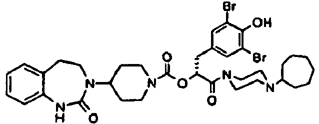 |
| (4) | 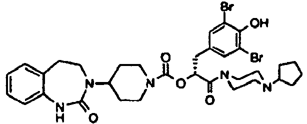 |
| (5) | 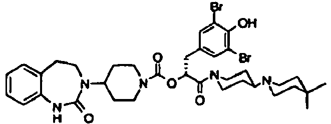 |
| (6) | 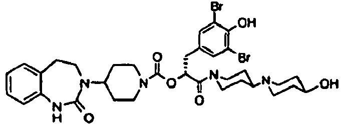 |
| (7) | 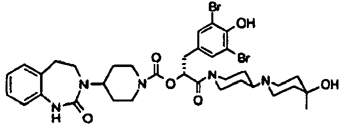 |
| (8) | 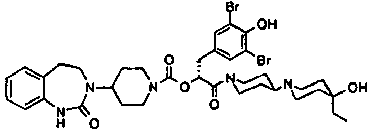 |
| (9) | 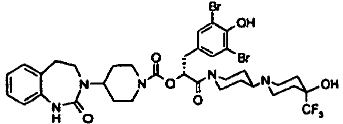 |
| (10) | 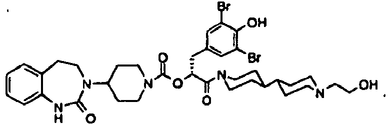 |
| (11) | 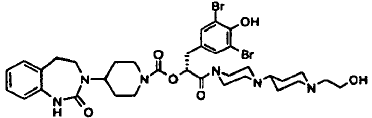 |
| (12) | 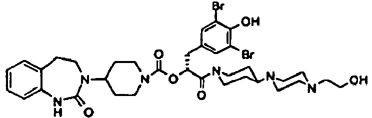 |
| (13) | 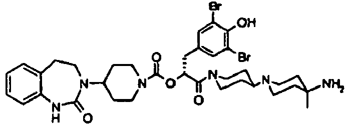 |
| (14) | 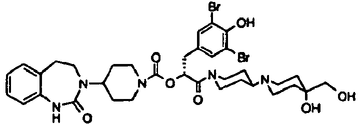 |
| (15) | 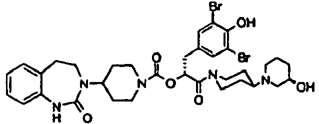 |
| (16) | 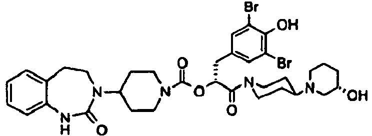 |
| (17) | 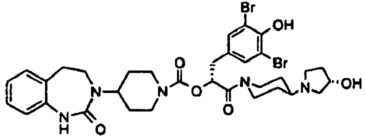 |
| (18) | 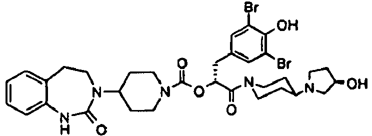 |
| (19) | 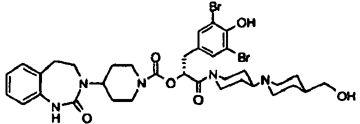 |
| (20) | 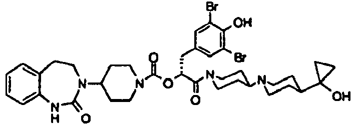 |
| (21) | 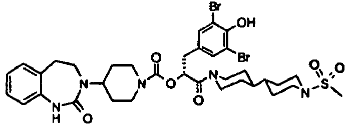 |
| (22) | 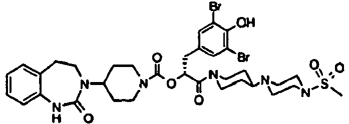 |
| (23) | 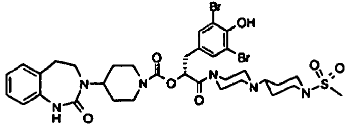 |
| (24) | 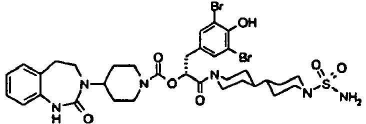 |
| (25) | 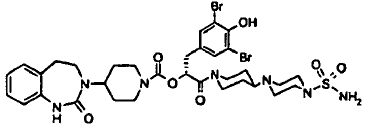 |
| (26) | 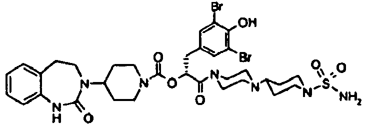 |
| (27) | 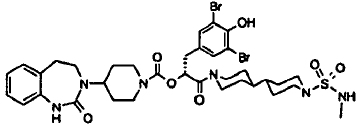 |
| (28) | 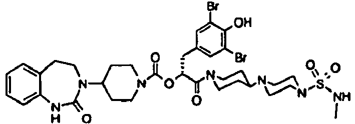 |
| (29) | 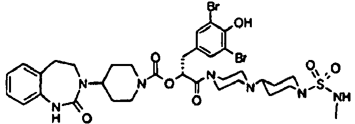 |
| (30) | 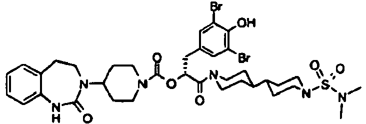 |
| (31) | 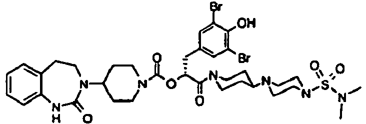 |
| (32) | 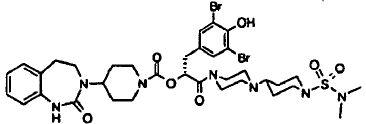 |
| (33) | 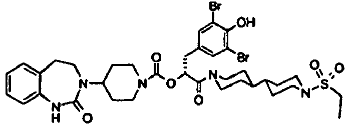 |
| (34) | 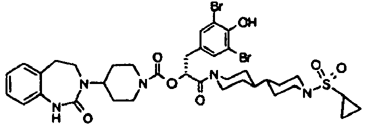 |
| (35) | 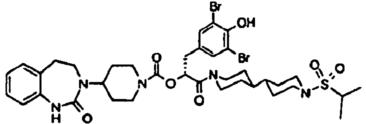 |
| (36) | 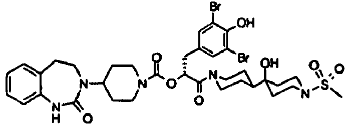 |
| (37) | 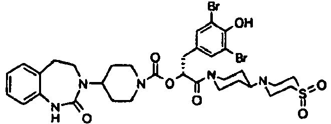 |
| (38) | 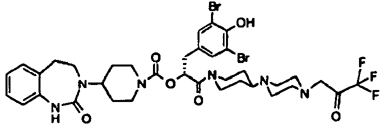 |
| (39) | 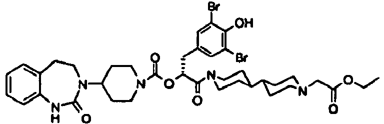 |
| (40) | 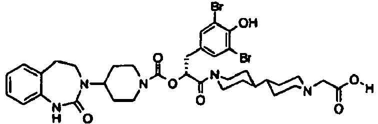 |
| (41) | 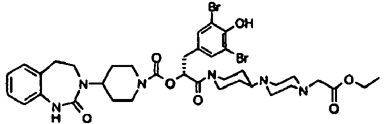 |
| (42) | 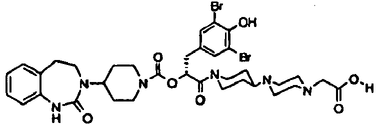 |
| (43) | 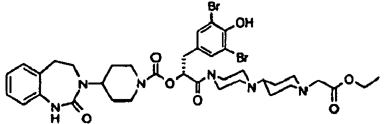 |
| (44) | 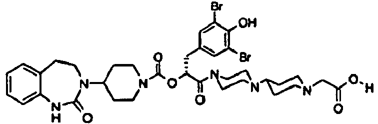 |
| (45) | 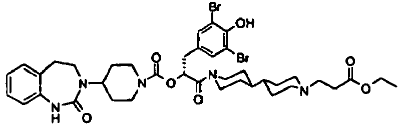 |
| (46) | 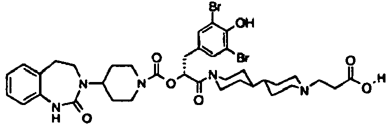 |
| (47) | 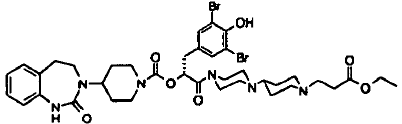 |
| (48) | 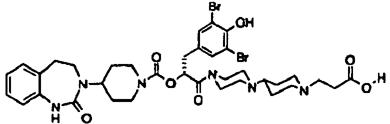 |
| (49) | 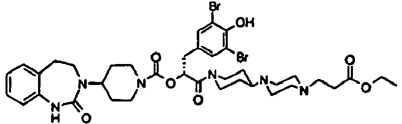 |
| (50) | 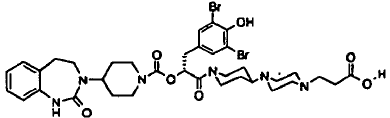 |
| (51) | 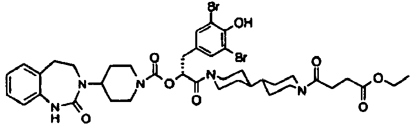 |
| (52) | 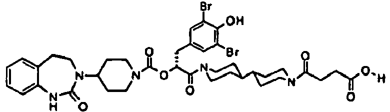 |
| (53) | 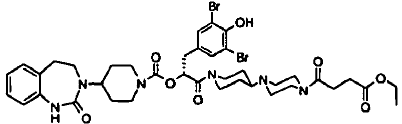 |
| (54) | 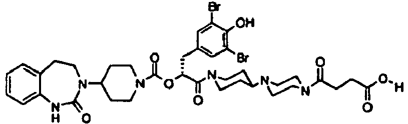 |
| (55) | 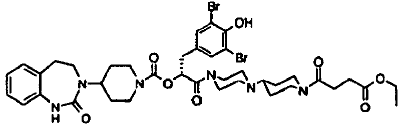 |
| (56) | 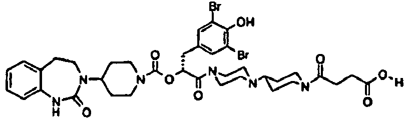 |
| (57) | 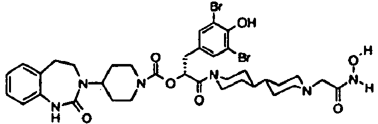 |
| (58) | 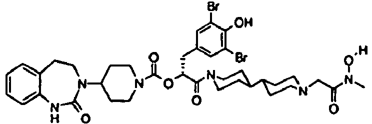 |
| (59) | 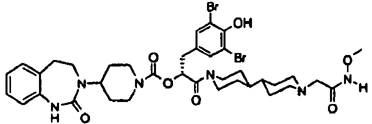 |
| (60) | 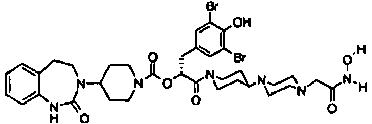 |
| (61) | 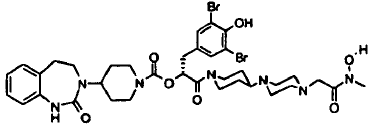 |
| (62) | 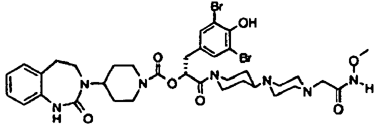 |
| (63) | 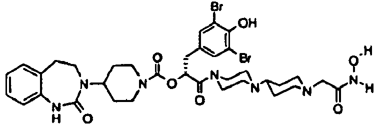 |
| (64) | 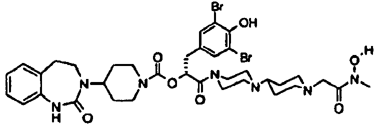 |
| (65) | 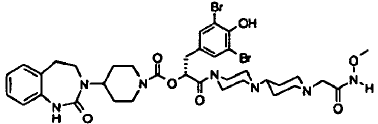 |
| (66) | 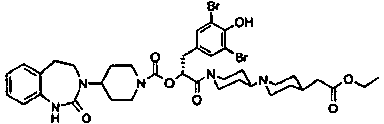 |
| (67) | 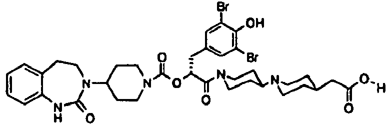 |
| (68) | 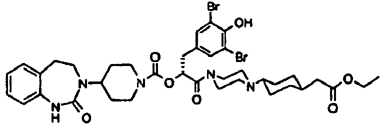 |
| (69) | 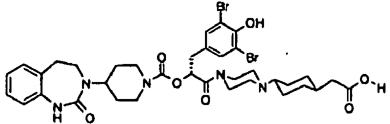 |
| (70) | 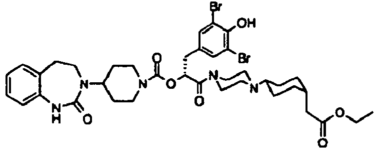 |
| (71) | 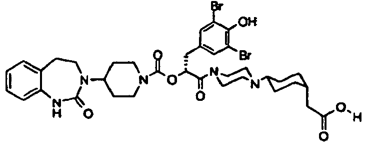 |
| (72) | 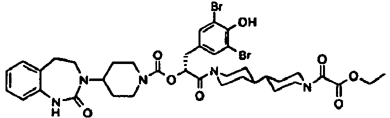 |
| (73) | 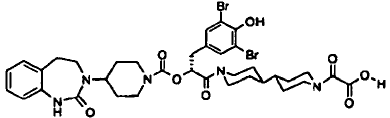 |
| (74) | 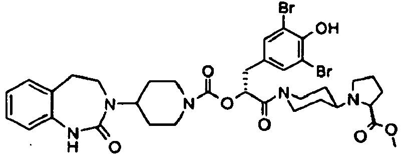 |
| (75) | 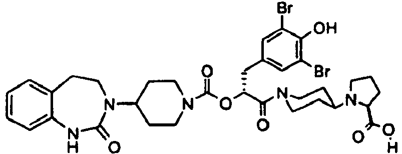 |
| (76) | 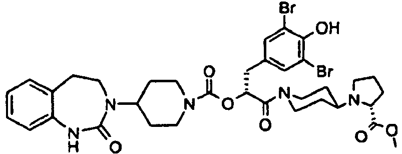 |
| (77) | 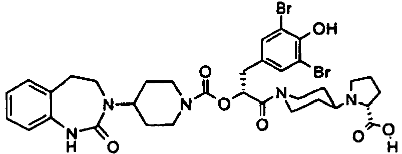 |
| (78) | 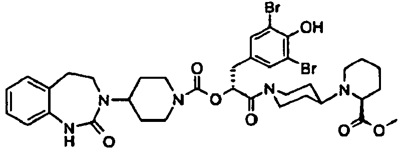 |
| (79) | 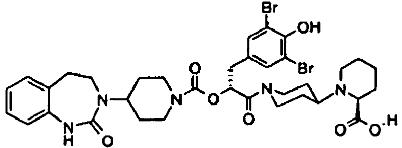 |
| (80) | 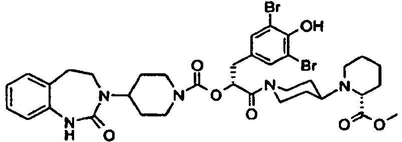 |
| (81) | 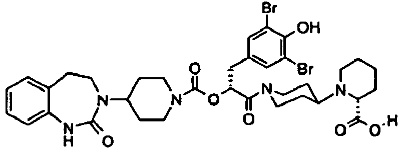 |
| (82) | 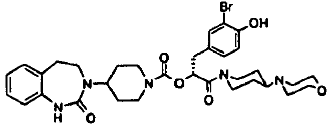 |
| (83) | 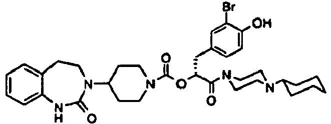 |
| (84) | 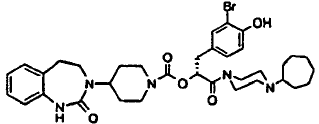 |
| (85) | 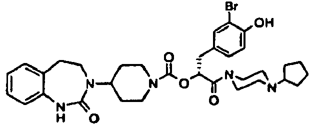 |
| (86) | 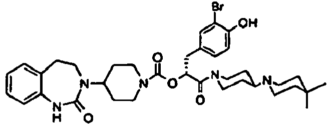 |
| (87) | 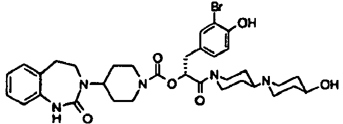 |
| (88) | 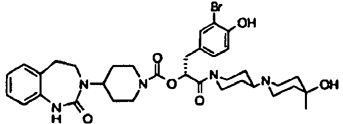 |
| (89) | 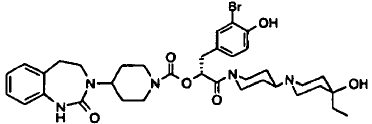 |
| (90) | 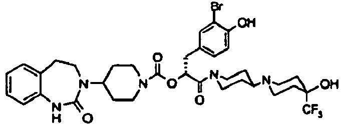 |
| (91) | 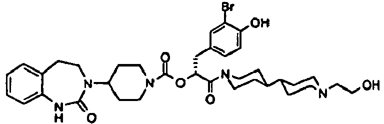 |
| (92) | 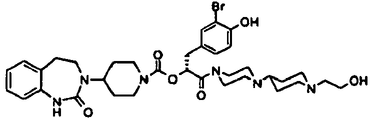 |
| (93) | 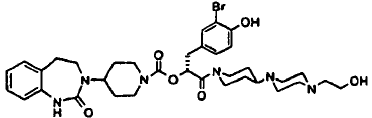 |
| (94) | 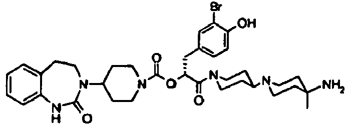 |
| (95) | 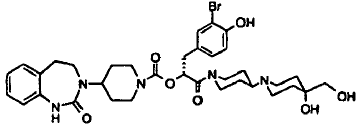 |
| (96) | 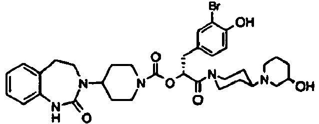 |
| (97) | 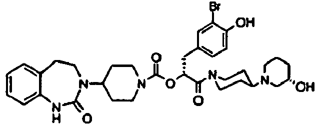 |
| (98) | 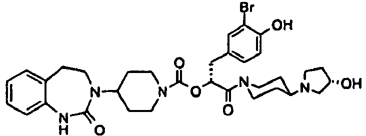 |
| (99) | 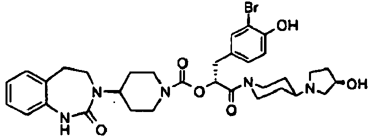 |
| (100) | 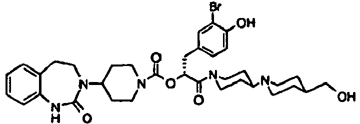 |
| (101) | 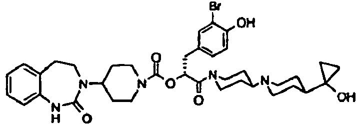 |
| (102) | 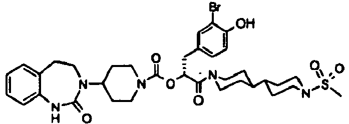 |
| (103) | 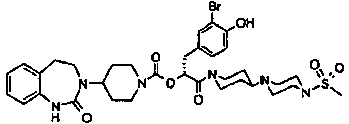 |
| (104) | 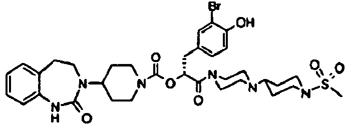 |
| (105) | 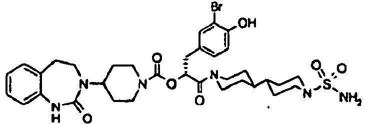 |
| (106) | 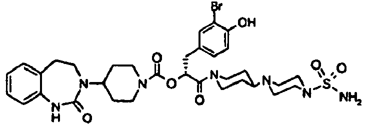 |
| (107) | 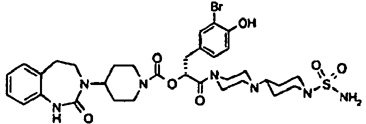 |
| (108) | 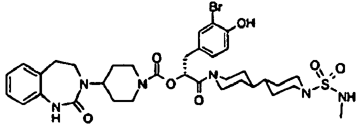 |
| (109) | 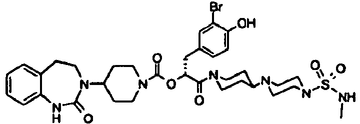 |
| (110) | 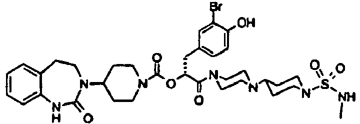 |
| (111) | 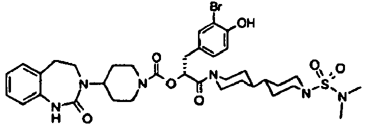 |
| (112) | 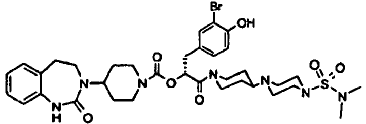 |
| (113) | 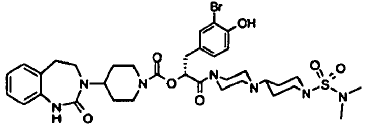 |
| (114) | 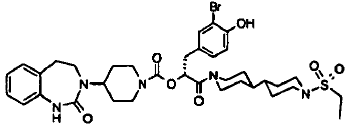 |
| (115) | 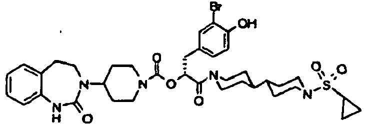 |
| (116) | 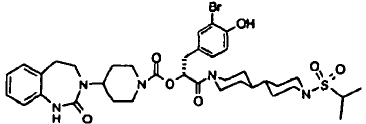 |
| (117) | 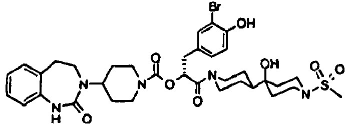 |
| (118) | 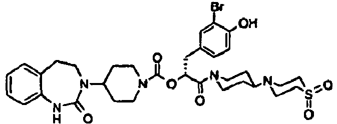 |
| (119) | 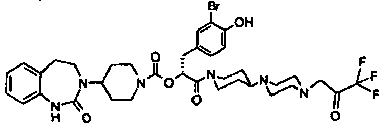 |
| (120) | 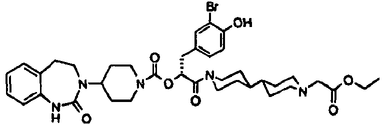 |
| (121) | 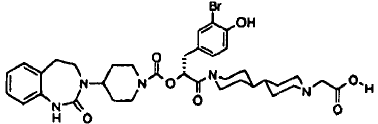 |
| (122) | 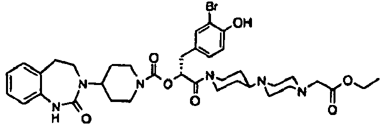 |
| (123) | 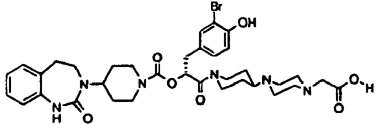 |
| (124) | 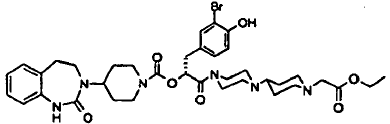 |
| (125) | 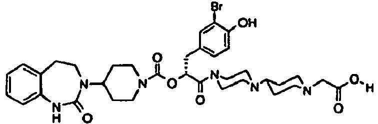 |
| (126) | 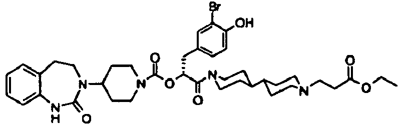 |
| (127) | 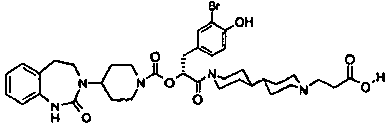 |
| (128) | 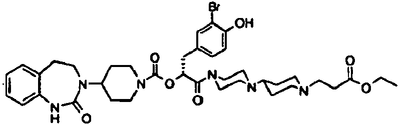 |
| (129) | 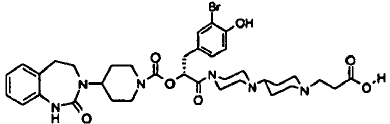 |
| (130) | 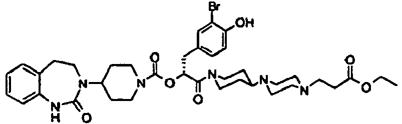 |
| (131) | 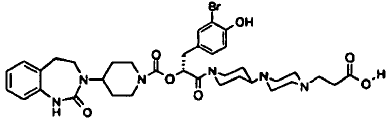 |
| (132) | 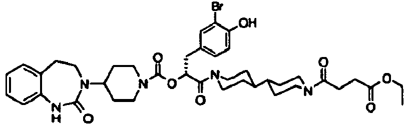 |
| (133) | 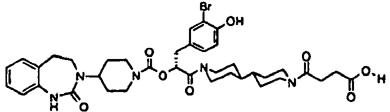 |
| (134) | 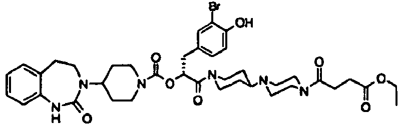 |
| (135) | 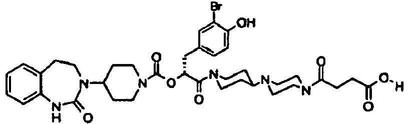 |
| (136) | 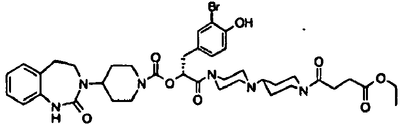 |
| (137) | 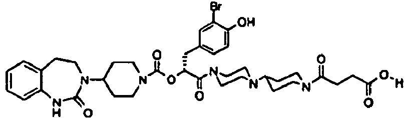 |
| (138) | 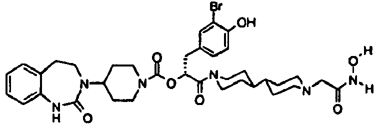 |
| (139) | 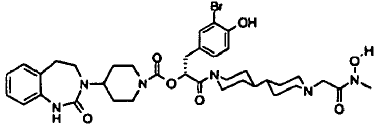 |
| (140) | 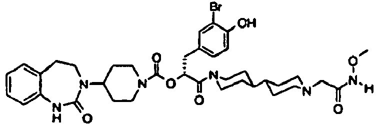 |
| (141) | 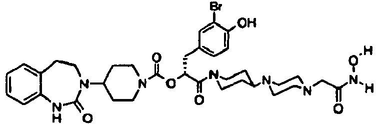 |
| (142) | 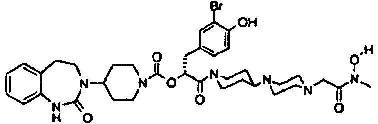 |
| (143) | 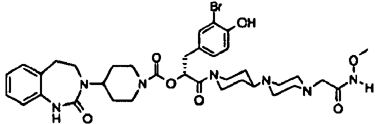 |
| (144) | 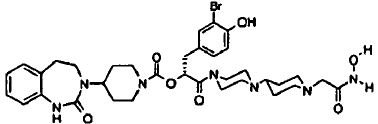 |
| (145) | 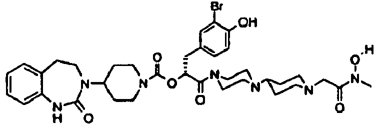 |
| (146) | 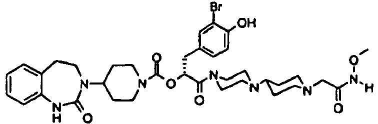 |
| (147) | 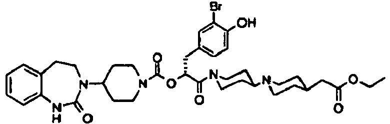 |
| (148) | 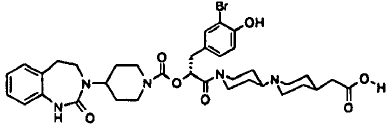 |
| (149) | 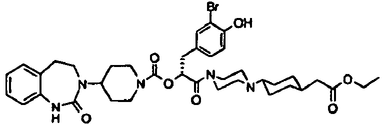 |
| (150) | 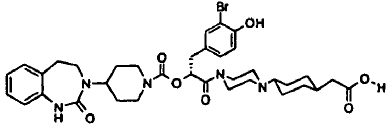 |
| (151) | 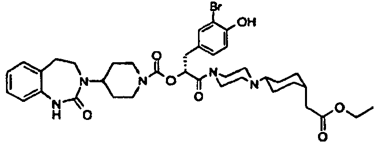 |
| (152) | 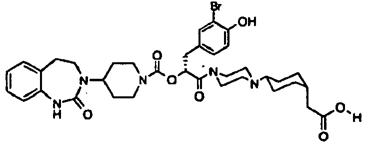 |
| (153) | 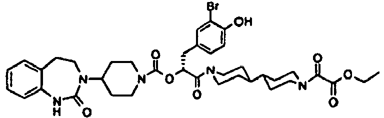 |
| (154) | 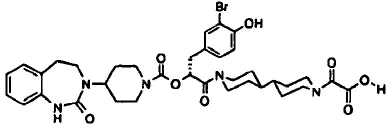 |
| (155) | 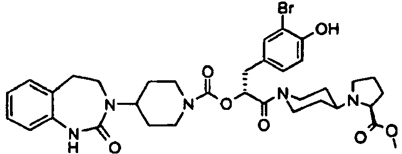 |
| (156) | 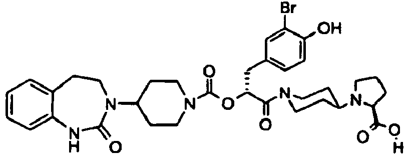 |
| (157) | 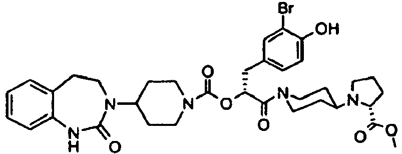 |
| (158) | 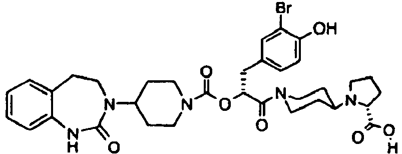 |
| (159) | 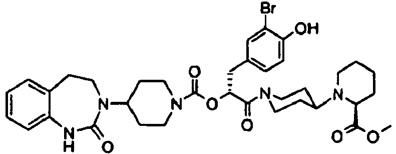 |
| (160) | 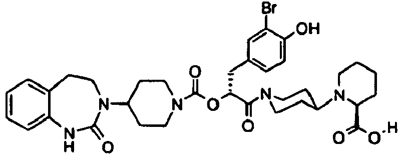 |
| (161) | 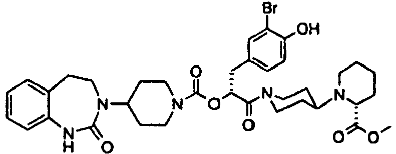 |
| (162) | 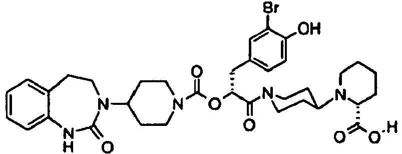 |
| (163) | 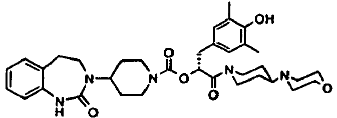 |
| (164) | 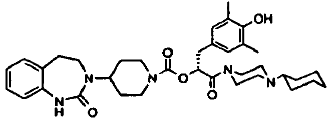 |
| (165) | 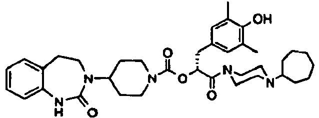 |
| (166) | 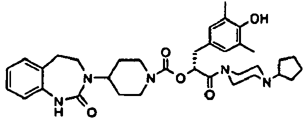 |
| (167) | 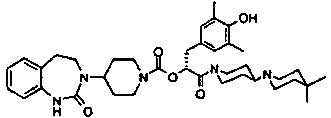 |
| (168) | 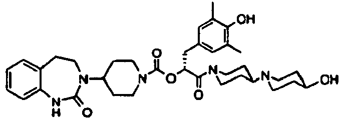 |
| (169) | 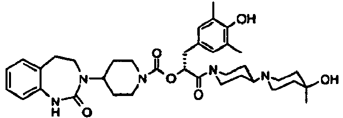 |
| (170) | 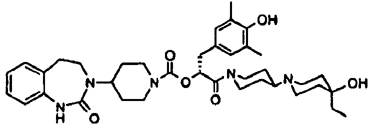 |
| (171) | 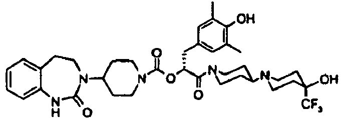 |
| (172) | 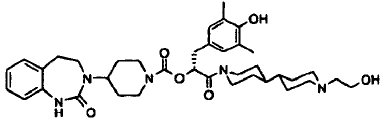 |
| (173) | 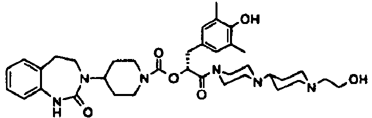 |
| (174) | 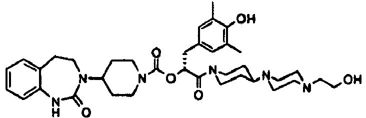 |
| (175) | 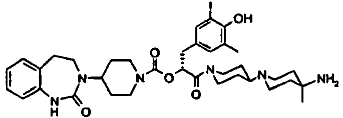 |
| (176) | 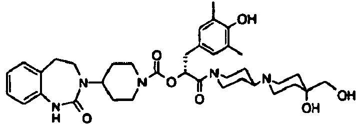 |
| (177) | 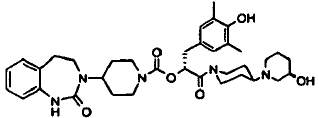 |
| (178) | 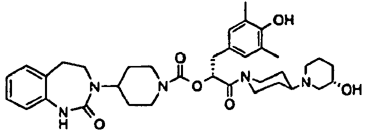 |
| (179) | 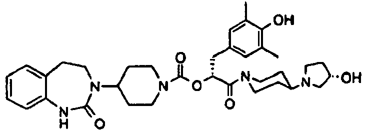 |
| (180) | 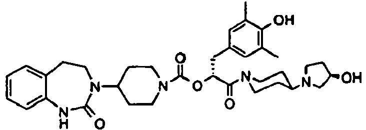 |
| (181) | 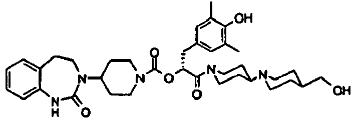 |
| (182) | 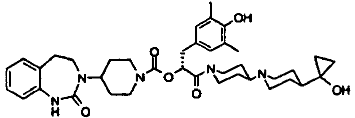 |
| (183) | 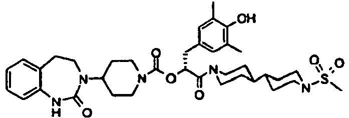 |
| (184) | 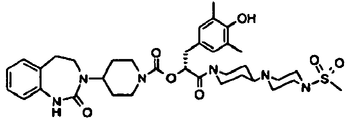 |
| (185) | 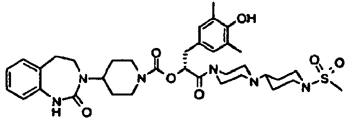 |
| (186) | 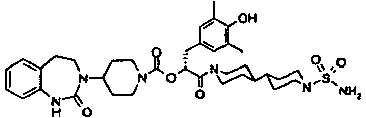 |
| (187) | 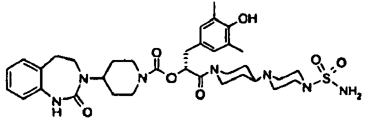 |
| (188) | 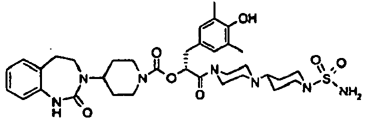 |
| (189) | 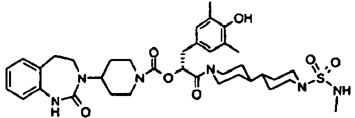 |
| (190) | 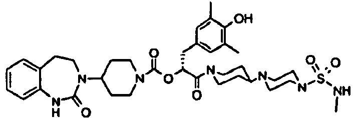 |
| (191) | 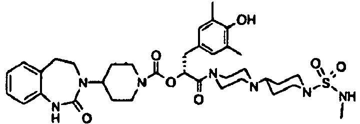 |
| (192) | 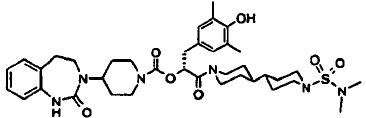 |
| (193) | 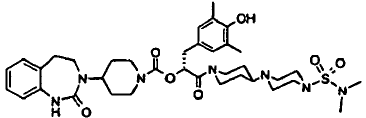 |
| (194) | 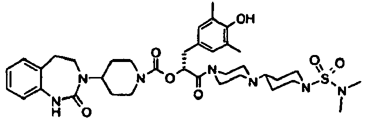 |
| (195) | 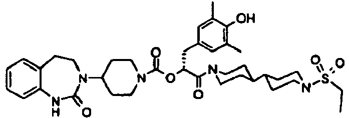 |
| (196) | 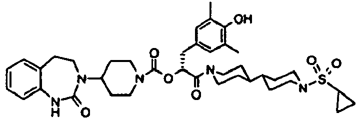 |
| (197) | 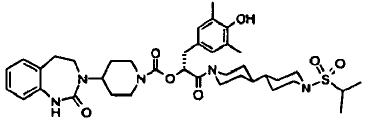 |
| (198) | 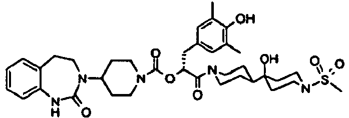 |
| (199) | 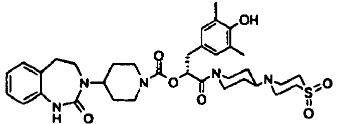 |
| (200) | 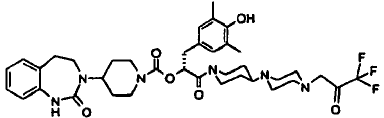 |
| (201) | 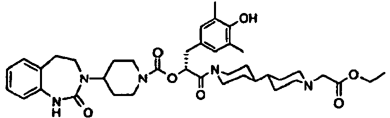 |
| (202) | 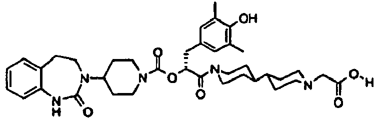 |
| (203) | 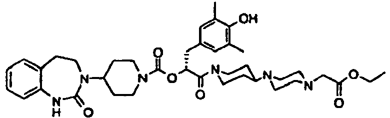 |
| (204) | 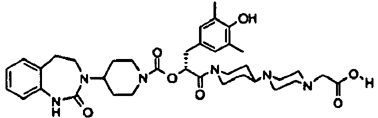 |
| (205) | 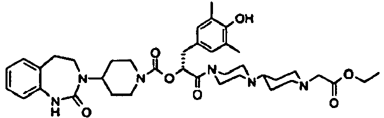 |
| (206) | 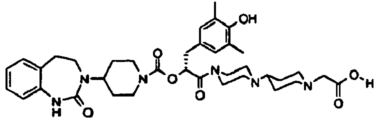 |
| (207) | 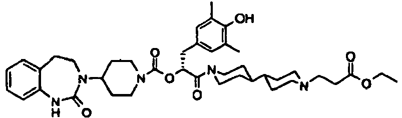 |
| (208) | 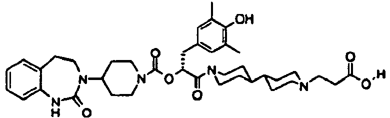 |
| (209) | 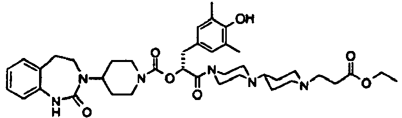 |
| (210) | 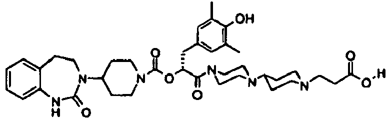 |
| (211) | 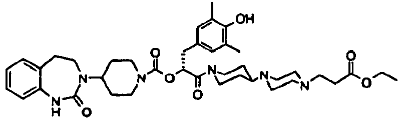 |
| (212) | 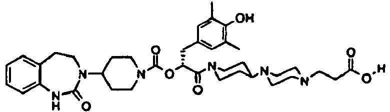 |
| (213) | 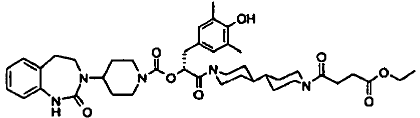 |
| (214) | 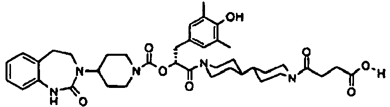 |
| (215) | 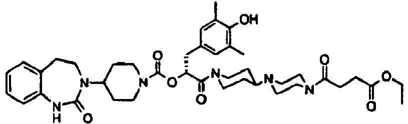 |
| (216) | 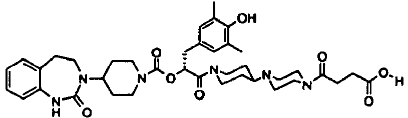 |
| (217) | 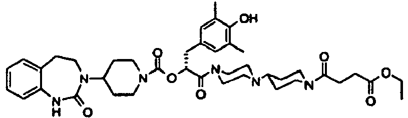 |
| (218) | 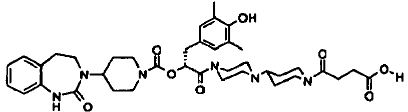 |
| (219) | 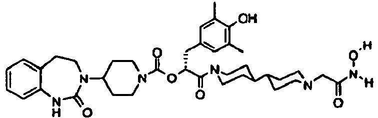 |
| (220) | 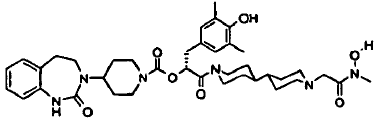 |
| (221) | 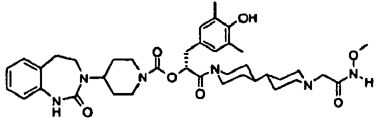 |
| (222) | 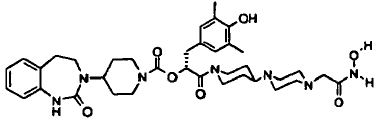 |
| (223) | 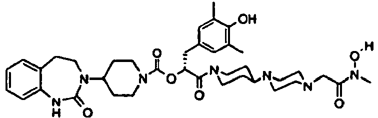 |
| (224) | 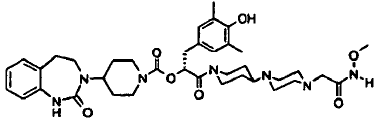 |
| (225) | 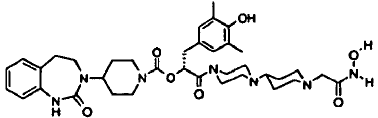 |
| (226) | 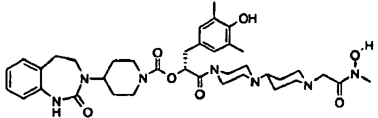 |
| (227) | 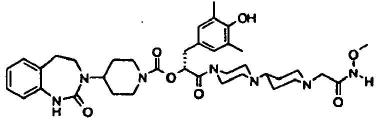 |
| (228) | 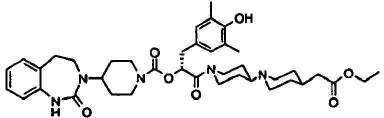 |
| (229) | 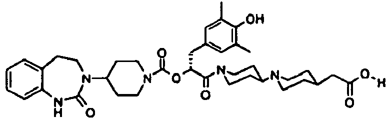 |
| (230) | 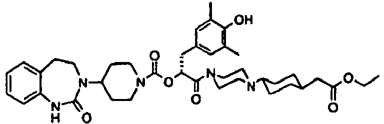 |
| (231) | 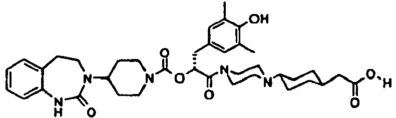 |
| (232) | 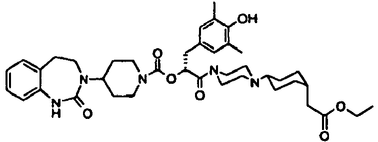 |
| (233) | 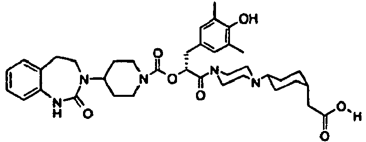 |
| (234) | 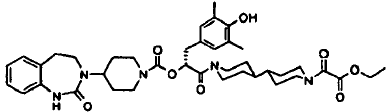 |
| (235) | 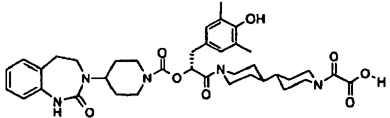 |
| (236) | 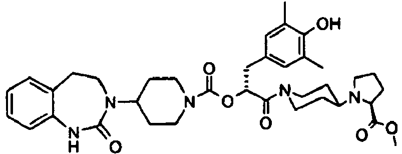 |
| (237) | 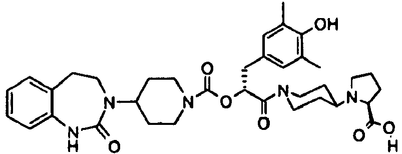 |
| (238) | 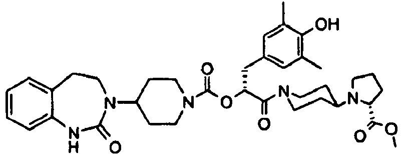 |
| (239) | 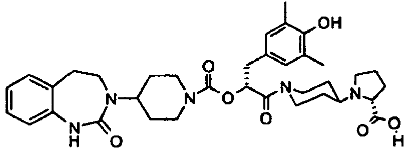 |
| (240) | 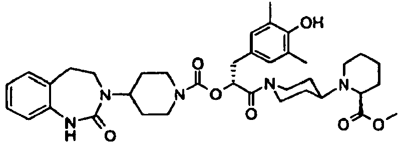 |
| (241) | 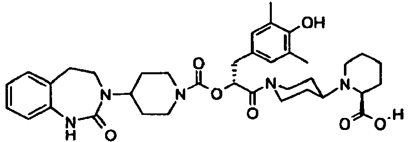 |
| (242) | 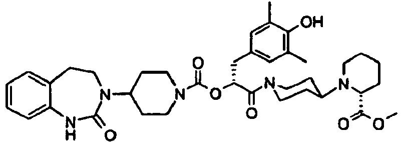 |
| (243) | 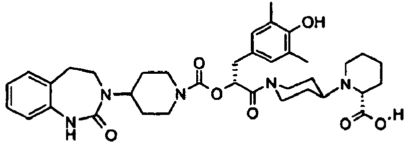 |
| (244) | 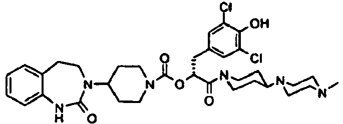 |
| (245) | 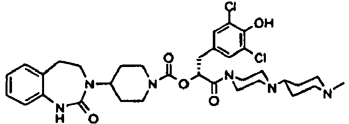 |
| (246) | 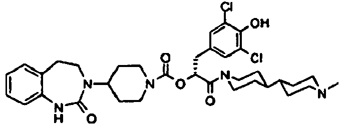 |
| (247) | 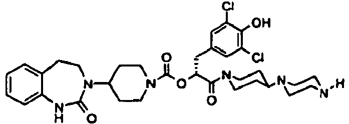 |
| (248) | 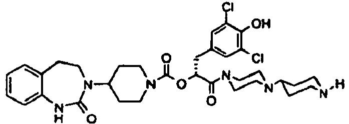 |
| (249) | 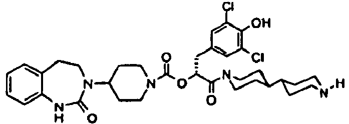 |
| (250) | 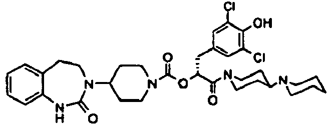 |
| (251) | 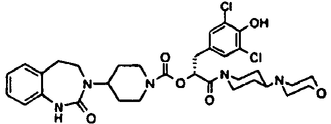 |
| (252) | 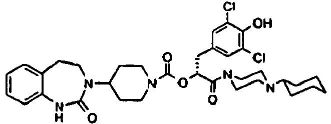 |
| (253) | 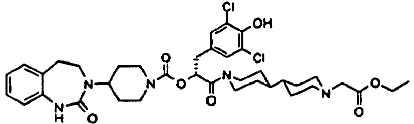 |
| (254) | 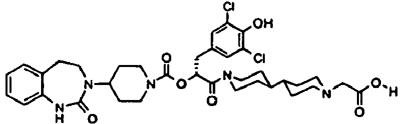 |
| (255) | 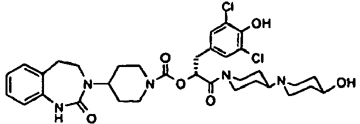 |
| (256) | 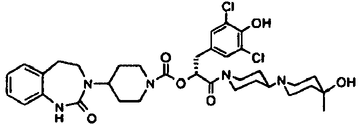 |
| (257) | 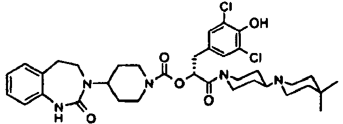 |
| (258) | 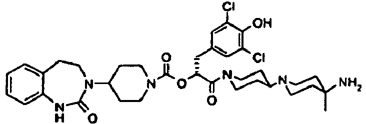 |
| (259) | 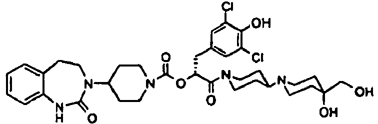 |
| (260) | 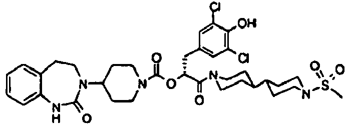 |
| (261) | 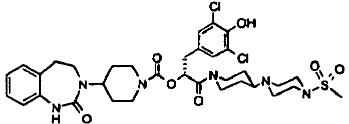 |
| (262) | 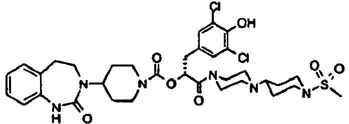 |
| (263) | 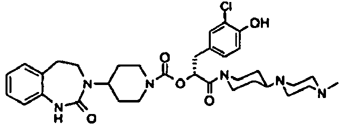 |
| (264) | 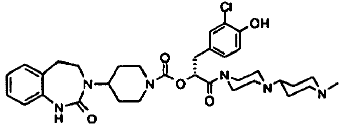 |
| (265) | 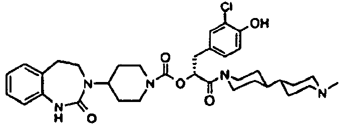 |
| (266) | 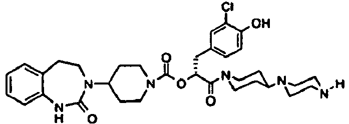 |
| (267) | 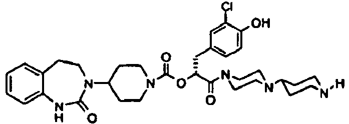 |
| (268) | 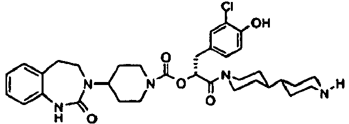 |
| (269) | 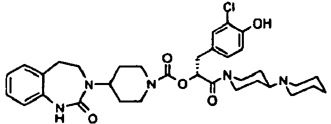 |
| (270) | 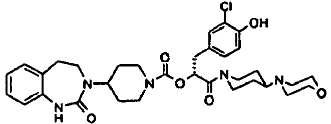 |
| (271) | 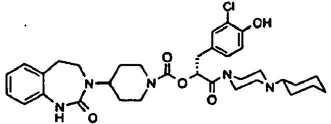 |
| (272) | 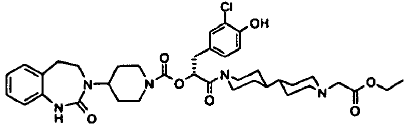 |
| (273) | 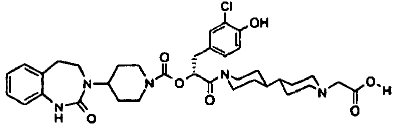 |
| (274) | 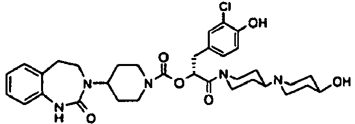 |
| (275) | 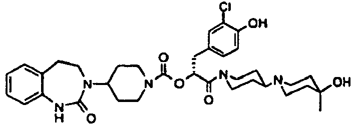 |
| (276) | 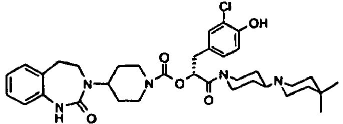 |
| (277) | 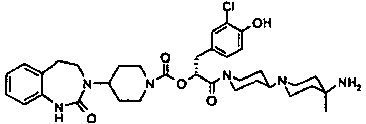 |
| (278) | 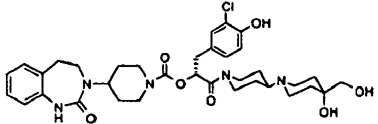 |
| (279) | 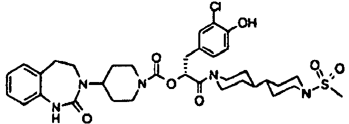 |
| (280) | 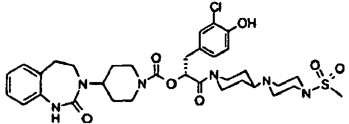 |
| (281) | 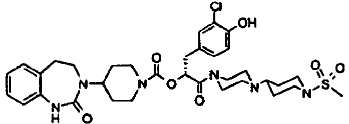 |
| (282) | 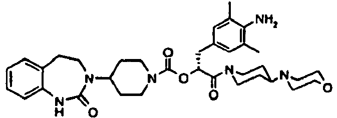 |
| (283) | 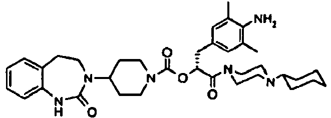 |
| (284) | 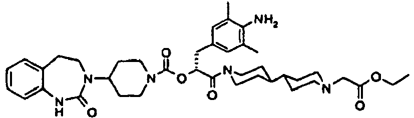 |
| (285) | 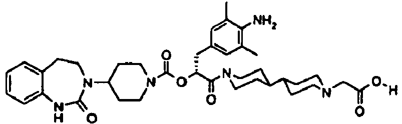 |
| (286) | 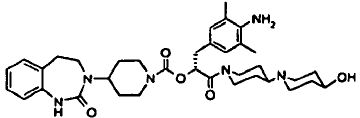 |
| (287) | 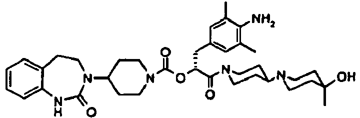 |
| (288) | 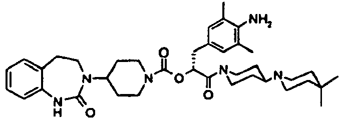 |
| (289) | 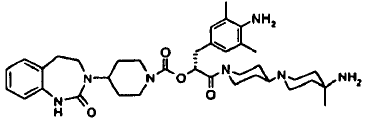 |
| (290) | 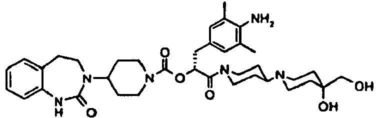 |
| (291) | 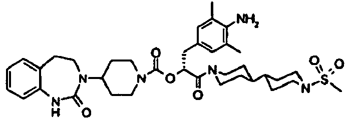 |
| (292) | 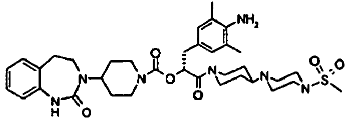 |
| (293) | 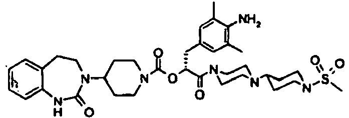 |
| (294) | 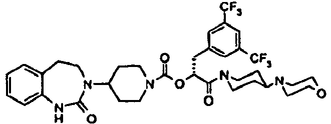 |
| (295) | 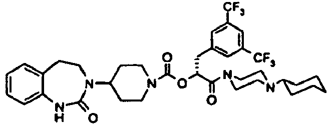 |
| (296) | 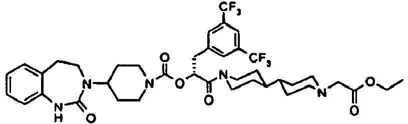 |
| (297) | 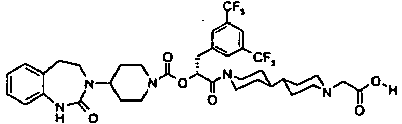 |
| (298) | 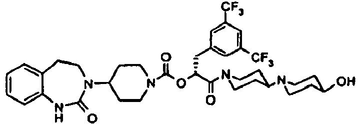 |
| (299) | 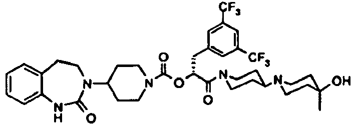 |
| (300) | 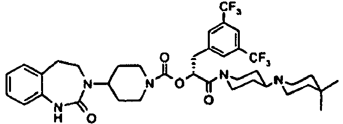 |
| (301) | 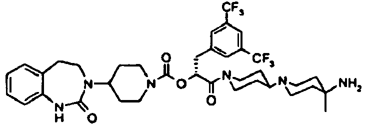 |
| (302) | 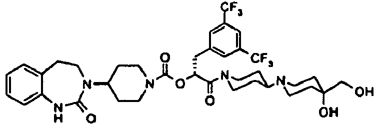 |
| (303) | 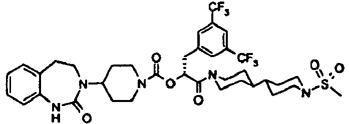 |
| (304) | 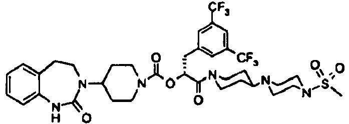 |
| (305) | 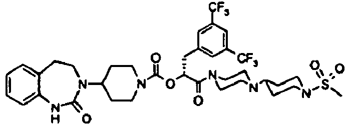 |
| (306) | 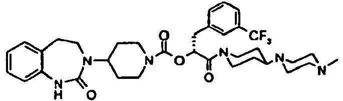 |
| (307) | 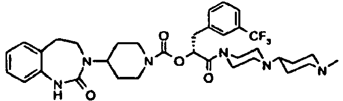 |
| (308) | 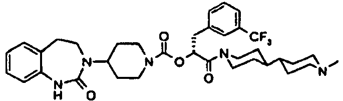 |
| (309) | 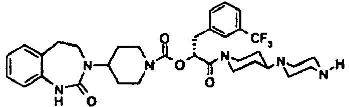 |
| (310) | 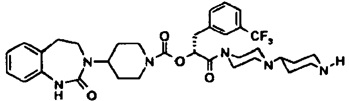 |
| (311) | 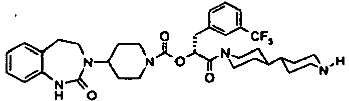 |
| (312) | 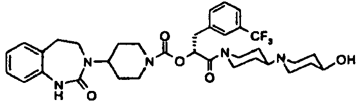 |
| (313) | 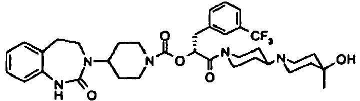 |
| (314) | 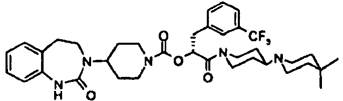 |
| (315) | 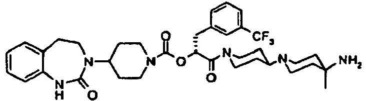 |
| (316) | 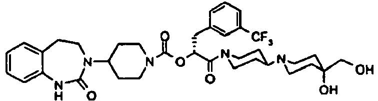 |
| (317) | 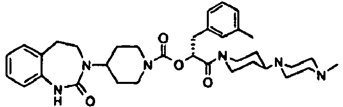 |
| (318) | 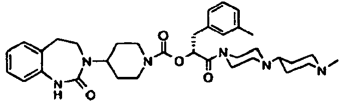 |
| (319) | 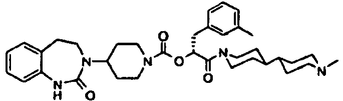 |
| (320) | 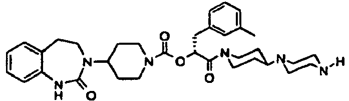 |
| (321) | 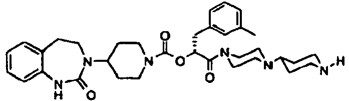 |
| (322) | 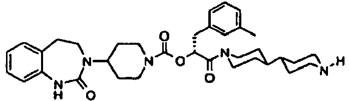 |
| (323) | 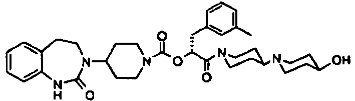 |
| (324) | 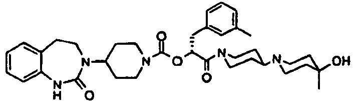 |
| (325) | 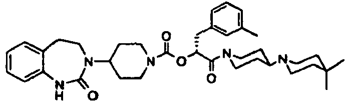 |
| (326) | 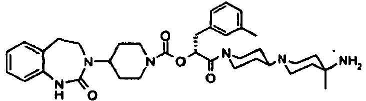 |
| (327) | 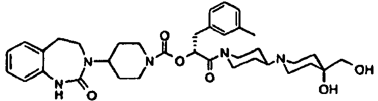 |

2. Physiologisch verträgliche Salze der Verbindungen nach Anspruch 1 mit anorganischen oder organischen Säuren oder Basen.

3. Arzneimittel, enthaltend eine Verbindung nach Anspruch 1 oder ein physiologisch verträgliches Salz gemäß Anspruch 2 neben gegebenenfalls einem oder mehreren inerten Trägerstoffen und/oder Verdünnungsmitteln.

4. Verwendung einer Verbindung nach einem der Ansprüche 1 bis 2 zur Herstellung eines Arzneimittels zur akuten und prophylaktischen Behandlung von Kopfschmerzen, insbesondere Migräne- bzw. Cluster-Kopfschmerz, zur Behandlung von nicht-insulinabhängigem Diabetes mellitus ("NIDDM"), des complex regional pain syndrome (CRPS1), cardiovaskulärer Erkrankungen, Morphintoleranz, Clostritiumtoxin-bedingten Durchfallerkrankungen, Erkrankungen der Haut, insbesondere thermische und strahlenbedingte Hautschäden inklusive Sonnenbrand, entzündlicher Erkrankungen, z.B. entzündlicher Gelenkerkrankungen (Arthritis), neurogenen Entzündungen der oralen Mucosa, entzündlichen Lungenerkrankungen, allergischer Rhinitis, Asthma, Erkrankungen, die mit einer überschießenden Gefäßerweiterung und dadurch bedingter verringerter Gewebedurchblutung einhergehen, z.B. Schock und Sepsis, zur Linderung von Schmerzzuständen, oder zur präventiven oder akut-therapeutisch Behandlung der Symptomatik menopausaler, durch Gefäßervveiterung und erhöhten Blutfluss verursachter Hitzewallungen östrogendefizienter Frauen sowie hormonbehandelter Prostatakarzinompatienten.

5. Verfahren zur Herstellung eines Arzneimittels gemäß Anspruch 3, **dadurch gekennzeichnet, dass** auf nichtchemischem Weg eine Verbindung nach einem der Ansprüche 1 bis 2 in einen oder mehrere inerte Trägerstoffe und/oder Verdünnungsmittel eingearbeitet wird.

6. Verfahren zur Herstellung der Verbindungen der allgemeinen Formel **I** nach einem der Ansprüche 1 bis 2, **dadurch gekennzeichnet, dass**
(a) ein Piperidin der allgemeinen Formel **III** 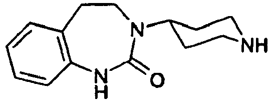 mit einem Kohlensäurederivat der allgemeinen Formel IV 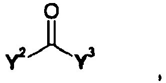 in der **Y²** und **Y³** nucleofuge Gruppen bedeuten, die gleich oder verschieden sein können, und mit einer Verbindung der allgemeinen Formel **V** 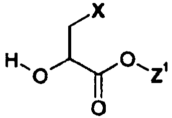 in der **B** wie in Anspruch 1 definiert ist und **Z¹** eine Schutzgruppe für eine Carboxygruppe darstellt, umgesetzt wird, oder
(b) eine Carbonsäure der allgemeinen Formel **VI** 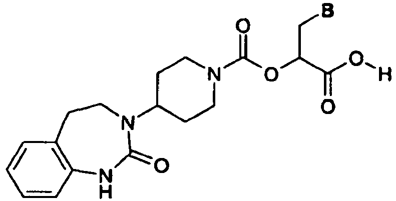 in der **B** wie in Anspruch 1 definiert ist, mit einem Amin der allgemeinen Formel **VII**
H-NR¹R²,
in der **R¹** und **R²** wie in Anspruch 1 definiert sind, gekuppelt wird, wobei vor Durchführung der Reaktion eine in den Resten **R¹** und **R²** des Amins der allgemeinen Formel VII gegebenenfalls vorhandene Carbonsäurefunktion, primäre oder sekundäre Aminofunktion oder Hydroxyfunktion durch übliche Schutzreste geschützt und gegebenenfalls verwendete Schutzreste nach Durchführung der Reaktion wieder abgespalten werden, oder
(c) eine Verbindung der allgemeinen Formel **VIII** 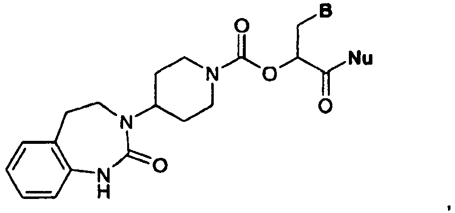 in der **B** wie in Anspruch 1 definiert ist und Nu eine Austrittsgruppe darstellt, mit einem Amin der allgemeinen Formel **VII**
H-N**R¹R²**,
in der **R¹** und **R²** wie in Anspruch 1 definiert sind, gekuppelt wird, wobei vor Durchführung der Reaktion eine in den Resten **R¹** und **R²** des Amins der allgemeinen Formel VII gegebenenfalls vorhandene Carbonsäurefunktion, primäre oder sekundäre Aminofunktion oder Hydroxyfunktion durch übliche Schutzreste geschützt und gegebenenfalls verwendete Schutzreste nach Durchführung der Reaktion wieder abgespalten werden, und
gewünschtenfalls eine so erhaltene Verbindung der allgemeinen Formel 1 in ihre Stereoisomere aufgetrennt wird und/oder
eine so erhaltene Verbindung der allgemeinen Formel **I** in ihre Salze, insbesondere für die pharmazeutische Anwendung in ihre physiologisch verträglichen Salze übergeführt wird.

## Claims

1. CGRP antagonists of general formula **I** 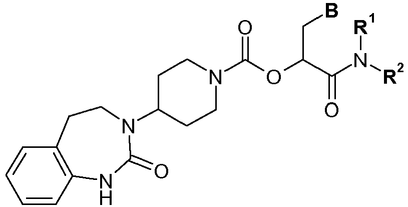 wherein
**B** denotes a group selected from 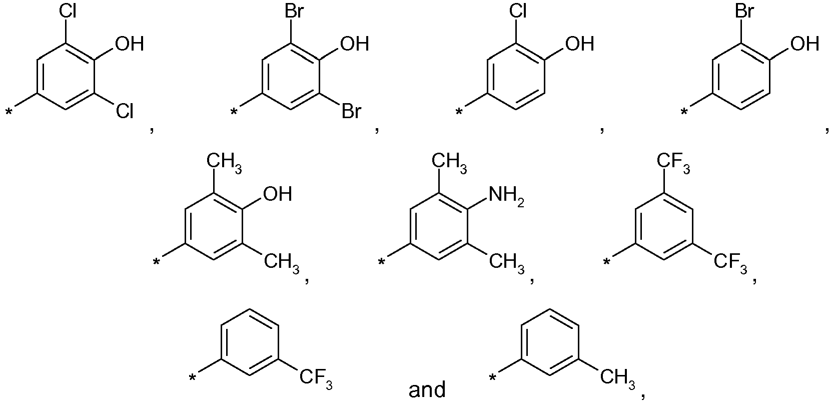
**R¹** and **R²** together with the enclosed nitrogen atom denote a group of general formula **II** 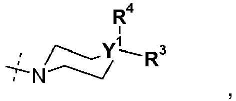
wherein
**Y¹** denotes the carbon atom or, if **R⁴** denotes a free pair of electrons, it may also denote the nitrogen atom,
**R³** denotes a cyclopentyl, cyclohexyl or cycloheptyl group or
**R³** denotes a heterocycle selected from a morpholin-4-yl, 1,1-dioxothiomorholin-4-yl, piperidin-1-yl, piperidin-4-yl, piperazin-1-yl or pyrrolidin-1-yl group,
wherein the above-mentioned monocyclic heterocycles in the ring may be mono- or disubstituted by hydroxy, methyl, ethyl, trifluoromethyl, hydroxymethyl or hydroxyethyl groups, or may optionally additionally be monosubstituted by a hydroxycyclopropyl, trifluoromethylcarbonylmethyl, amino, carboxy-carbonyl, methoxycarbonyl, ethoxycarbonyl-carbonyl, carboxymethyl, carboxyethyl, ethoxycarbonylmethyl, ethoxycarbonylethyl, carboxy-ethylcarbonyl, ethoxycarbonyl-ethylcarbonyl, aminosulphonyl, methylaminosulphonyl, dimethylaminosulphonyl, methylsulphonyl, ethylsulphonyl, isopropylsulphonyl, cyclopropylsulphonyl, (hydroxyamino)-carbonylmethyl, hydroxy-(methyl)-aminocarbonyl-methyl or methoxyaminocarbonyl-methyl group, wherein the substituents may be identical or different and may be bound to a ring carbon or ring nitrogen atom, or
the above-mentioned monocyclic heterocycles in the ring may be monosubstituted by a carboxy group, if this carboxy group is not bound via a nitrogen atom, and
**R⁴** denotes the hydrogen atom, if **Y¹** denotes the carbon atom, or
**R⁴** denotes a free pair of electrons, if **Y¹** denotes the nitrogen atom,
the tautomers, the diastereomers, the enantiomers, the hydrates, the mixtures thereof and the salts thereof as well as the hydrates of the salts, particularly the physiologically acceptable salts thereof with inorganic or organic acids or bases, with the proviso that the following compounds, the tautomers, diastereomers, enantiomers, hydrates, mixtures and salts thereof and the hydrates of the salts are excluded from the scope of the claim:
| **No.** | **Structure** |
|---|---|
| (1) | 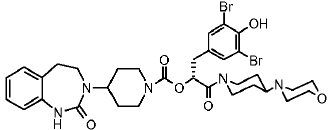 |
| (2) | 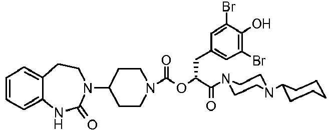 |
| (3) | 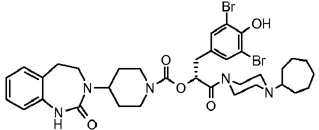 |
| (4) | 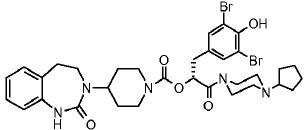 |
| (5) | 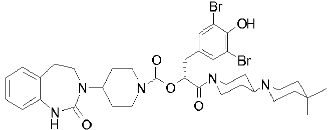 |
| (6) | 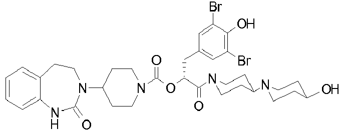 |
| (7) | 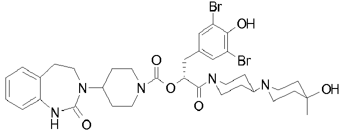 |
| (8) | 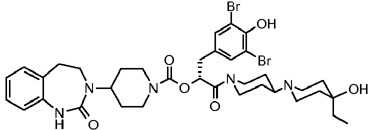 |
| (9) | 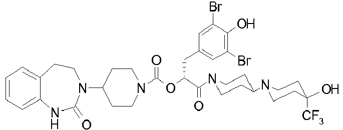 |
| (10) | 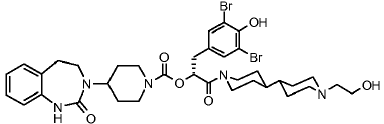 |
| (11) | 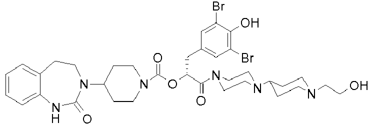 |
| (12) | 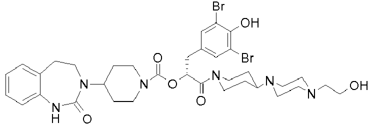 |
| (13) | 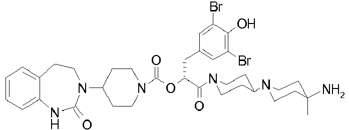 |
| (14) | 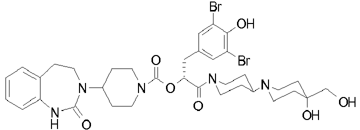 |
| (15) | 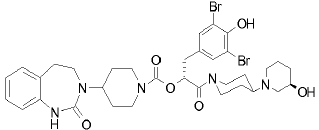 |
| (16) | 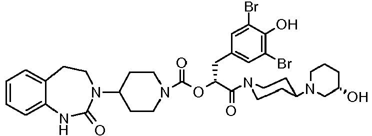 |
| (17) | 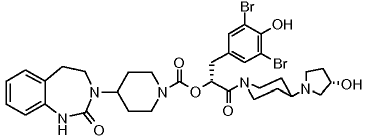 |
| (18) | 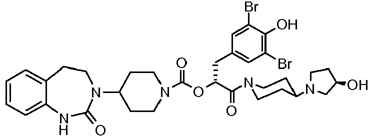 |
| (19) | 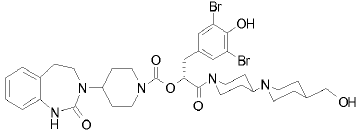 |
| (20) | 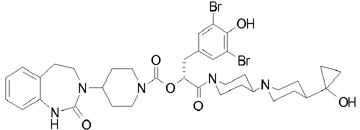 |
| (21) | 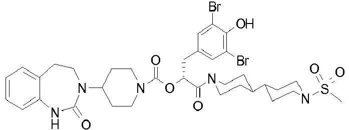 |
| (22) | 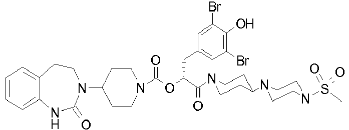 |
| (23) | 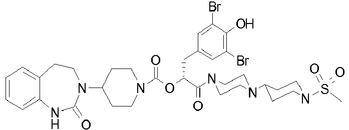 |
| (24) | 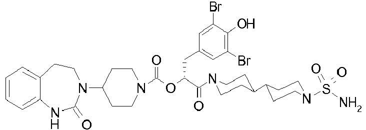 |
| (25) | 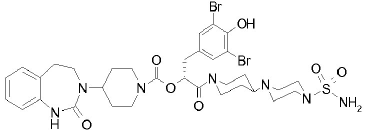 |
| (26) | 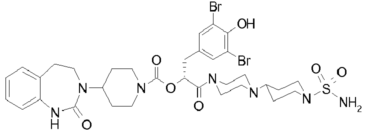 |
| (27) | 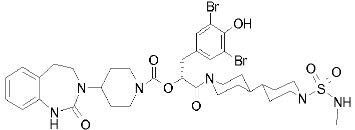 |
| (28) | 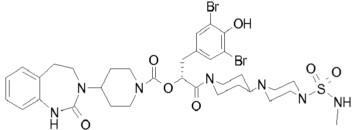 |
| (29) | 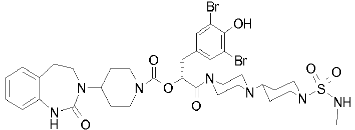 |
| (30) | 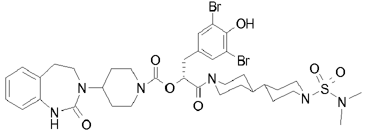 |
| (31) | 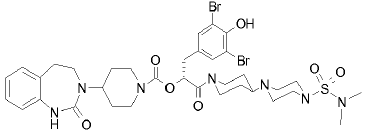 |
| (32) | 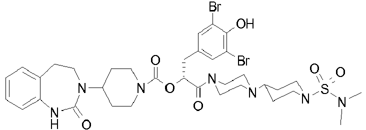 |
| (33) | 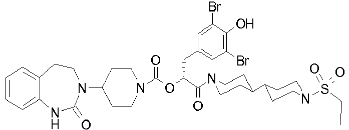 |
| (34) | 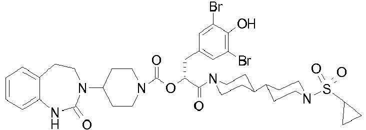 |
| (35) | 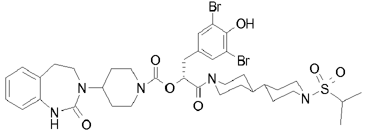 |
| (36) | 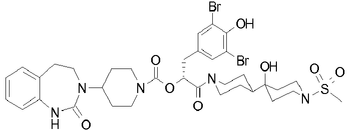 |
| (37) | 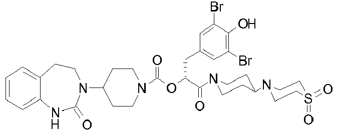 |
| (38) | 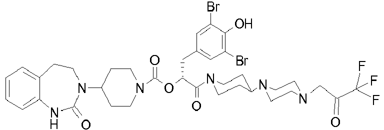 |
| (39) | 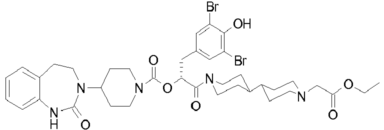 |
| (40) | 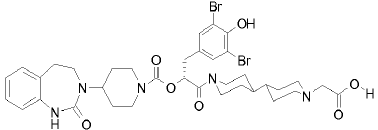 |
| (41) | 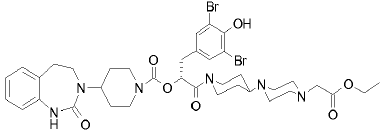 |
| (42) | 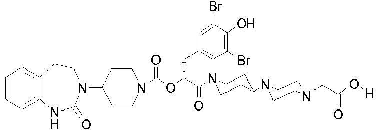 |
| (43) | 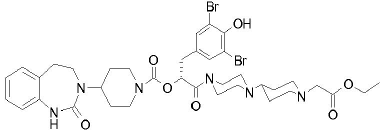 |
| (44) | 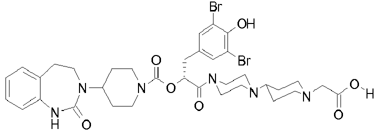 |
| (45) | 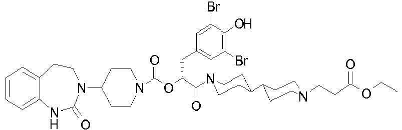 |
| (46) | 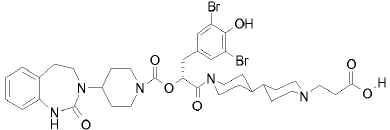 |
| (47) | 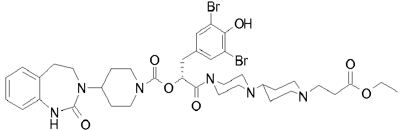 |
| (48) | 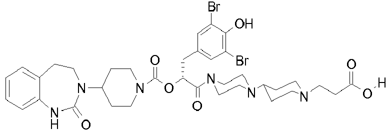 |
| (49) | 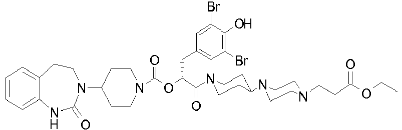 |
| (50) | 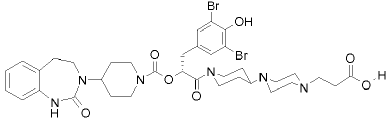 |
| (51) | 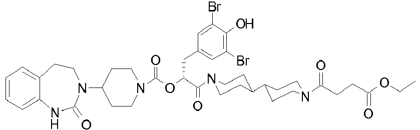 |
| (52) | 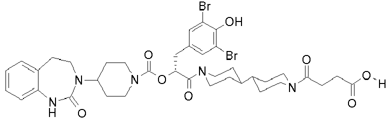 |
| (53) | 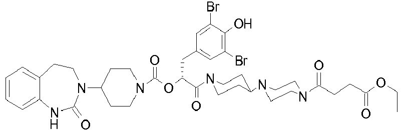 |
| (54) | 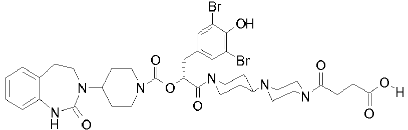 |
| (55) | 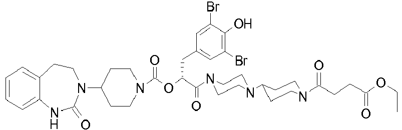 |
| (56) | 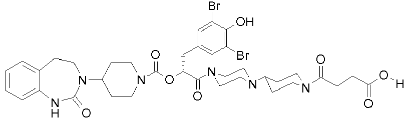 |
| (57) | 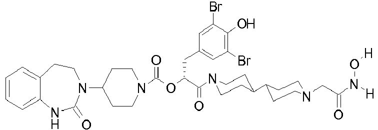 |
| (58) | 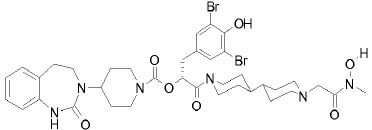 |
| (59) | 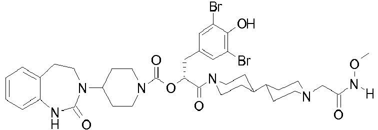 |
| (60) | 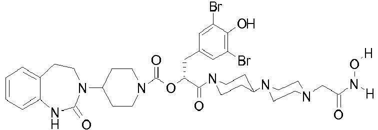 |
| (61) | 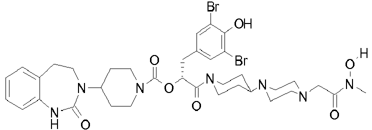 |
| (62) | 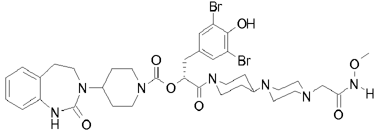 |
| (63) | 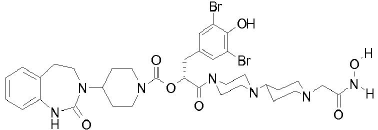 |
| (64) | 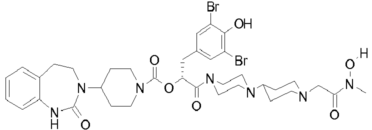 |
| (65) | 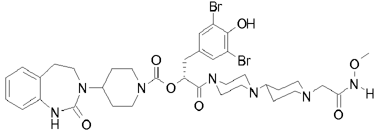 |
| (66) | 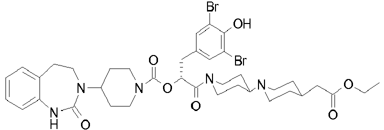 |
| (67) | 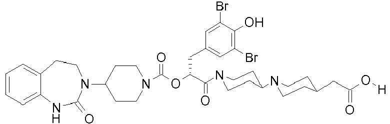 |
| (68) | 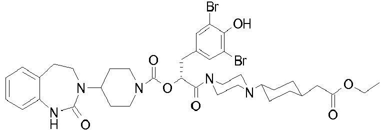 |
| (69) | 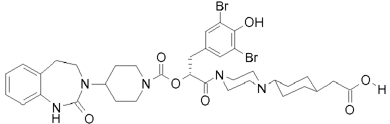 |
| (70) | 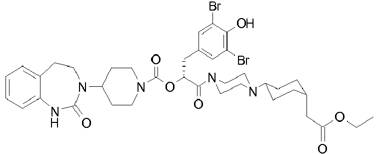 |
| (71) | 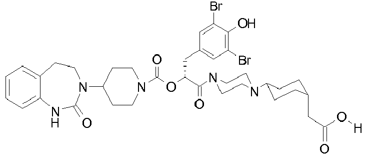 |
| (72) | 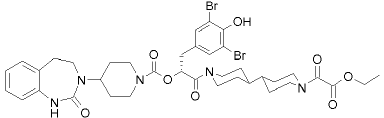 |
| (73) | 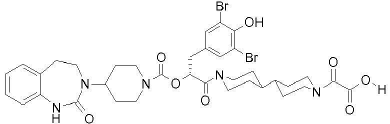 |
| (74) | 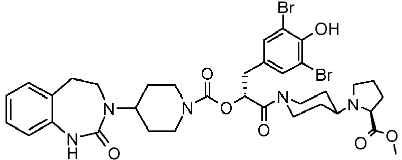 |
| (75) | 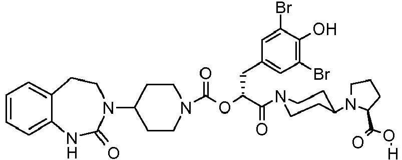 |
| (76) | 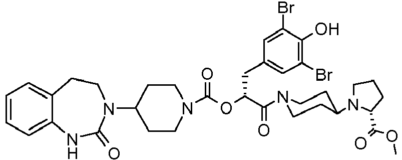 |
| (77) | 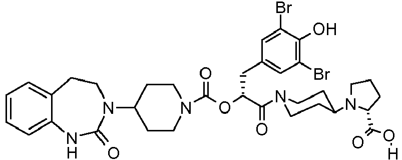 |
| (78) | 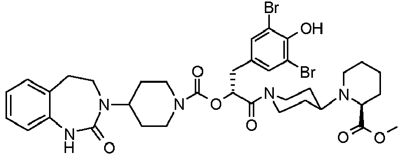 |
| (79) | 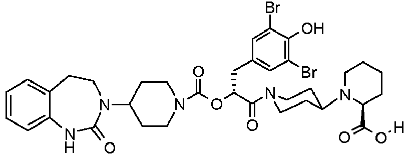 |
| (80) | 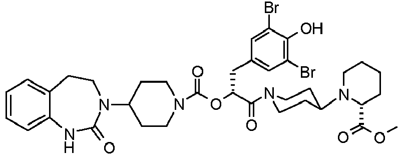 |
| (81) | 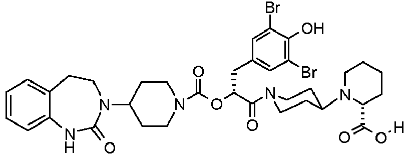 |
| (82) | 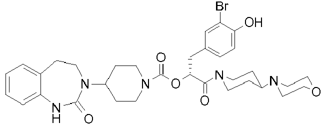 |
| (83) | 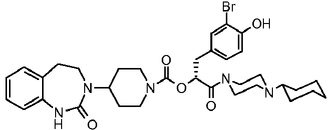 |
| (84) | 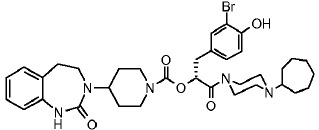 |
| (85) | 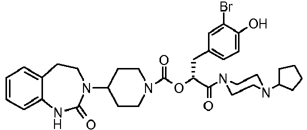 |
| (86) | 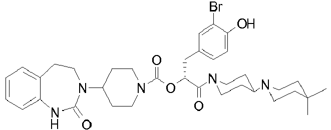 |
| (87) | 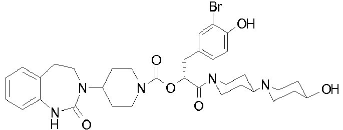 |
| (88) | 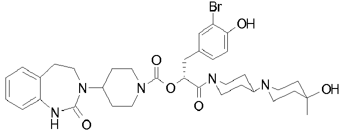 |
| (89) | 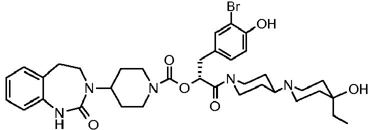 |
| (90) | 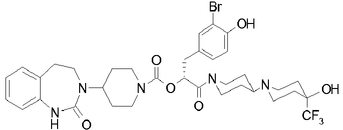 |
| (91) | 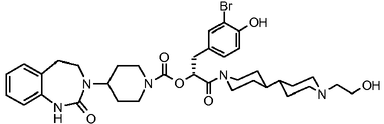 |
| (92) | 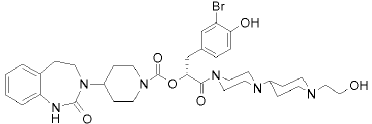 |
| (93) | 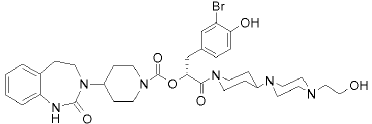 |
| (94) | 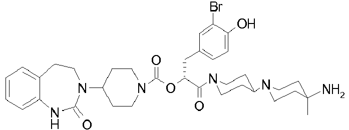 |
| (95) | 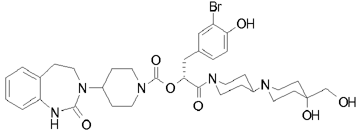 |
| (96) | 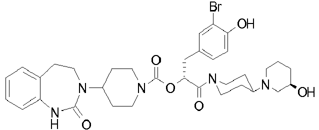 |
| (97) | 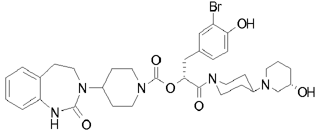 |
| (98) | 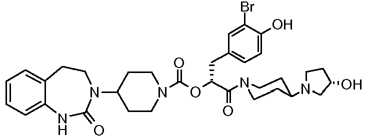 |
| (99) | 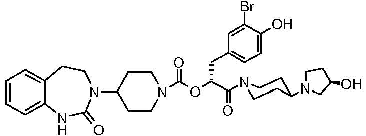 |
| (100) | 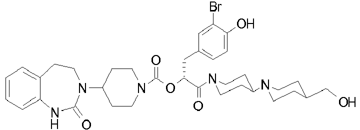 |
| (101) | 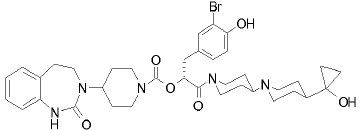 |
| (102) | 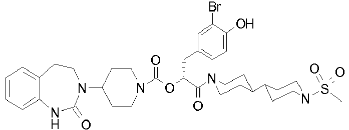 |
| (103) | 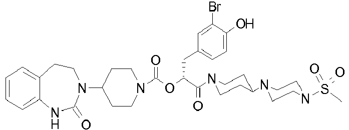 |
| (104) | 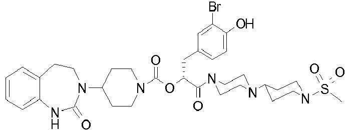 |
| (105) | 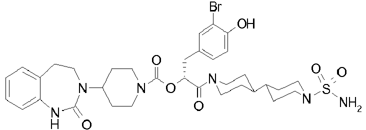 |
| (106) | 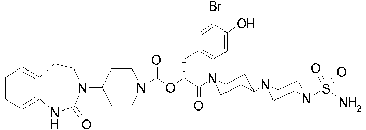 |
| (107) | 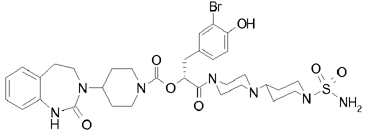 |
| (108) | 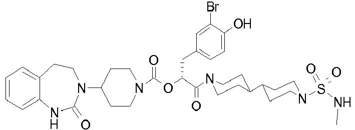 |
| (109) | 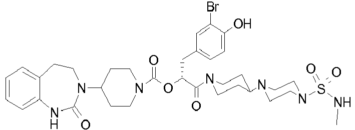 |
| (110) | 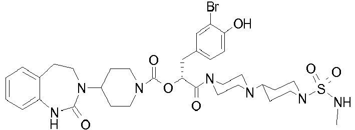 |
| (111) | 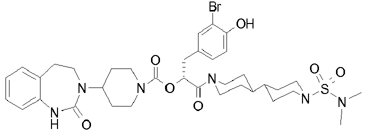 |
| (112) | 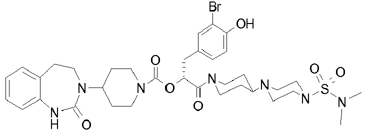 |
| (113) | 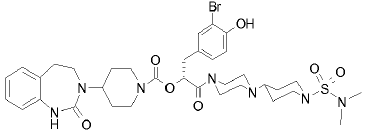 |
| (114) | 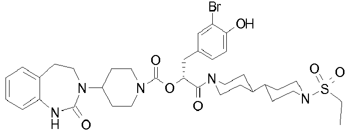 |
| (115) | 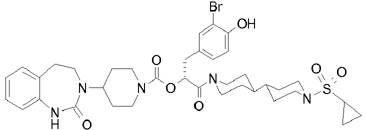 |
| (116) | 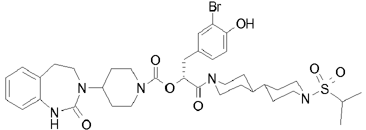 |
| (117) | 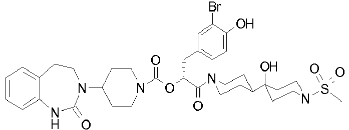 |
| (118) | 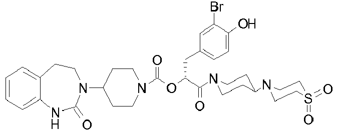 |
| (119) | 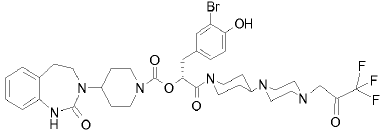 |
| (120) | 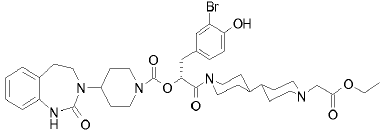 |
| (121) | 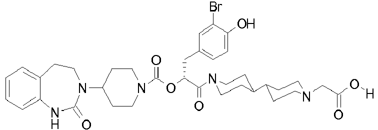 |
| (122) | 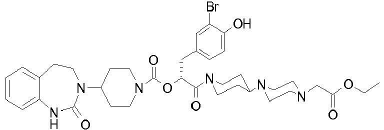 |
| (123) | 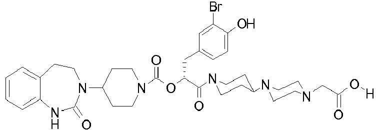 |
| (124) | 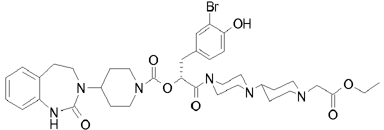 |
| (125) | 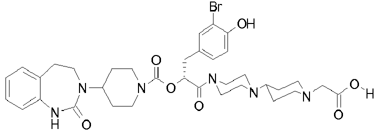 |
| (126) | 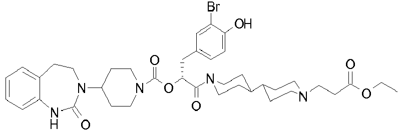 |
| (127) | 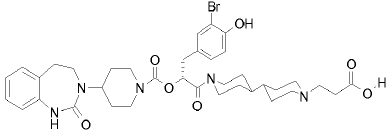 |
| (128) | 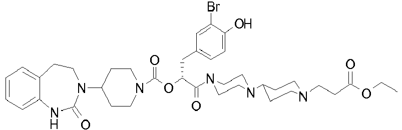 |
| (129) | 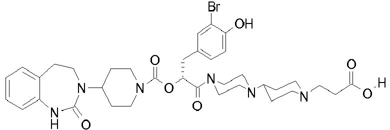 |
| (130) | 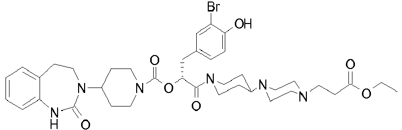 |
| (131) | 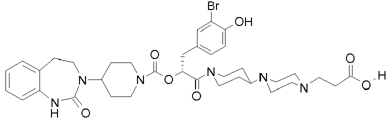 |
| (132) | 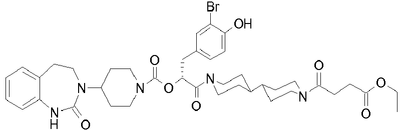 |
| (133) | 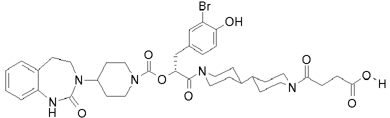 |
| (134) | 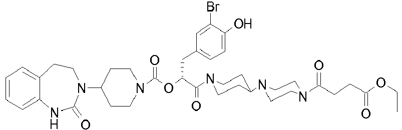 |
| (135) | 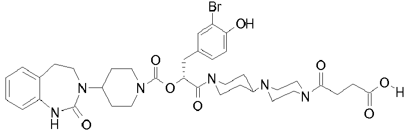 |
| (136) | 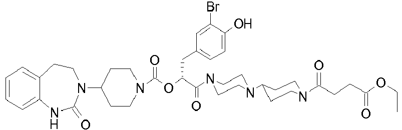 |
| (137) | 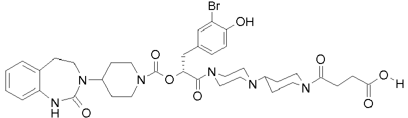 |
| (138) | 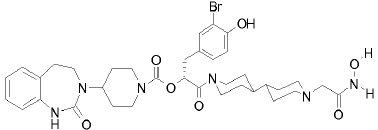 |
| (139) | 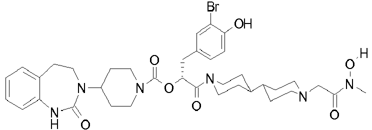 |
| (140) | 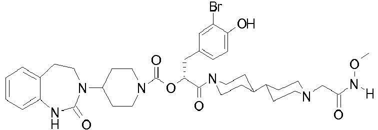 |
| (141) | 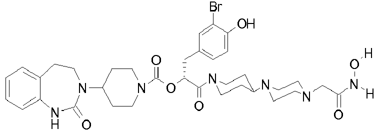 |
| (142) | 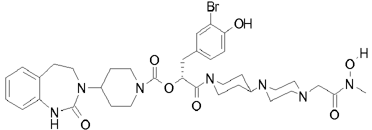 |
| (143) | 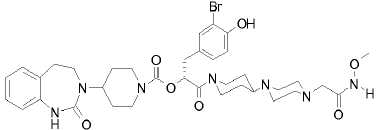 |
| (144) | 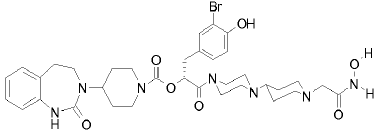 |
| (145) | 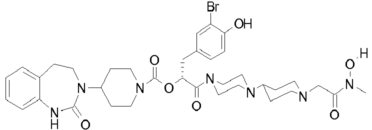 |
| (146) | 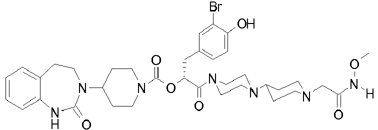 |
| (147) | 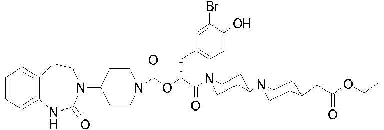 |
| (148) | 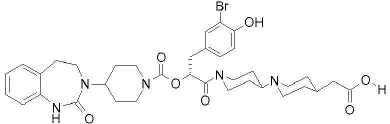 |
| (149) | 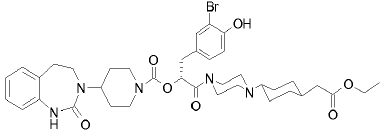 |
| (150) | 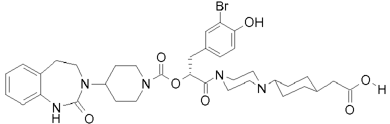 |
| (151) | 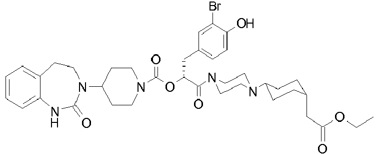 |
| (152) | 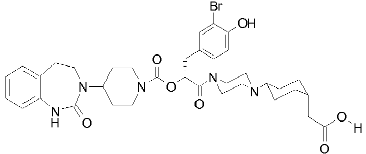 |
| (153) | 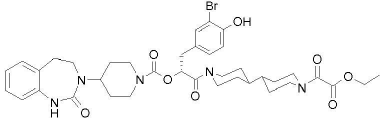 |
| (154) | 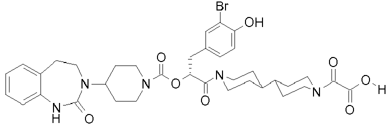 |
| (155) | 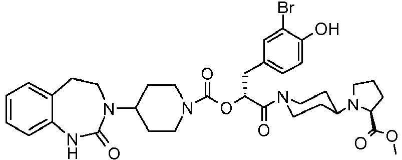 |
| (156) | 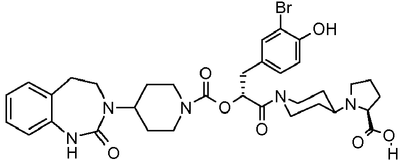 |
| (157) | 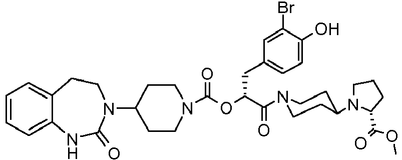 |
| (158) | 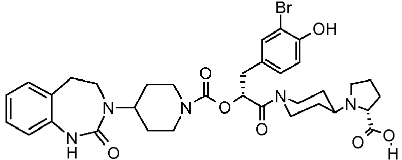 |
| (159) | 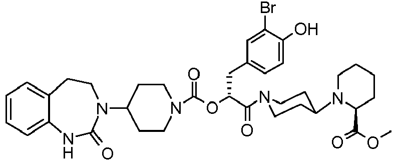 |
| (160) | 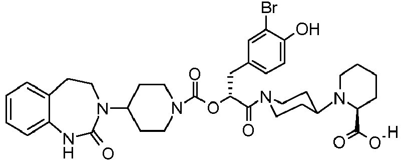 |
| (161) | 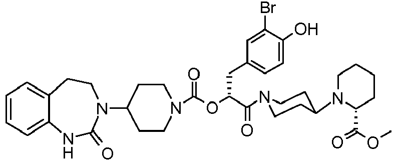 |
| (162) | 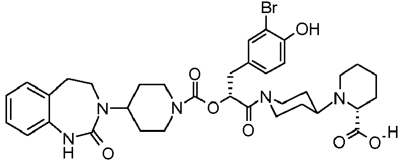 |
| (163) | 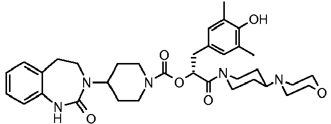 |
| (164) | 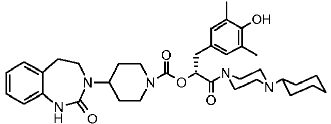 |
| (165) | 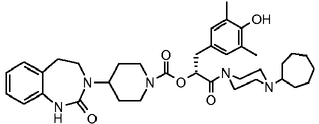 |
| (166) | 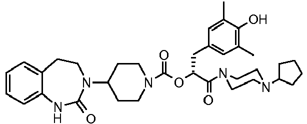 |
| (167) | 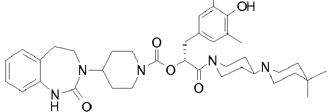 |
| (168) | 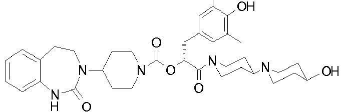 |
| (169) | 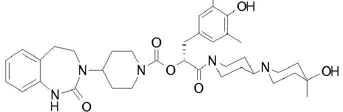 |
| (170) | 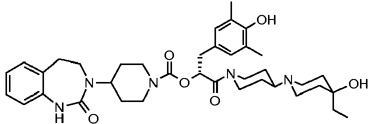 |
| (171) | 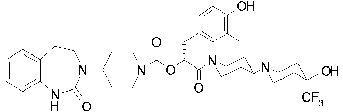 |
| (172) | 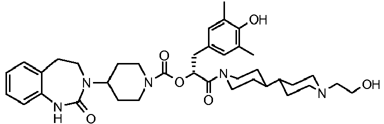 |
| (173) | 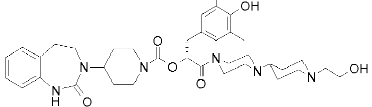 |
| (174) | 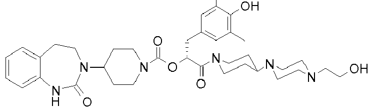 |
| (175) | 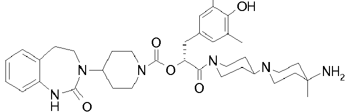 |
| (176) | 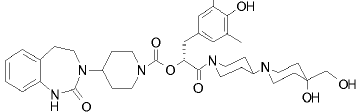 |
| (177) | 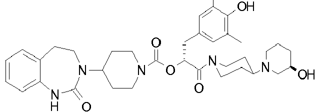 |
| (178) | 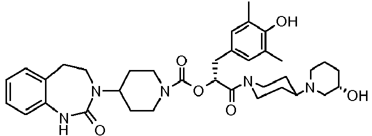 |
| (179) | 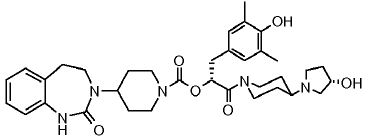 |
| (180) | 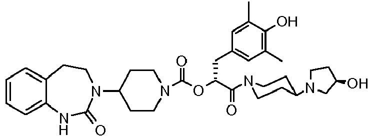 |
| (181) | 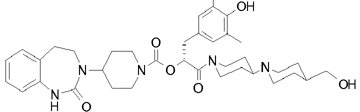 |
| (182) | 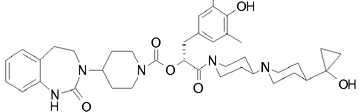 |
| (183) | 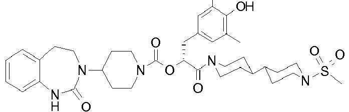 |
| (184) | 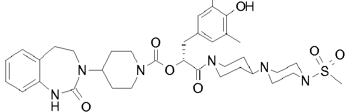 |
| (185) | 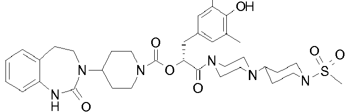 |
| (186) | 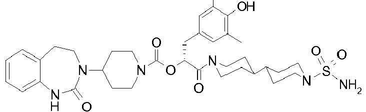 |
| (187) | 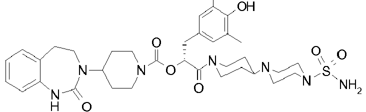 |
| (188) | 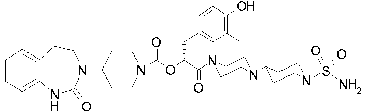 |
| (189) | 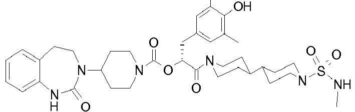 |
| (190) | 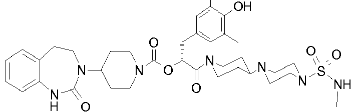 |
| (191) | 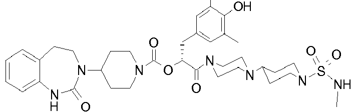 |
| (192) | 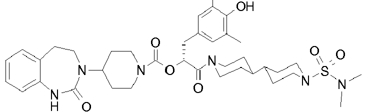 |
| (193) | 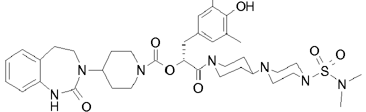 |
| (194) | 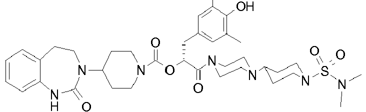 |
| (195) | 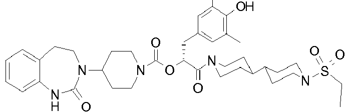 |
| (196) | 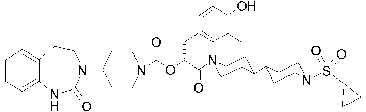 |
| (197) | 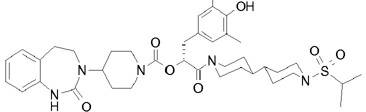 |
| (198) | 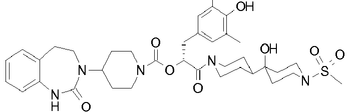 |
| (199) | 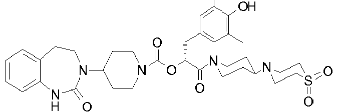 |
| (200) | 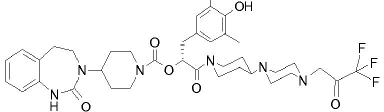 |
| (201) | 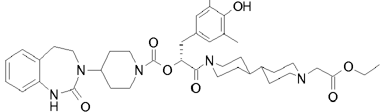 |
| (202) | 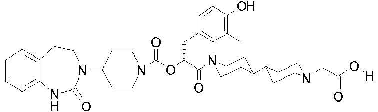 |
| (203) | 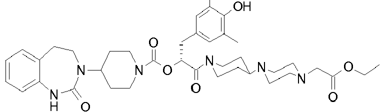 |
| (204) | 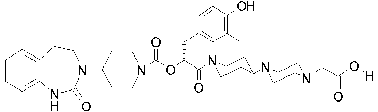 |
| (205) | 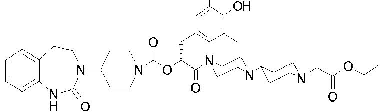 |
| (206) | 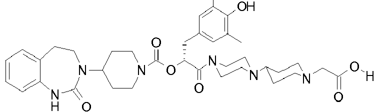 |
| (207) | 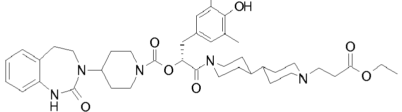 |
| (208) | 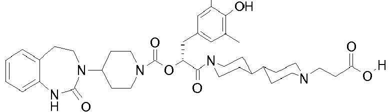 |
| (209) | 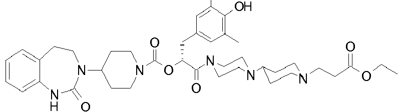 |
| (210) | 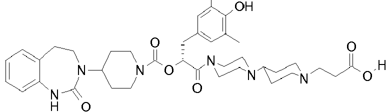 |
| (211) | 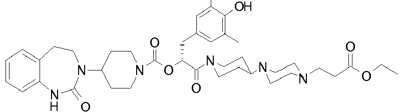 |
| (212) | 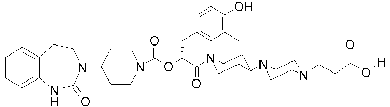 |
| (213) | 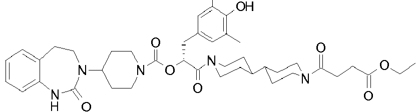 |
| (214) | 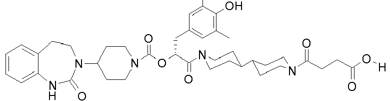 |
| (215) | 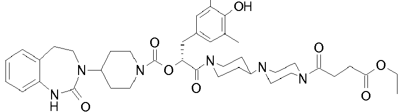 |
| (216) | 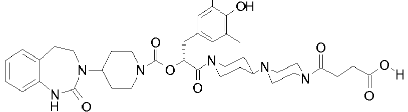 |
| (217) | 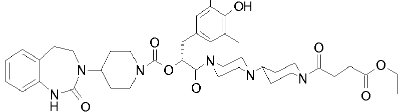 |
| (218) | 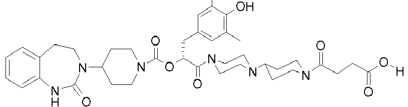 |
| (219) | 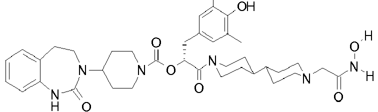 |
| (220) | 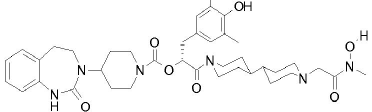 |
| (221) | 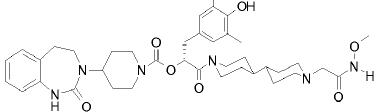 |
| (222) | 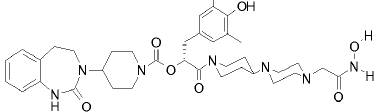 |
| (223) | 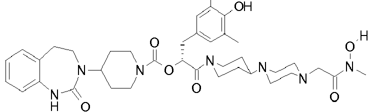 |
| (224) | 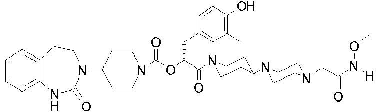 |
| (225) | 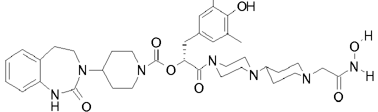 |
| (226) | 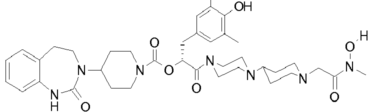 |
| (227) | 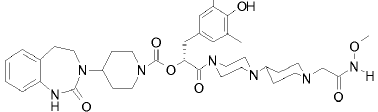 |
| (228) | 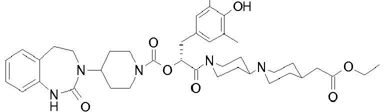 |
| (229) | 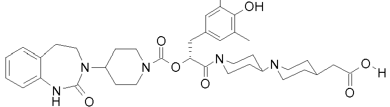 |
| (230) | 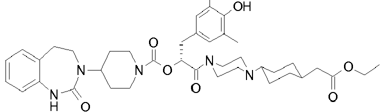 |
| (231) | 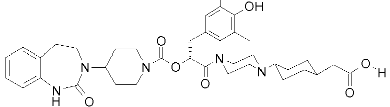 |
| (232) | 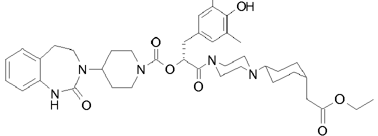 |
| (233) | 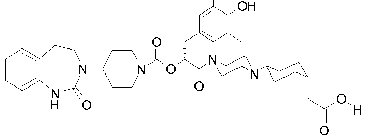 |
| (234) | 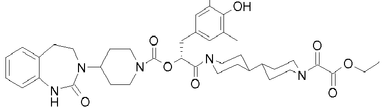 |
| (235) | 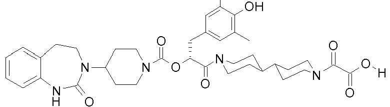 |
| (236) | 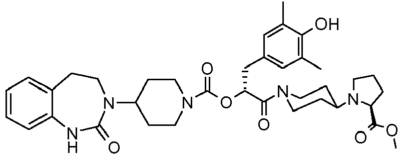 |
| (237) | 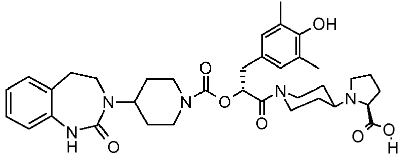 |
| (238) | 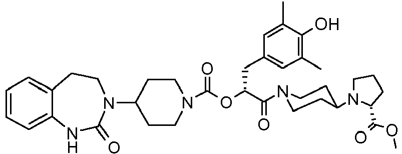 |
| (239) | 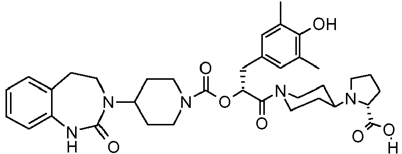 |
| (240) | 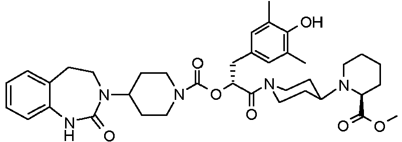 |
| (241) | 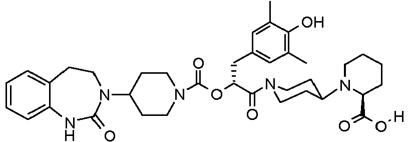 |
| (242) | 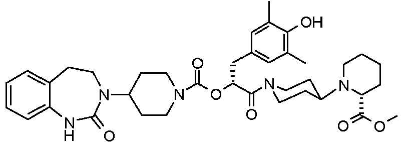 |
| (243) | 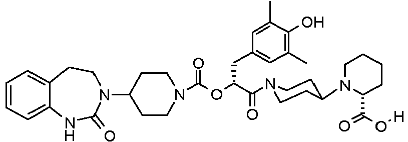 |
| (244) | 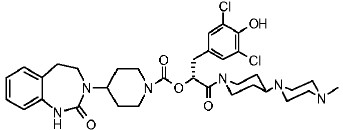 |
| (245) | 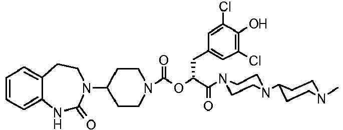 |
| (246) | 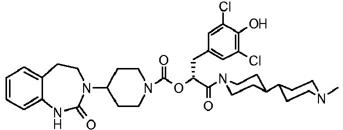 |
| (247) | 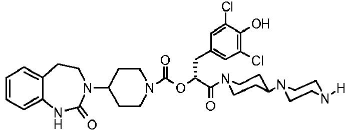 |
| (248) | 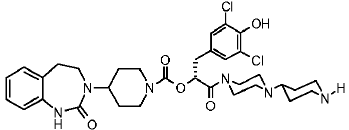 |
| (249) | 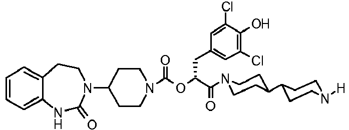 |
| (250) | 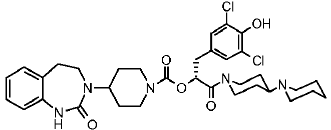 |
| (251) | 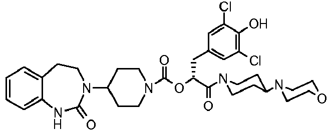 |
| (252) | 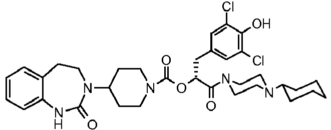 |
| (253) | 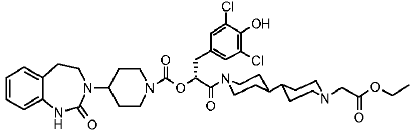 |
| (254) | 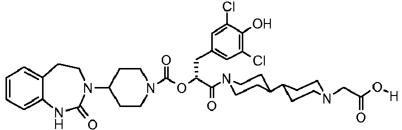 |
| (255) | 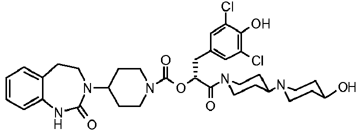 |
| (256) | 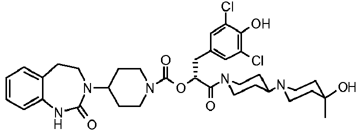 |
| (257) | 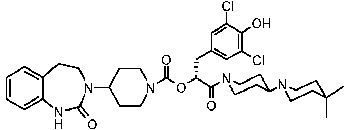 |
| (258) | 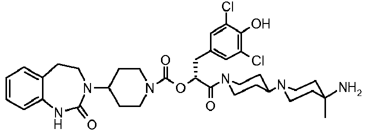 |
| (259) | 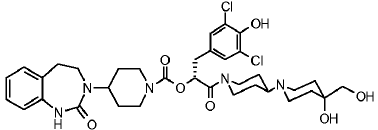 |
| (260) | 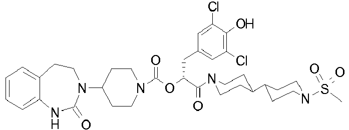 |
| (261) | 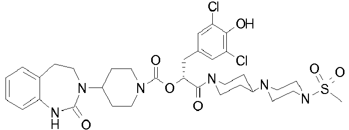 |
| (262) | 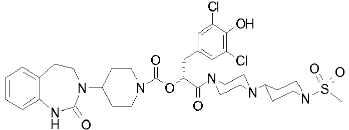 |
| (263) | 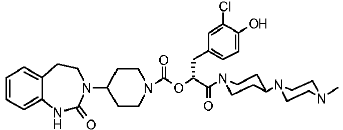 |
| (264) | 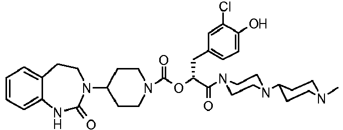 |
| (265) | 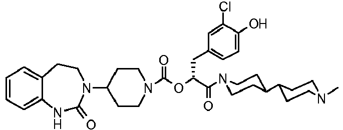 |
| (266) | 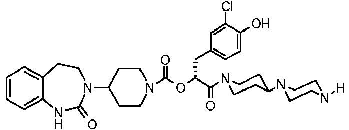 |
| (267) | 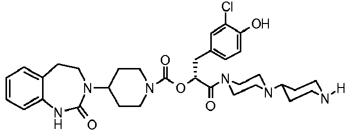 |
| (268) | 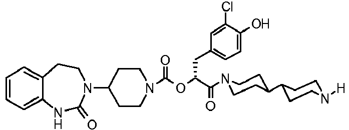 |
| (269) | 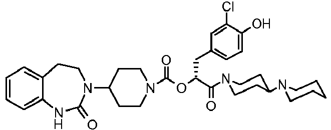 |
| (270) | 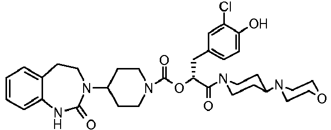 |
| (271) | 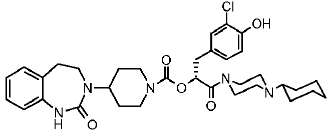 |
| (272) | 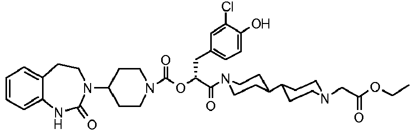 |
| (273) | 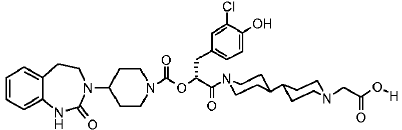 |
| (274) | 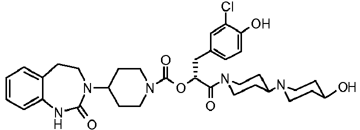 |
| (275) | 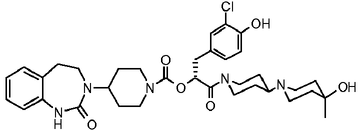 |
| (276) | 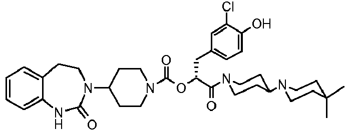 |
| (277) | 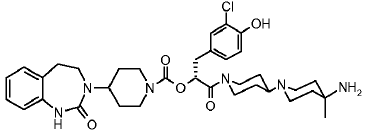 |
| (278) | 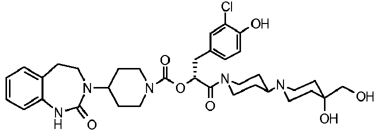 |
| (279) | 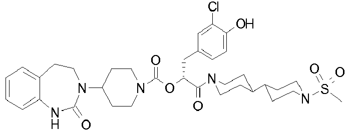 |
| (280) | 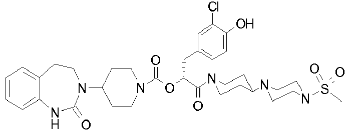 |
| (281) | 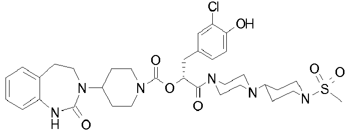 |
| (282) | 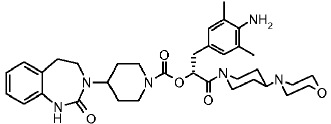 |
| (283) | 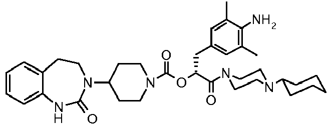 |
| (284) | 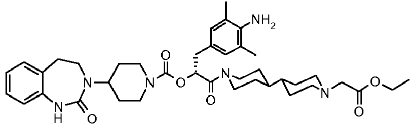 |
| (285) | 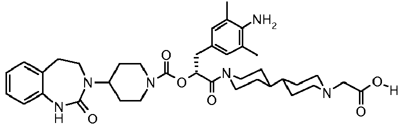 |
| (286) | 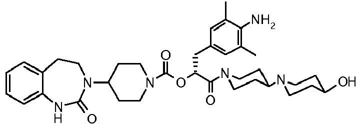 |
| (287) | 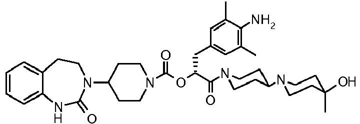 |
| (288) | 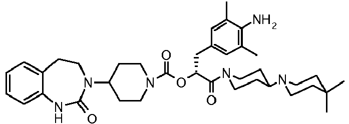 |
| (289) | 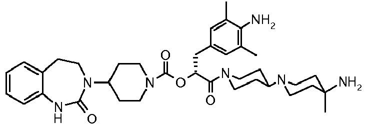 |
| (290) | 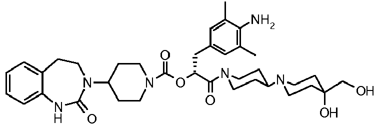 |
| (291) | 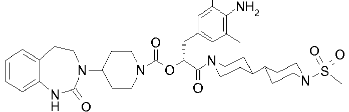 |
| (292) | 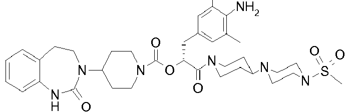 |
| (293) | 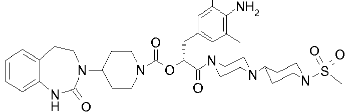 |
| (294) | 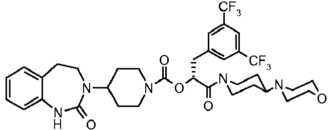 |
| (295) | 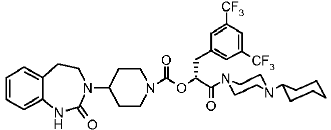 |
| (296) | 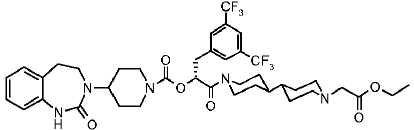 |
| (297) | 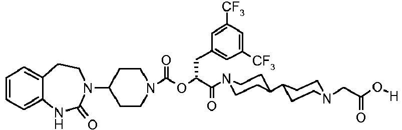 |
| (298) | 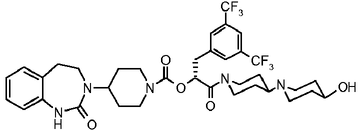 |
| (299) | 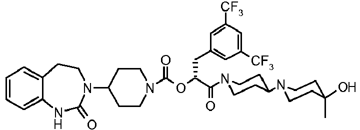 |
| (300) | 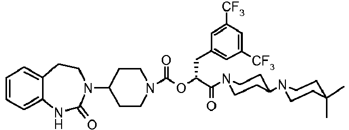 |
| (301) | 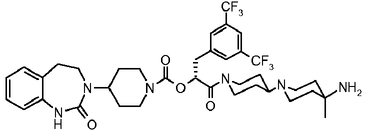 |
| (302) | 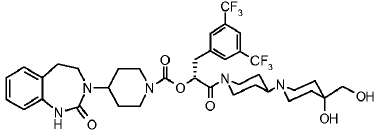 |
| (303) | 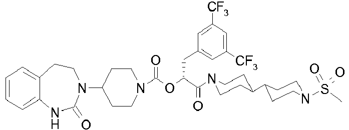 |
| (304) | 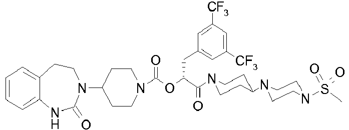 |
| (305) | 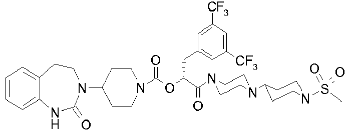 |
| (306) | 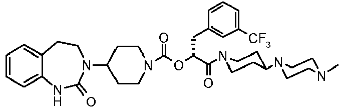 |
| (307) | 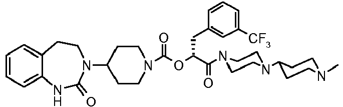 |
| (308) | 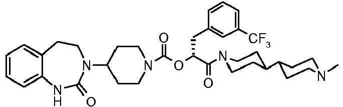 |
| (309) | 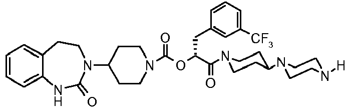 |
| (310) | 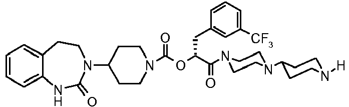 |
| (311) | 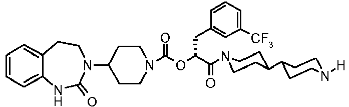 |
| (312) | 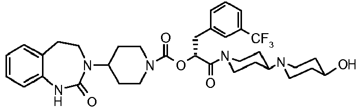 |
| (313) | 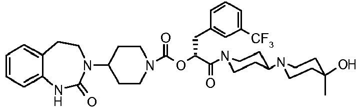 |
| (314) | 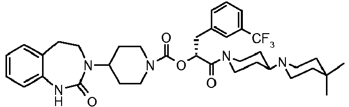 |
| (315) | 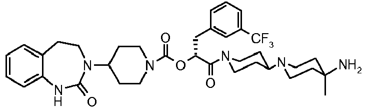 |
| (316) | 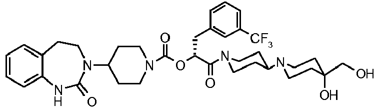 |
| (317) | 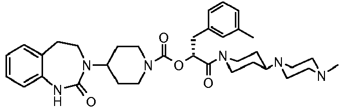 |
| (318) | 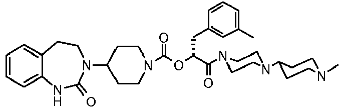 |
| (319) | 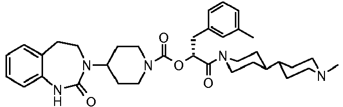 |
| (320) | 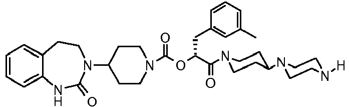 |
| (321) | 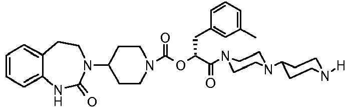 |
| (322) | 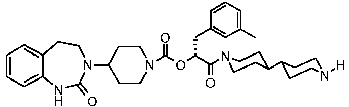 |
| (323) | 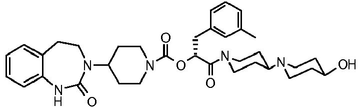 |
| (324) | 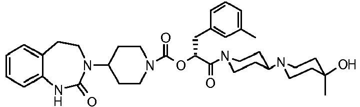 |
| (325) | 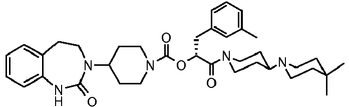 |
| (326) | 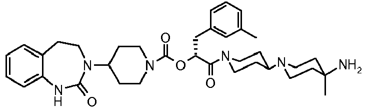 |
| (327) | 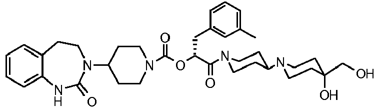 |

2. Physiologically acceptable salts of the compounds according to claim 1 with inorganic or organic acids or bases.

3. Medicaments containing a compound according to claim 1 or a physiologically acceptable salt according to claim 2 optionally together with one or more inert carriers and/or diluents.

4. Use of a compound according to one of claims 1 to 2 for preparing a medicament for the acute and prophylactic treatment of headaches, particularly migraine or cluster headaches, for the treatment of non-insulin-dependent diabetes mellitus ("NIDDM"), complex regional pain syndrome (CRPS1), cardiovascular diseases, morphine tolerance, diarrhoea caused by clostridium toxin, skin diseases, particularly thermal and radiation-induced skin damage including sunburn, inflammatory diseases, e.g. inflammatory diseases of the joints (arthritis), neurogenic inflammation of the oral mucosa, inflammatory lung diseases, allergic rhinitis, asthma, diseases accompanied by excessive vasodilatation and resultant reduced circulation of the blood, e.g. shock and sepsis, for alleviating pain or for preventive or acute therapeutic treatment of the symptoms of menopausal hot flushes caused by vasodilatation and increased blood flow in oestrogen-deficient women and hormone-treated patients with prostate carcinoma.

5. Process for preparing a medicament according to claim 3, **characterised in that** a compound according to one of claims 1 to 2 is incorporated in one or more inert carriers and/or diluents by a non-chemical method.

6. Process for preparing the compounds of general formula **I** according to one of claims 1 to 2, **characterised in that**
(a) a piperidine of general formula **III** 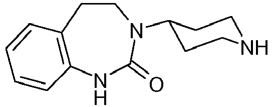 is reacted with a carbonic acid derivative of general formula **IV** 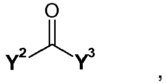 wherein **Y²** and **Y³** represent nucleofugic groups, which may be identical or different, and with a compound of general formula **V** 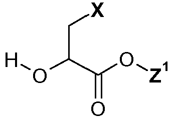 wherein **B** is defined as in claim 1 and **Z¹** denotes a protective group for a carboxy group, or
(b) a carboxylic acid of general formula **VI** 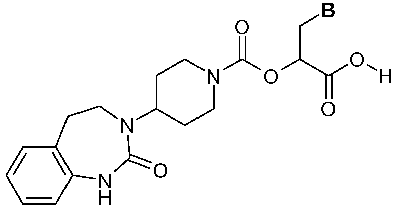 wherein **B** is defined as in claim 1, is coupled with an amine of general formula **VII**
H-N**R¹R²**,
wherein **R¹** and **R²** are defined as in claim 1, wherein before the reaction is carried out any carboxylic acid function, primary or secondary amino function or hydroxy function present in the groups **R¹** and **R²** of the amine of general formula **VII** is protected by conventional protecting groups and any protecting groups used are cleaved again after the reaction, or
(c) a compound of general formula **VIII** 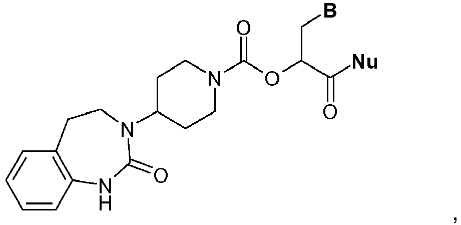 wherein **B** is defined as in claim 1 and **Nu** denotes a leaving group, is coupled with an amine of general formula **VII**
H-N**R¹R²**,
wherein **R¹** and **R²** are defined as in claim 1, wherein before the reaction is carried out any carboxylic acid function, primary or secondary amino function or hydroxy function present in the groups **R¹** and **R²** of the amine of general formula **VII** is protected by conventional protecting groups and any protecting groups used are cleaved again after the reaction, and
if desired a compound of general formula **I** thus obtained is resolved into its stereoisomers and/or
a compound of general formula **I** thus obtained is converted into the salts thereof, particularly for pharmaceutical use into the physiologically acceptable salts thereof.

## Revendications

1. Antagonistes de CGRP de formule générale **1** 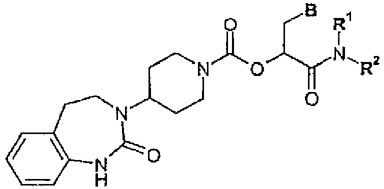 où
**B** représente un groupe choisi parmi 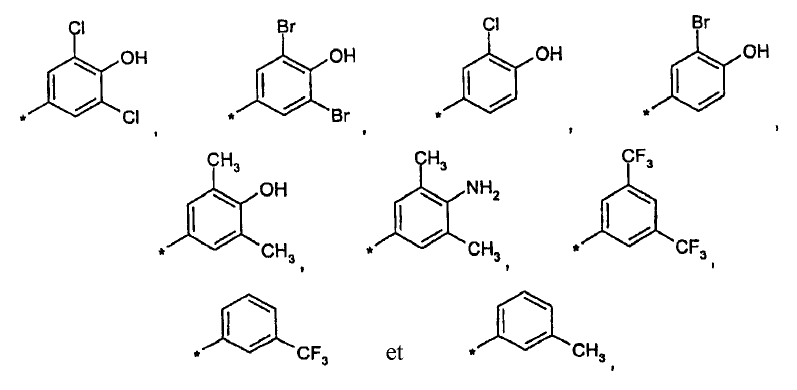 **R¹** et **R²** représentent avec l'atome d'azote inclus un groupement de formule générale **II** 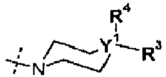 où
**Y¹** représente un atome de carbone ou, quand **R⁴** représente une paire d'électrons libres, également un atome d'azote,
**R³** représente un groupe cyclopentyle, cyclohexyle ou cycloheptyle ou
**R³** représente un hétérocycle choisi parmi un groupe morpholin-4-yle, 1,1-dioxothiomorpholin-4-yle, pipéridin-1-yle, pipéridin-4-yle, pipérazin-1-yle ou pyrrolidin-1-yle,
où les hétérocycles monocycliques cités précédemment peuvent être substitués dans le cycle une ou deux fois par des groupes hydroxyle, méthyle, éthyle, trifluorométhyle, hydroxyméthyle ou hydroxyéthyle, ou, éventuellement en plus, une fois par un groupe hydroxycyclopropyle, trifluorométhylcarbonylméthyle, amino, carboxy-carbonyle, méthoxycarbonyle, éthoxycarbonyl-carbonyle, carboxyméthyle, carboxyéthyle, éthoxycarbonylméthyle, éthoxycarbonyléthyle, carboxy-éthylcarbonyle, éthoxycarbonyl-éthylcarbonyle, aminosulfonyle, méthylaminosulfonyle, diméthylaminosulfonyle, méthylsulfonyle, éthylsulfonyle, isopropylsulfonyle, cyclopropylsulfonyle, (hydroxyamino)-carbonylméthyle, hydroxy-(méthyl)-aminocarbonyl-méthyle ou méthoxyaminocarbonyl-méthyle, où les substituants peuvent être identiques ou différents et peuvent être liés à un atome de carbone du cycle ou un atome d'azote du cycle, ou
les hétérocycles monocycliques cités précédemment peuvent être substitués une fois dans le cycle par un groupe carboxyle quand ce groupe carboxyle n'est pas lié par le biais d'un atome d'azote, et
**R⁴** représente un atome d'hydrogène quand **Y¹** représente un atome de carbone, ou **R⁴** représente une paire d'électrons libres quand **Y¹** représente un atome d'azote, leurs tautomères, leurs diastéréoisomères, leurs énantiomères, leurs hydrates, leurs mélanges et leurs sels ainsi que les hydrates des sels, en particulier leurs sels physiologiquement acceptables avec des acides ou bases inorganiques ou organiques, à condition que les composés suivants, leurs tautomères, leurs diastéréoisomères, leurs énantiomères, leurs hydrates, leurs mélanges et leurs sels ainsi que les hydrates des sels soient exclus de l'étendue de la revendication :
| **nº.** | **Structure** |
|---|---|
| (1) | 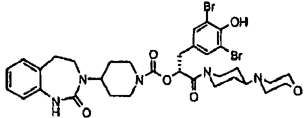 |
| (2) | 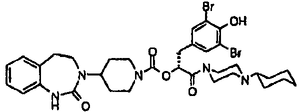 |
| (3) | 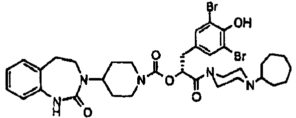 |
| (4) | 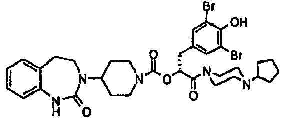 |
| (5) | 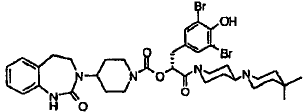 |
| (6) | 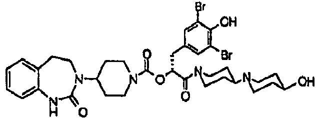 |
| (7) | 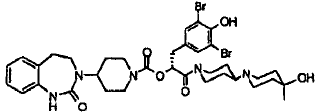 |
| (8) | 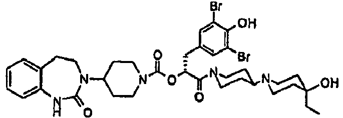 |
| (9) | 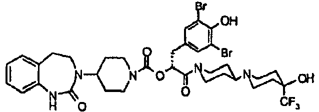 |
| (10) | 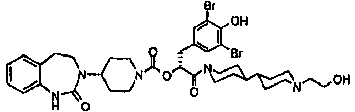 |
| (11) | 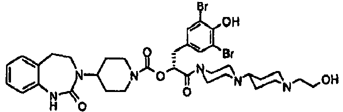 |
| (12) | 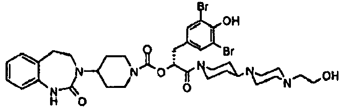 |
| (13) | 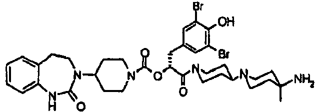 |
| (14) | 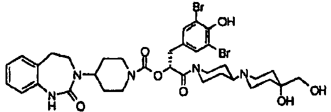 |
| (15) | 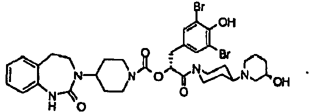 |
| (16) | 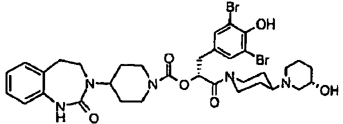 |
| (17) | 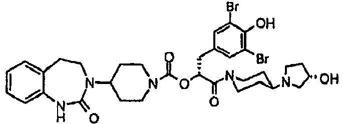 |
| (18) | 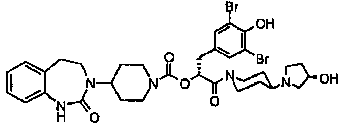 |
| (19) | 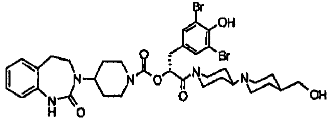 |
| (20) | 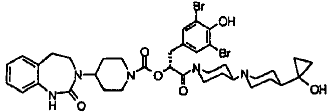 |
| (21) | 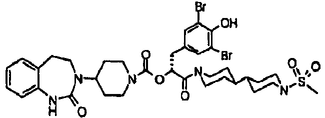 |
| (22) | 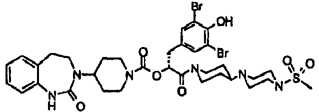 |
| (23) | 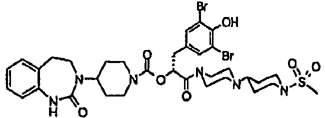 |
| (24) | 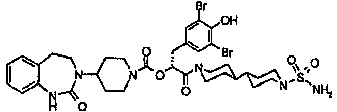 |
| (25) | 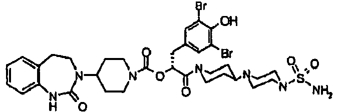 |
| (26) | 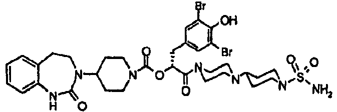 |
| (27) | 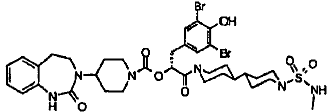 |
| (28) | 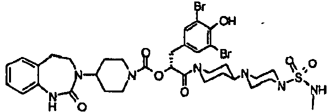 |
| (29) | 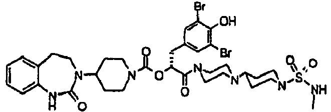 |
| (30) | 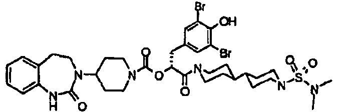 |
| (31) | 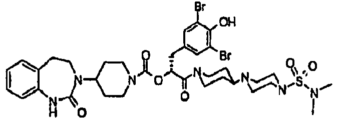 |
| (32) | 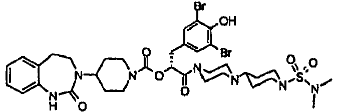 |
| (33) | 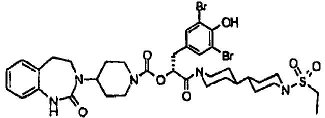 |
| (34) | 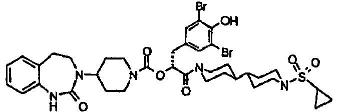 |
| (35) | 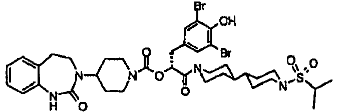 |
| (36) | 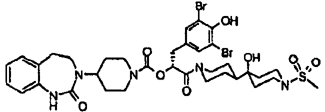 |
| (37) | 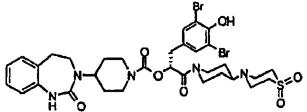 |
| (38) | 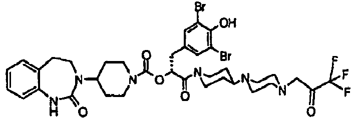 |
| (39) | 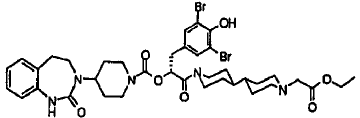 |
| (40) | 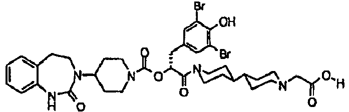 |
| (41) | 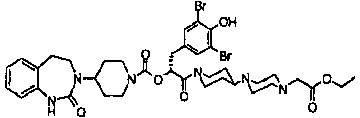 |
| (42) | 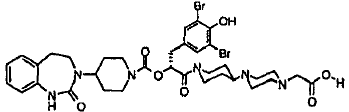 |
| (43) | 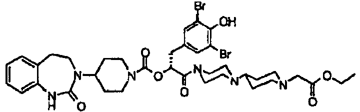 |
| (44) | 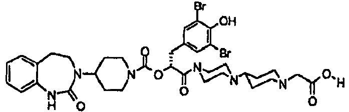 |
| (45) | 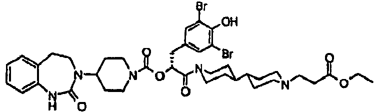 |
| (46) | 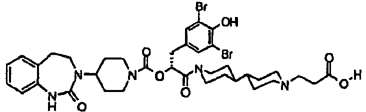 |
| (47) | 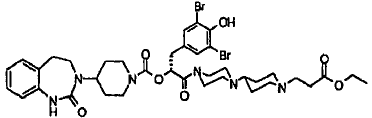 |
| (48) | 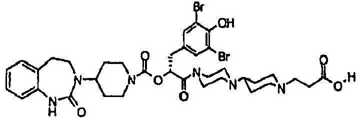 |
| (49) | 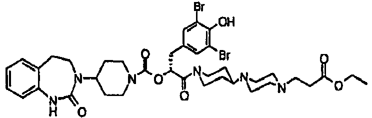 |
| (50) | 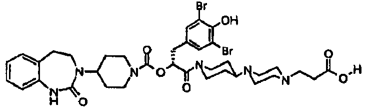 |
| (51) | 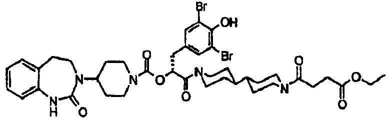 |
| (52) | 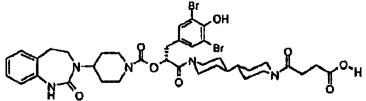 |
| (53) | 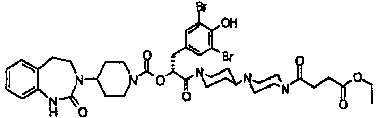 |
| (54) | 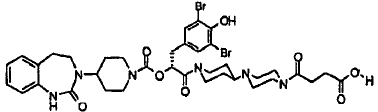 |
| (55) | 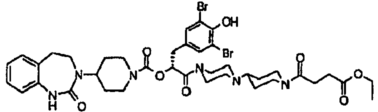 |
| (56) | 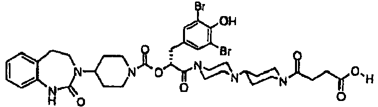 |
| (57) | 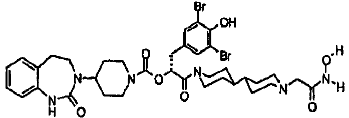 |
| (58) | 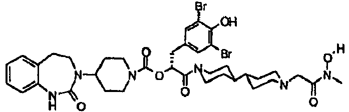 |
| (59) | 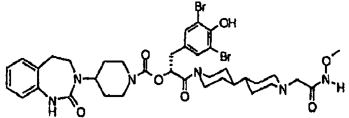 |
| (60) | 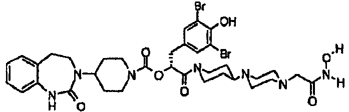 |
| (61) | 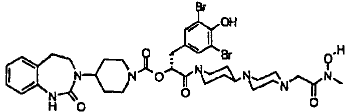 |
| (62) | 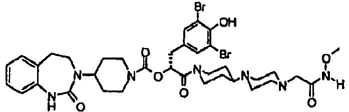 |
| (63) | 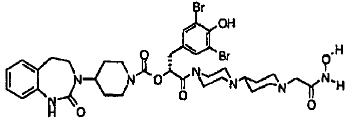 |
| (64) | 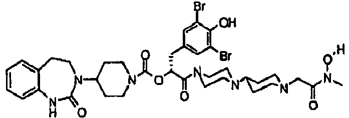 |
| (65) | 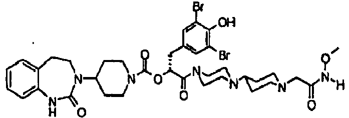 |
| (66) | 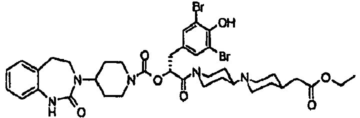 |
| (67) | 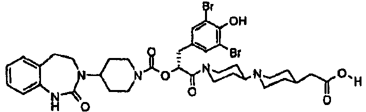 |
| (68) | 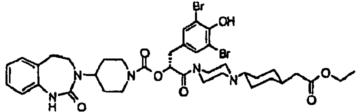 |
| (69) | 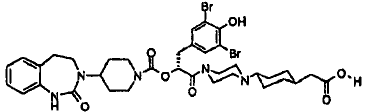 |
| (70) | 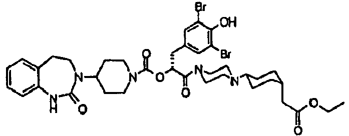 |
| (71) | 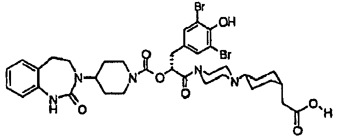 |
| (72) | 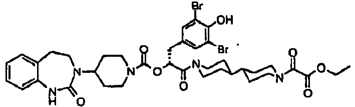 |
| (73) | 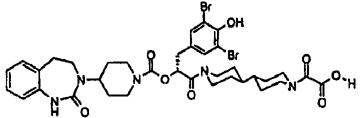 |
| (74) | 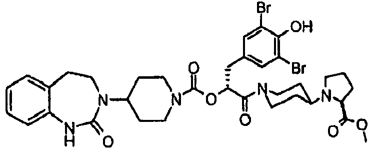 |
| (75) | 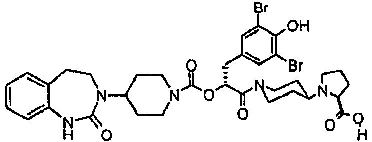 |
| (76) | 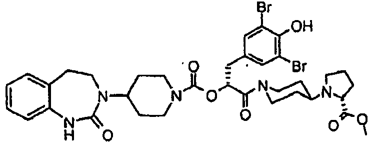 |
| (77) | 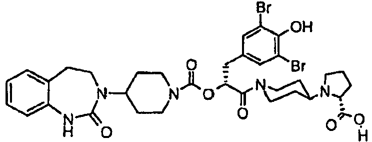 |
| (78) | 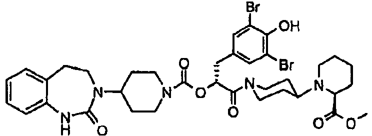 |
| (79) | 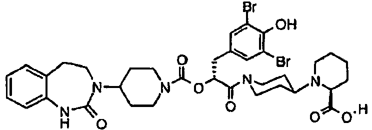 |
| (80) | 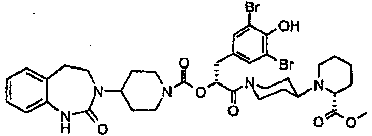 |
| (81) | 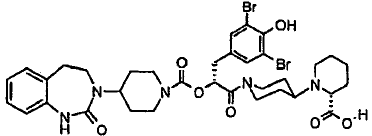 |
| (82) | 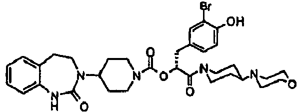 |
| (83) | 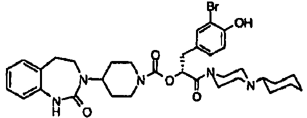 |
| (84) | 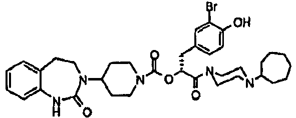 |
| (85) | 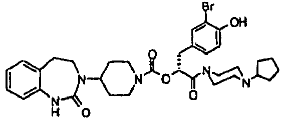 |
| (86) | 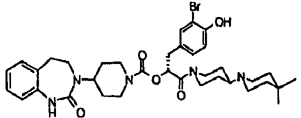 |
| (87) | 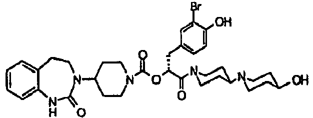 |
| (88) | 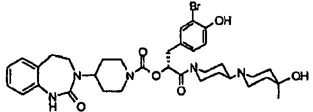 |
| (89) | 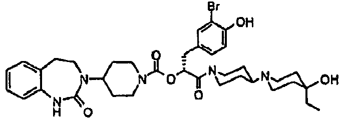 |
| (90) | 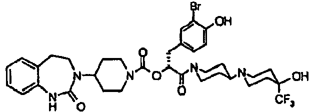 |
| (91) | 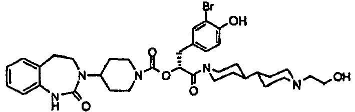 |
| (92) | 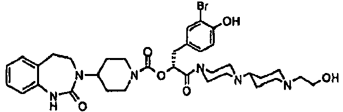 |
| (93) | 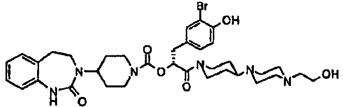 |
| (94) | 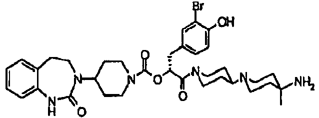 |
| (95) | 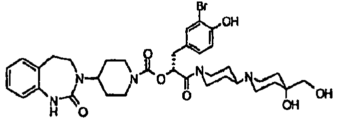 |
| (96) | 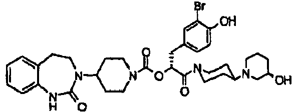 |
| (97) | 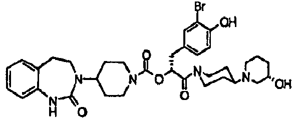 |
| (98) | 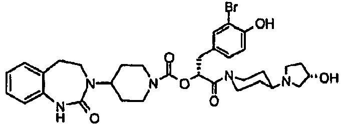 |
| (99) | 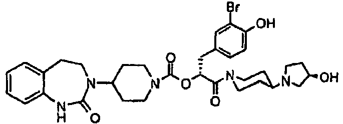 |
| (100) | 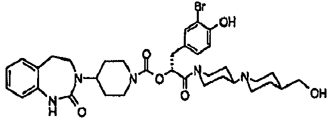 |
| (101) | 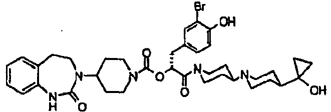 |
| (102) | 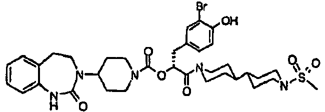 |
| (103) | 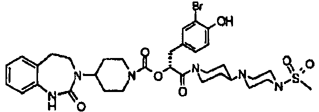 |
| (104) | 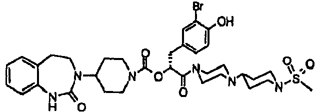 |
| (105) | 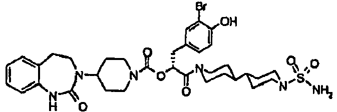 |
| (106) | 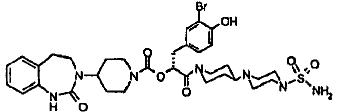 |
| (107) | 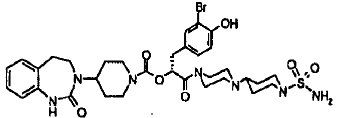 |
| (108) | 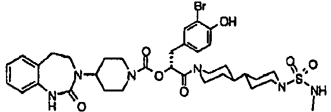 |
| (109) | 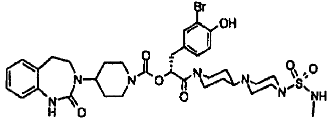 |
| (110) | 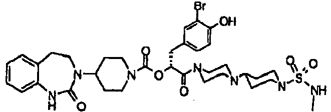 |
| (111) | 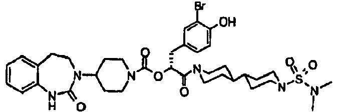 |
| (112) | 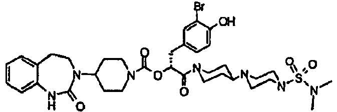 |
| (113) | 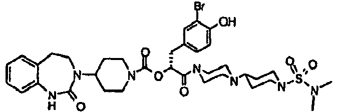 |
| (114) | 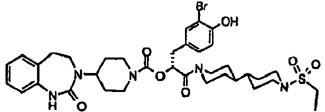 |
| (115) | 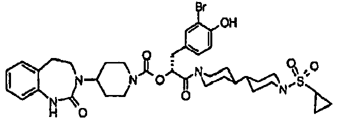 |
| (116) | 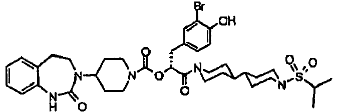 |
| (117) | 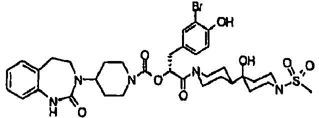 |
| (118) | 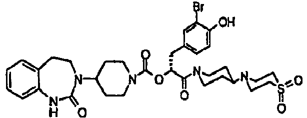 |
| (119) | 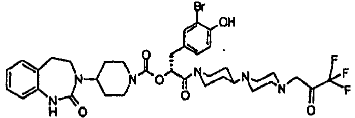 |
| (120) | 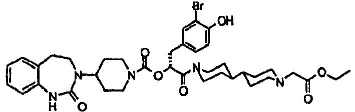 |
| (121) | 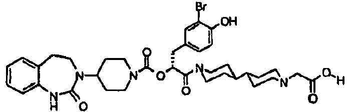 |
| (122) | 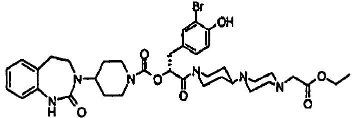 |
| **n°.** | **Structure** |
|---|---|
| (123) | 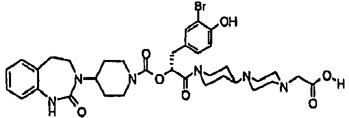 |
| (124) | 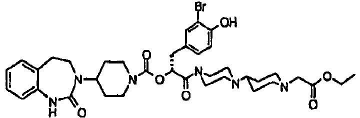 |
| (125) | 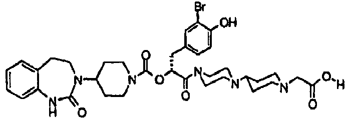 |
| (126) | 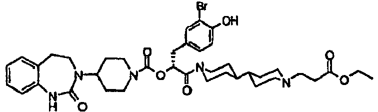 |
| (127) | 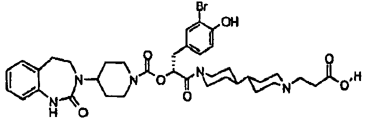 |
| (128) | 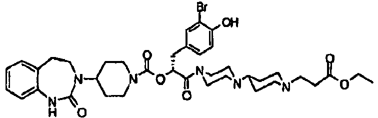 |
| (129) | 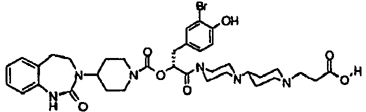 |
| (130) | 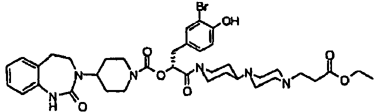 |
| **n°.** | **Structure** |
| (131) | 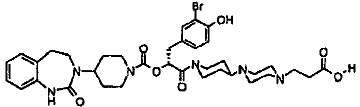 |
| (132) | 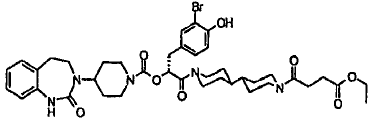 |
| (133) | 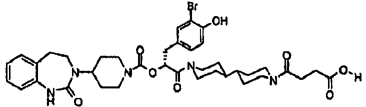 |
| (134) | 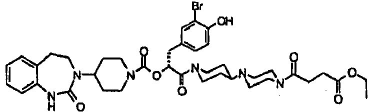 |
| (135) | 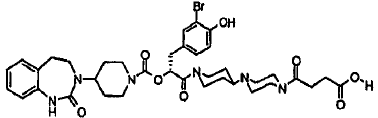 |
| (136) | 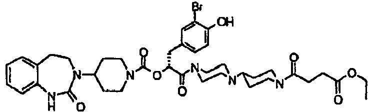 |
| (137) | 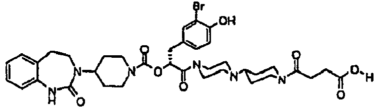 |
| (138) | 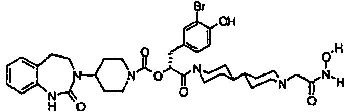 |
| (139) | 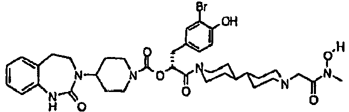 |
| (140) | 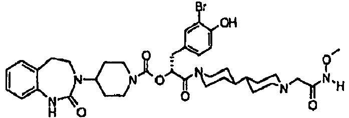 |
| (141) | 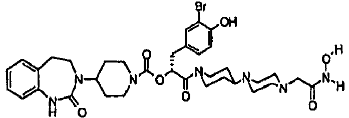 |
| (142) | 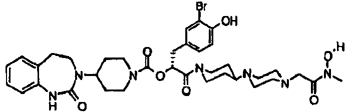 |
| (143) | 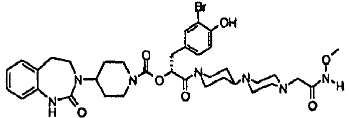 |
| (144) | 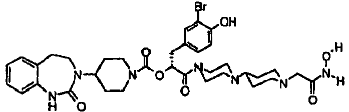 |
| (145) | 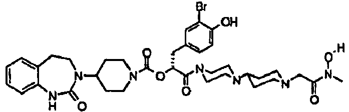 |
| (146) | 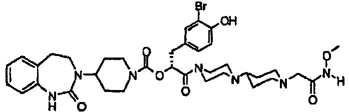 |
| (147) | 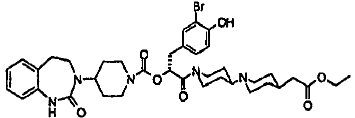 |
| (148) | 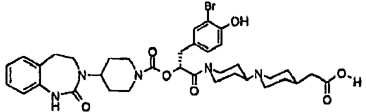 |
| (149) | 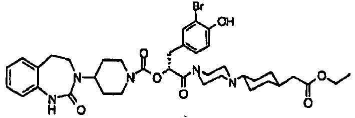 |
| (150) | 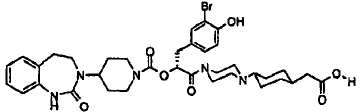 |
| (151) | 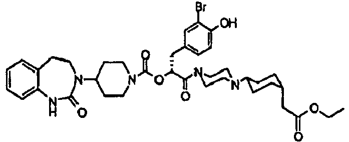 |
| (152) | 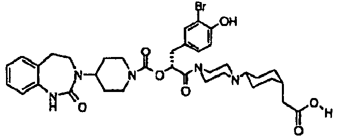 |
| (153) | 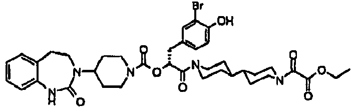 |
| (154) | 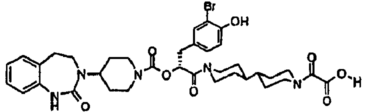 |
| (155) | 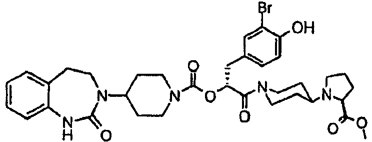 |
| (156) | 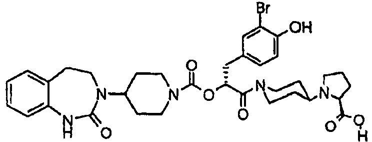 |
| (157) | 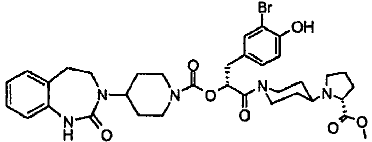 |
| (158) | 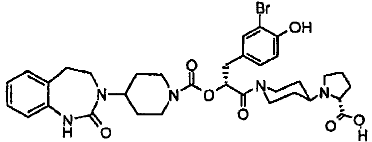 |
| (159) | 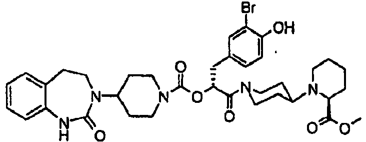 |
| (160) | 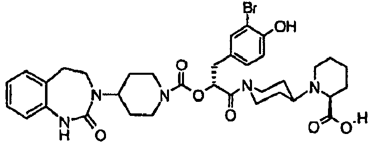 |
| **(161)** | 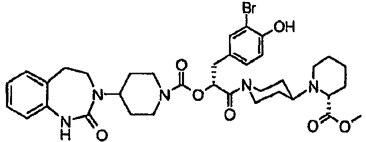 |
| (162) | 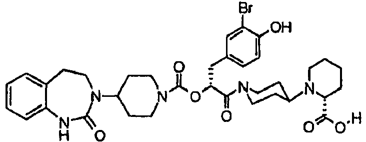 |
| (163) | 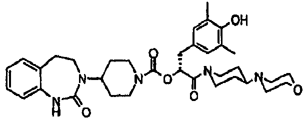 |
| (164) | 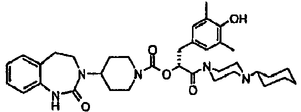 |
| (165) | 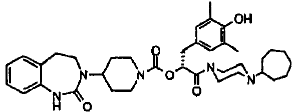 |
| (166) | 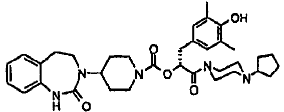 |
| (167) | 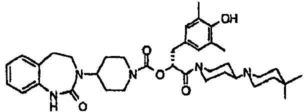 |
| (168) | 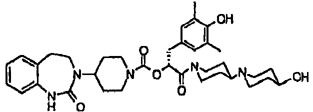 |
| (169) | 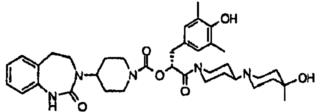 |
| (170) | 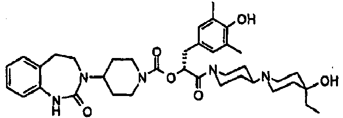 |
| (171) | 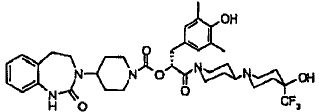 |
| (172) | 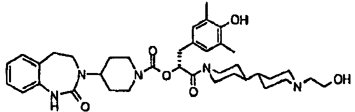 |
| (173) | 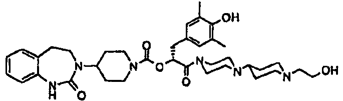 |
| (174) | 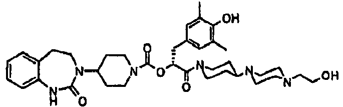 |
| (175) | 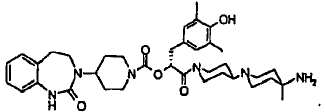 |
| (176) | 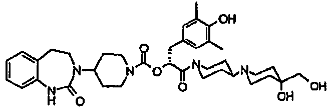 |
| (177) | 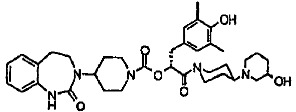 |
| (178) | 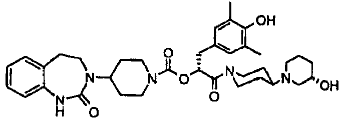 |
| (179) | 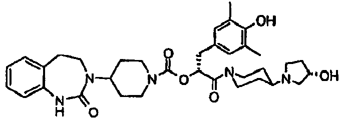 |
| (180) | 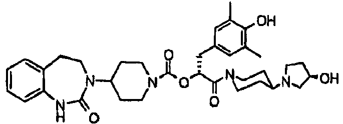 |
| (181) | 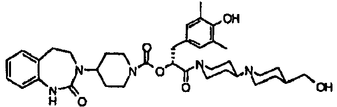 |
| (182) | 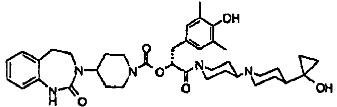 |
| (183) | 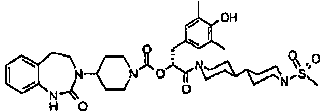 |
| (184) | 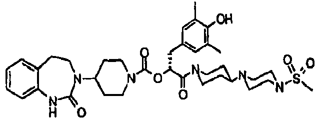 |
| (185) | 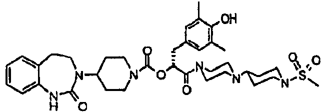 |
| (186) | 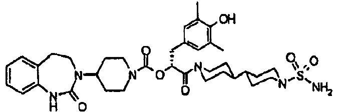 |
| (187) | 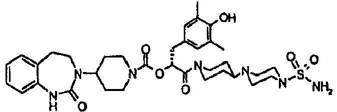 |
| (188) | 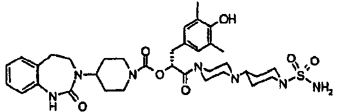 |
| (189) | 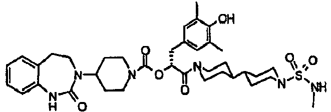 |
| (190) | 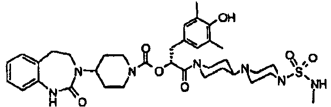 |
| (191) | 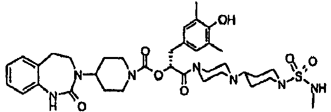 |
| (192) | 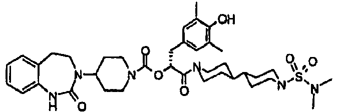 |
| (193) | 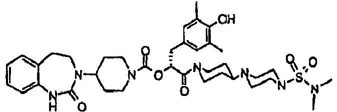 |
| (194) | 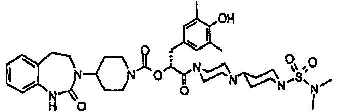 |
| (195) | 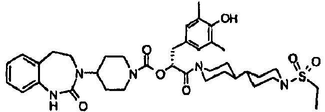 |
| (196) | 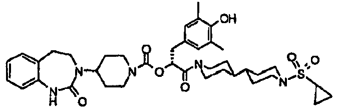 |
| (197) | 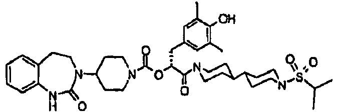 |
| (198) | 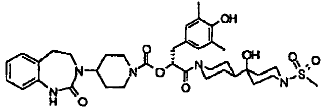 |
| (199) | 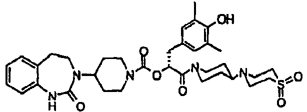 |
| (200) | 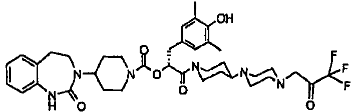 |
| (201) | 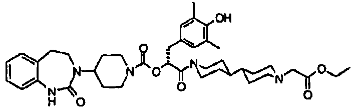 |
| (202) | 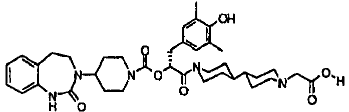 |
| (203) | 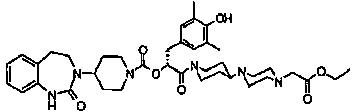 |
| (204) | 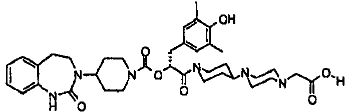 |
| (205) | 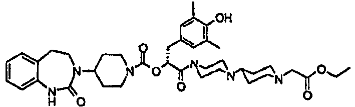 |
| (206) | 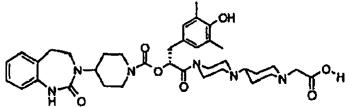 |
| (207) | 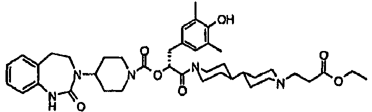 |
| (208) | 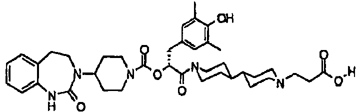 |
| (209) | 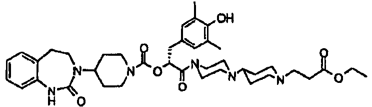 |
| (210) | 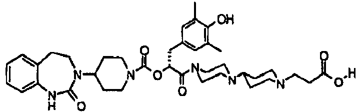 |
| (211) | 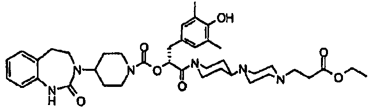 |
| (212) | 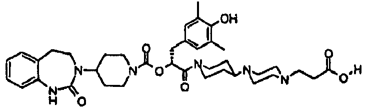 |
| (213) | 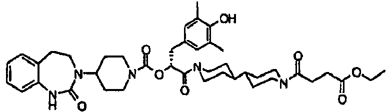 |
| (214) | 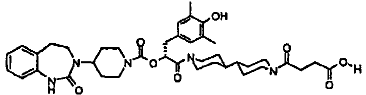 |
| (215) | 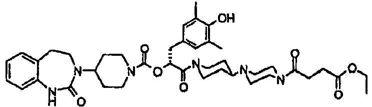 |
| (216) | 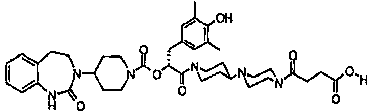 |
| (217) | 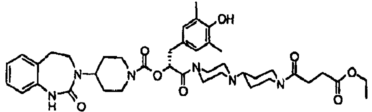 |
| (218) | 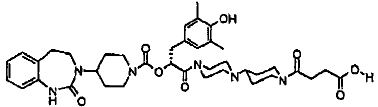 |
| (219) | 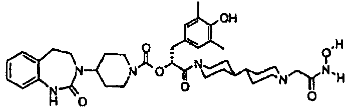 |
| (220) | 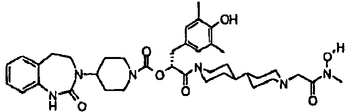 |
| (221) | 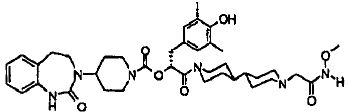 |
| (222) | 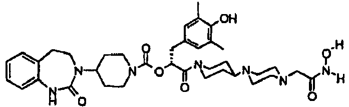 |
| (223) | 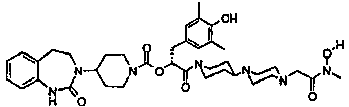 |
| (224) | 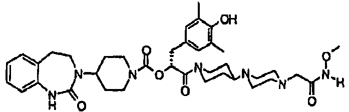 |
| (225) | 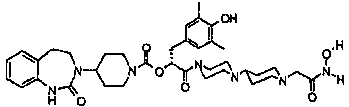 |
| (226) | 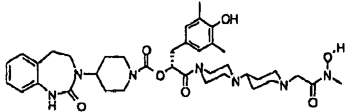 |
| (227) | 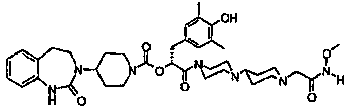 |
| (228) | 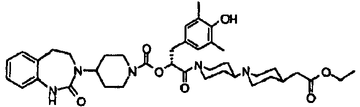 |
| (229) | 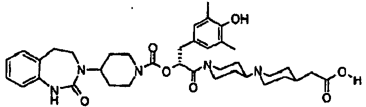 |
| (230) | 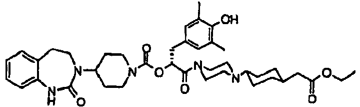 |
| (231) | 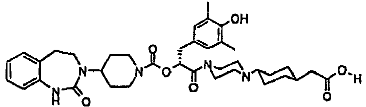 |
| (232) | 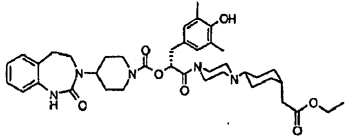 |
| (233) | 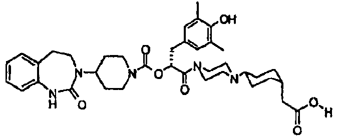 |
| (234) | 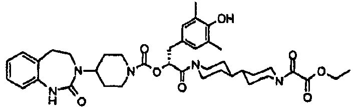 |
| (235) | 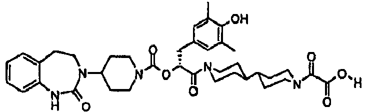 |
| (236) | 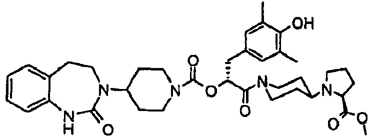 |
| (237) | 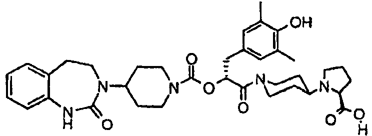 |
| (238) | 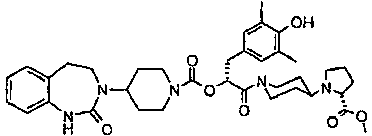 |
| (239) | 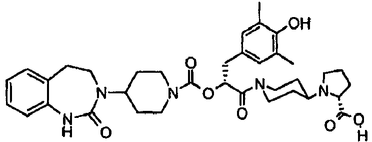 |
| (240) | 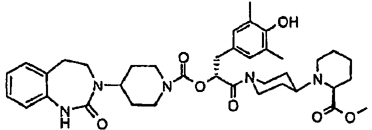 |
| (241) | 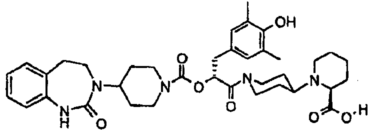 |
| (242) | 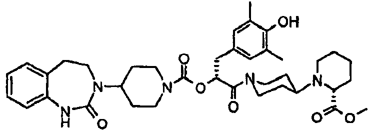 |
| (243) | 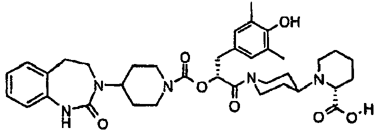 |
| (244) | 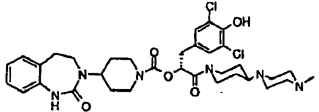 |
| (245) | 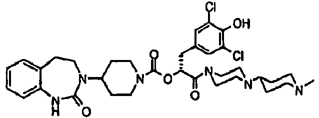 |
| (246) | 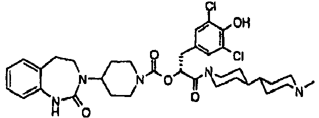 |
| (247) | 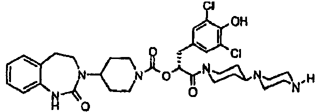 |
| (248) | 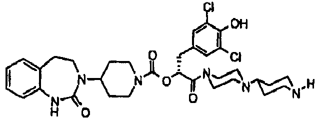 |
| (249) | 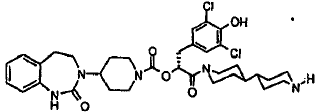 |
| (250) | 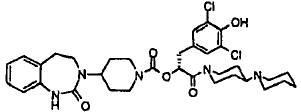 |
| (251) | 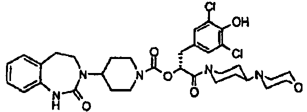 |
| (252) | 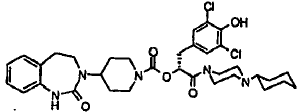 |
| (253) | 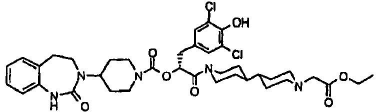 |
| (254) | 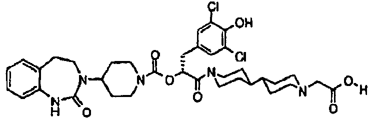 |
| (255) | 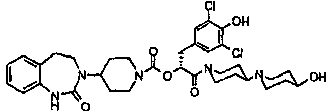 |
| (256) | 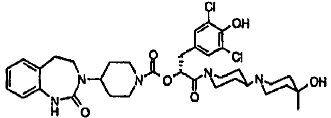 |
| (257) | 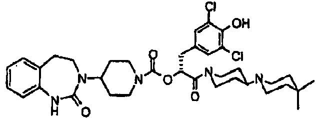 |
| (258) | 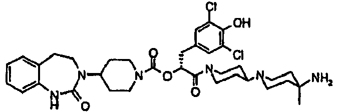 |
| (259) | 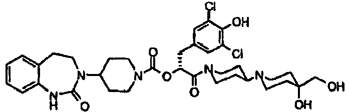 |
| (260) | 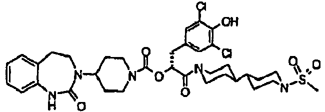 |
| (261) | 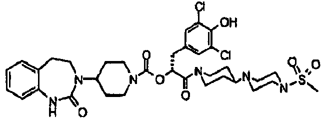 |
| (262) | 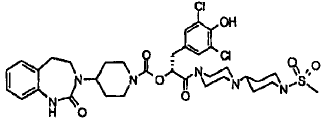 |
| (263) | 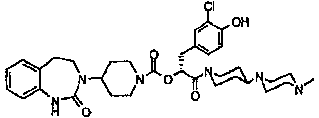 |
| (264) | 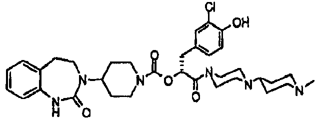 |
| (265) | 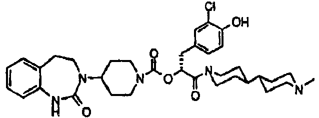 |
| (266) | 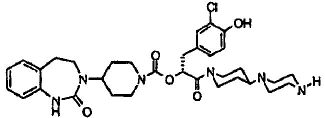 |
| (267) | 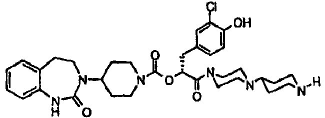 |
| (268) | 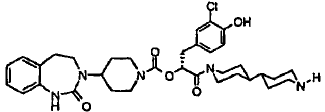 |
| (269) | 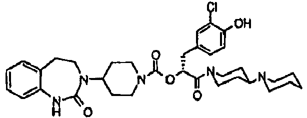 |
| (270) | 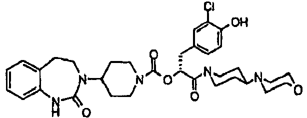 |
| (271) | 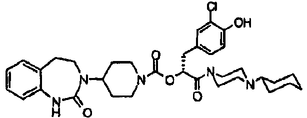 |
| (272) | 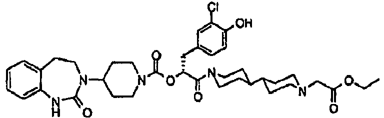 |
| (273) | 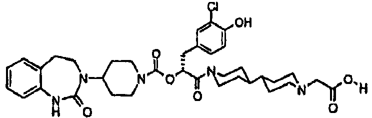 |
| (274) | 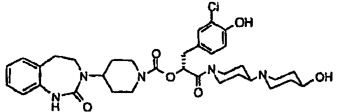 |
| (275) | 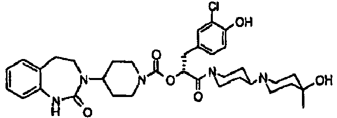 |
| (276) | 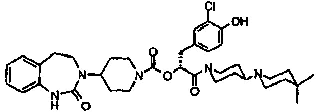 |
| (277) | 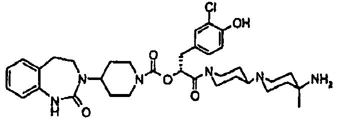 |
| (278) | 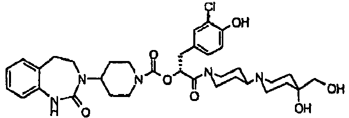 |
| (279) | 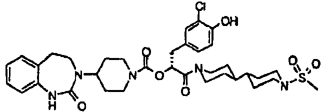 |
| (280) | 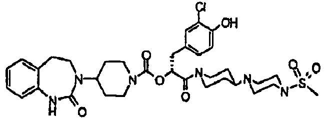 |
| (281) | 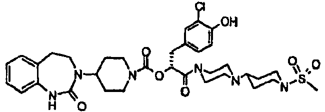 |
| (282) | 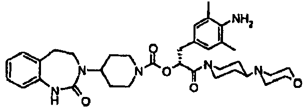 |
| (283) | 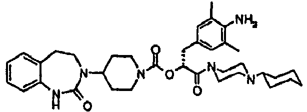 |
| (284) | 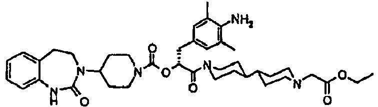 |
| (285) | 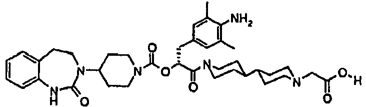 |
| (286) | 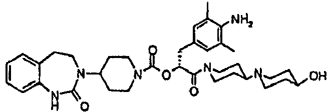 |
| (287) | 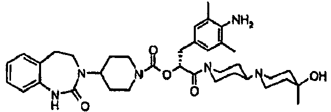 |
| (288) | 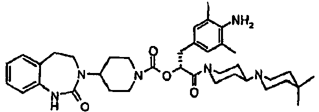 |
| (289) | 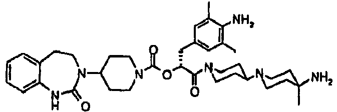 |
| (290) | 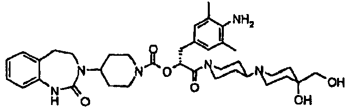 |
| (291) | 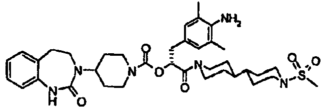 |
| (292) | 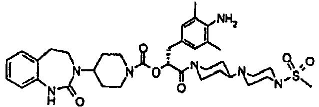 |
| (293) | 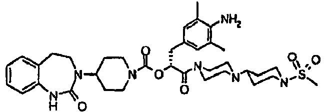 |
| (294) | 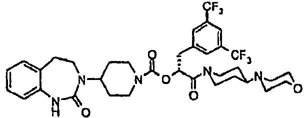 |
| (295) | 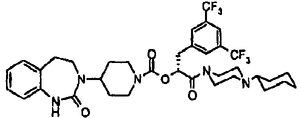 |
| (296) | 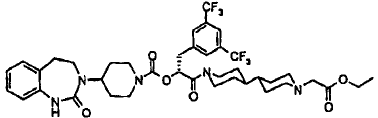 |
| (297) | 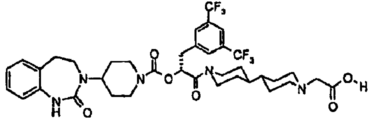 |
| (298) | 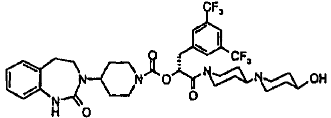 |
| (299) | 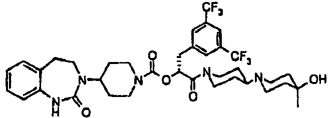 |
| (300) | 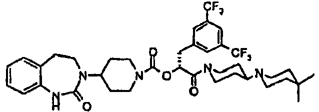 |
| (301) | 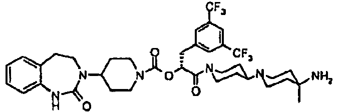 |
| (302) | 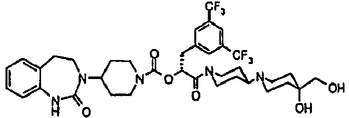 |
| (303) | 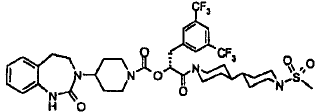 |
| (304) | 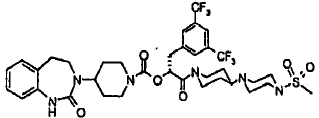 |
| (305) | 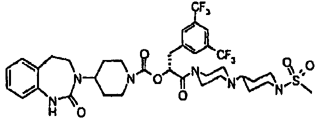 |
| (306) | 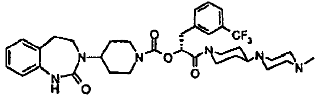 |
| (307) | 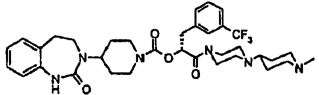 |
| (308) | 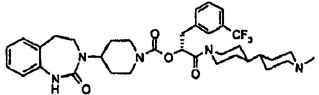 |
| (309) | 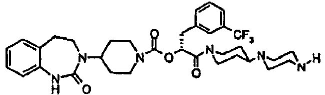 |
| (310) | 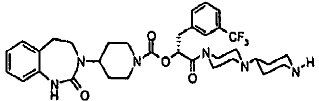 |
| (311) | 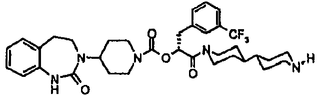 |
| (312) | 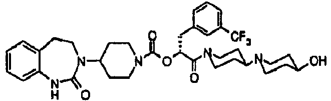 |
| (313) | 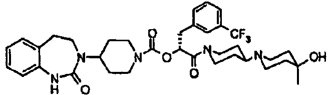 |
| (314) | 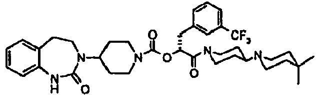 |
| (315) | 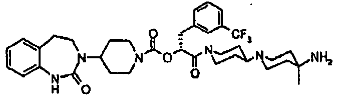 |
| (316) | 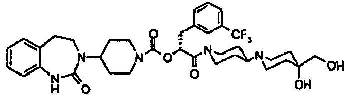 |
| (317) | 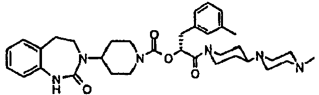 |
| (318) | 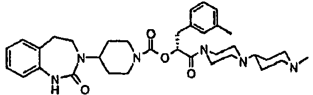 |
| (319) | 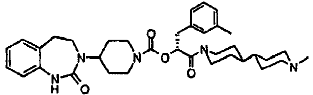 |
| (320) | 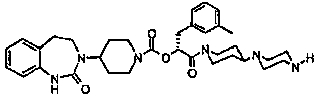 |
| (321) | 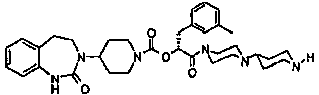 |
| (322) | 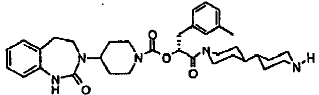 |
| (323) | 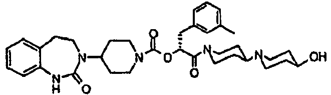 |
| (324) | 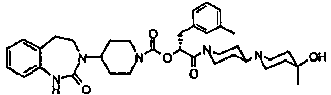 |
| (325) | 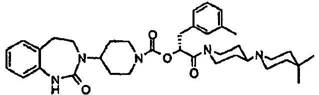 |
| (326) | 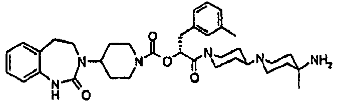 |
| (327) | 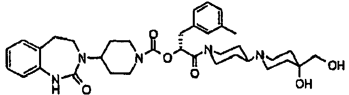 |

2. Sels physiologiquement acceptables des composés selon la revendication 1 avec des acides ou bases inorganiques ou organiques.

3. Médicament contenant un composé selon la revendication 1 ou un sel physiologiquement acceptable selon la revendication 2 outre éventuellement un ou plusieurs vecteurs et/ou diluants inertes.

4. Utilisation d'un composé selon l'une des revendications 1 à 2 pour la production d'un médicament pour le traitement aigu et prophylactique des céphalées, en particulier des céphalées de type migraine et des céphalées vasculaires de Horton, pour le traitement du diabète sucré non-insulinodépendant (« DSNID »), du complex regional pain syndrome (CRPS1), des maladies cardiovasculaires, de la tolérance à la morphine, des maladies diarrhéiques dues à la toxine clostridiale, des maladies de la peau, en particulier des lésions cutanées thermiques et dues aux rayonnements y compris les coups de soleil, des maladies inflammatoires, par exemple des maladies articulaires inflammatoires (arthrite), des inflammations neurogènes de la muqueuse buccale, des maladies pulmonaires inflammatoires, de la rhinite allergique, de l'asthme, des maladies qui se développent avec un élargissement excessif des vaisseaux et une irrigation sanguine réduite des tissus résultante, par exemple le choc et la septicémie, pour soulager les états douloureux, ou pour le traitement préventif ou thérapeutique aigu de la symptomatique des bouffées de chaleur ménopausiques, provoquées par un élargissement des vaisseaux et un débit sanguin accru des femmes carencées en estrogènes et des patients ayant un cancer de la prostate sous traitement hormonal.

5. Procédé de production d'un médicament selon la revendication 3 **caractérisé en ce que**, par voie non chimique, un composé selon l'une des revendications 1 à 2 est incorporé dans un ou plusieurs vecteurs et/ou diluants inertes.

6. Procédé de production des composés de formule générale **I** selon l'une des revendications 1 à 2 **caractérisé en ce que**
(a) une pipéridine de formule générale **III** 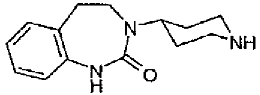 est mise à réagir avec un dérivé de l'acide carbonique de formule générale **IV** 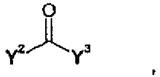 où **Y**² et **Y**³ représentent des groupes nucléofuges, qui peuvent être identiques ou différents, et avec un composé de formule générale **V** 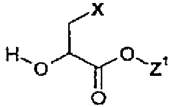 où **B** est défini comme dans la revendication 1 et **Z**¹ représente un groupe protecteur pour un groupe carboxyle, ou
(b) un acide carboxylique de formule générale **VI** 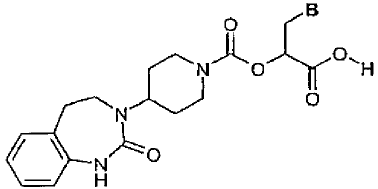 où **B** est défini comme dans la revendication 1, est couplé avec une amine de formule générale **VII**
H-N**R**¹**R**²**,**
où **R**¹ et **R**² sont définis comme dans la revendication 1, où, avant la conduite de la réaction, une fonction acide carboxylique, une fonction amino primaire ou secondaire ou une fonction hydroxyle éventuellement présente dans les groupements **R**¹ et **R**² de l'amine de formule générale **VII** est protégée par des groupements protecteurs courants et les groupements protecteurs éventuellement utilisés sont clivés après la conduite de la réaction, ou
(c) un composé de formule générale **VIII** 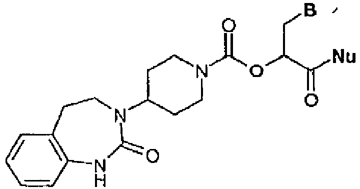 où **B** est défini comme dans la revendication 1 et **Nu** représente un groupe partant, est couplé avec une amine de formule générale **VII**
H-N**R**¹**R**²**,**
où **R**¹ et **R**² sont définis comme dans la revendication 1, où, avant la conduite de la réaction, une fonction acide carboxylique, une fonction amino primaire ou secondaire ou une fonction hydroxyle éventuellement présente dans les groupements **R**¹ et **R**² de l'amine de formule générale **VII** est protégée par des groupements protecteurs courants et les groupements protecteurs éventuellement utilisés sont clivés après la conduite de la réaction, et
si on le souhaite un composé de formule générale **I** ainsi obtenu est séparé en ses stéréoisomères et/ou
un composé de formule générale **I** ainsi obtenu est converti en ses sels, en particulier pour l'utilisation pharmaceutique en ses sels physiologiquement acceptables.
